(19) Europäisches Patentamt
European Patent Office
Office européen des brevets

(11) EP 4 737 450 A1

(12) EUROPEAN PATENT APPLICATION
published in accordance with Art. 153(4) EPC

(43) Date of publication:
**06.05.2026 Bulletin 2026/19**

(21) Application number: **24830602.9**

(22) Date of filing: **20.06.2024**

(51) International Patent Classification (IPC):
***C07D 405/14*** (2006.01) ***C07D 403/12*** (2006.01)
***C07D 403/14*** (2006.01) ***A61K 31/4439*** (2006.01)
***A61P 7/02*** (2006.01)

(52) Cooperative Patent Classification (CPC):
**A61K 31/4439; A61P 7/02; C07D 403/12; C07D 403/14; C07D 405/14**

(86) International application number:
**PCT/CN2024/100315**

(87) International publication number:
**WO 2025/001956 (02.01.2025 Gazette 2025/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **30.06.2023 CN 202310808231**

(71) Applicant: **Longwood Pharmaceuticals (Hangzhou) Co., Ltd**
**Hangzhou, Zhejiang 311199 (CN)**

(72) Inventors:
- **WANG, Yonggang**
  **Hangzhou, Zhejiang 311199 (CN)**
- **YU, Fei**
  **Hangzhou, Zhejiang 311199 (CN)**
- **GAO, Yanshan**
  **Hangzhou, Zhejiang 311199 (CN)**
- **FU, Dengrong**
  **Hangzhou, Zhejiang 311199 (CN)**
- **SUN, Xicheng**
  **Hangzhou, Zhejiang 311199 (CN)**
- **WU, Gongxiong**
  **Hangzhou, Zhejiang 311199 (CN)**

(74) Representative: **dompatent**
**Partnerschaft von Patentanwälten und Rechtsanwälten mbB**
**Deichmannhaus am Dom**
**Bahnhofsvorplatz 1**
**50667 Köln (DE)**

(54) **HETEROAROMATIC FORMAMIDE COMPOUNDS AND USES THEREOF IN MEDICINE**

(57) The present invention relates to heteroaromatic formamide compounds and the uses thereof in medicine. In particular, the present invention relates to heteroaromatic formamide compounds represented by general formula (I), a preparation method thereof, a pharmaceutical composition containing said compounds, and the use of the same as a therapeutic agent, in particular, the use as a selective inhibitor for plasma kallikrein (PKa) and the use in treating and/or preventing diseases which may benefit from the inhibition of plasma kallikrein, especially the use in treating and/or preventing diabetic macular edema (DME), age-related macular degeneration (AMD), choroidal neovascularization, hereditary angioedema (HAE), cerebral edema after stroke, etc.

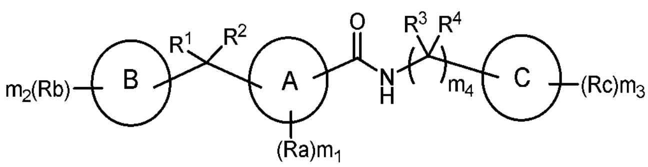

(I)

EP 4 737 450 A1

## Description

### Technical field

The invention belongs to the field of biomedicine, and relates to heteroaromatic formamide compounds and uses thereof in medicine. Specifically, the invention relates to a heteroaromatic formamide compound represented by general formula (I), a pharmaceutical composition containing the compound, and the use as a therapeutic agent, in particular to synthesis methods of the compound and pharmaceutically acceptable salts of the compound, and the use thereof as a selective inhibitor for human plasma kallikrein.

### Background

The kallikrein-kinin system (KKS) consists of two independently regulated proteolytic pathways, mediated by tissue kinin (TK) and tissue kinin (PK), that produce bradykinin and stimulate the bradykinin receptor, respectively. The effect of KKS on retinal vascular permeability is mainly mediated by bradykinin. Both TK and PK are widely distributed in various tissues and may be regulated by different mechanisms. The components of plasma KKS are mainly synthesized in the liver and secreted into the blood. The plasma prekallikrein (PPK) in KKS signaling pathway is an abundant serine protease proenzyme, which can be converted to the catalytically active plasma kallikrein (PKa) by factor XIIa (FXIIa), and participates in inflammatory reactions. PKa cleaves kininogen to produce bradykinin. Bradykinin (BK) is a non-peptide that stimulates the BK receptor, which is abundantly expressed in vascular, glial, and neuronal cells. BK is a potent inflammatory factor, and activation of the BK receptor allows it to bind to $B_1R$ and $B_2R$, can promote increase of bradykinin level to further mediate various signal cascades by stimulating release of mediators such as eicosanoids, endothelium-derived hyperpolarizing factor, NO, allyl alcohol, tissue-type plasminogen activator (tPA) and glucose transporter-1/2 (glu1/2). These mediators, together with inflammation, angiogenesis, vasodilation, and increased vascular permeability, can induce DME formation.

Plasma kallikrein plays a role in a variety of inflammatory conditions and may have many effects in the conditions, such as hereditary angioedema (HAE), retinopathy or diabetic retinopathy, proliferative and non-proliferative retinopathy, diabetic macular edema (DME), clinically important macular edema, cystoid macular edema, CME after cataract extraction, CME induced by cryotherapy, CME induced by uveitis, endophthalmitis, vascular embolism, retinal edema, complications associated with cataract surgery for diabetic retinopathy, hypertensive retinopathy, retinal damage, dry and wet age-related macular degeneration (AMD), polypoidal choroidal vasculopathy, vitreous detachment following choroidal neovascularization, ischemia-reperfusion injury such as in all kinds of scenarios associated with tissue and/or organ transplantation, operation-induced cerebral injury, cerebral ischemia, global ischemia, edema associated with glioma, spinal cord injury, pain, ischemia, cerebral ischemia, neurological and cognitive deficits, deep vein embolism, stroke, myocardial infarction, acquired angioedema, high-altitude cerebral edema, cytotoxic cerebral edema, osmotic cerebral edema, obstructive hydrocephalus, radiation-induced edema, lymphedema, traumatic brain injury, hemorrhagic stroke, intracerebral hemorrhage, hemorrhagic transformation of ischemic stroke, blood coagulation disorders such as thrombus, itching, disorders of inflammatory components such as multiple sclerosis, encephalitis, Alzheimer’s disease, increased blood pressure associated with diabetes or hyperlipidemia, renal insufficiency, chronic kidney disease, heart failure, microalbuminuria, Albuminuria, proteinuria, cerebral hemorrhage, deep vein thrombosis, coagulation after fibrinolytic therapy, angina, angioedema, sepsis, lupus, gout, psoriasis, inflammatory bowel, diabetes, diabetic complications, Parkinson’s disease, amyotrophic lateral sclerosis, Creutzfeldt-Jakob disease, stroke, epilepsy, allergic edema, airflow blockage such as chronic allergic sinusitis or perennial rhinitis, airflow blockage in acute asthma, and other diseases.

Plasma kallikrein inhibitors are considered as suitable for the treatment of a wide range of conditions, in particular for the treatment of edema formation in diseases, such as edema formation associated with ischemic reperfusion injury of stroke, retinopathy or edema associated diseases such as hereditary angioedema, macular edema and cerebral edema.

At present, the treatment of acute ischemic stroke is mainly general treatment and specific treatment. Specific treatment is the key to the treatment of ischemic stroke, mainly including the improvement of cerebral blood circulation and neuroprotection. When stroke occurs, the earlier neuroprotection is carried out to reduce the progress of brain nerve death, the more effective it is to prevent the damage of harmful substances to the brain and win more time for the brain. At present, edaravone and butylphthalide are the mainstream neuroprotective drugs in domestic clinic, and others include citicoline, monosialotetrahexosylganglioside sodium, cinepazide, etc., but the overall therapeutic effect is not ideal, and it is difficult to remove the hat of adjuvant drugs, so it is urgent to develop new, effective and safe anti-stroke drugs or pharmaceutical compositions to meet the urgent clinical needs. Because cerebral ischemic injury is related to many mechanisms, such as excessive formation of free radicals, toxic effects of excitatory amino acids, intracellular calcium overload, inflammatory reaction etc., no matter what the mechanism is, the final outcome will lead to nerve cell death, functional damage and formation of cerebral infarction. The kallikrein-kinin system (KKS) mediates thrombosis, vascular permeability, and blood pressure changes during cerebral ischemia. The contact kinin pathway is initiated by activation of coagulation factor XII

(FXIIa), which cleaves plasma prekallikrein to plasma kallikrein (PK). Subsequently, PK acts on high molecular weight kininogen to induce release of the pro-inflammatory peptide hormone bradykinin (BK). Binding of BK to its endothelial bradykinin receptors 1 and 2 (B 1R/B2R) initiates several inflammatory signaling cascades leading to thrombin generation and influx of circulating immune cells, ultimately leading to thrombosis and neuroinflammation. Inhibition of PK after morbidity of stroke can significantly reduce cerebral thrombosis and stabilize the blood-brain barrier, thereby reducing the number of brain infiltrating immune cells (Seminars in Immunopathology 2023, 45:389 - 410). Simultaneously, PK inhibition also reduces NMDA receptor-mediated excitotoxicity and ischemia-induced neuronal death (Stroke 2014, Vol 45, Issue suppl_1, Abstract W P210).

At present, the clinical benefits of conventional neuroprotective mechanisms are limited. KKS system is closely related to stroke-related hemorrhage, edema, inflammation, neuroprotection, etc. PK inhibition can significantly reduce cerebral thrombosis caused by stroke, stabilize the blood-brain barrier, thereby reducing the number of brain infiltrating immune cells, also can inhibit NMDA receptor-mediated excitotoxicity and ischemic neuronal death, and significantly reduce the range of cerebral infarction.

Plasma kallikrein inhibitors are considered as particularly useful in the treatment of retinopathies, such as retinopathic macular edema (DME) associated with diabetes and/or hypertension.

Proteomic analysis of vitreous samples from DME patients revealed that PPK (plasma prekallikrein) and plasma kallikrein were elevated 11.0-fold ($p < 0.0001$) compared to control vitreous from macular hole patients (Diabetes 2015, 64, 3588-3599). Systemic continuous administration of PKal inhibitors has been shown to reduce retinal vascular permeability and retinal blood flow abnormalities in diabetic mice (Diabetes 2011, 60, 590-1598). Additional preclinical evidence suggests that plasma kallikrein (PK) may contribute to VEGF-mediated retinal edema (Investig. Ophthalmol. Vis. Sci. 2016, 57, 2390 - 2399). Current therapeutic strategies for DR and DME are mainly limited to vascular endothelial growth factor (VEGF) inhibitors and laser photocoagulation. These treatment modalities are not universally effective for all patients, and potential side effects persist in a significant proportion of patients. At present, the treatment of DME mainly relies on anti-VEGF therapy, but some patients (about 50%) do not respond to VEGF therapy. This means that nearly 50% of patients do not receive effective treatment. Preclinical studies have shown that activation of KKS in the eye induces retinal vascular permeability, vasodilation, and retinal thickening. Proteomic analysis of the DME vitreous suggests that the KKS and VEGF pathways are potentially independent biological pathways. In addition, the correlation between DME-related proteins in vitreous and KKS pathway was significantly higher than that of VEGF pathway. Preclinical experiments in diabetic animals have shown that inhibition of the KKS system is an effective way to reduce retinal vascular permeability. (Invest Ophthalmol Vis Sci. 2016; 57(6): 2390-2399).

The invention provides a preparation method of a novel effective PKa small molecule inhibitor with a high selectivity.

**Summary of the invention**

The invention provides a novel and selective small molecule inhibitor for plasma kallikrein, and provides an effective treatment solution for diseases such as stroke, HAE, diabetic macular edema (DME) caused by retinal microvascular disorder and age-related macular degeneration (AMD).

Specifically, the present invention provides a compound represented by the general formula (I) or a pharmaceutically acceptable prodrug or salt thereof:

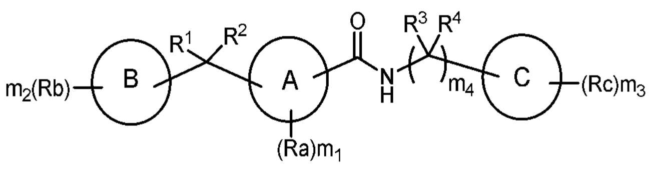

(I)

wherein,

ring A is 5-6 membered heteroaryl or phenyl;
ring B is 5-10 membered monocyclic or bicyclic aryl or heteroaryl;
$m_4$ is 0 or 1;
when $m_4$ is 1, ring C is selected from the group consisting of 5-6 membered heteroaryl, phenyl, and 9-10 membered bicyclic heteroaryl;
when $m_4$ is 0, ring C is selected from the group consisting of 5-6 membered heteroaryl fused to 5-6 membered heterocyclyl, phenyl fused to 5-6 membered heterocyclyl, 5-6 membered heteroaryl fused to $C_{3-6}$cycloalkyl, and

phenyl fused to $C_{3-6}$cycloalkyl;

$R^1$, $R^2$, $R^3$, and $R^4$ are each independently selected from the group consisting of H atom, halogen, $C_{1-6}$alkyl, $C_{3-6}$cycloalkyl, -($C_{1-6}$alkylene)$_n$-$C_{1-6}$alkoxy, and $C_{1-6}$hydroxyalkyl;

each Ra is independently selected from the group consisting of -($C_{1-6}$alkylene)$_n$-$C_{1-6}$alkoxy, -($C_{1-6}$alkylene)$_n$-O-$C_{1-6}$alkylene-$C_{1-6}$alkoxy, $C_{1-6}$haloalkyl, $C_{1-6}$hydroxyalkyl, $C_{1-6}$alkyl, $C_{1-6}$haloalkoxy, -( $C_{1-6}$alkylene)$_n$-$C_{3-6}$cycloalkyl, -($C_{1-6}$alkylene)$_n$-O-$C_{3-6}$cycloalkyl, halogen, -OH, -SH, -$NH_2$, -C(O)$NH_2$, -$NO_2$, -CN, $C_{2-6}$alkenyl, $C_{2-6}$alkynyl, -$C_{1-6}$alkylene-O-$C_{1-6}$alkylene-C(O)-$NH_2$, -$C_{1-6}$alkylene-O-$C_{1-6}$alkylene-C(O)-N($C_{1-6}$alkyl)$_2$, -$C_{1-6}$alkylene-O-$C_{1-6}$alkylene-C(O)-NH-$C_{1-6}$alkyl, -($C_{1-6}$alkylene)$_n$-5-6 membered heteroaryl, -O-($C_{1-6}$alkylene)$_n$-5-6 membered heteroaryl, -($C_{1-6}$alkylene)$_n$-4-7 membered monocyclic or bicyclic heterocyclyl, -($C_{1-6}$alkylene)$_n$-O-($C_{1-6}$alkylene)$_n$-4-7 membered monocyclic or bicyclic heterocyclyl, -NH-4-7 membered monocyclic or bicyclic heterocyclyl, -N($C_{1-6}$alkyl)-4-7 membered monocyclic or bicyclic heterocyclyl, -$C_{1-6}$alkylene-O-$C_{1-6}$alkylene-$NH_2$, -$C_{1-6}$alkylene-O-$C_{1-6}$alkylene-N($C_{1-6}$alkyl)$_2$ and -$C_{1-6}$alkylene-O-$C_{1-6}$alkylene-NH-$C_{1-6}$alkyl, and the $C_{1-6}$alkylene, 5-6 membered heteroaryl, 4-7 membered monocyclic or bicyclic heterocyclyl, and $C_{3-6}$cycloalkyl are each independently optionally substituented with one or more substituents selected from the group consisting of $C_{1-6}$alkyl, oxo, -($C_{1-6}$alkylene)$_n$-$C_{1-6}$alkoxy, -C(O)-$C_{1-6}$alkyl, -C(O)-$C_{1-6}$alkoxy, halogen, and -OH;

each Rb is independently selected from the group consisting of halogen, -CN, -($C_{1-6}$alkylene)$_n$-$C_{3-6}$cycloalkyl, -O-($C_{1-6}$alkylene)$_n$-$C_{3-6}$cycloalkyl, -C(O)-$C_{3-6}$cycloalkyl, $C_{1-6}$alkyl, $C_{1-6}$haloalkyl, -($C_{1-6}$alkylene)$_n$-4-7 membered monocyclic or bicyclic heterocyclyl, -($C_{1-6}$alkylene)$_n$-5-6 membered heteroaryl, -($C_{1-6}$alkylene)$_n$-9-10 membered bicyclic heteroaryl, -$C_{2-6}$alkenyl-$C_{3-6}$cycloalkyl, phenyl, $C_{1-6}$hydroxyalkyl, $C_{1-6}$haloalkoxy, -($C_{1-6}$alkylene)$_n$-$C_{1-6}$alkoxy, -OH, -SH, -$NH_2$, -C(O)$NH_2$, -$NO_2$, $C_{2-6}$alkenyl, $C_{2-6}$alkynyl, -($C_{1-6}$alkylene)$_n$-4-7 membered monocyclic or bicyclic heterocyclyl fused to phenyl and -O-phenyl, and the $C_{3-6}$cycloalkyl, 4-7 membered monocyclic or bicyclic heterocyclyl, 9-10 membered bicyclic heteroaryl, phenyl, and 5-6 membered heteroaryl are each independently optionally substituented with one or more substituents selected from the group consisting of halogen, oxo, $C_{1-6}$alkyl, $C_{1-6}$haloalkyl, $C_{1-6}$haloalkoxy, -($C_{1-6}$alkylene)$_n$-$C_{1-6}$alkoxy, $C_{1-6}$hydroxyalkyl, -CN, -OH, -SH, -$NH_2$, -C(O)$NH_2$, and -$NO_2$;

each Rc is independently selected from the group consisting of $C_{1-6}$alkyl, $C_{1-6}$haloalkyl, halogen, -$NH_2$, -NH-$C_{1-6}$alkyl, -N($C_{1-6}$alkyl)$_2$, -($C_{1-6}$alkylene)$_n$-$C_{1-6}$alkoxy, formamidinyl, N-hydroxyformamidinyl, -C(=NH)-NH-$C_{1-6}$alkyl, -C(=NH)-N($C_{1-6}$alkyl)$_2$, $C_{1-6}$haloalkoxy, $C_{1-6}$hydroxyalkyl, -CN, -OH, -SH, -S-$C_{1-6}$ alkyl, -C(O)$NH_2$, - $NO_2$, -$C_{1-6}$alkylene-$NH_2$, -$C_{1-6}$alkylene-NH-$C_{1-6}$alkyl, $C_{2-6}$alkenyl, $C_{2-6}$alkynyl, $C_{3-6}$cycloalkyl, 3-6-membered heterocyclyl, -NH-($C_{1-6}$alkylene)$_n$-$C_{3-6}$cycloalkyl, -NH-($C_{1-6}$alkylene)$_n$-3-6 membered heterocyclyl and 5-6 membered heteroaryl, and the 3-6 membered heterocyclyl and 5-6 membered heteroaryl are each independently optionally substituted with one or more substituents selected from the group consisting of $C_{1-6}$alkyl, oxo, -($C_{1-6}$alkylene)$_n$-$C_{1-6}$alkoxy, -C(O)-$C_{1-6}$alkyl, -C(O)-$C_{1-6}$alkoxy, halogen and -OH;

or, two adjacent Rc together with the atoms to which they are attached form a $C_{3-6}$cycloalkyl or 5-6 membered heterocyclyl, and the $C_{3-6}$cycloalkyl or 5-6 membered heterocyclyl are each independently optionally substituted with one or more substituents selected from the group consisting of $C_{1-6}$alkyl, oxo, -($C_{1-6}$alkylene)$_n$-$C_{1-6}$alkoxy, -C(O)-$C_{1-6}$alkyl, -C(O)-$C_{1-6}$alkoxy, halogen and -OH;

the heterocyclyl and heteroaryl each containing 1, 2, 3 or 4 heteroatoms selected from the group consisting of N, O and S;

$m_1$ is 0, 1 or 2;

$m_2$ is 0, 1, 2, or 3;

$m_3$ is 0, 1, 2, or 3; and

each n is independently 0 or 1.

In some embodiments of the present invention, the compound represented by the general formula (I) or a pharmaceutically acceptable prodrug or salt thereof is a compound represented by the general formula (II) or a pharmaceutically acceptable prodrug or salt thereof,

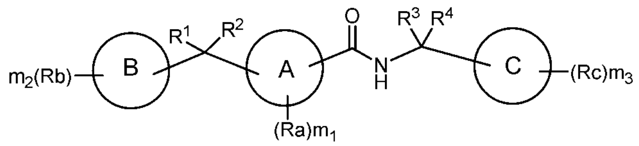

(II)

wherein,

ring A, ring B, ring C, $R^1$, $R^2$, $R^3$, $R^4$, Ra, Rb, Rc, $m_1$, $m_2$ and $m_3$ are as defined in formula (I).

In some embodiments of the present invention, the compound represented by the formula (I) or (II) or a

pharmaceutically acceptable prodrug or salt thereof is a compound represented by formula (III) or a pharmaceutically acceptable prodrug or salt thereof,

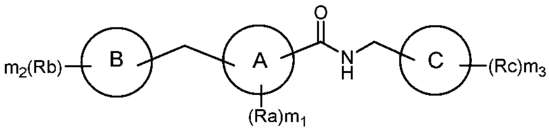

(III)

wherein,

ring A, ring B, ring C, Ra, Rb, Rc, $m_1$, $m_2$ and $m_3$ are as defined in formula (I).

In some embodiments of the present invention, the compound represented by the general formula (I) or (II) or a pharmaceutically acceptable prodrug or salt thereof is a compound represented by general formula (IV) or a pharmaceutically acceptable prodrug or salt thereof,

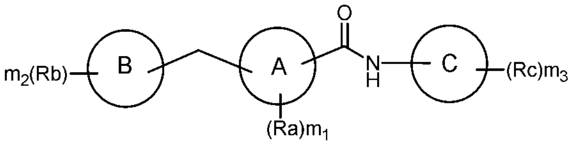

(IV)

wherein,

ring A, ring B, Ra, Rb, Rc, $m_1$, $m_2$ and $m_3$ are as defined in formula (I);

ring C is selected from the group consisting of 5-6 membered heteroaryl fused to 5-6 membered heterocyclyl, phenyl fused to 5-6 membered heterocyclyl, 5-6 membered heteroaryl fused to $C_{3-6}$cycloalkyl, and phenyl fused to $C_{3-6}$cycloalkyl; and

in particular, ring C is selected from the group consisting of cyclopentopyridinyl, cyclopentoimidazolyl and dihydrofuropyridinyl.

In some embodiments of the invention, in a compound represented by the general formula (I), (II), (III), or (IV), or a pharmaceutically acceptable prodrug or salt thereof,

ring A is selected from triazolyl, pyrazolyl, thiazolyl, oxadiazolyl, oxazolyl, thienyl, thiadiazolyl, pyridyl, isoxazolyl, imidazolyl, pyrrolyl, furyl, isothiazolyl, phenyl, pyrimidinyl, pyrazinyl, pyridazinyl, triazinyl, and tetrazolyl;

in partivular,

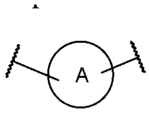

is selected from the group consisting of

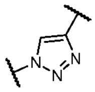 , 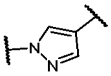 , 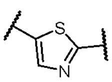 ,

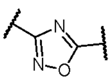 , 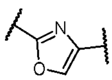 , 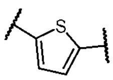 , 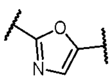 , 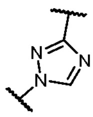 , 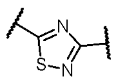 , 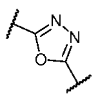 , 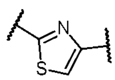 , 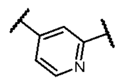 ,

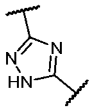, 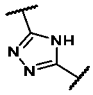, 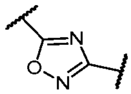, 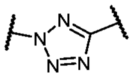, 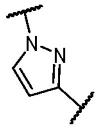, 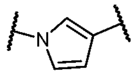, 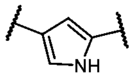 and 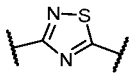.

In some embodiments of the invention, in a compound represented by the general formula (I), (II), (III), or (IV), or a pharmaceutically acceptable prodrug or salt thereof,

ring B is selected from the group consisting of phenyl, quinolinyl, imidazopyridinyl, pyridinyl, imidazolyl, naphthyl, triazolyl, pyrazolyl, thiazolyl, oxadiazolyl, oxazolyl, thienyl, thiadiazolyl, isoxazolyl, pyrrolyl, furanyl, isothiazolyl, phenyl, pyrimidinyl, pyrazinyl, pyridazinyl, triazinyl, isoquinolinyl, indolyl, indazolyl, quinoxalinyl, phthalazinyl, benzimidazolyl, benzothiophenyl, quinazolinyl, and benzothiazolyl;
in particular,

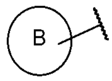

is selected from the group consisting of

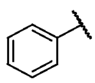, 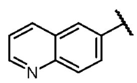, 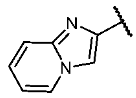,

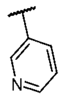 and 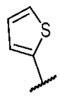.

In some embodiments of the invention, in a compound represented by the general formula (I), (II), (III), or (IV), or a pharmaceutically acceptable prodrug or salt thereof,

ring C is selected from the group consisting of pyridinyl, phenyl, pyrimidinyl, thiadiazolyl, thiazolyl, triazolyl, pyrazolyl, oxadiazolyl, oxazolyl, thienyl, isoxazolyl, imidazolyl, pyrrolyl, furanyl, isothiazolyl, pyrazinyl, pyridazinyl, triazinyl, isoquinolinyl, pyrrolopyridinyl, imidazopyridinyl, triazolopyridinyl, benzisoxazolyl, benzothiazolyl and pyrazolopyridinyl;
in particular,

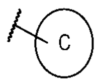

is selected from the group consisting of

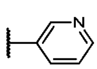, 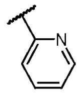, 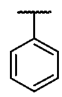, 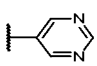,

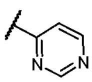, 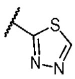, 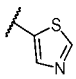, 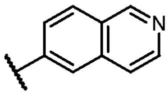, 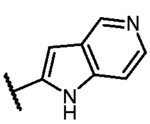, 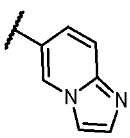, 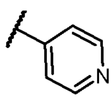, 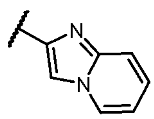,

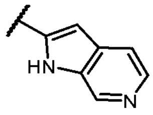, 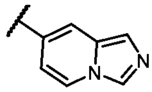, 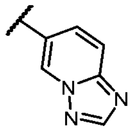, 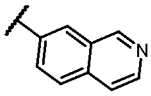, 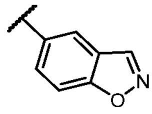, 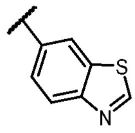, 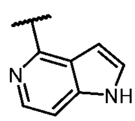,

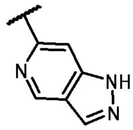, 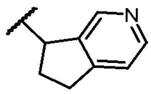, 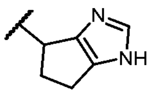 and 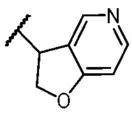.

In some embodiments of the invention, in a compound represented by the general formula (I), (II), (III), or (IV), or a pharmaceutically acceptable prodrug or salt thereof,

each Ra is independently selected from the group consisting of -($C_{1-6}$alkylene)$_n$-$C_{1-6}$alkoxy, -($C_{1-6}$alkylene)$_n$-O-$C_{1-6}$alkylene-$C_{1-6}$alkoxy, -($C_{1-6}$alkylene)$_n$-4-7 membered monocyclic or bicyclic heterocyclyl, -($C_{1-6}$alkylene)$_n$-O-($C_{1-6}$alkylene)$_n$-4-7 membered monocyclic or bicyclic heterocyclyl, -NH-4-7 membered monocyclic or bicyclic heterocyclyl, $C_{1-6}$haloalkyl, $C_{1-6}$hydroxyalkyl, $C_{1-6}$alkyl, $C_{1-6}$haloalkoxy, -($C_{1-6}$alkylene)$_n$-$C_{3-6}$cycloalkyl, -($C_{1-6}$alkylene)$_n$-O-$C_{3-6}$cycloalkyl, halogen, -CN, -$C_{1-6}$alkylene-O-$C_{1-6}$alkylene-C(O)-N($C_{1-6}$alkyl)$_2$, -($C_{1-6}$alkylene)$_n$-5-6 membered heteroaryl, -O-($C_{1-6}$alkylene)$_n$-5-6 membered heteroaryl, and -$C_{1-6}$alkylene-O-$C_{1-6}$alkylene-N($C_{1-6}$alkyl)$_2$, and the $C_{1-6}$alkylene, 5-6 membered heteroaryl, 4-7 membered monocyclic or bicyclic heterocyclyl, and $C_{3-6}$cycloalkyl are each independently optionally substituted with one, two, three or four substituents selected from the group consisting of $C_{1-6}$alkyl, oxo, -($C_{1-6}$alkylene)$_n$-$C_{1-6}$alkoxy, -C(O)-$C_{1-6}$alkyl, -C(O)-$C_{1-6}$alkoxy, halogen, and -OH, each n is independently 0 or 1, the 4-7 membered monocyclic or bicyclic heterocyclyl and the 5-6 membered heteroaryl each independently contain 1, 2, or 3 heteroatoms selected from the group consisting of N, O, and S, preferably selected from the group consisting of pyrimidinyl, pyrazolyl, pyridinyl, pyrrolidinyl, azetidinyl, piperidinyl, piperazinyl, oxetanyl, 2-oxa-6-azaspiro[3.3]heptyl, 2-azabicyclo[2.2.1]heptyl, isoxazolyl, tetrahydropyranyl, pyrazinyl and 1-oxa-6-azaspiro[3.3]heptyl;

in particular, each Ra is independently selected from the group consisting of

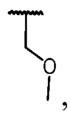,

tetrahydrofuryl, oxetanyl, difluoromethyl, trifluoromethyl, methyl, Cl atom, -CN,

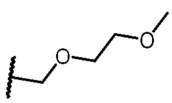, 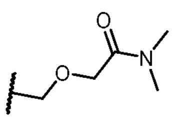, 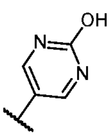, 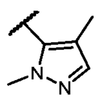, 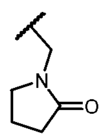, 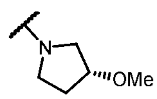, 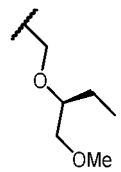,

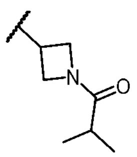, 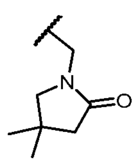, 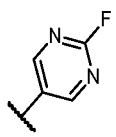, 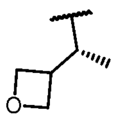, 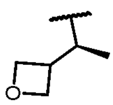, 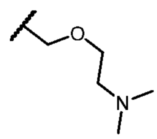, 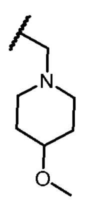,

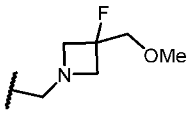, 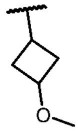, 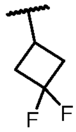, 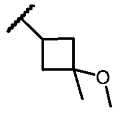, 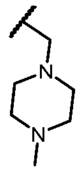, 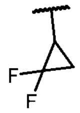, 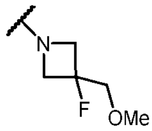,

morpholinyl,

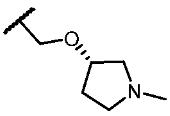, 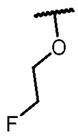, 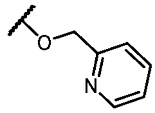, 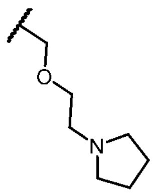, 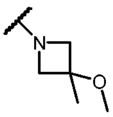, 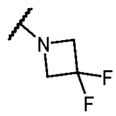, 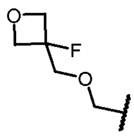,

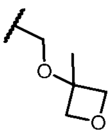, 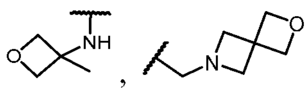, 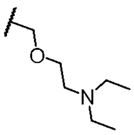, 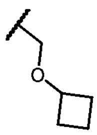, 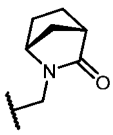, 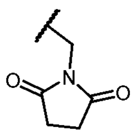, 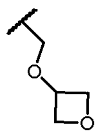,

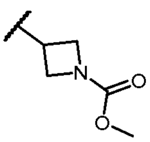, 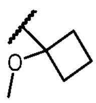, 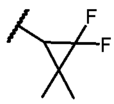, 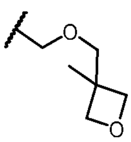, 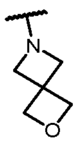, 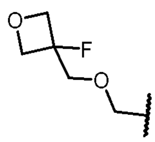, 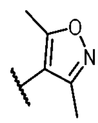, 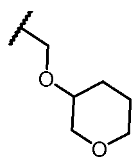,

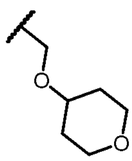, 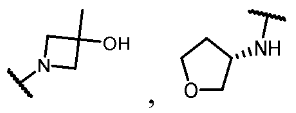, 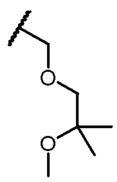,

pyrazinyl, pyrimidinyl,

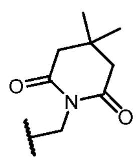,

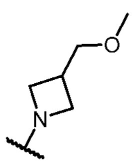, 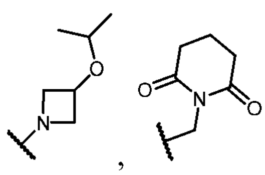, 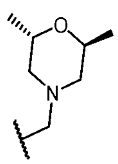, 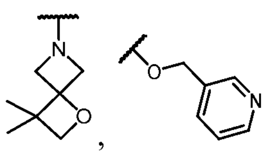, 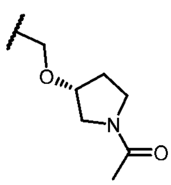, 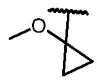,

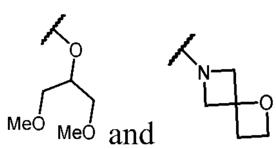.

In some embodiments of the invention, in a compound represented by the general formula (I), (II), (III), or (IV), or a pharmaceutically acceptable prodrug or salt thereof,

each Rb is independently selected from the group consisting of halogen, -CN, -($C_{1-6}$alkylene)$_n$-$C_{3-6}$cycloalkyl, -O-$C_{1-6}$alkylene-$C_{3-6}$cycloalkyl, -C(O)-$C_{3-6}$cycloalkyl, $C_{1-6}$alkyl, $C_{1-6}$haloalkyl, $C_{1-6}$alkoxy, -($C_{1-6}$alkylene)$_n$-4-7 membered monocyclic or bicyclic heterocyclyl, -$C_{1-6}$alkylene-5-6 membered heteroaryl, -$C_{1-6}$alkylene-9-10 membered bicyclic heteroaryl, -$C_{2-6}$alkenyl-$C_{3-6}$cycloalkyl, -$C_{1-6}$alkylene-4-7 membered monocyclic or bicyclic heterocyclyl fused to phenyl and -O-phenyl, each n being independently 0 or 1, the 4-7 membered monocyclic or bicyclic heterocyclyl, 9-10 membered bicyclic heteroaryl, phenyl, and 5-6 membered heteroaryl are each independently optionally substituted with one, two or three substituents selected from the group consisting of halogen, oxo, $C_{1-6}$alkyl, and $C_{1-6}$haloalkyl, and the 4-7 membered monocyclic or bicyclic heterocyclyl, the 9-10 membered bicyclic heteroaryl, and the 5-6 membered heteroaryl each independently contain 1, 2, or 3 heteroatoms selected from the group consisting of N, O, and S, preferably selected from the group consisting of dihydropyridinyl, 3-azabicyclo[3.1.0]hexyl, pyrazolyl, morpholinyl, piperidinyl, pyrrolopyridinyl, 7-azabicyclo[2.2.1]heptyl, azepinyl, azetidinyl, 2-azabicyclo [2.2.1]heptyl, pyrrolidinyl, 5-azaspiro[2.4]heptyl, thiazolidinyl, indazolyl, dihydropyrimidinyl and triazolyl;
in particular, each Rb is independently selected from the group consisting of

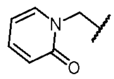,

Cl atom, -CN,

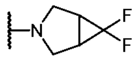,

cyclopropyl, F atom,

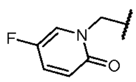,

methyl, ethyl,

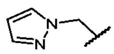, 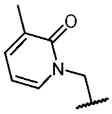, 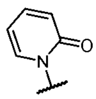, 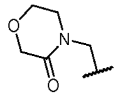,

Cl atom,

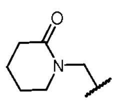, 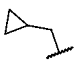, 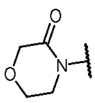, 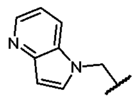,

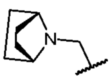, 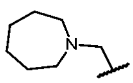, 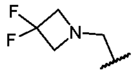, 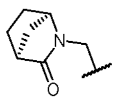, 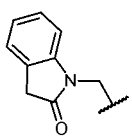, 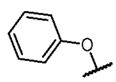, 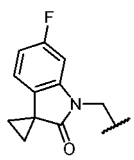,

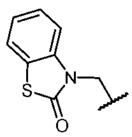, 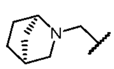, 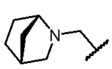,

trifluoromethyl, cyclohexyl,

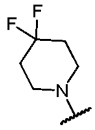, 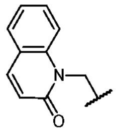,

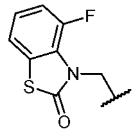, 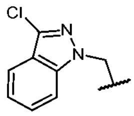, 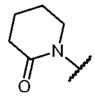, 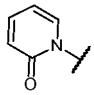, 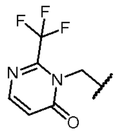,

methoxy, ethoxy,

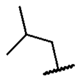, 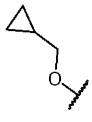,

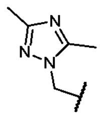, 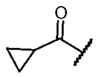, 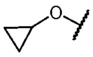, 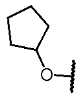 and 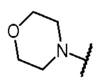.

In some embodiments of the invention, in a compound represented by the general formula (I), (II), (III), or (IV), or a pharmaceutically acceptable prodrug or salt thereof,

each Rc is independently selected from the group consisting of $C_{1-6}$alkyl, $C_{1-6}$haloalkyl, halogen, $-NH_2$, $-C_{1-6}$alkylene-$NH_2$, $-C_{1-6}$alkylene-NH-$C_{1-6}$alkyl, $-(C_{1-6}alkylene)_n-C_{1-6}$alkoxy, formamidinyl, N-hydroxyformamidinyl, -C(=NH)-NH-$C_{1-6}$ alkyl, -S-$C_{1-6}$alkyl, -NH-$C_{1-6}$alkyl, $-N(C_{1-6}alkyl)_2$, $C_{1-6}$hydroxyalkyl, -CN, $C_{3-6}$cycloalkyl, 3-6 membered heterocyclyl, $-NH-(C_{1-6}alkylene)_n-C_{3-6}$cycloalkyl, $-NH-(C_{1-6}alkylene)_n$-3-6 membered heterocyclyl, and 5-6 membered heteroaryl, each n is independently 0 or 1, the 3-6 membered heterocyclyl and 5-6 membered heteroaryl are each independently optionally substituted with one, two or three substituents selected from the group consisting of $C_{1-6}$alkyl, oxo, $C_{1-6}$alkoxy, halogen, and -OH, the 3-6 membered heterocyclyl and 5-6 membered heteroaryl each independently containing 1, 2 or 3 heteroatoms selected from the group consisting of N, O and S, preferably selected from pyrrolidinyl, azetidinyl, oxetanyl and triazolyl;

or two adjacent Rc together with the atoms to which they are attached form piperazinyl, piperidinyl, pyrrolidinyl, or dihydrofuran, each independently optionally substituted with one, two, or three substituents selected from the group consisting of $C_{1-6}$alkyl, oxo, $C_{1-6}$alkoxy, halogen, and -OH;

in particular, each Rc is independently selected from the group consisting of methyl, $-NH_2$, $-CH_2NH_2$, $-CH_2NHCH_3$, F atom, methoxy, difluoromethyl, formamidinyl, N-hydroxyformamidinyl, Cl atom, ethyl, methylthio, $-N(CH_3)_2$, -NH-$CH_3$, -CN,

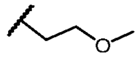

, pyrrolidinyl, cyclopropyl,

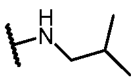,

-C(=NH)-NH-$CH_3$, -NH-cyclobutyl, -NH-$CH_2$-cyclopropyl,

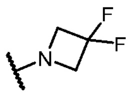,

-NH-$CH_2$-oxetanyl, azetidinyl,

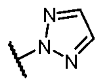

and hydroxyethyl.

In some embodiments of the invention, in a compound represented by the general formula (I), (II), (III), or (IV), or a pharmaceutically acceptable prodrug or salt thereof,

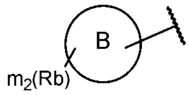

is selected from the group consisting of

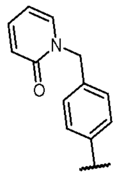, 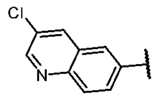,

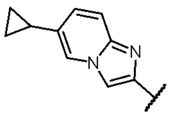, 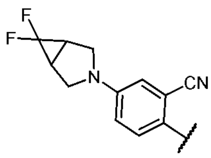, 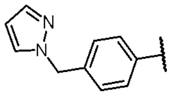, 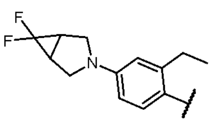, 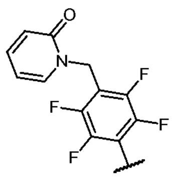,

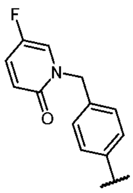, 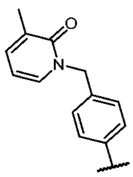, 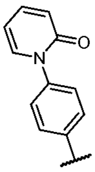, 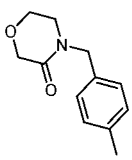, 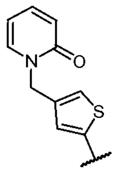, 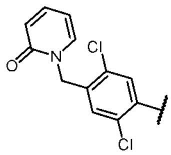, 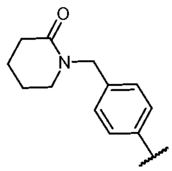,

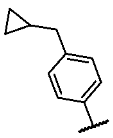, 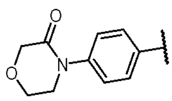, 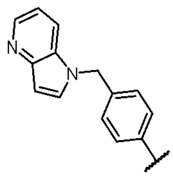, 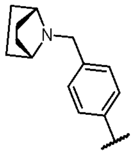, 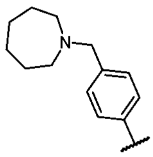, 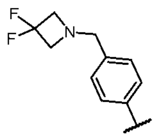,

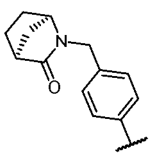, 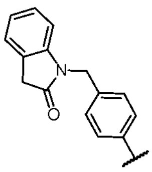, 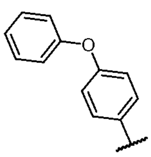, 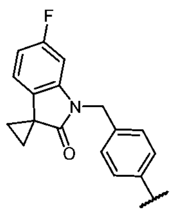, 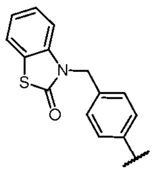, 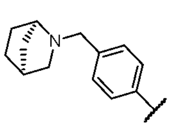,

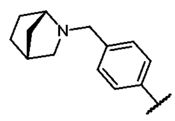, 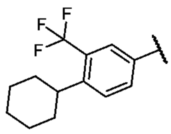, 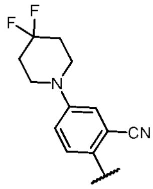, 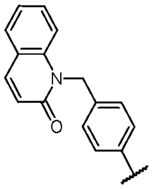, 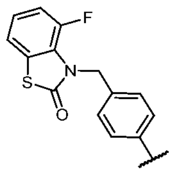, 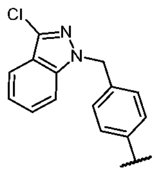,

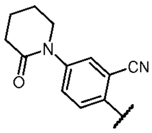, 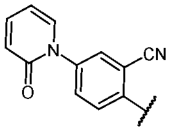, 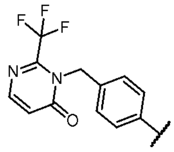, 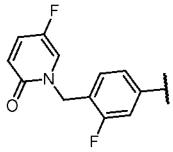, 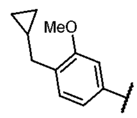, 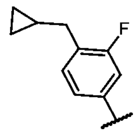,

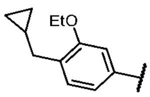, 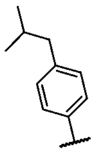, 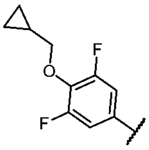, 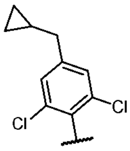, 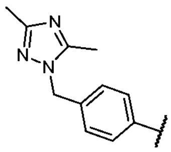, 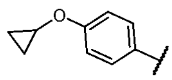, 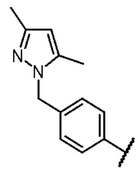,

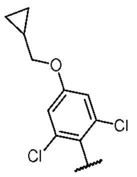, 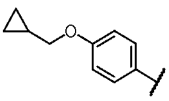, 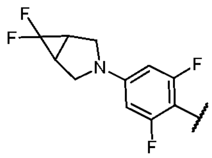, 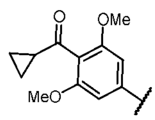, 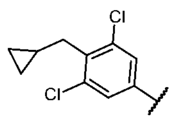, 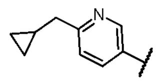,

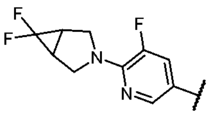, 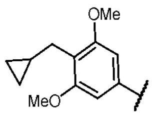, 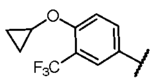, 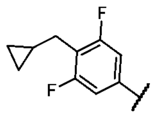, 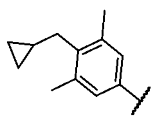, 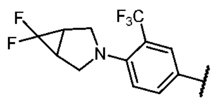,

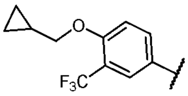, 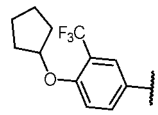, 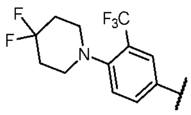, 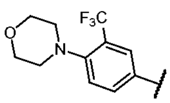, 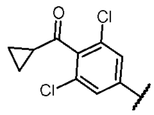

and

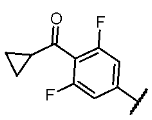.

In some embodiments of the invention, in a compound represented by the general formula (I), (II), (III), or (IV), or a pharmaceutically acceptable prodrug or salt thereof,

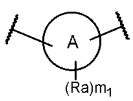

is selected from the group consisting of

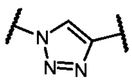, 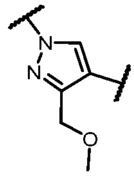,

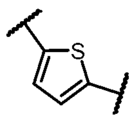, 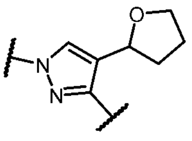, 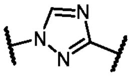, 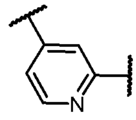, 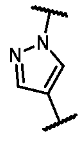, 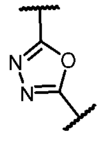, 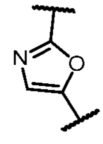, 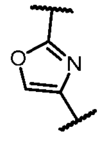,

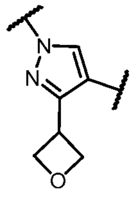, 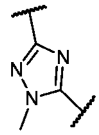, 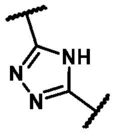, 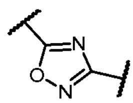, 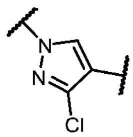, 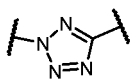, 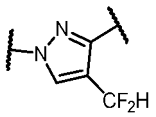,

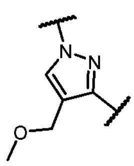, 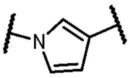, 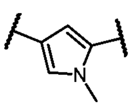, 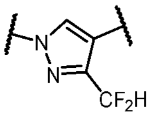, 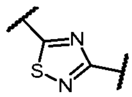, 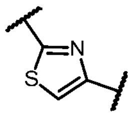, 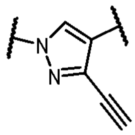,

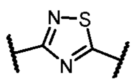, 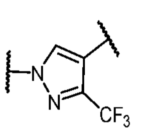, 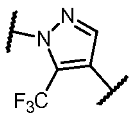, 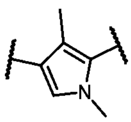, 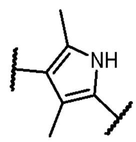, 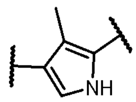,

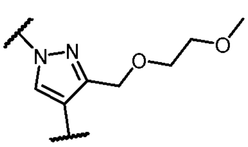, 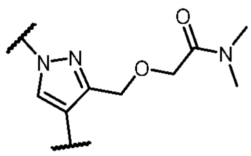, 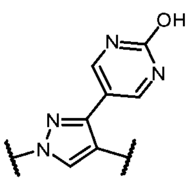, 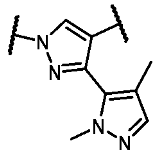, 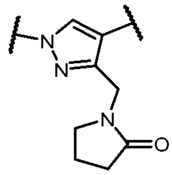,

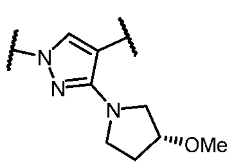, 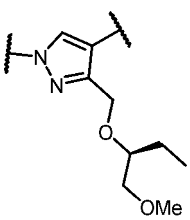, 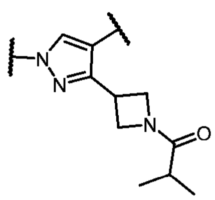, 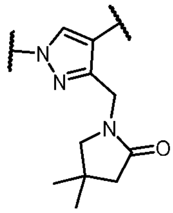, 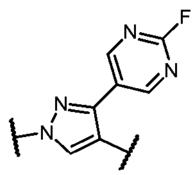,

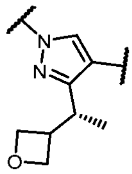, 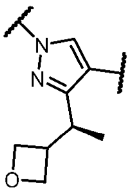, 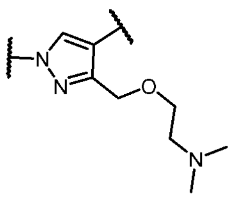, 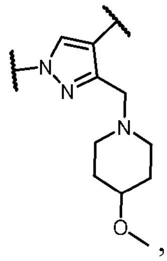, 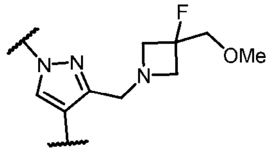, 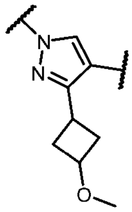

, 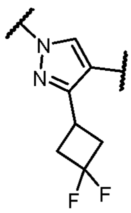, 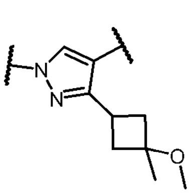, 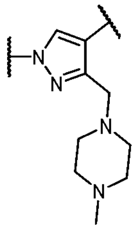, 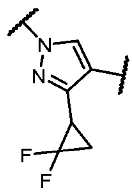, 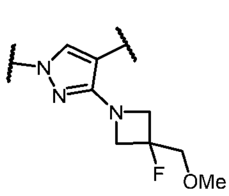, 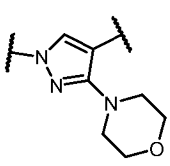,

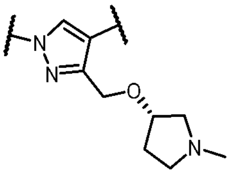, 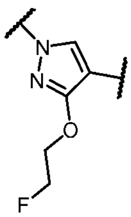, 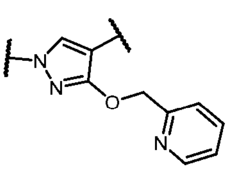, 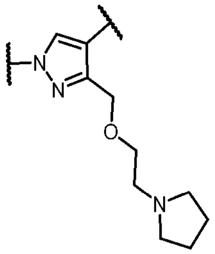, 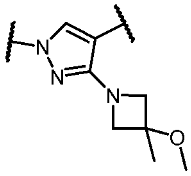,

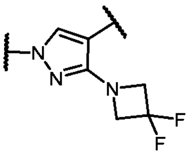, 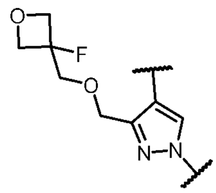, 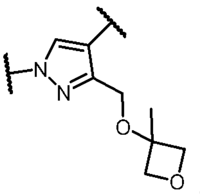, 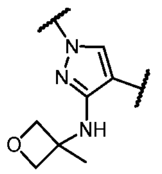, 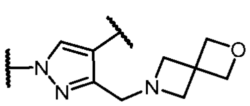,

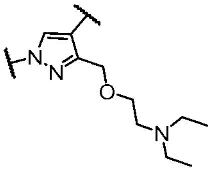, 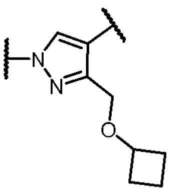, 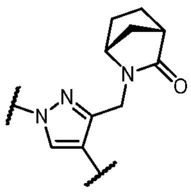, 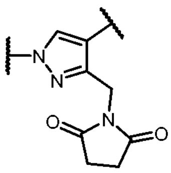, 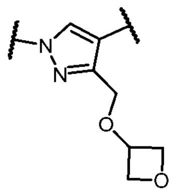,

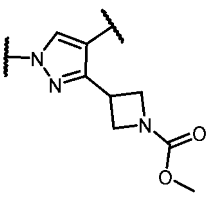, 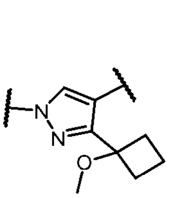, 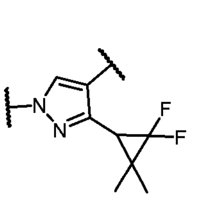, 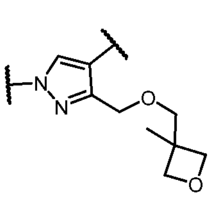, 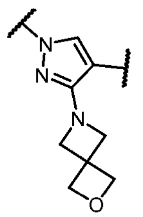,

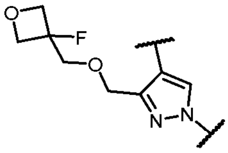, 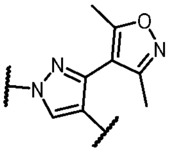, 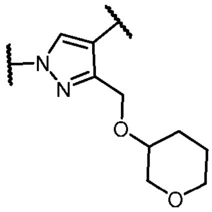, 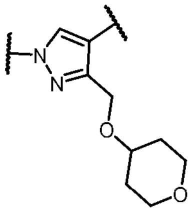, 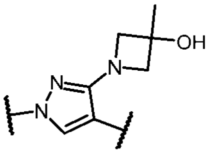,

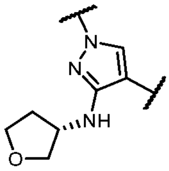, 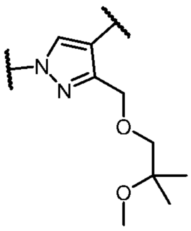, 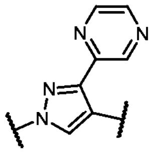, 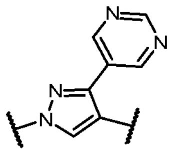, 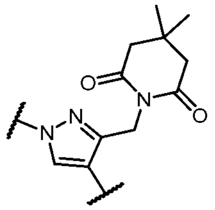,

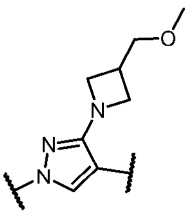, 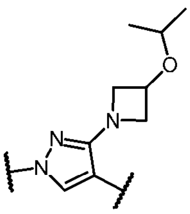, 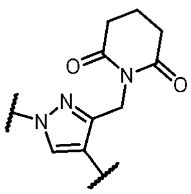, 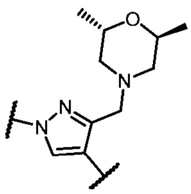, 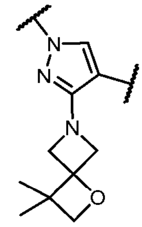,

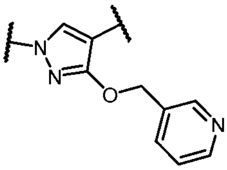, 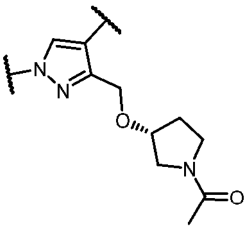, 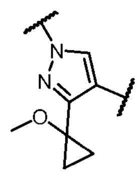, 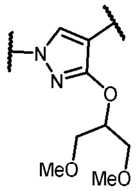 and 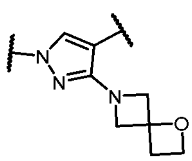.

In some embodiments of the invention, in a compound represented by the general formula (I), (II), (III), or (IV), or a pharmaceutically acceptable prodrug or salt thereof,

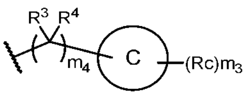, 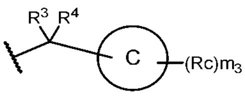 or 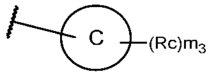

is selected from the group consisting of

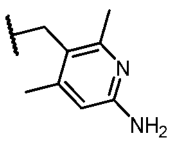, 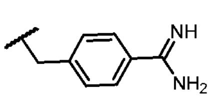, 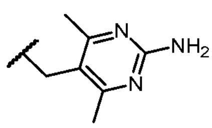, 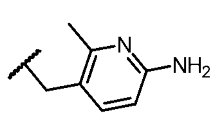,

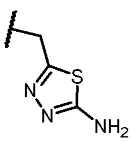, 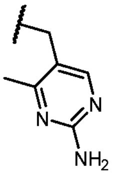, 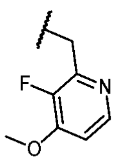, 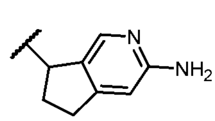, 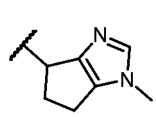, 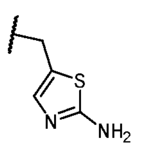,

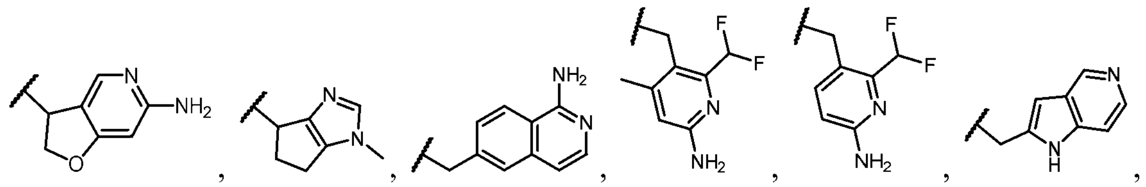

, , , , , ,

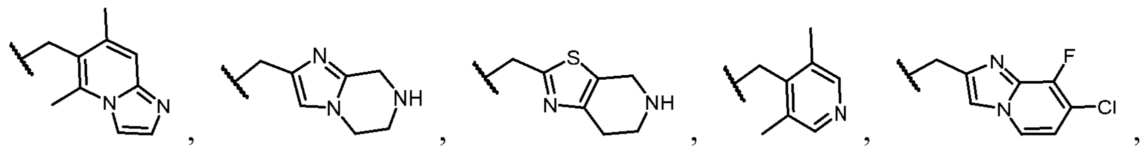

, , , , ,

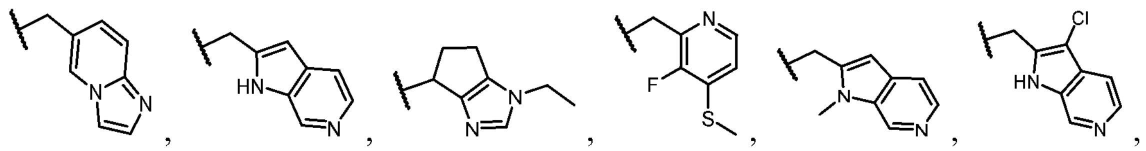

, , , , , ,

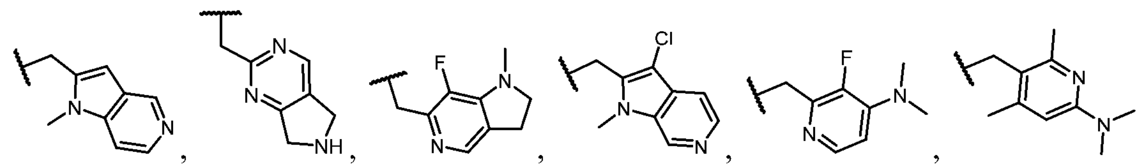

, , , , , ,

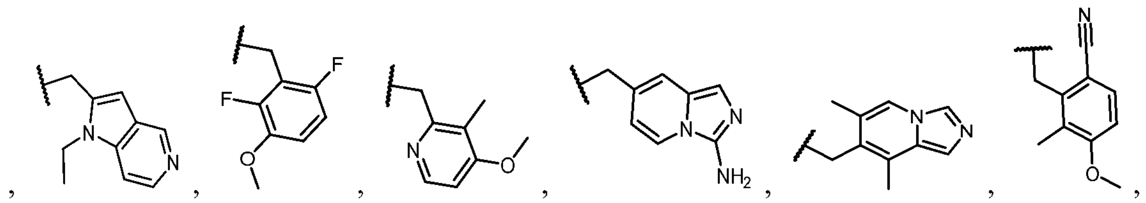

, , , , , , ,

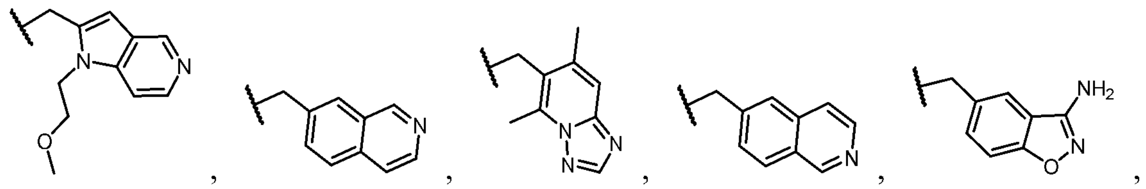

, , , , ,

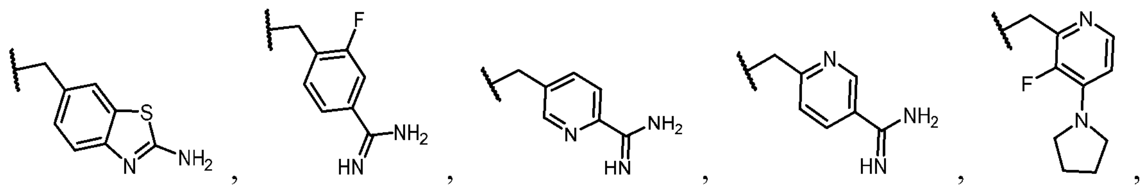

, , , , ,

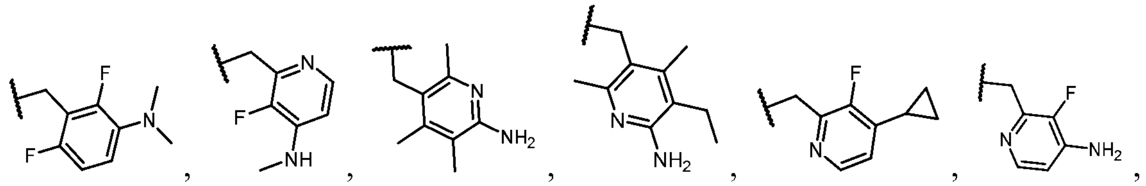

, , , , , ,

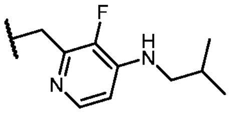, 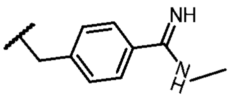, 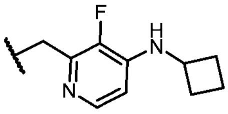, 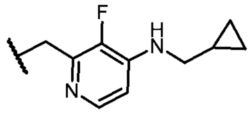, 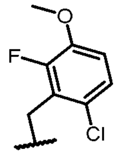,

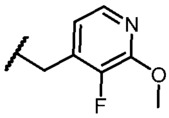, 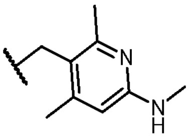, 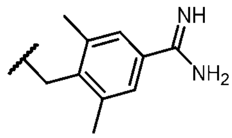, 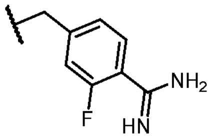, 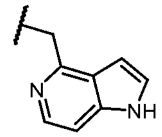,

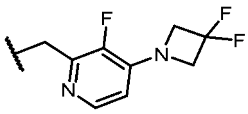, 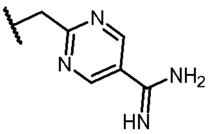, 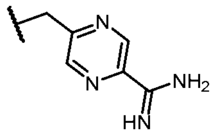, 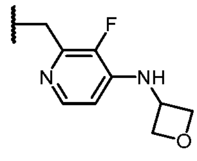, 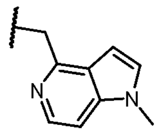,

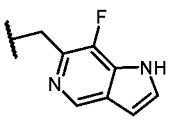, 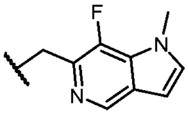, 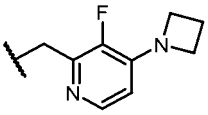, 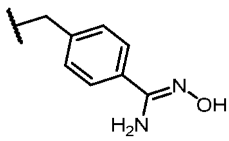, 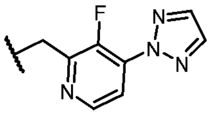,

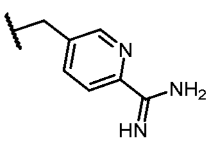, 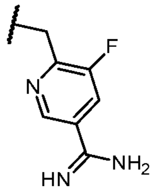, 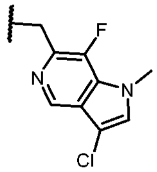, 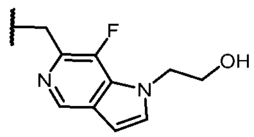, 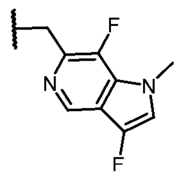,

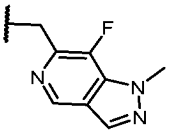, 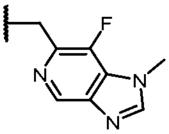, 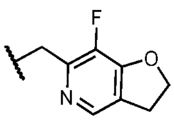, 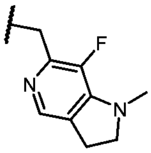 and 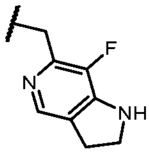.

In some embodiments of the invention, in a compound represented by the general formula (I), (II), (III), or (IV), or a pharmaceutically acceptable prodrug or salt thereof,
when each Rc is selected from the group consisting of formamidinyl, N-hydroxyformamidinyl, or $-NH_2$, the pharmaceutically acceptable prodrug of the compound is selected from the group consisting of:

$C_{1-6}$carbonate of formamidinyl and $-NH_2$;
$C_{1-6}$carbonate, $C_{1-6}$carboxylate, phenylcarboxylate or pyridylcarboxylate of N-hydroxyformamidinyl, and phenylcarboxylate and pyridylcarboxylate may be substituted with one or more halogens, $C_{1-6}$alkyl or $C_{1-6}$alkoxy;
and/or
the pharmaceutically acceptable salt is selected from the group consisting of tosylate, mesylate, hydrochloride, acetate, and citrate.

Typical compounds of the present application include, but not limited to:

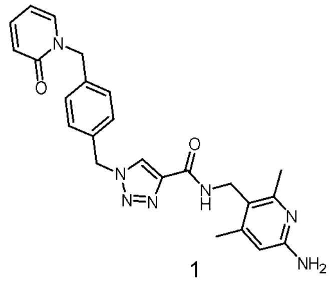
1 ,

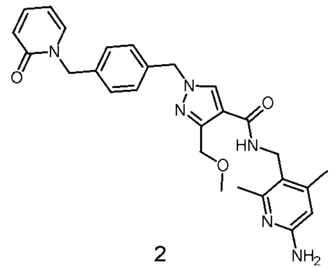
2 ,

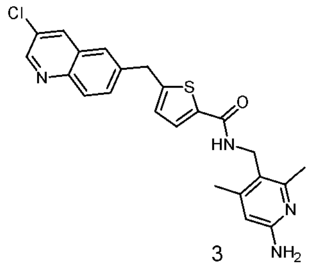
3 ,

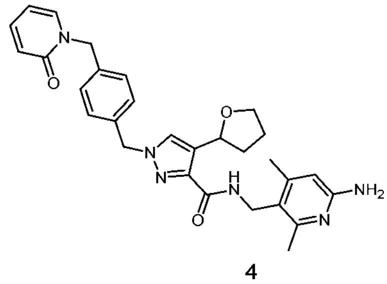
4 ,

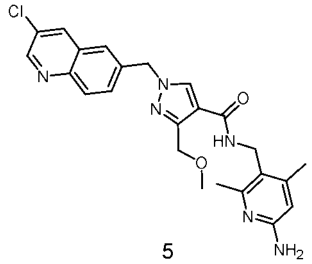
5 ,

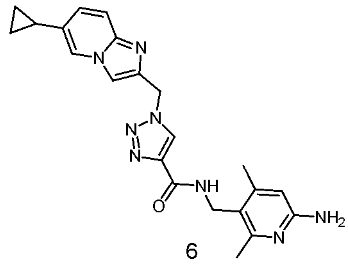
6 ,

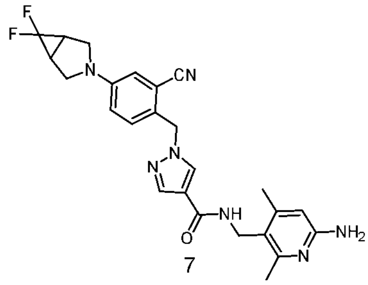
7 ,

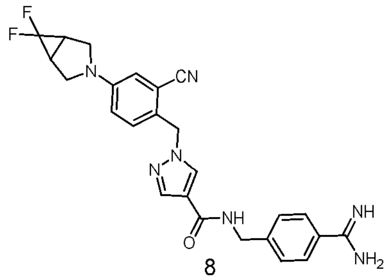
8 ,

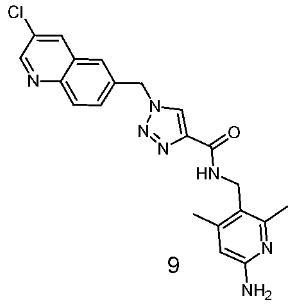
9 ,

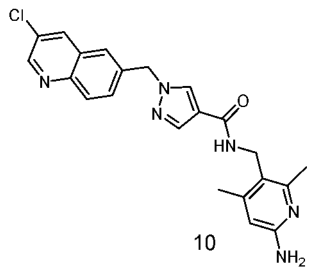
10 ,

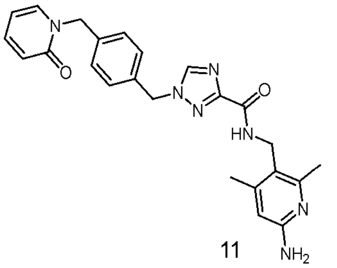
11 ,

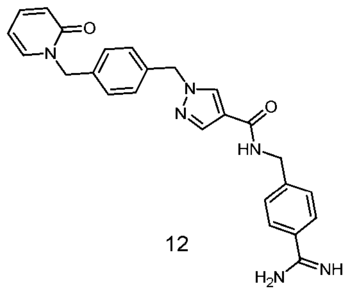
12 ,

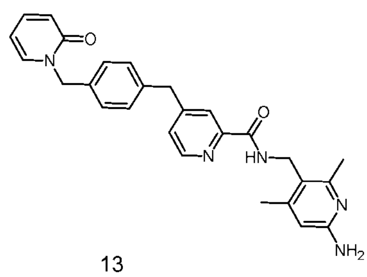
13 ,

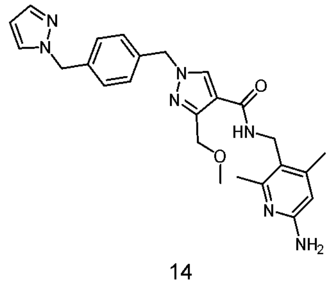
14 ,

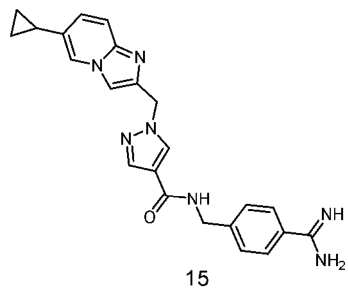
15 ,

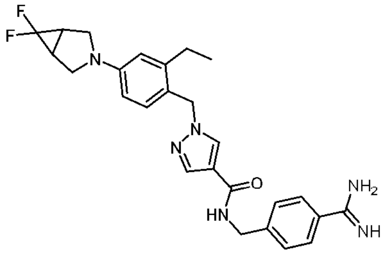
16,

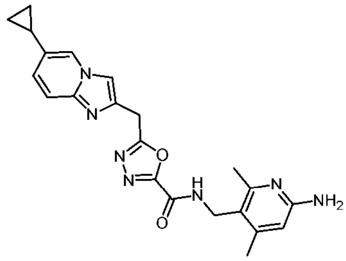
17,

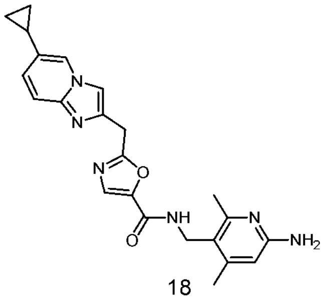
18,

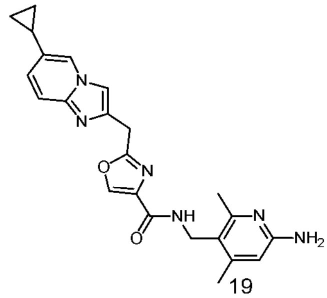
19,

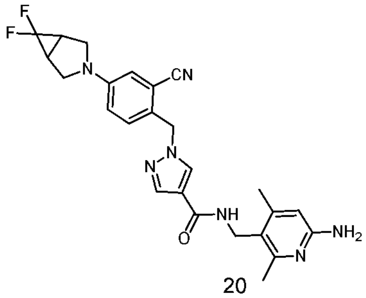
20,

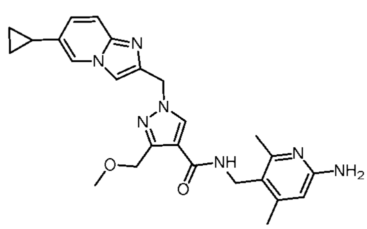
21,

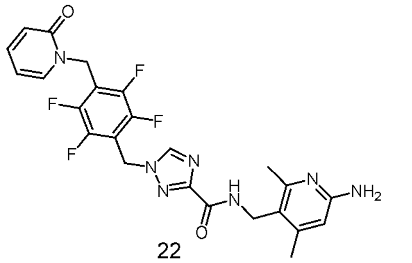
22,

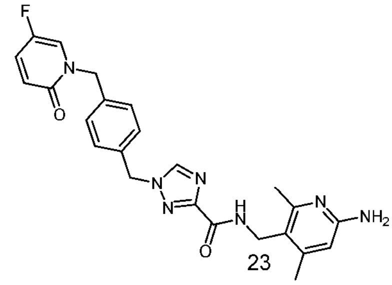
23,

20

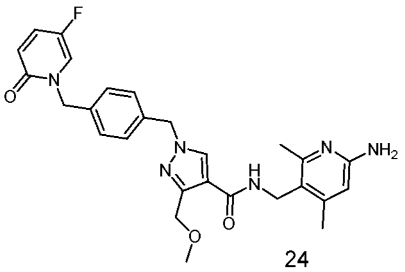
24,

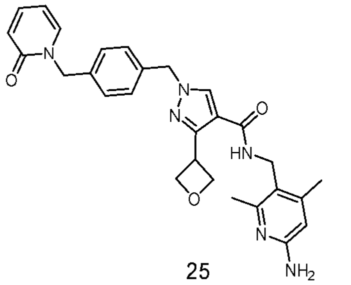
25,

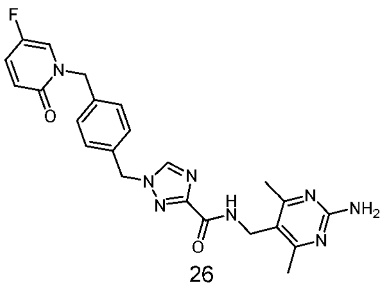
26,

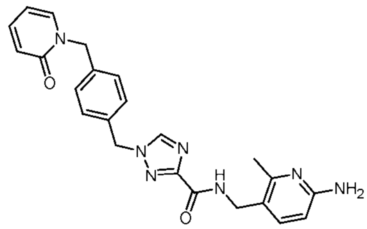
27,

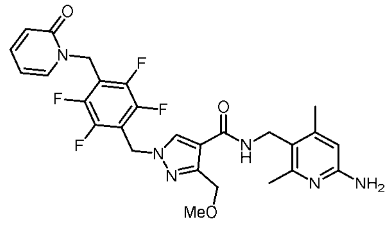

28 ,

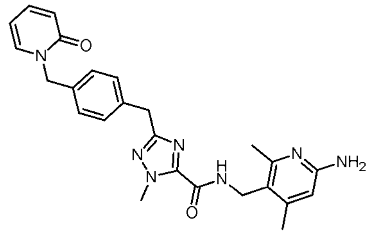

29 ,

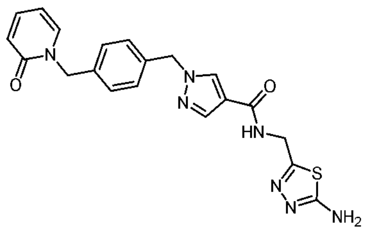

30 ,

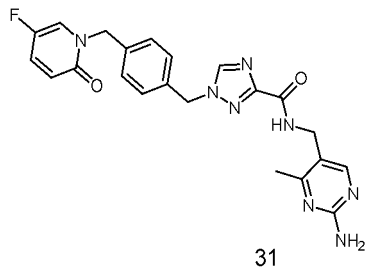

31 ,

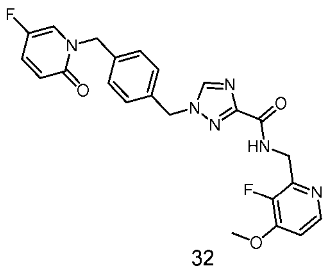

32 ,

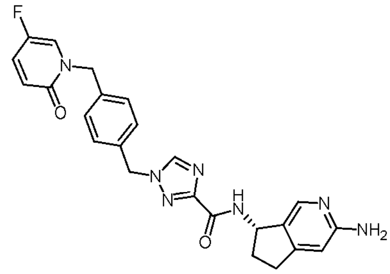

33 ,

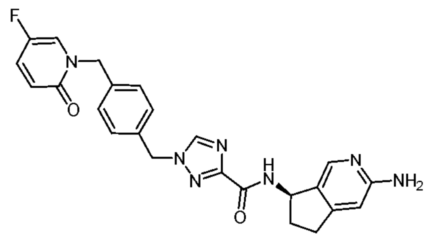

34 ,

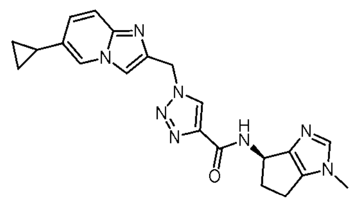

35 ,

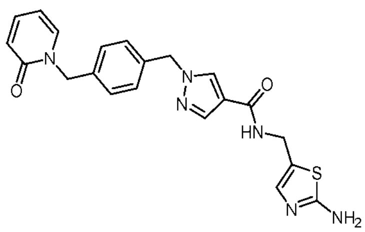

36 ,

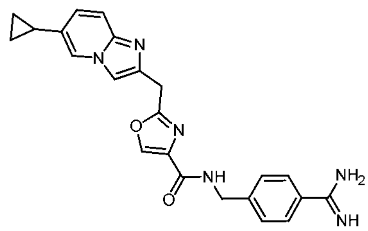

37 ,

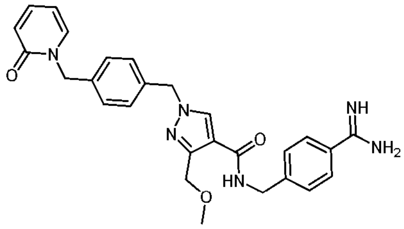

38 ,

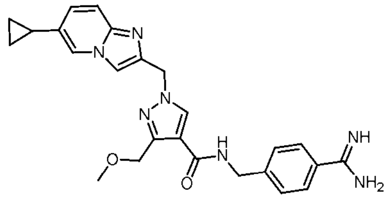

39 ,

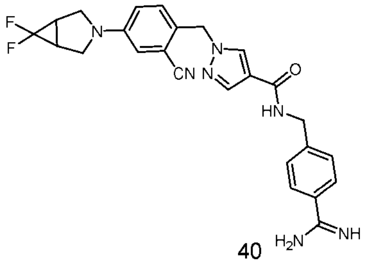
40 ,

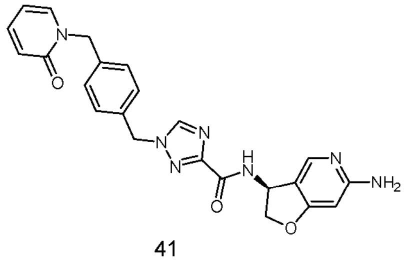
41 ,

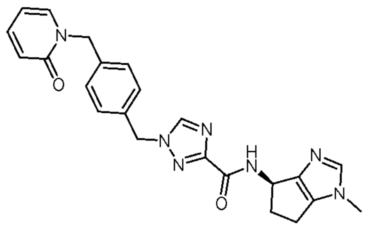
42 ,

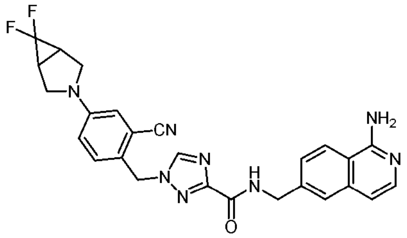
43 ,

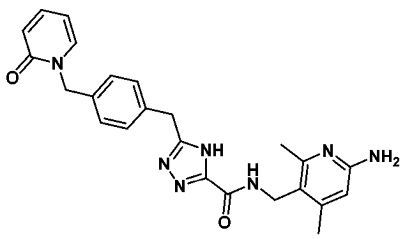
44 ,

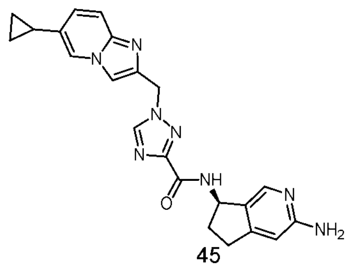
45 ,

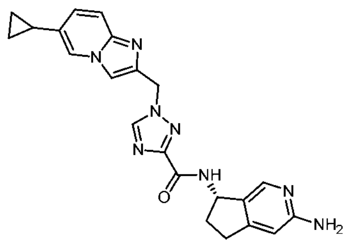
46

, 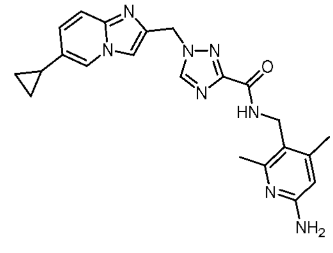
47 ,

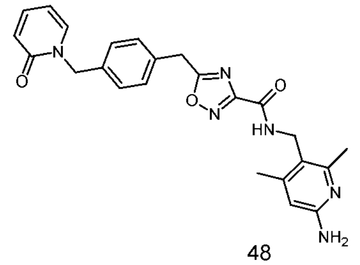
48 ,

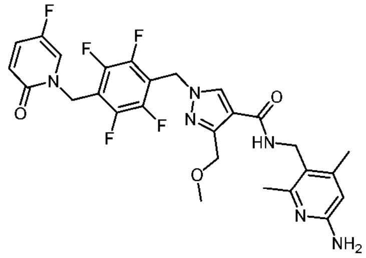
49 ,

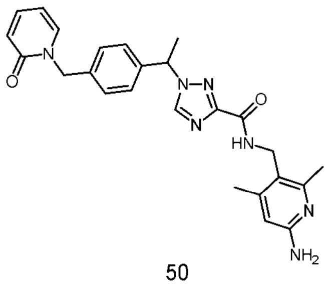
50 ,

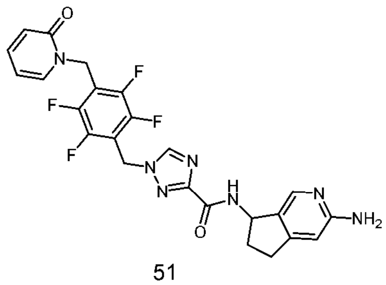
51 ,

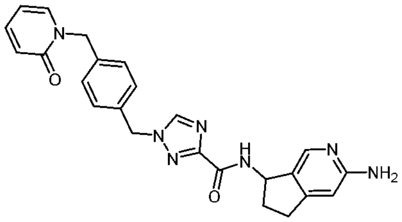

52 ,

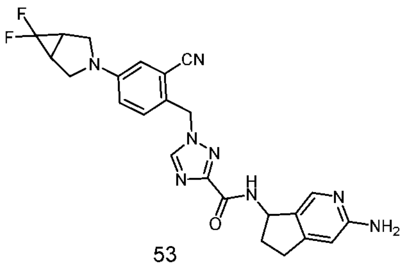

53 ,

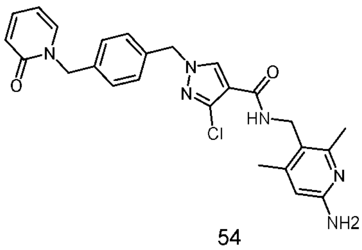

54 ,

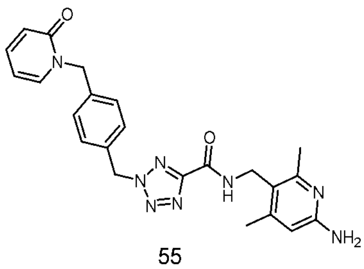

55 ,

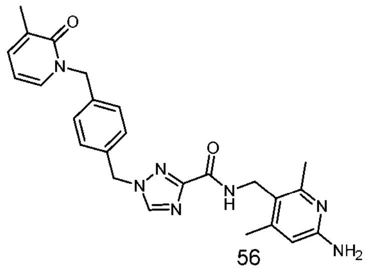

56 ,

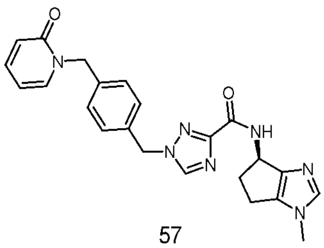

57 ,

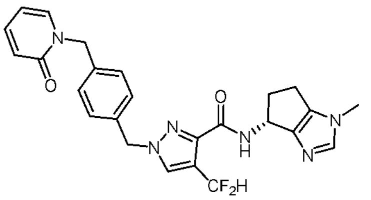

58 ,

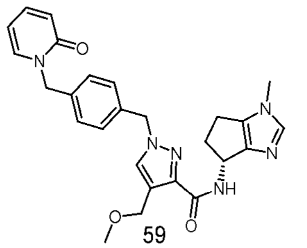

59 ,

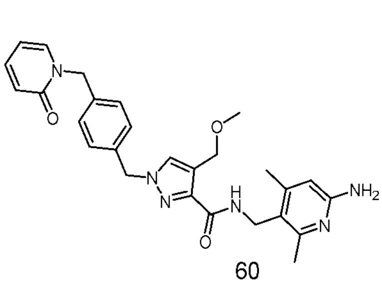

60 ,

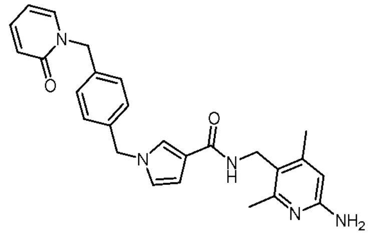

61 ,

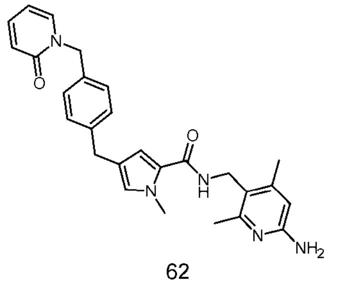

62 ,

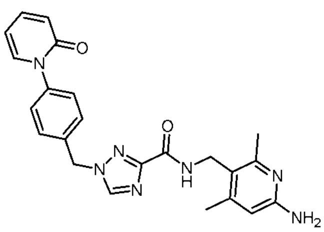

63 ,

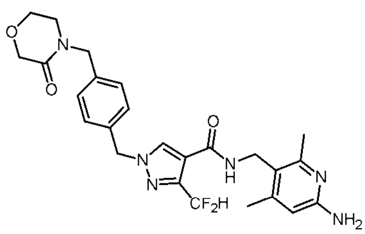

64 ,

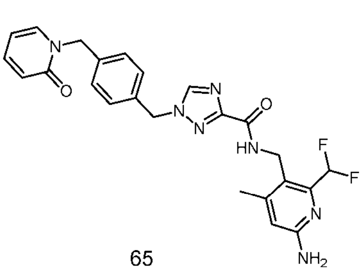

65 ,

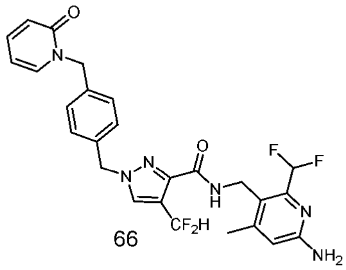
66 ,

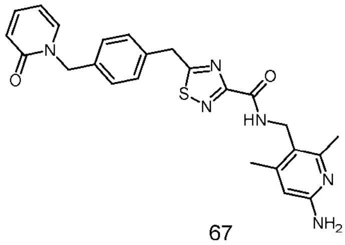
67 ,

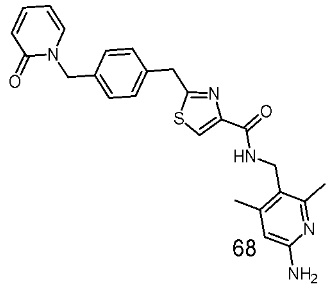
68 ,

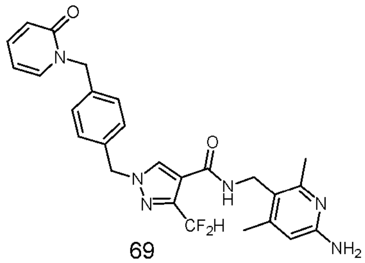
69 ,

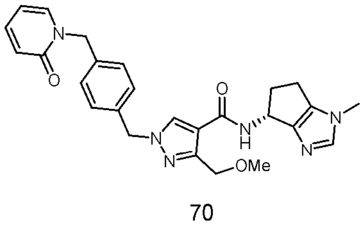
70 ,

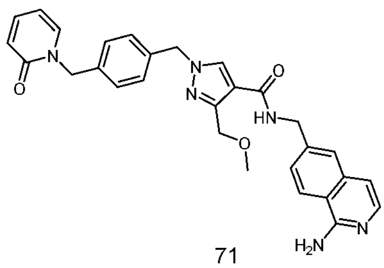
71 ,

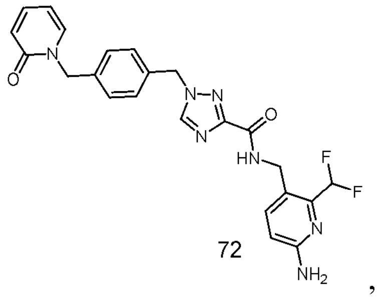
72 ,

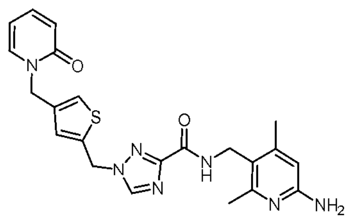
73 ,

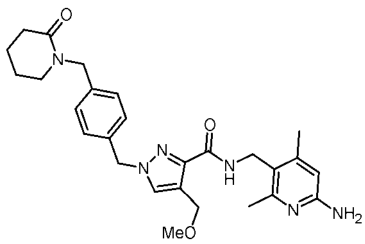
74 ,

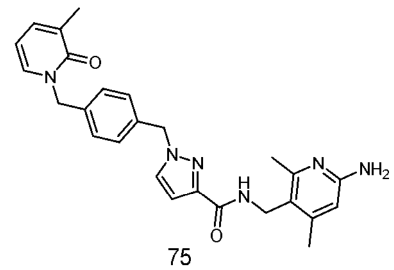
75 ,

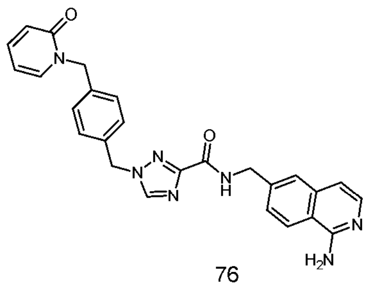
76 ,

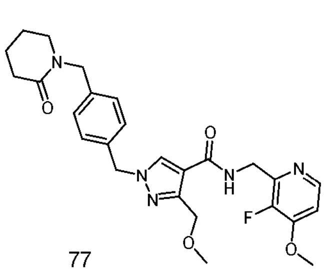
77 ,

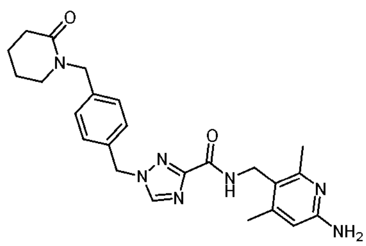
78 ,

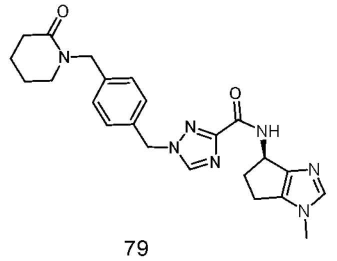
79 ,

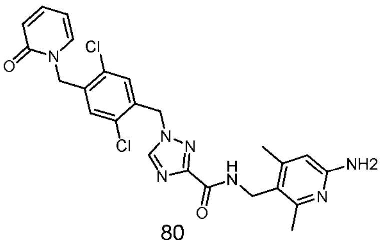
80 ,

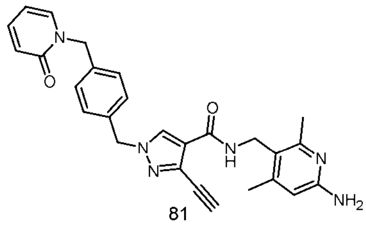
81 ,

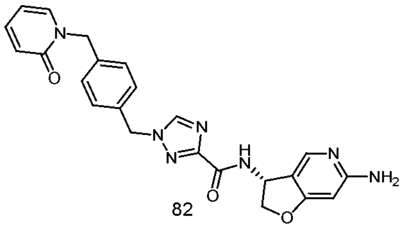
82 ,

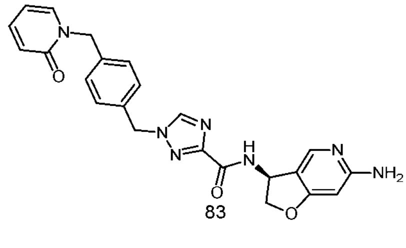
83 ,

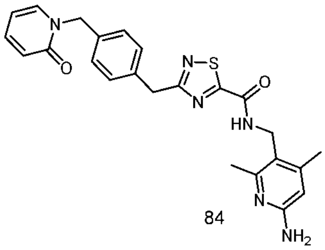
84 ,

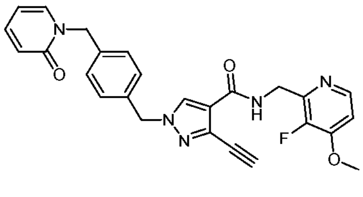
85 ,

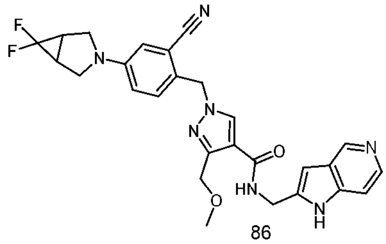
86 ,

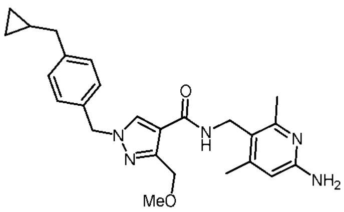
87 ,

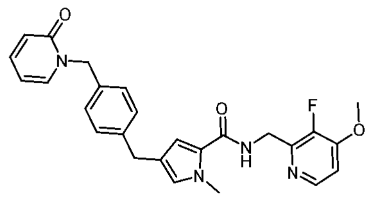
88 ,

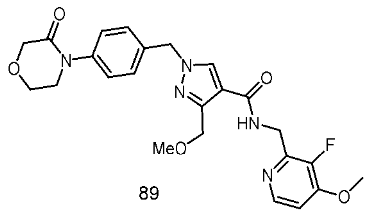
89 ,

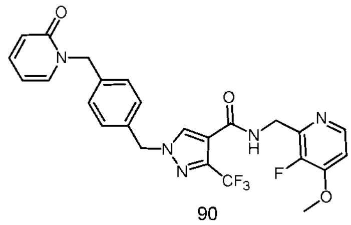
90 ,

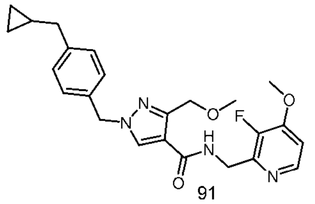
91 ,

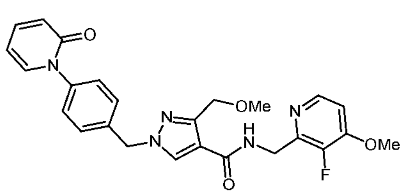
92

, 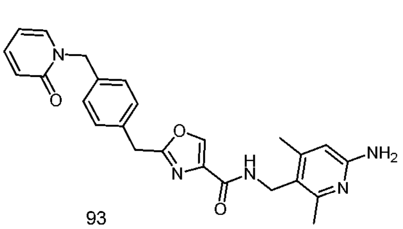
93 ,

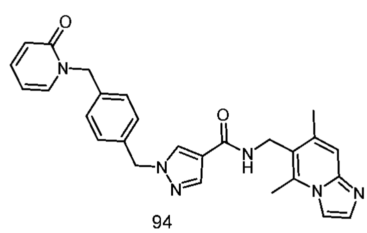
94 ,

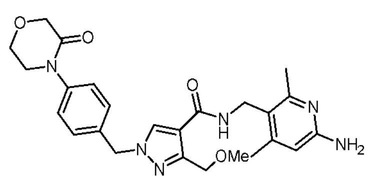
95 ,

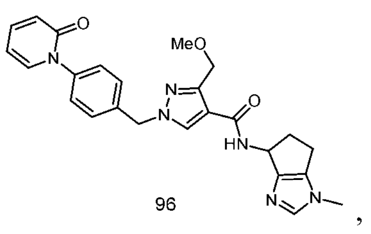
96 ,

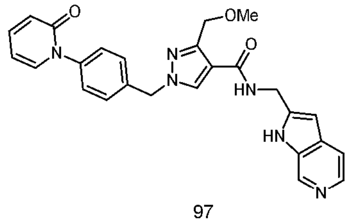
97 ,

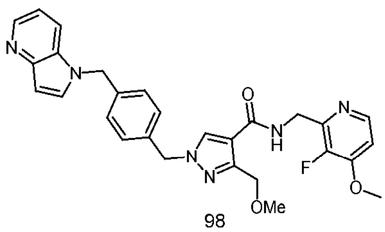
98 ,

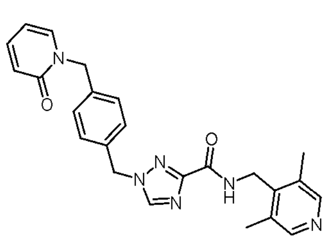
99 ,

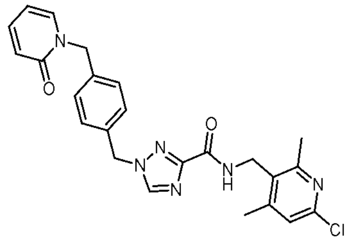
100 ,

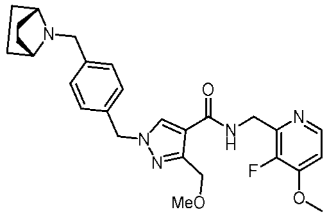
101 ,

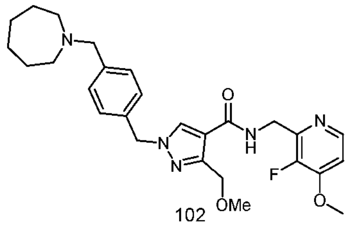
102 ,

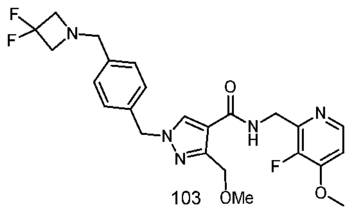
103 ,

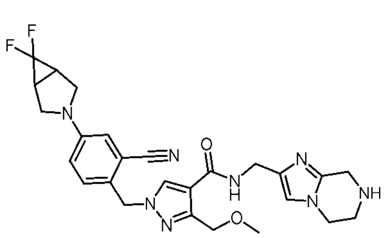
104 ,

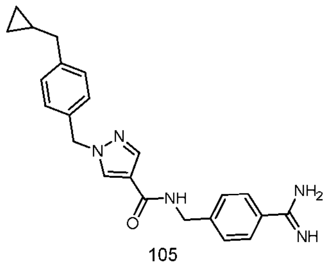
105 ,

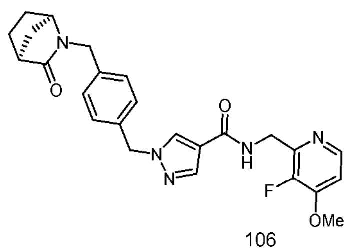
106 ,

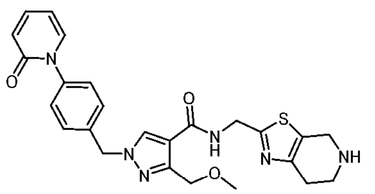
107 ,

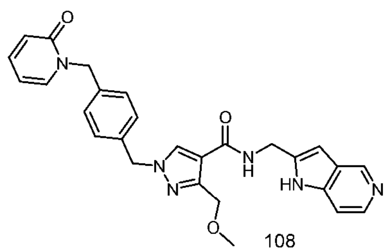
108 ,

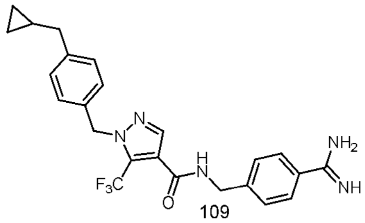
109 ,

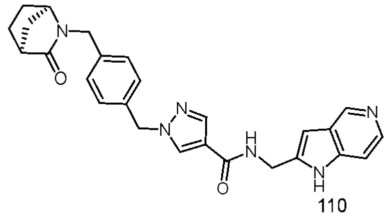
110 ,

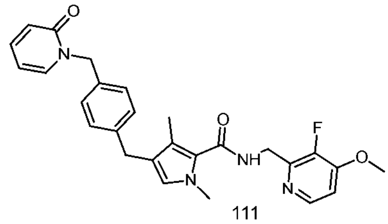
111
,

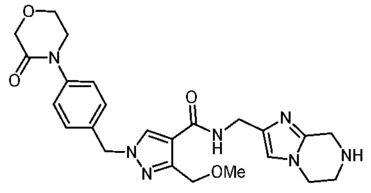
112
,

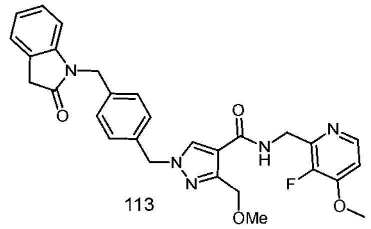
113
,

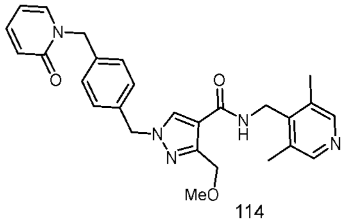
114
,

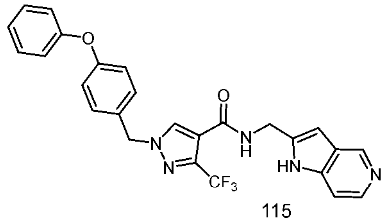
115
,

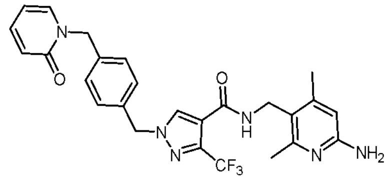
116
,

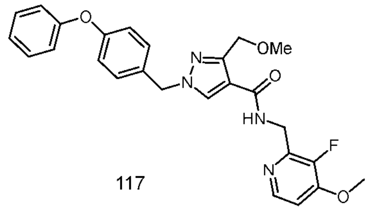
117
,

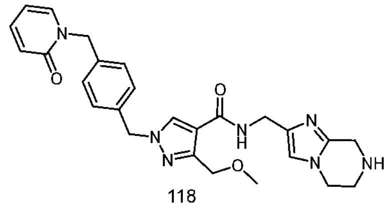
118
,

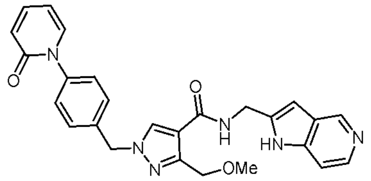
119
,

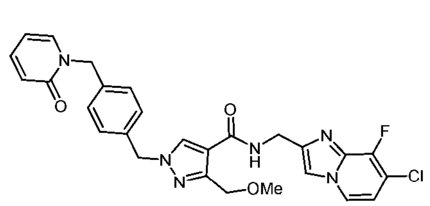
120
,

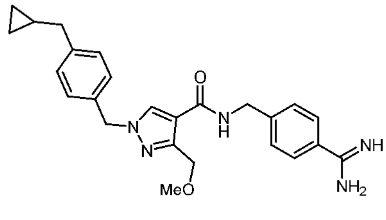
121
,

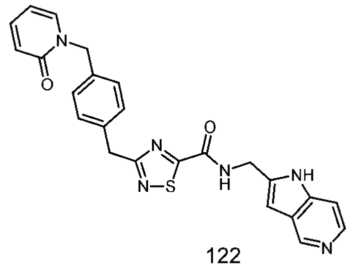
122
,

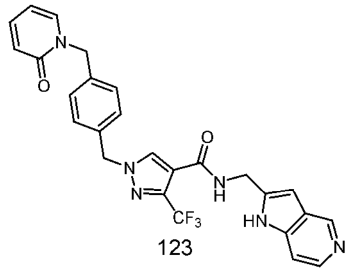
123
,

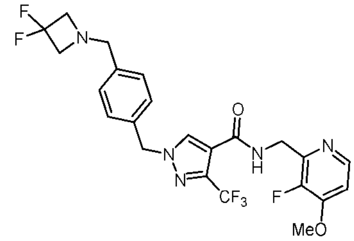
124
,

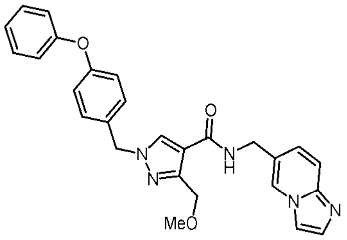
125 ,
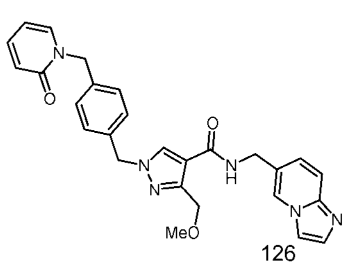
126 ,
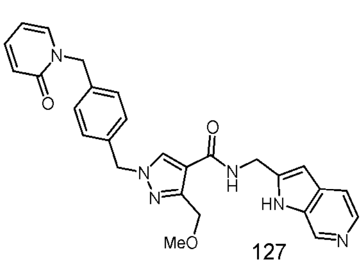
127 ,
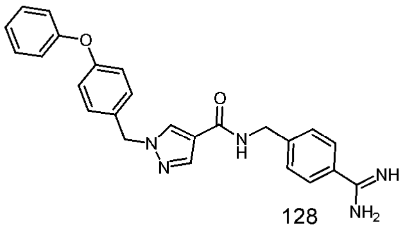
128 ,
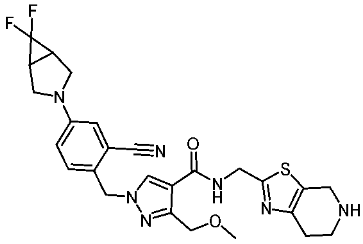
129 ,
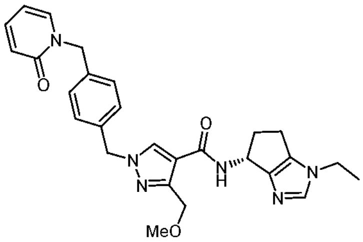
130 ,
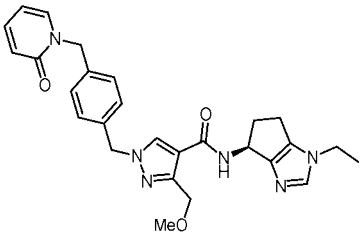
131 ,
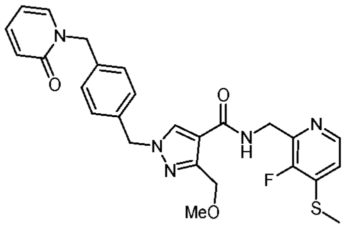
132 ,
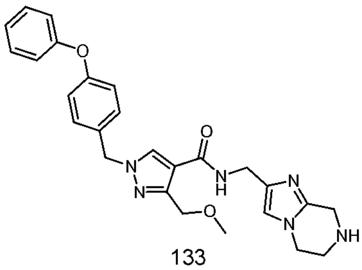
133 ,
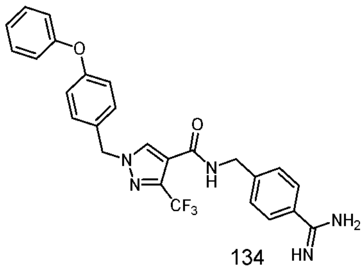
134 ,
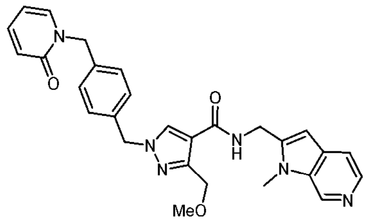
135 ,
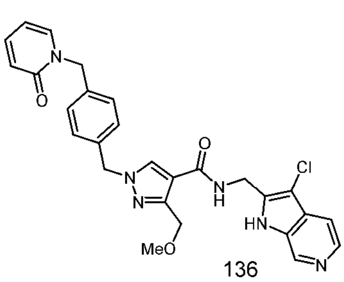
136 ,
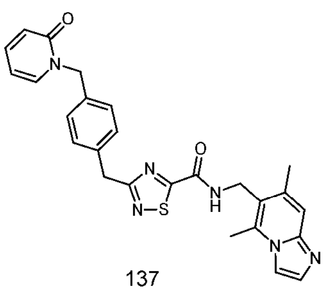
137 ,
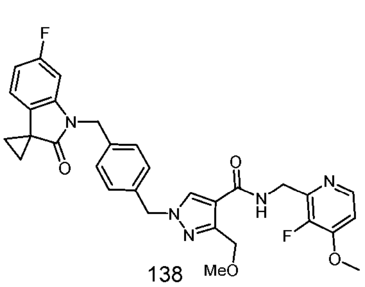
138 ,

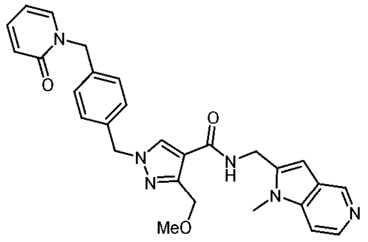
139 ,

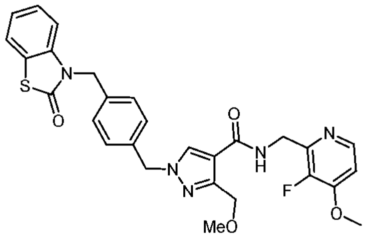
140 ,

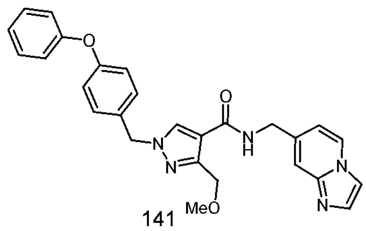
141 ,

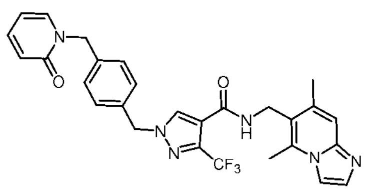
142 ,

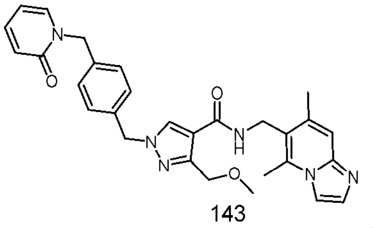
143 ,

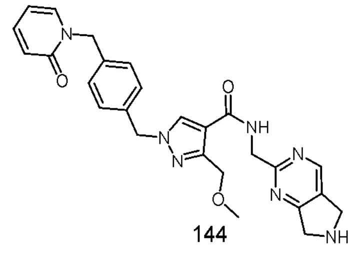
144 ,

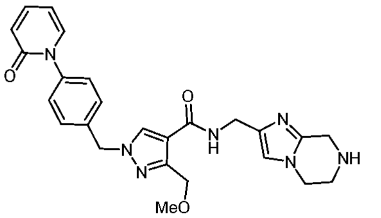
145 ,

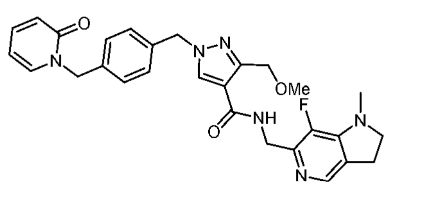
146 ,

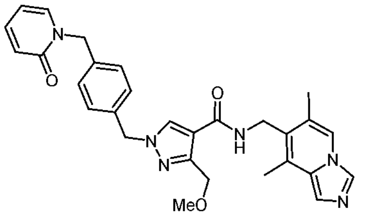
147 ,

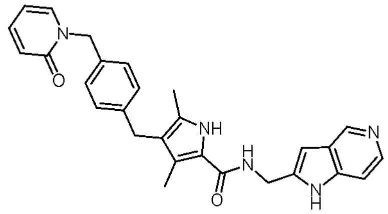
148 ,

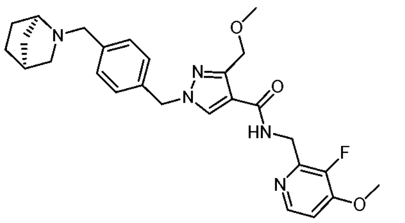
149 ,

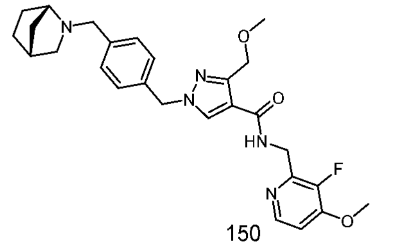
150 ,

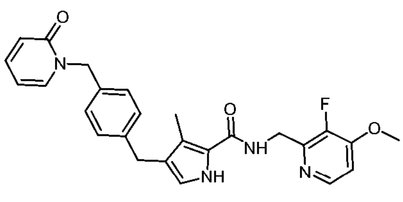
151 ,

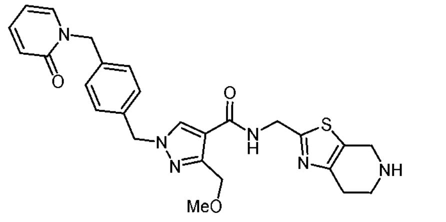
152 ,

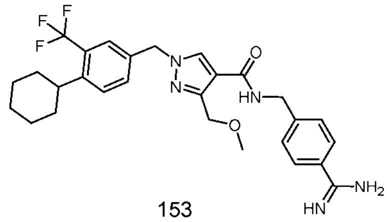
153
,

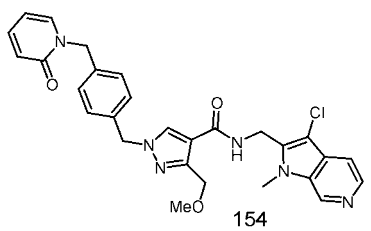
154
,

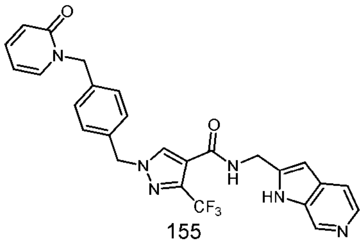
155
,

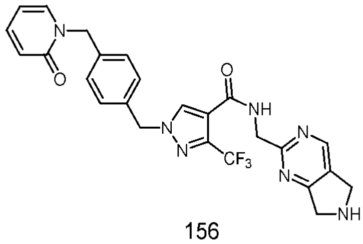
156
,

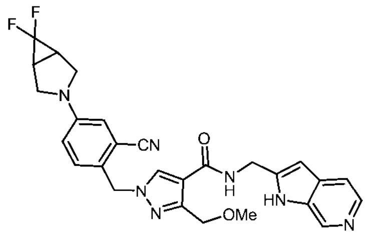
157
,

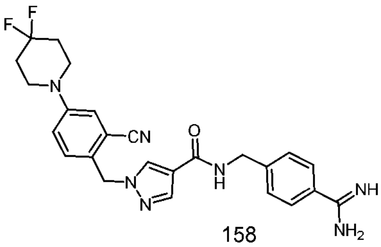
158
,

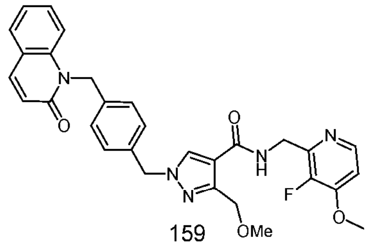
159
,

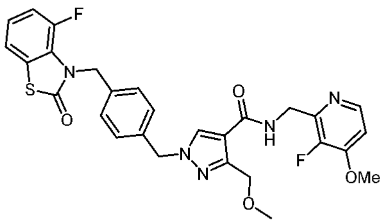
160
,

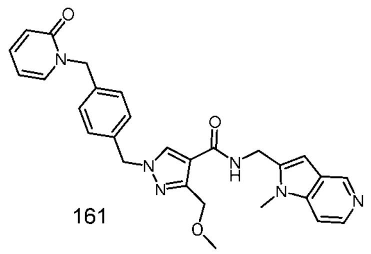
161
,

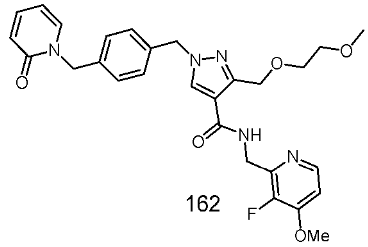
162
,

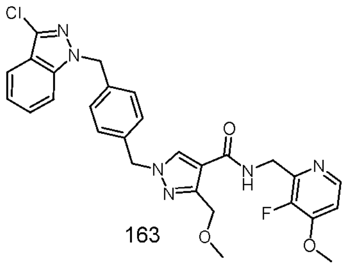
163
,

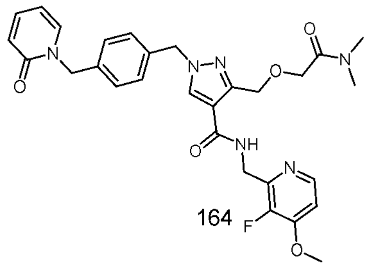
164
,

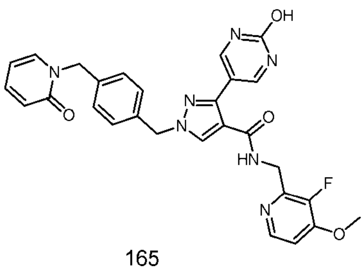
165
,

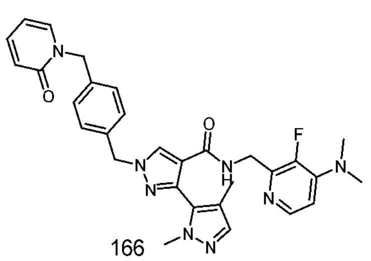
166
,

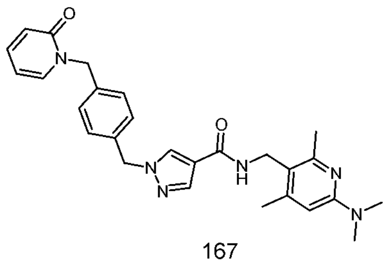

167,

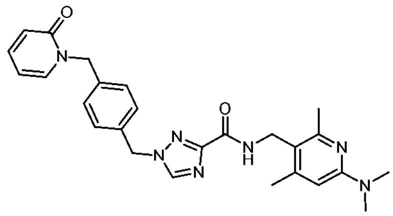

168,

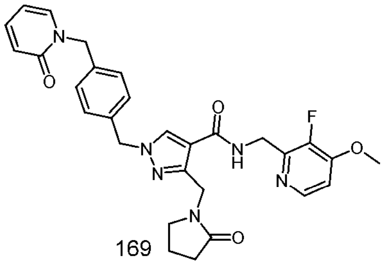

169,

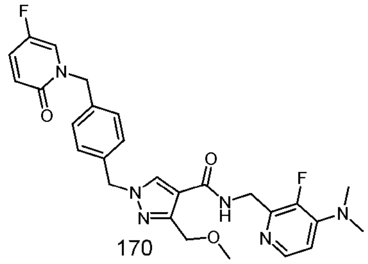

170,

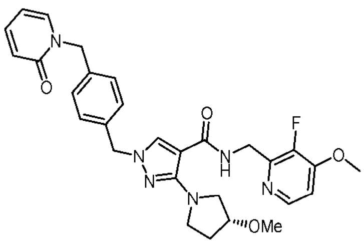

171,

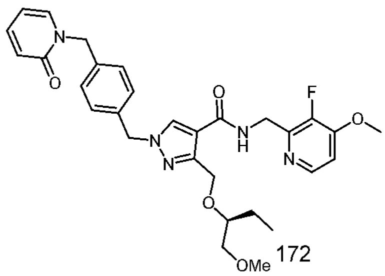

172,

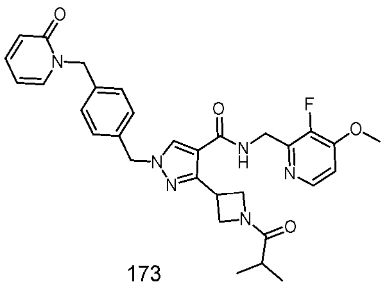

173,

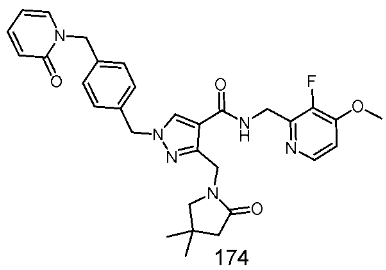

174,

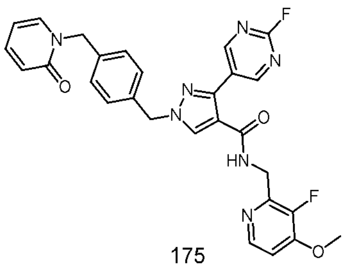

175,

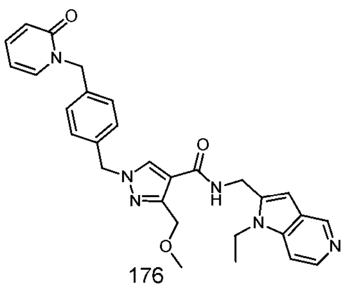

176,

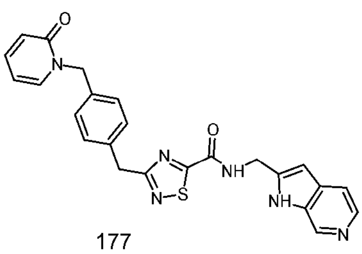

177,

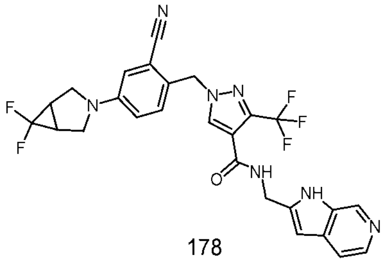
178 ,

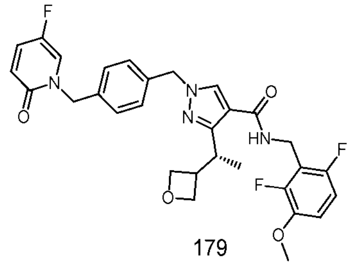
179 ,

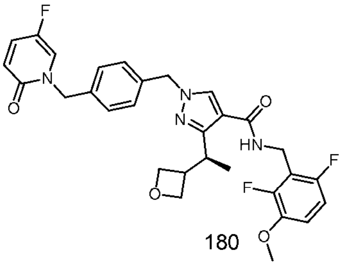
180 ,

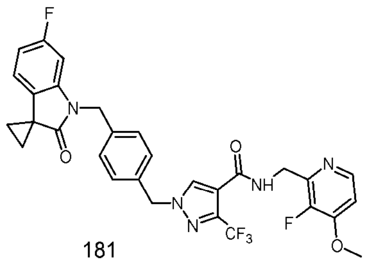
181 ,

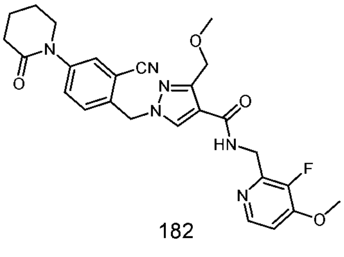
182 ,

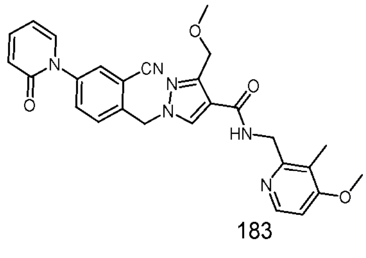
183 ,

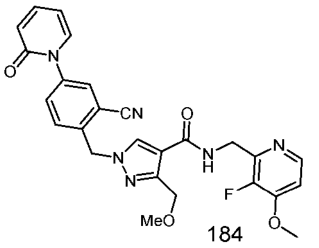
184 ,

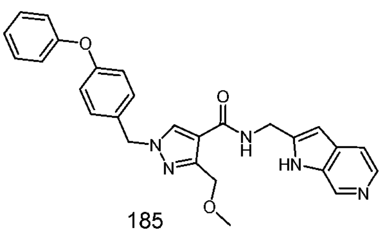
185 ,

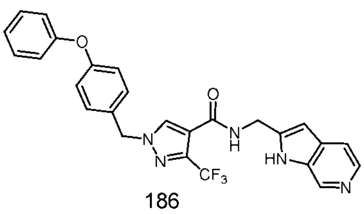
186 ,

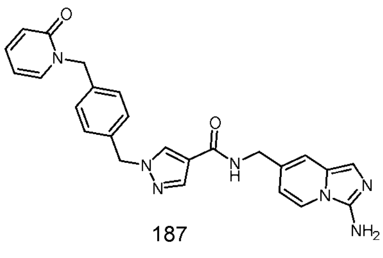
187 ,

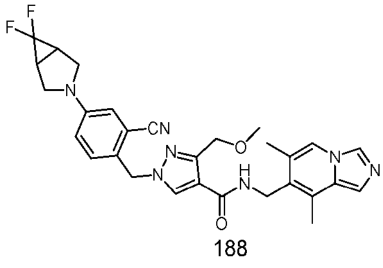
188 ,

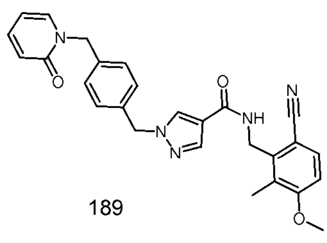
189 ,

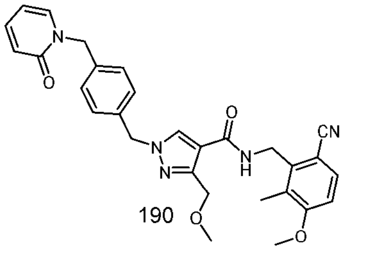
190 ,

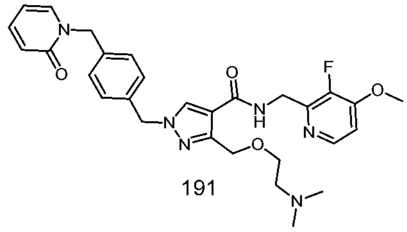
191 ,

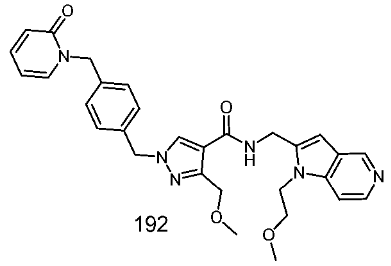

192 ,

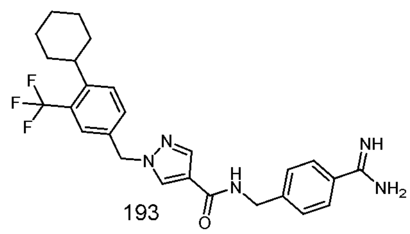

193 ,

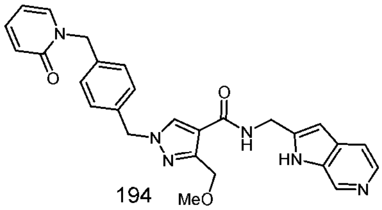

194 ,

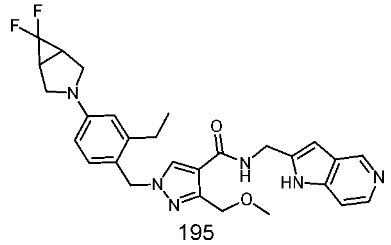

195 ,

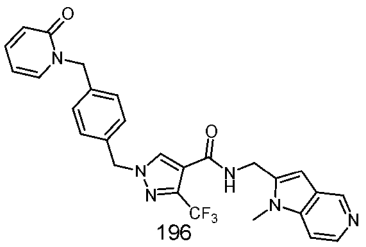

196 ,

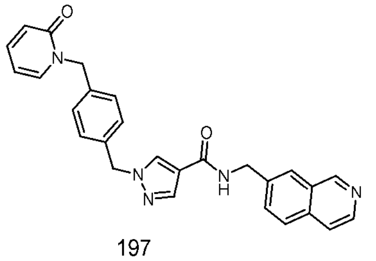

197 ,

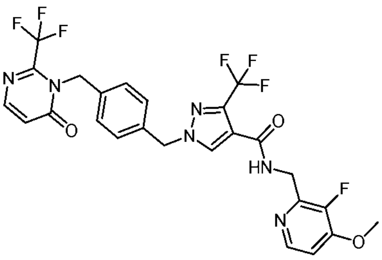

198 ,

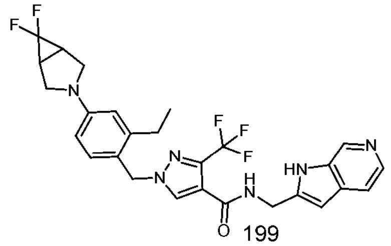

199 ,

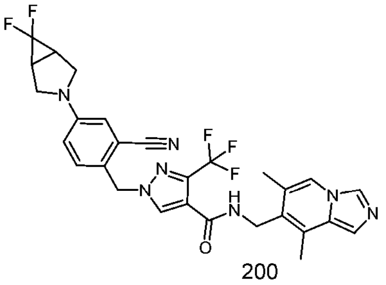

200 ,

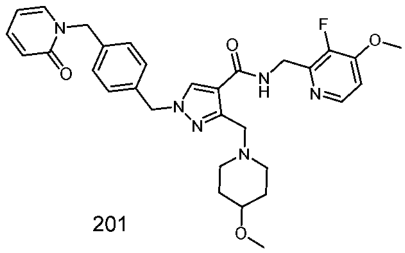

201 ,

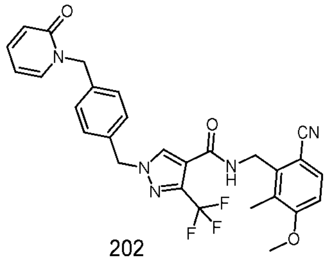
202 ,

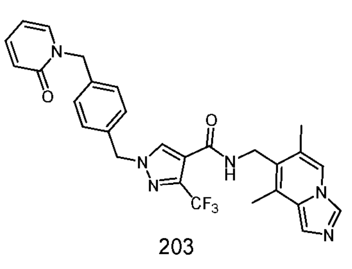
203 ,

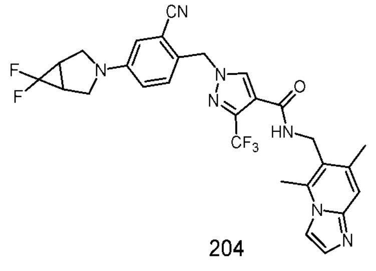
204 ,

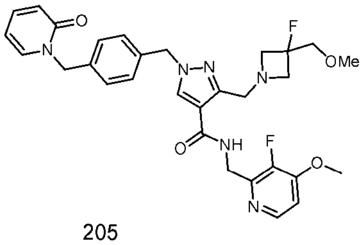
205 ,

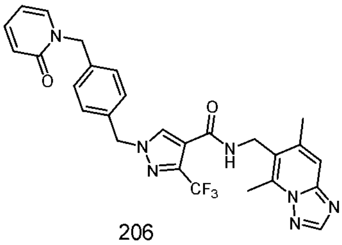
206 ,

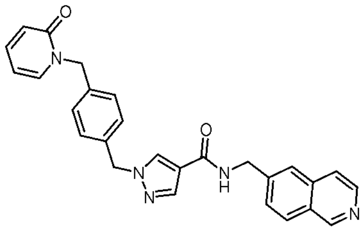
207 ,

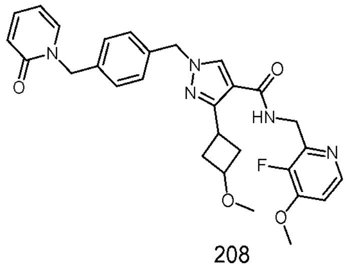
208 ,

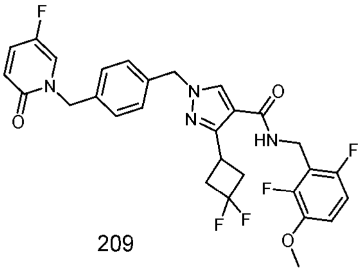
209 ,

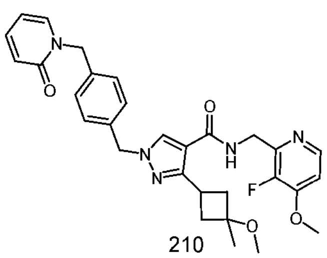
210 ,

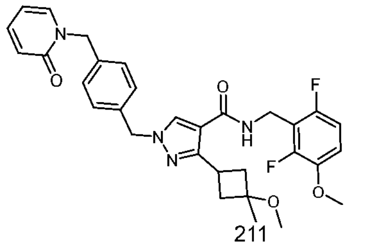
211 ,

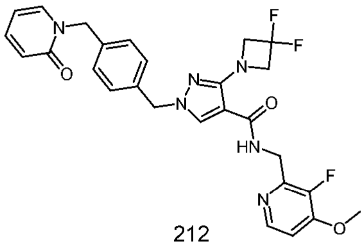
212 ,

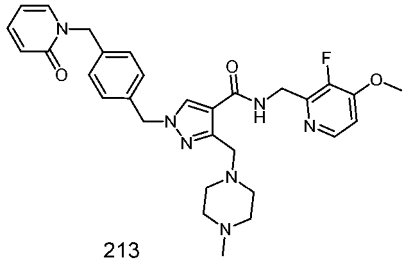
213 ,

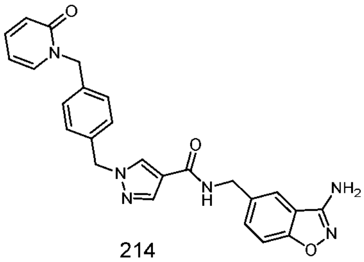
214 ,

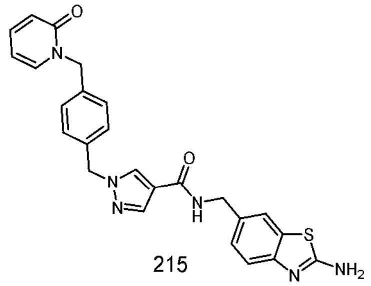
215,

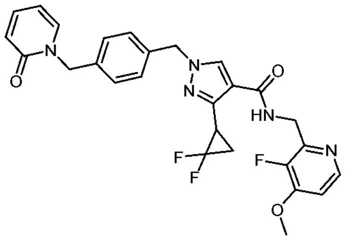
216,

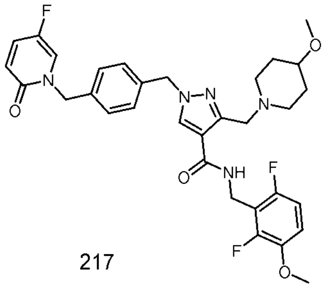
217,

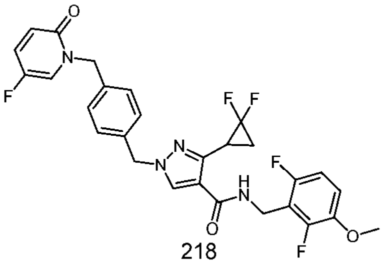
218,

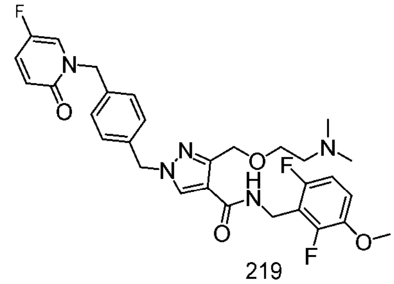
219,

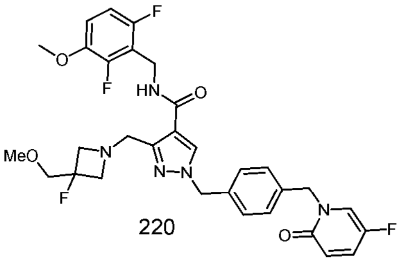
220,

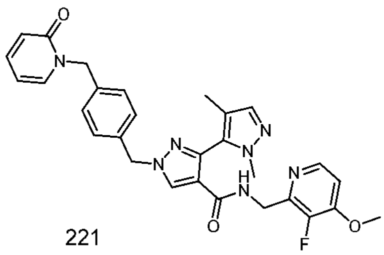
221,

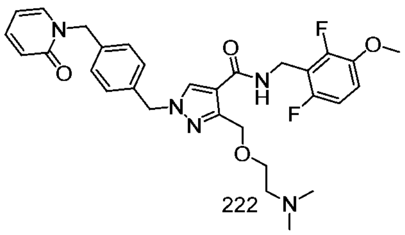
222,

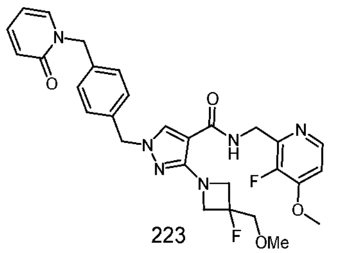
223,

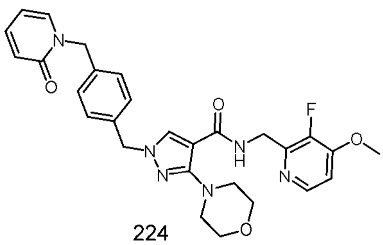
224,

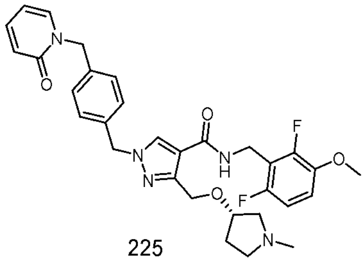
225,

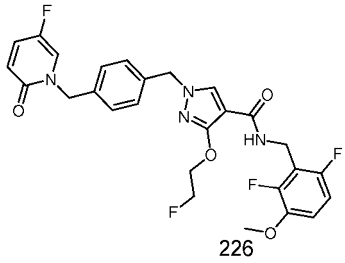
226,

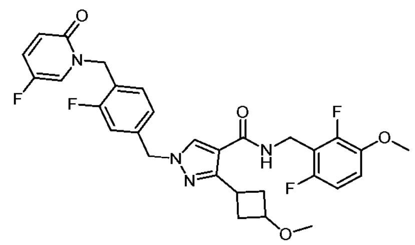

227,

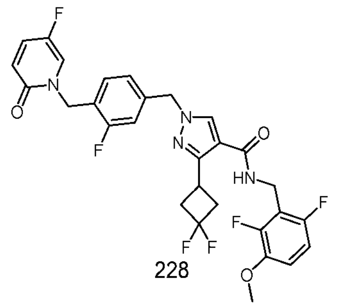

228,

225

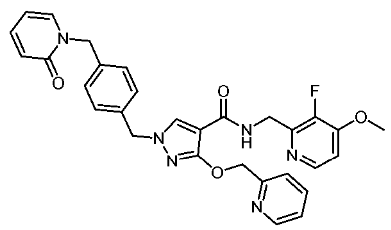

229,

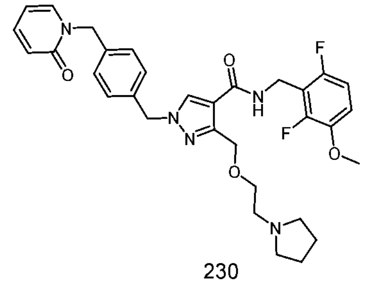

230,

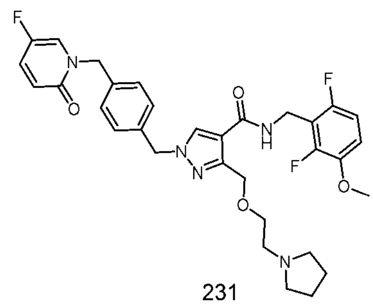

231,

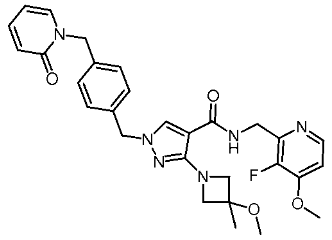

232,

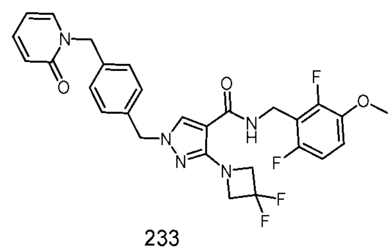

233,

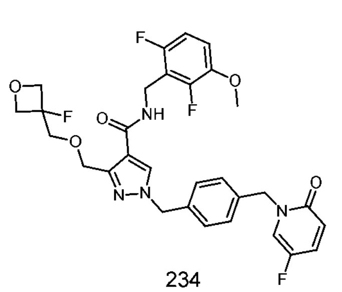

234,

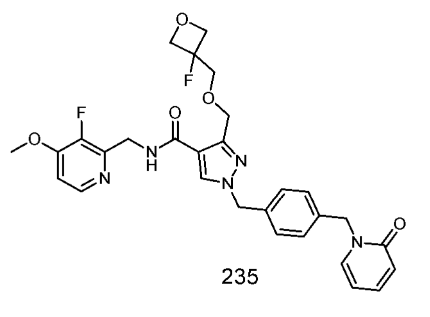

235,

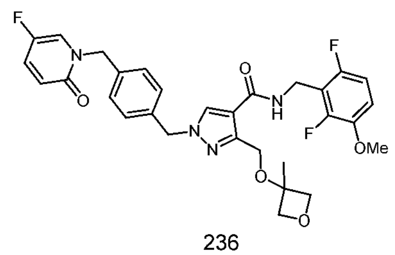

236,

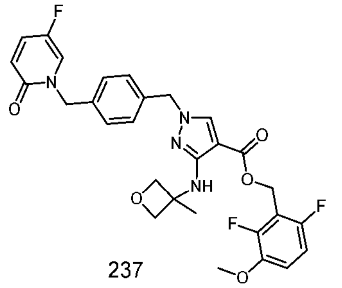

237,

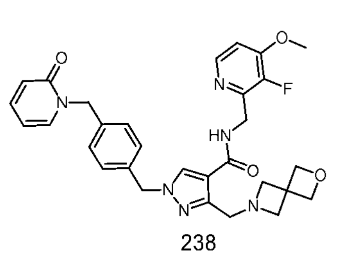

238,

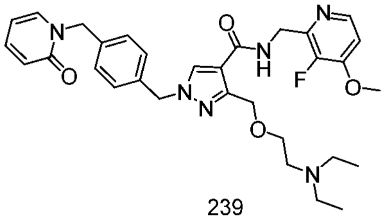
239 ,

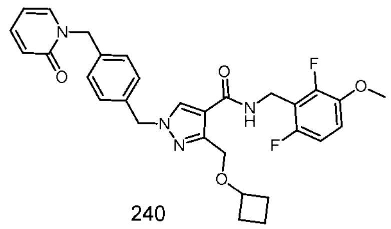
240 ,

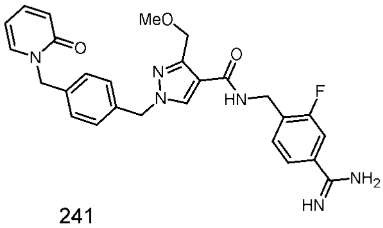
241 ,

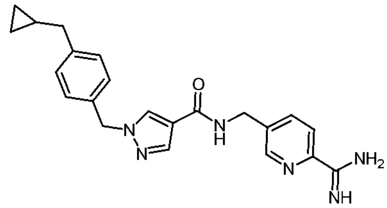
242 ,

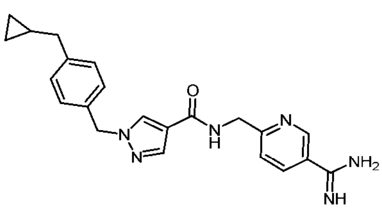
243 ,

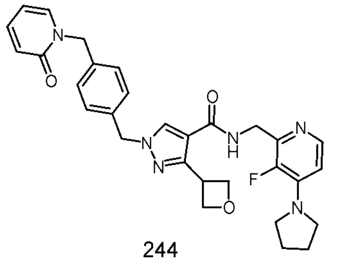
244 ,

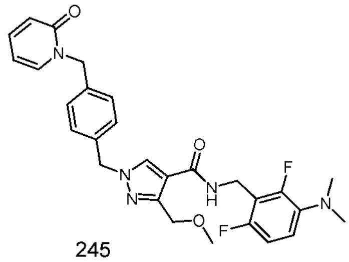
245 ,

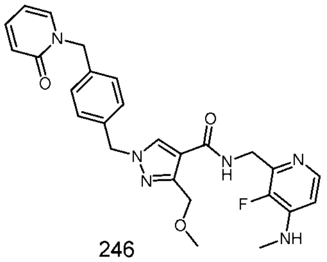
246 ,

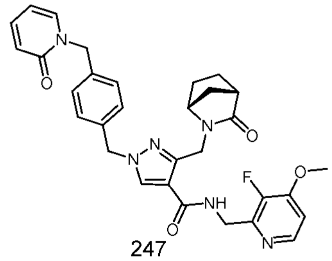
247 ,

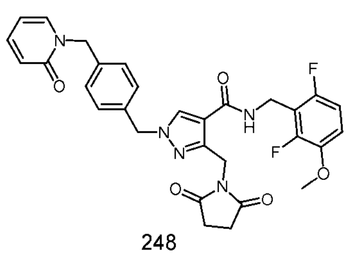
248 ,

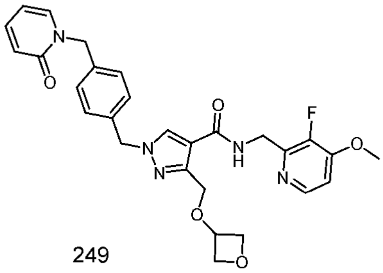
249 ,

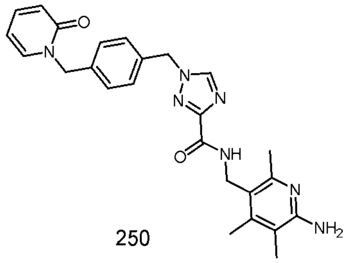
250 ,

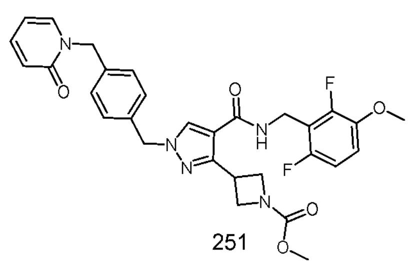
251,

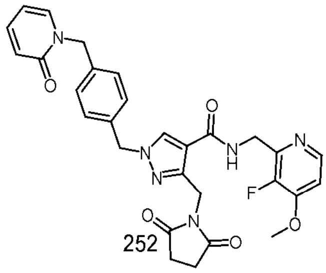
252,

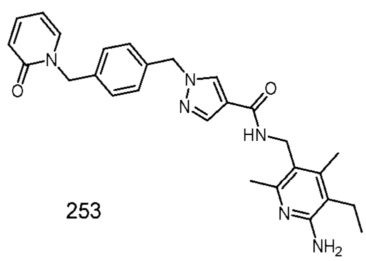
253,

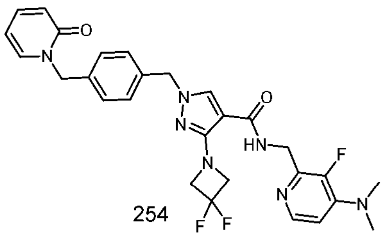
254,

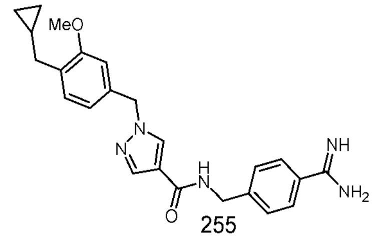
255,

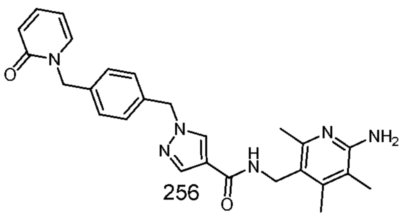
256,

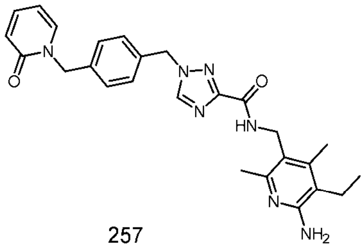
257,

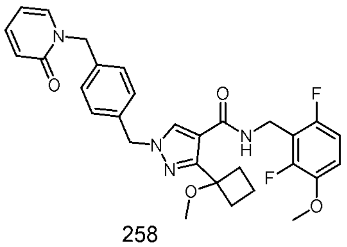
258,

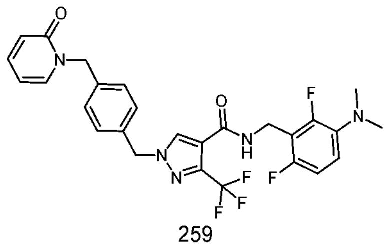
259,

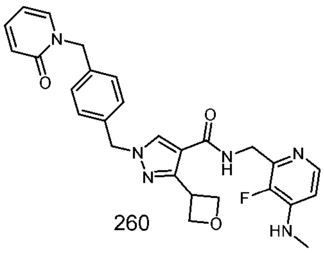
260,

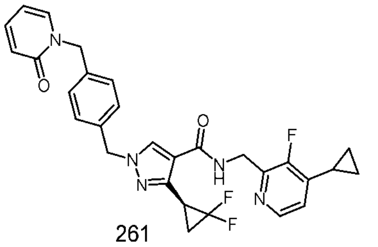
261,

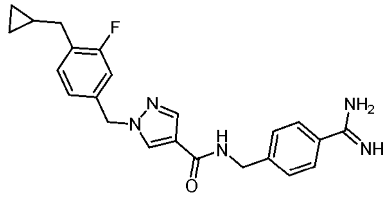
262,

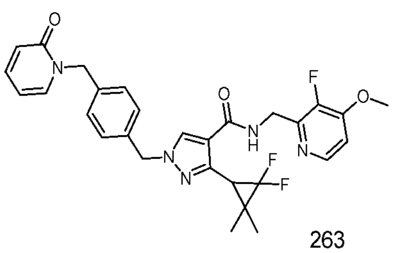
263,

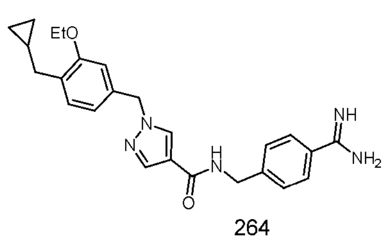
264,

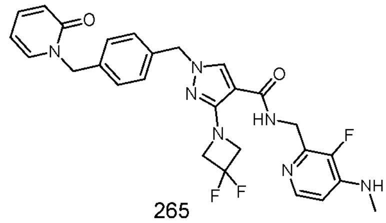
265
,
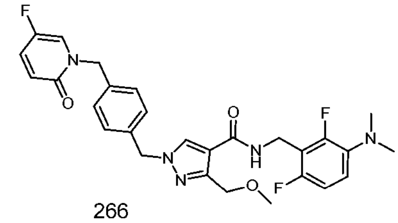
266
,
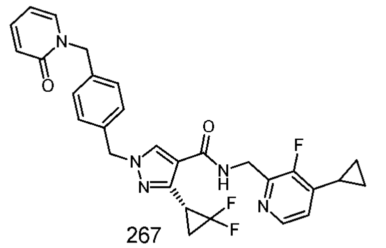
267
,
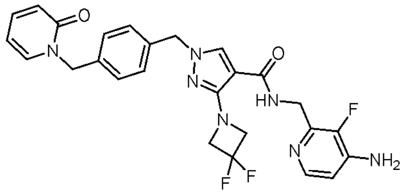
268
,
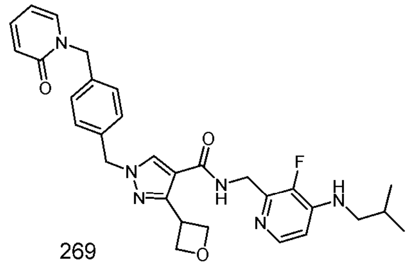
269
,
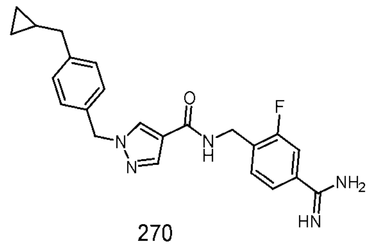
270
,
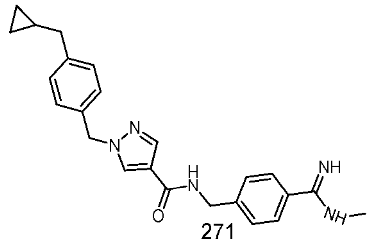
271
,
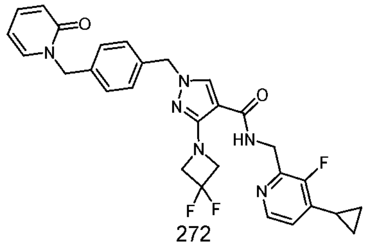
272
,
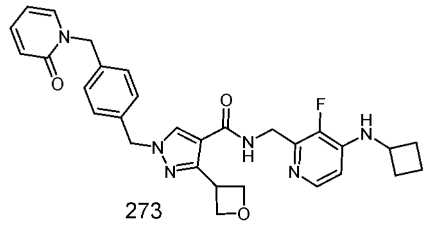
273
,
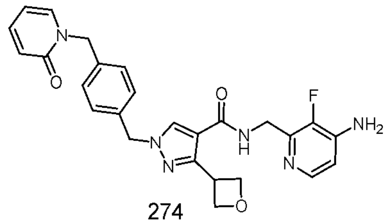
274
,
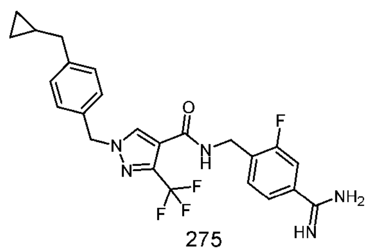
275
,
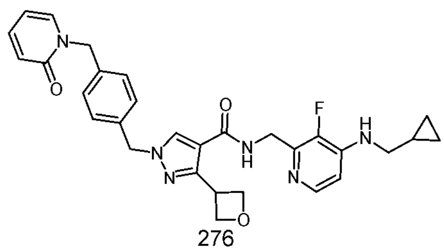
276
,

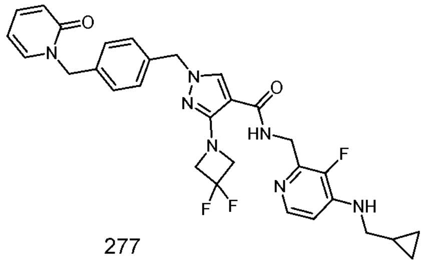
277
,

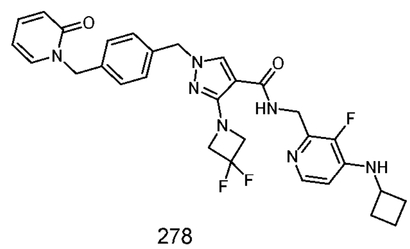
278
,

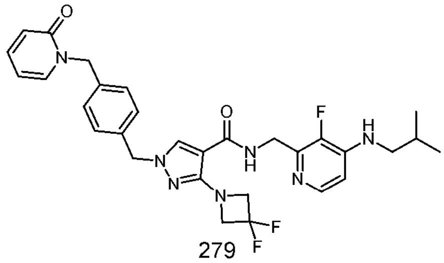
279
,

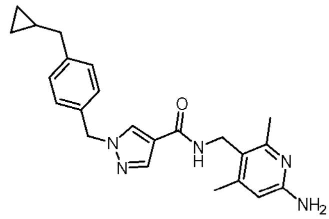
280
,

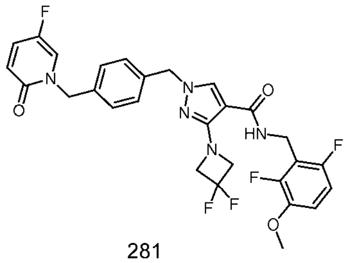
281
,

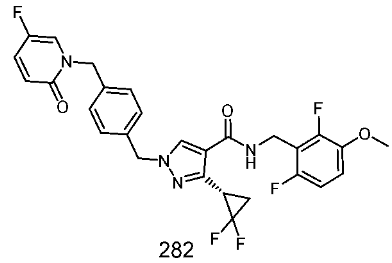
282
,

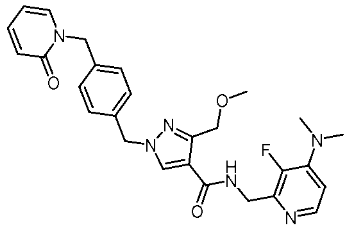
283
,

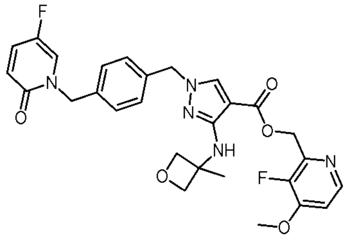
284
,

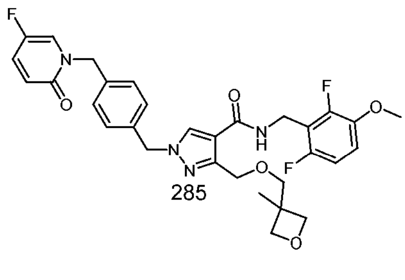
285
,

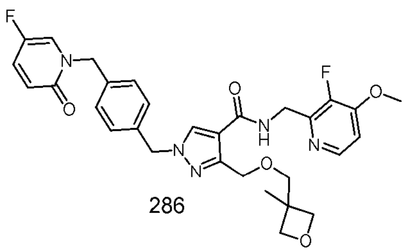
286
,

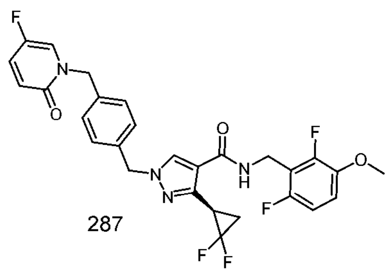
287
,

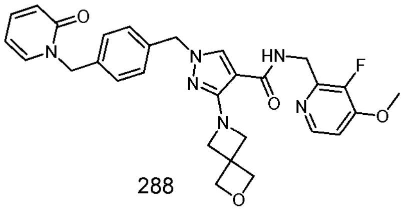
288
,

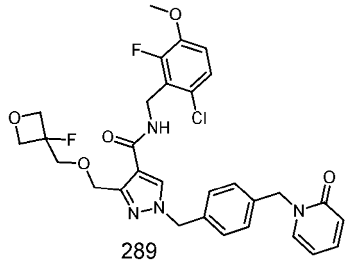
289
,

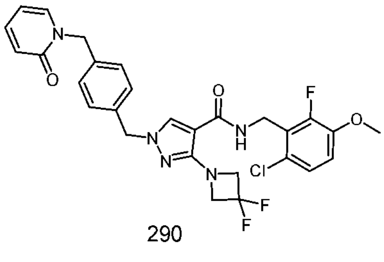
290
,

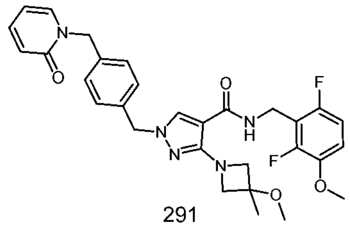
291
,

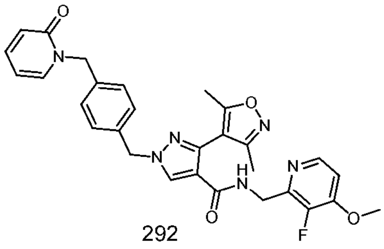
292
,

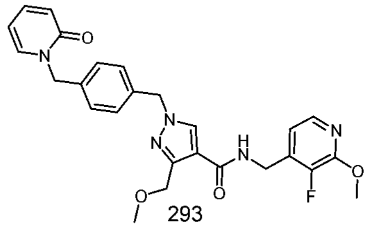
293
,

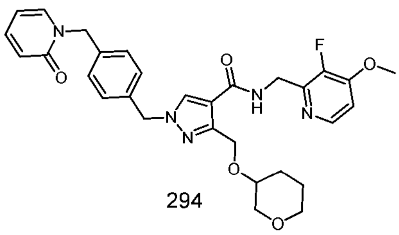
294
,

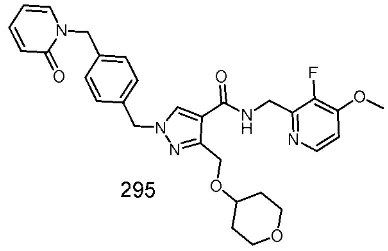
295
,

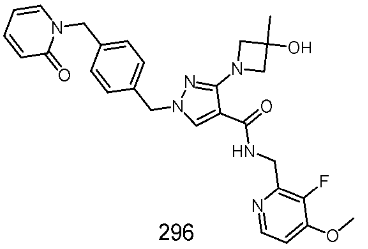
296
,

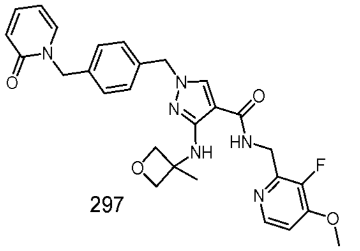
297
,

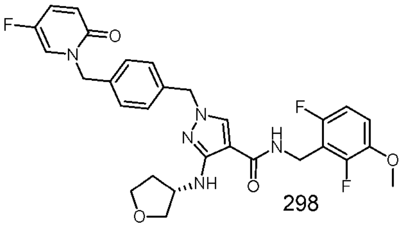
298
,

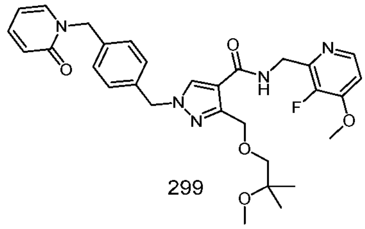
299
,

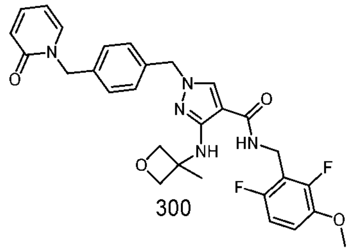
300 ,

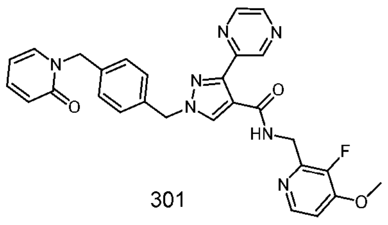
301 ,

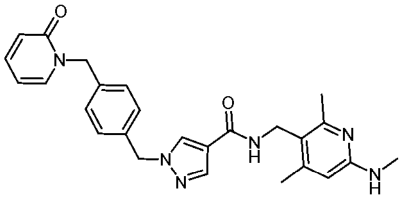
302 ,

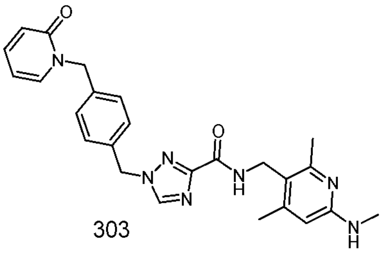
303 ,

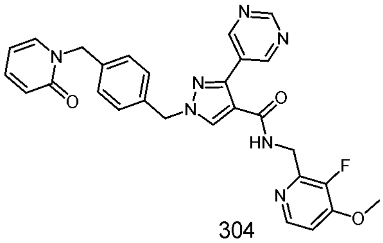
304 ,

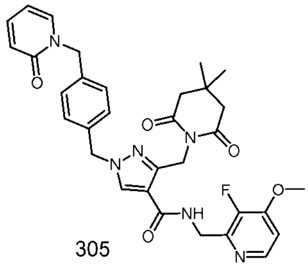
305 ,

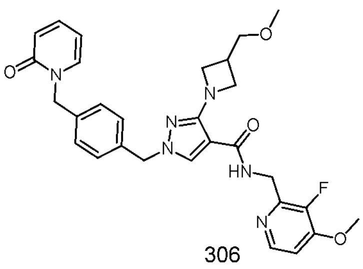
306 ,

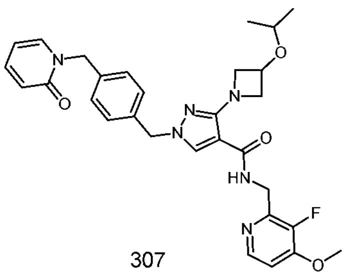
307 ,

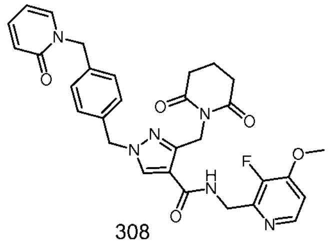
308 ,

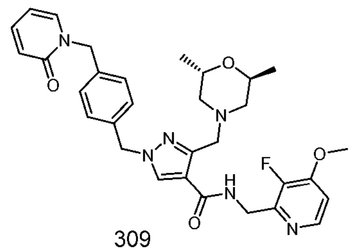
309 ,

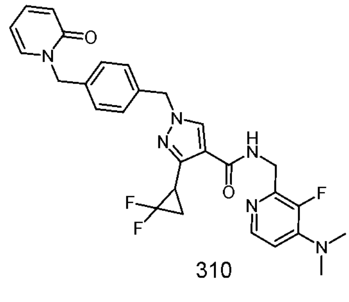
310 ,

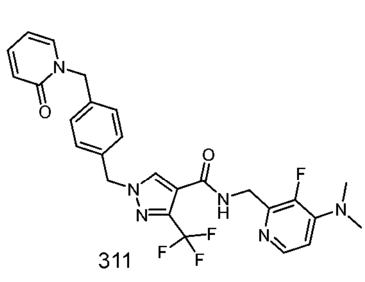
311 ,

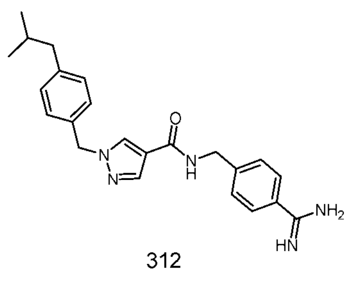
312 ,

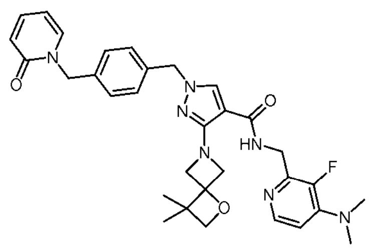
313,

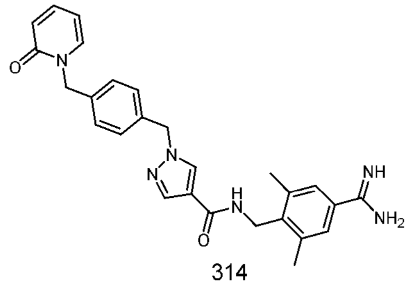
314,

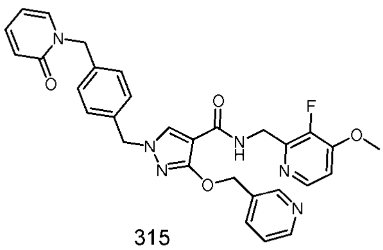
315,

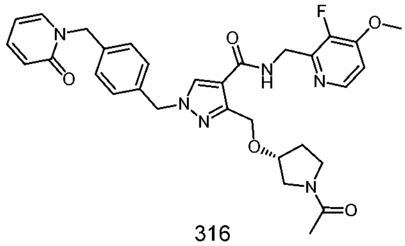
316,

313

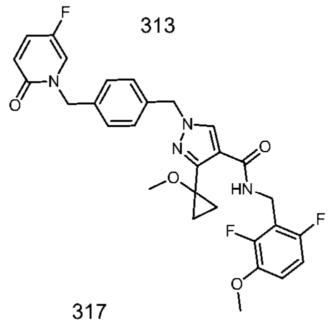
317,

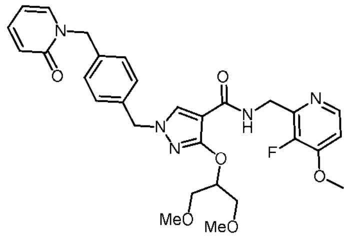
318,

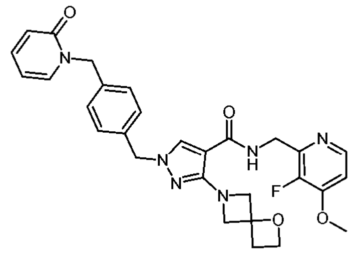
319,

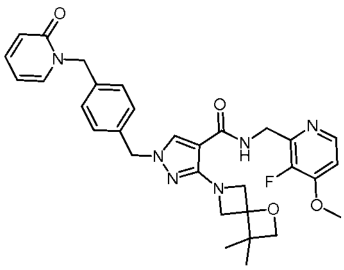
320,

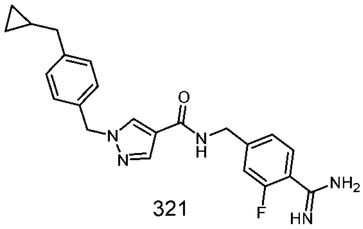
321,

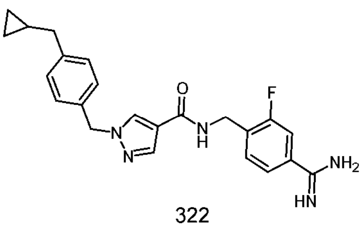
322,

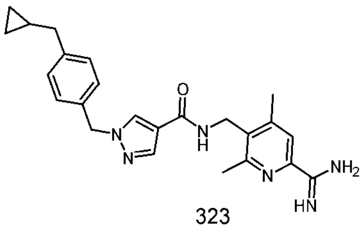
323,

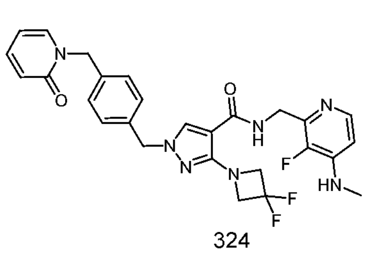
324,

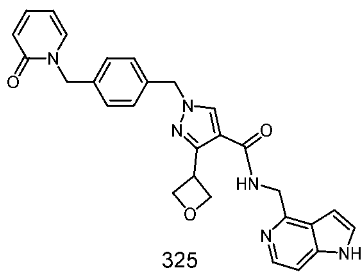
325,

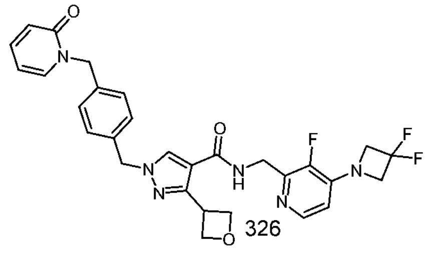
326,

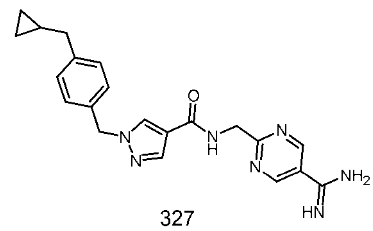
327,

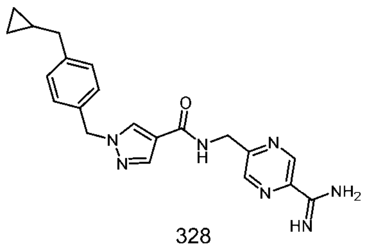
328,

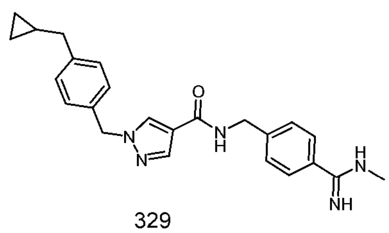
329,

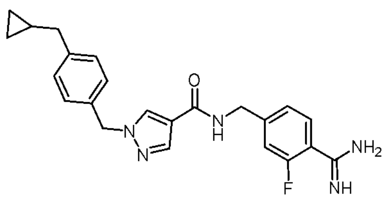
330,

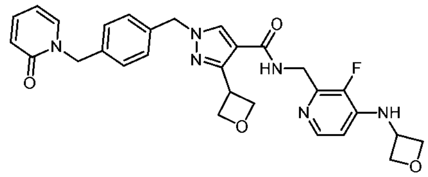
331,

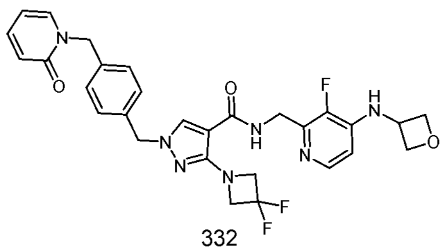
332,

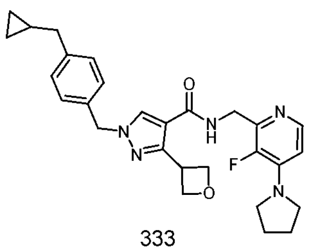
333,

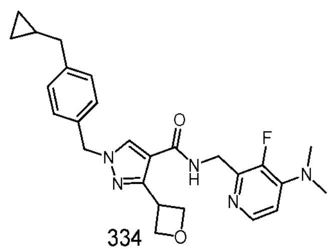
334,

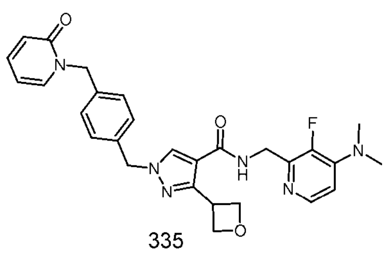
335,

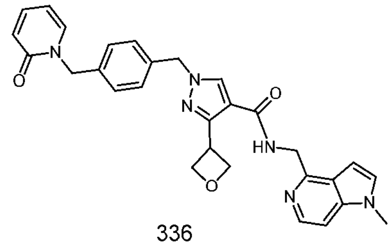
336,

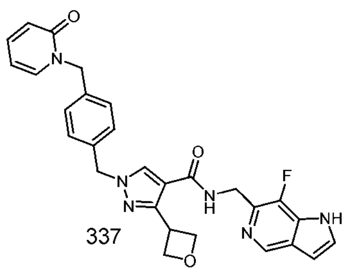
337,

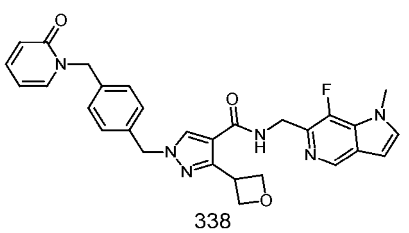
338,

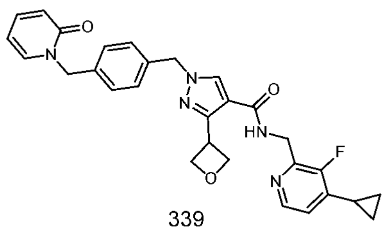
339 ,

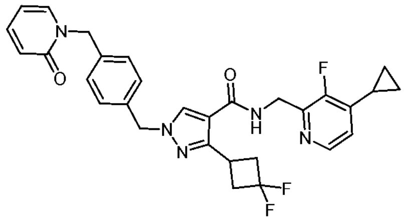
340 ,

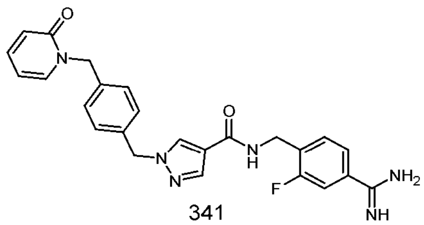
341 ,

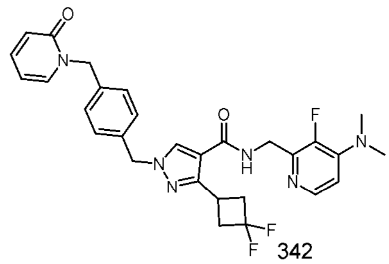
342 ,

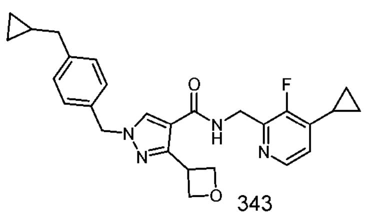
343 ,

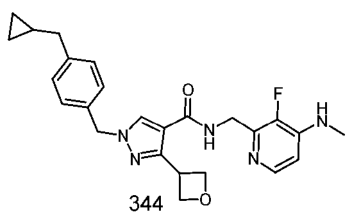
344 ,

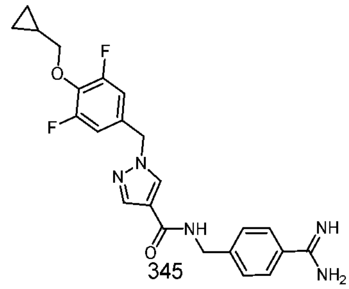
345 ,

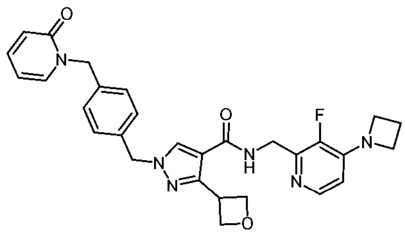
346 ,

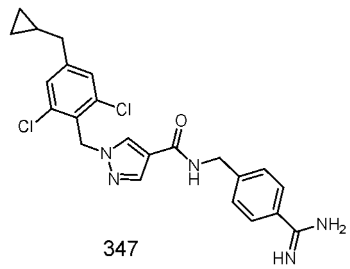
347 ,

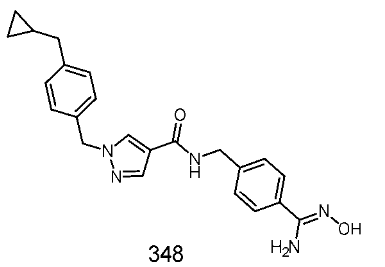
348 ,

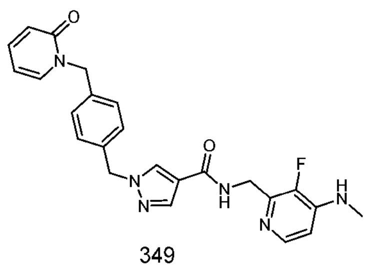
349 ,

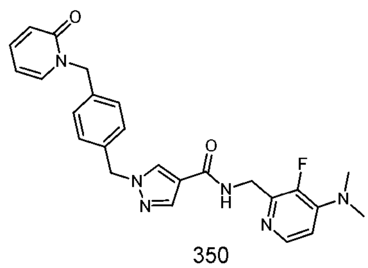
350 ,

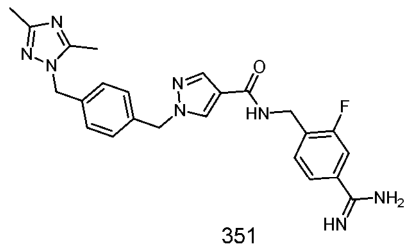

351 ,

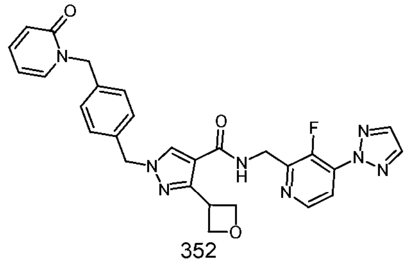

352 ,

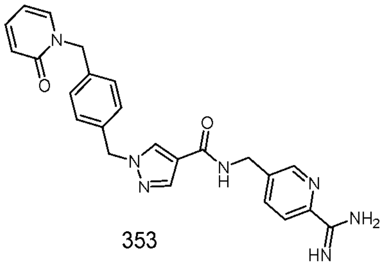

353 ,

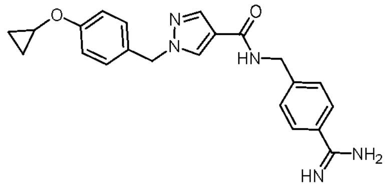

354 ,

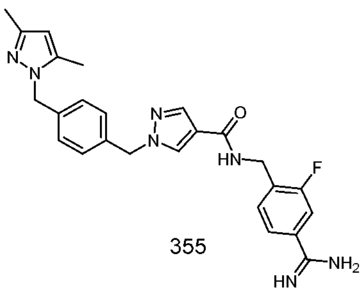

355 ,

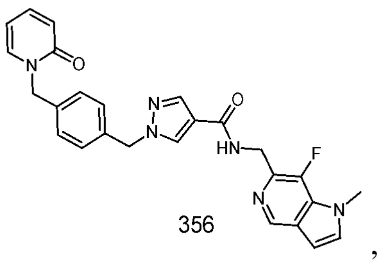

356 ,

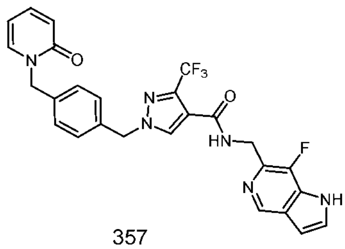

357 ,

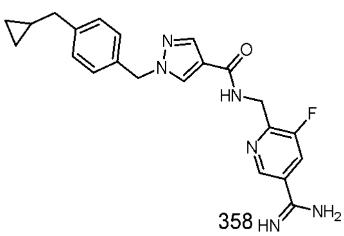

358 ,

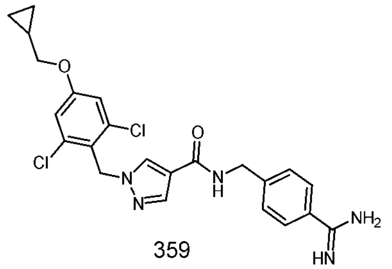

359 ,

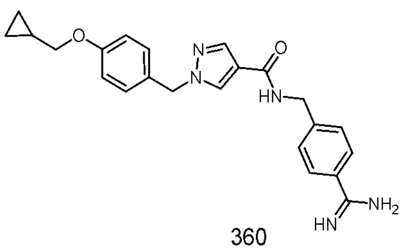

360 ,

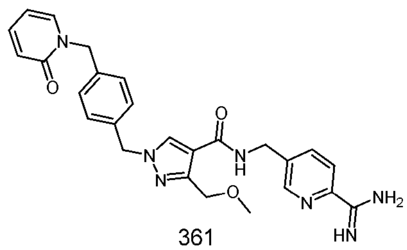

361 ,

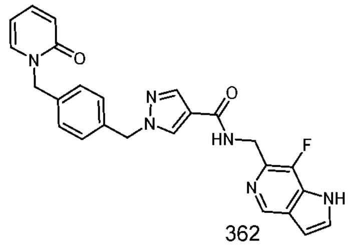
362 ,

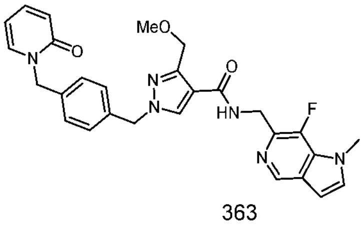
363 ,

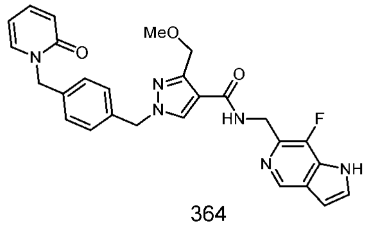
364 ,

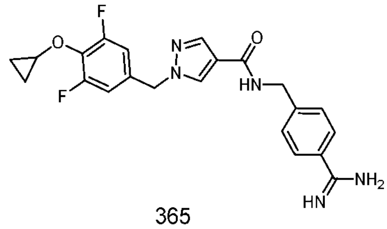
365 ,

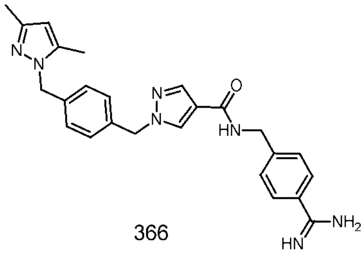
366 ,

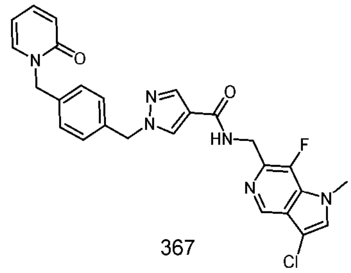
367 ,

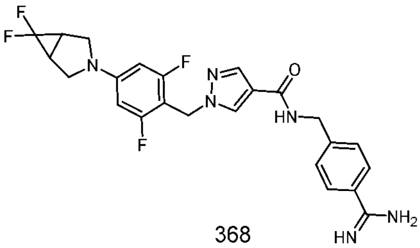
368 ,

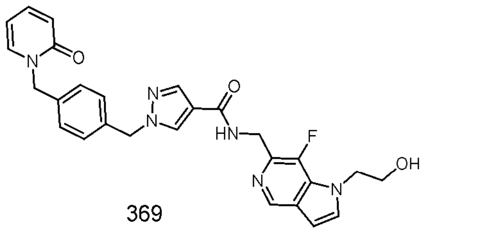
369 ,

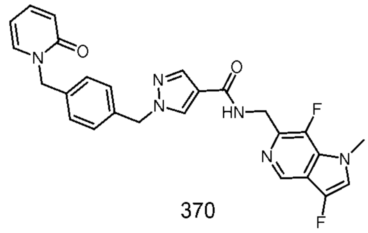
370 ,

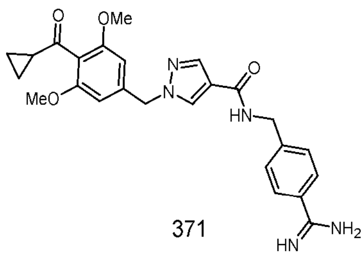
371 ,

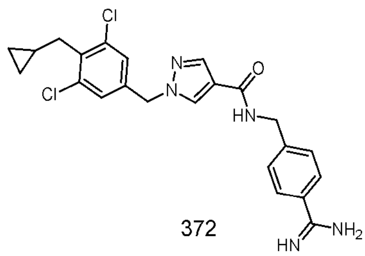
372 ,

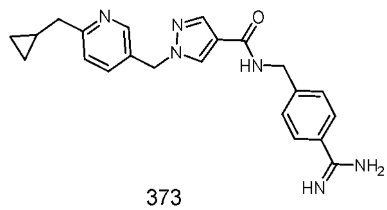
373 ,

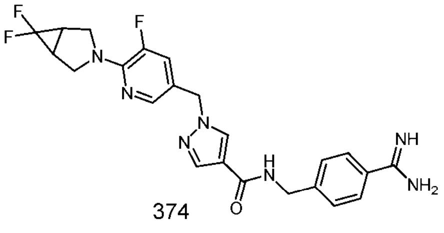
374 ,

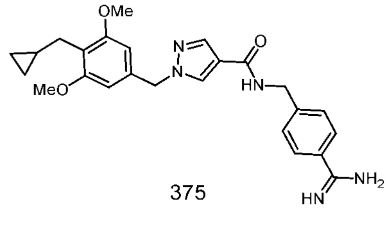
375 ,

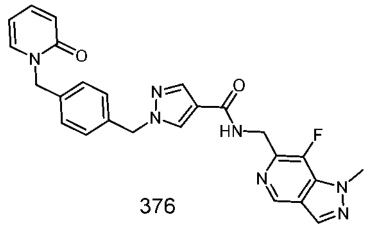
376 ,

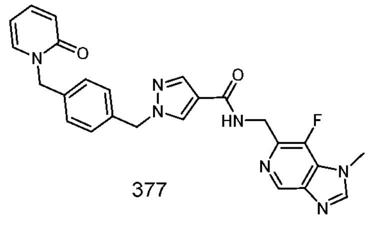
377
,

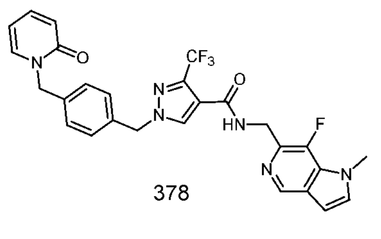
378
,

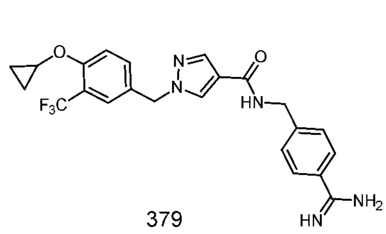
379
,

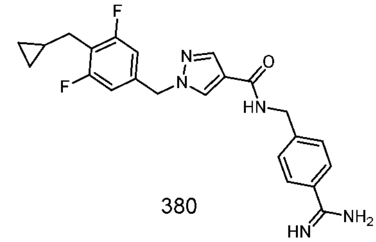
380
,

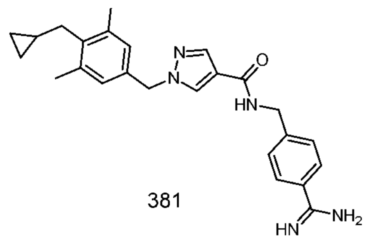
381
,

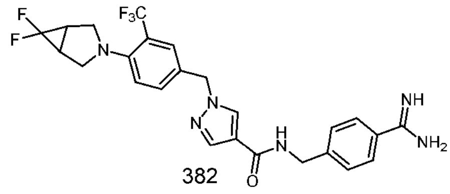
382
,

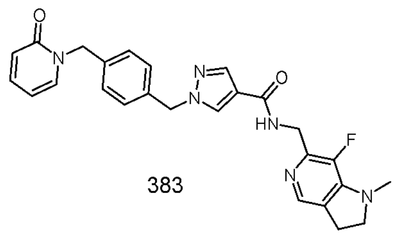
383
,

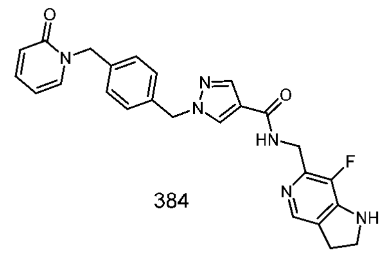
384
,

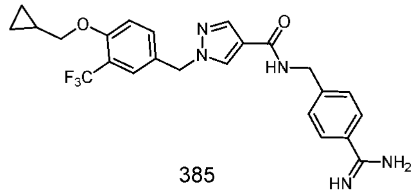
385
,

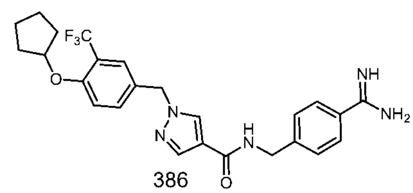
386
,

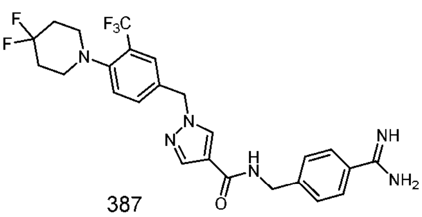
387
,

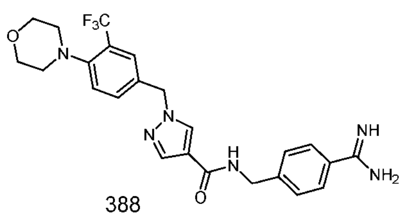
388
,

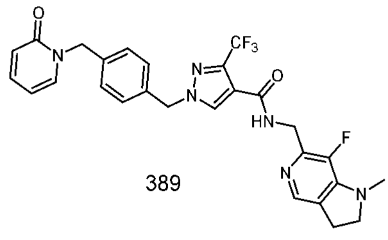
389

,

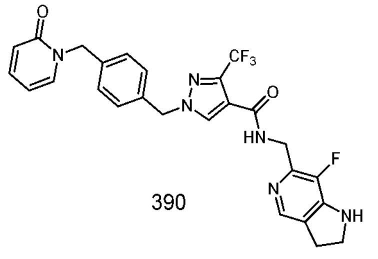
390

,

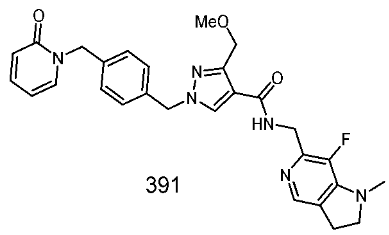
391

,

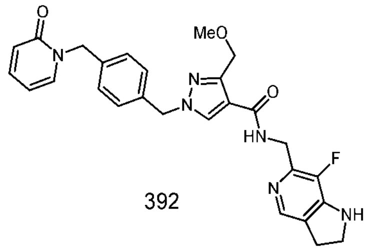
392

,

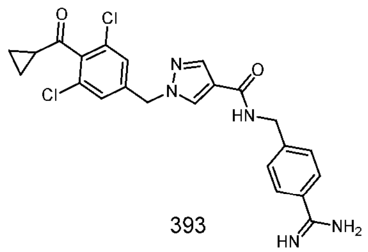
393

,

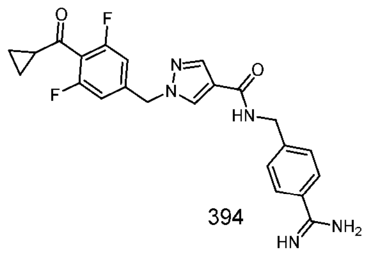
394

,

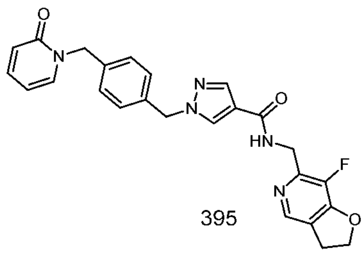
395

and

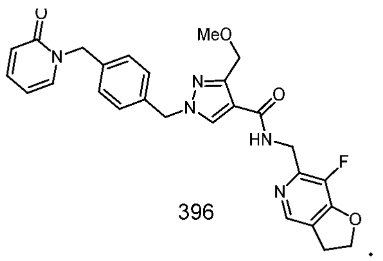
396

.

The present invention also provides a process for preparing a compound represented by the general formula (I), comprising:

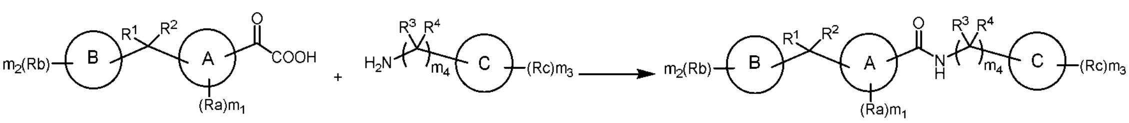
(IA) (IB) (I)

The compound represented by the general formula (IA) and the compound represented by the general formula (IB) can be subjected to amidation reaction in the presence of a condensing agent such as HATU, TCFH, DCC, CDI, BEP, PyBOP or DMTMM to obtain the compound represented by the general formula (I);

wherein,

ring A, ring B, ring C, $R^1$-$R^4$, Ra, Rb, Rc, $m_1$, $m_2$, $m_3$ and $m_4$ are as defined in the general formula (I).

The present invention also provides a process for preparing a compound represented by the general formula (II) or a pharmaceutically acceptable salt thereof, which comprises:

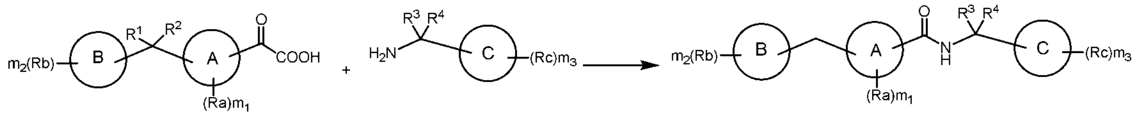

(IA) (IIB) (II)

The compound represented by the general formula (IA) and the compound represented by the general formula (IIB) can be subjected to amidation reaction in the presence of a condensing agent such as HATU, TCFH, DCC, CDI, BEP, PyBOP or DMTMM to obtain the compound represented by the general formula (II);
wherein,
ring A, ring B, ring C, $R^1$-$R^4$, Ra, Rb, Rc, $m_1$, $m_2$ and $m_3$ are as defined in the general formula (I).

The present invention also provides a process for preparing a compound represented by the general formula (III) or a pharmaceutically acceptable salt thereof, which comprises:

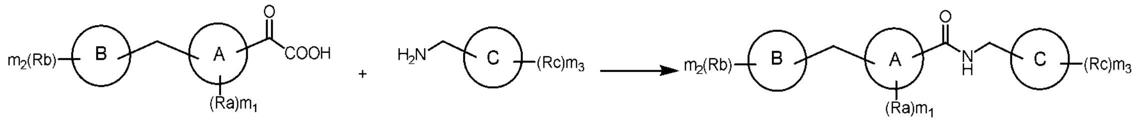

(IIIA) (IIIB) (III)

The compound represented by the general formula (IIIA) and the compound represented by the general formula (IIIB) can be subjected to amidation reaction in the presence of a condensing agent such as HATU, TCFH, DCC, CDI, BEP, PyBOP or DMTMM to obtain the compound represented by the general formula (III);
wherein,
ring A, ring B, ring C, Ra, Rb, Rc, $m_1$, $m_2$ and $m_3$ are as defined in the general formula (I).

The present invention also provides a process for preparing a compound represented by the general formula (IV) or a pharmaceutically acceptable salt thereof, which comprises:

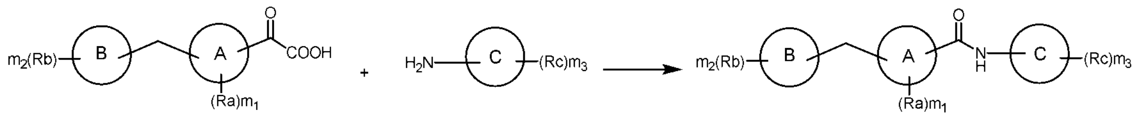

(IIIA) (IVB) (IV)

The compound represented by the general formula (IIIA) and the compound represented by the general formula (IVB) can be subjected to amidation reaction in the presence of a condensing agent such as HATU, TCFH, DCC, CDI, BEP, PyBOP or DMTMM to obtain the compound represented by the general formula (IV);
wherein,
ring A, ring B, ring C, Ra, Rb, Rc, $m_1$, $m_2$ and $m_3$ are as defined in the general formula (I).

The above reactions are preferably carried out in a solvent including, but not limit to, N,N-dimethylformamide, dichloromethane; alkaline reagent used includes, but are not limit to, pyridine, triethylamine, and N,N-diisopropylethylamine.

The invention also provides a pharmaceutical composition containing the compound represented by the general formula (I), (II), (III) or (IV) and a pharmaceutically acceptable carrier. The pharmaceutical composition contains a therapeutically effective amount of a compound represented by the general formula (I), (II), (III) or (IV), or a tautomer, a mesomer, a racemate, an enantiomer, a diastereoisomer, or a mixture thereof, a prodrug or a pharmaceutically acceptable salt thereof, and one or more pharmaceutically acceptable carriers, diluents, or excipients.

The invention also relates to use of the compound represented by the general formula (I), (II), (III) or (IV) or a pharmaceutically acceptable salt thereof or a pharmaceutical composition containing the same for the manufacture of a medicament for treating an ocular microangiopathy-associated disorder.

The invention also relates to the compound represented by the general formula (I), (II), (III) or (IV) or a pharmaceutically acceptable salt thereof or a pharmaceutical composition containing the same, which is used as a medicine.

The invention also relates to the compound represented by the general formula (I), (II), (III) or (IV) or a pharmaceutically acceptable salt thereof or a pharmaceutical composition containing the same, which is used for treating an ocular microangiopathy-associated disorder.

The present invention also relates to a method for treating an ocular microangiopathy-associated disorder comprising administering to a patient in need thereof a therapeutically effective amount of the compound represented by

the general formula (I), (II), (III) or (IV), or a pharmaceutically acceptable salt thereof, or a pharmaceutical composition comprising the same.

In some embodiments of the present invention, the ocular microangiopathy-associated disorder is selected from the group consisting of diabetic retinopathy (DR), diabetic macular edema (DME), retinal vein occlusion (RVO), central retinal vein occlusion (CRVO), macular degeneration, retinopathy of prematurity (ROP), neovascular glaucoma, retinitis pigmentosa (RP), proliferative and non-proliferative retinopathy, retinal angiomatous hyperplasia, macular telangiectasia, ischemic retinopathy, iris neovascularization, intraocular neovascularization, corneal neovascularization, retinal neovascularization, polypoidal choroidal vasculopathy (PCV), choroidal neovascularization, retinal degeneration, diabetic complications of retinal vascular permeability associated with diabetic retinopathy and diabetic macular edema; in particular selected from the group consisting of diabetic retinopathy (DR), diabetic macular edema (DME), retinal vein occlusion (RVO), central retinal vein occlusion (CRVO) and wet age-related macular degeneration (wet AMD).

The compounds represented by the general formulae (I) to (IV) can be converted into the corresponding salts by well-known methods. The salt is preferably a water soluble pharmaceutically acceptable non-toxic acid addition salt, and the exemple thereof is a salt of an amino group with an inorganic acid such as hydrochloric acid, hydrobromic acid, phosphoric acid, sulfuric acid, and perchloric acid, or with an organic acid such as acetic acid, oxalic acid, maleic acid, tartaric acid, citric acid, succinic acid, or malonic acid, or a salt formed by using other methods known in the art, such as an ion exchange method. Other pharmaceutically acceptable salts include adipate, alginate, ascorbate, aspartate, benzenesulfonate, tosylate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, caproate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectate, persulfate, 3-phenylpropionate, phosphate, picrate, pivalate, propionate, stearate, succinate, sulfate, tartrate, thiocyanate, p-toluenesulfonate, undecanoate, valerate, and the like. In particular, the salt is selected from the group consisting of tosylate, methanesulfonate, hydrochloride, acetate and citrate.

The compound of the present invention or a pharmaceutically acceptable salt thereof may be administered in a pure form or in an appropriate pharmaceutical composition by any acceptable mode of administration with similar effects. The pharmaceutical composition of the present invention can be prepared by combining the compound of the present invention with appropriate pharmaceutically acceptable carriers, diluents or excipients, and can be formulated into solid, semi-solid, liquid or gaseous form preparations such as tablets, capsules, powders, granules, nanoparticles, suspensions, emulsions, gels, liposomes, solutions, injections. Typical routes of administration of the pharmaceutical composition include, but are not limited to, oral or parenteral administration. As used herein, the term parenteral includes subcutaneous, intravenous, intramuscular, intraocular, or infusion technique. The pharmaceutical composition of the present invention is formulated to allow the active ingredients contained therein to be bioavailable upon administration of the composition to a patient. A composition to be administered to an individual or patient may be in the form of one or more dosage units, wherein, for example, a tablet may be a single dosage unit and a container containing the compound of the invention in aerosol form may hold multiple dosage units. The actual methods of preparing such dosage forms are or will be known to those of skill in the art. The composition to be administered will in any case contain a therapeutically effective amount of the compound of the present invention or a pharmaceutically acceptable salt thereof to treat the disease or condition of interest in accordance with the teachings of the present invention.

The pharmaceutical composition of the present invention may be in solid or liquid form. In one aspect, the carrier is a particulate such that the composition is, for example, in the form of a tablet or powder. The carrier may be a liquid and the composition may be, for example, an oral syrup, an injectable liquid. When intended for oral administration, the pharmaceutical composition is preferably in solid or liquid form, with semi-solid, semi-liquid, suspension, and gel forms included among the forms considered herein as solid or liquid. For oral solid compositions, the pharmaceutical composition may be formulated in the form of powders, granules, compressed tablets, pills, capsules, chewable tablets, powder tablets, and the like. Such solid compositions typically contain one or more inert diluents or edible carriers. Additionally, one or more of the following may be present: binder such as carboxymethylcellulose, ethylcellulose, microcrystalline cellulose, xanthan gum or gelatin; excipient such as starch, lactose or dextrin; disintegrant such as alginic acid, sodium alginate, Primogel, corn starch, and the like; lubricant, such as magnesium stearate or hydrogenated vegetable oil (Sterotex); glidant, such as colloidal silicon dioxide; sweetener, such as sucrose or saccharin; flavoring agent, such as peppermint, methyl salicylate or orange flavoring agents; and colorant.

When the pharmaceutical composition is in the form of a capsule, such as a gelatin capsule, it may contain a liquid carrier, such as polyethylene glycol or vegetable oil, in addition to the types of materials described above. The pharmaceutical composition may be in liquid form, such as a tincture, syrup, solution, emulsion, or suspension. This liquid can be taken orally, or delivered by injection, as two examples. When intended for oral administration, the preferred composition contains, in addition to the compound of the present invention, one or more of a sweetener, a preservative, a dye/coloring agent, and a flavor enhancer. In compositions intended for administration by injection, one or more of surfactants, preservatives, wetting agents, dispersing agents, suspending agents, buffering agents, stabilizing agents,

and isotonic agents may be included.

The compound and pharmaceutical composition of the present invention may be further combined with one or more, preferably one, additional therapeutic agent. The compound of the present invention may be combined with one or more additional therapeutic agents selected from the group consisting of anti-VEGF monoclonal or diabody, anti-diabetic agents, agents useful in the treatment of overweight and/or obesity; agents for the treatment of hypertension, pulmonary hypertension, heart failure and/or atherosclerosis and agents for the treatment of ocular disorders.

Additional agents for the treatment of type II diabetes which may be combined with the compound of the present invention are one or more selected from the group consisting of GK agonists, GLP-1 receptor agonists, exenatide, liraglutide, somaglutide, dulaglutide, loxenatide, abilutide; alpha-glucosidase inhibitor, voglibose, acarbose; SGLT-2 inhibitors, Canagliflozin, Dapagliflozin, Empagliflozin, Ipragliflozin, Luseogliflozin and Tofogliflozin; DPP-4 inhibitors, sitagliptin, vildagliptin, saxagliptin, alogliptin, linagliptin, gemigliptin, teneligliptin and metformin. Pharmaceutical compositions thereof are useful for preventing, slowing the progression of, or treating one or more metabolic disorders selected from the group consisting of type I diabetes, type II diabetes, impaired glucose tolerance, hyperglycemia, postprandial hyperglycemia, impaired fasting glucose, overweight, obesity, hypertension, insulin resistance, and/or metabolic syndrome; or improve glycemic control and/or reduce fasting plasma glucose; or prevent, slow, delay, or reverse diabetic complications.

Additional agents for the treatment of hypertension which may be combined with the compound of the present invention are selected from the group consisting of angiotensin II receptor blockers (ARB), angiotensin converting enzyme inhibitors (ACEI), calcium channel blocker (CCB), beta receptor blockers, diuretics, alpha receptor blockers, etc., in particular, felodipine sustained-release tablets, nifedipine, nitrendipine, nisoldipine, nicardipine, lacidipine, lercanidipine, amlodipine, levamlodipine, verapamil, diltiazem, captopril, enalapril, benazepril, lisilopril, ramipril, fosinopril, cilazapril, perindopril, quinapril, imidapril, zofenopril, losartan, Valsartan, irbesartan, irbesartan, candesartan medoxomil, telmisartan, olmesartan medoxomil, etc.

Additional agents for the treatment of heart failure and/or atherosclerosis which may be combined with the compound of the present invention are selected from the group consisting of mevastatin, lovastatin, pravastatin, simvastatin, fluvastatin, cholestyramine, colestipol, aspirin, clopidogrel, cilostazol, omega-3 multivalent unsaturate fatty acids, omega-6 monovalent unsaturate fatty acids, heparin, heparan sulfate, chondroitin sulfate, dextran sulfate, fenelizone, proprotein convertase subtilisin 9 (PCSK9) type inhibitor ilotuzumab (Rebion, Evolocumab), Alirocumab, Inclisiran (Leqvio), etc.

Additional agents for the treatment of pulmonary hypertension which may be combined with the compound of the present invention are selected from the group consisting of bosentan, liocicept, maxitentan, selepag, etc.

Additional agents for the treatment of ocular disease which may be combined with the compound of the present invention include intravitreally administered corticosteroids, intravitreally administered anti-VEGF therapeutics, anti-Ang2 inhibitors, dual anti-VEGF/anti-Ang2 inhibitors, supplemental inhibitors (supplemental factor 3, 5, B and D inhibitors, etc.), anti-PDGF, dual anti-VEGF/anti-PDGF, VAP-1 inhibitors, bradykinin receptor 1 (BK1) antagonists, Tie-2 agonists, CCR-2 antagonists, etc.

Additional anti-VEGF monoclonal antibody or double antibody agents which may be combined with the compound of the present invention are selected from the group consisting of aflibercept (ziv-aflibercept, Eylea), Bevacizumab (Avastin), Ranibizumab (Lucentis) and Pegaptanib (Macugen), Faricimab (Vabysmo).

In some embodiments of the present invention, the stroke includes hemorrhagic and acute ischemic stroke, and additional agents which may be combined with the compound of the present invention for the treatment of stroke are selected from the group consisting of alteplase, cetiplase, edaravone, butylphthalide, citicoline, or dextroborneol, etc.

Description of terms

Unless stated to the contrary, terms used in the description and claims have the following meanings.

The term "alkyl" refers to a saturated linear or branched aliphatic hydrocarbon group having from 1 to 20 (e.g., 1, 2, 3, 4, 5, 6, 7, 8, 9, 10, 11, 12, 13, 14, 15, 16, 17, 18, 19, or 20) carbon atoms (i.e., $C_{1-20}$alkyl). The alkyl group is preferably an alkyl group having 1 to 12 carbon atoms (i.e., $C_{1-12}$alkyl group), and more preferably an alkyl group having 1 to 6 carbon atoms (i.e., $C_{1-6}$alkyl group). Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2, 3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof.

The term "alkylene" refers to a divalent alkyl group, wherein the alkyl group is as defined above, having from 1 to 20 (e.g., 1, 2, 3, 4, 5, 6, 7, 8, 9, 10, 11, 12, 13, 14, 15, 16, 17, 18, 19, or 20) carbon atoms (i.e., $C_{1-20}$alkylene). The alkylene group is preferably an alkylene group having 1 to 12 carbon atoms (i.e., $C_{1-12}$alkylene group), and more preferably an alkylene group having 1 to 6 carbon atoms (i.e., $C_{1-6}$alkylene group). Non-limiting examples include: $-CH_2-$, $-CH(CH_3)-$, $-C(CH_3)_2-$, $-CH_2CH_2-$, $-CH(CH_2CH_3)-$, $-CH_2CH(CH_3)-$, $-CH_2C(CH_3)_2-$, $-CH_2CH_2CH_2-$, $-CH_2CH_2CH_2CH_2-$, etc.

The term "alkenyl" refers to an alkyl group containing at least one carbon-carbon double bond in the molecule, wherein the alkyl group is as defined above and has from 2 to 12 (e.g., 2, 3, 4, 5, 6, 7, 8, 9, 10, 11, or 12) carbon atoms (i.e., the $C_{2-12}$alkenyl group). The alkenyl group is preferably an alkenyl group having 2 to 6 carbon atoms (i.e., $C_{2-6}$alkenyl group). Non-limiting examples include vinyl, propenyl, isopropenyl, butenyl, and the like.

The term "alkynyl" refers to an alkyl group containing at least one carbon-carbon triple bond in the molecule, wherein the alkyl group is as defined above and has from 2 to 12 (e.g., 2, 3, 4, 5, 6, 7, 8, 9, 10, 11, or 12) carbon atoms (i.e., $C_{2-12}$alkynyl). The alkynyl group is preferably an alkynyl group having 2 to 6 carbon atoms (i.e., $C_{2-6}$alkynyl). Non-limiting examples include: ethynyl, propynyl, butynyl, pentynyl, hexynyl, and the like.

The term "alkoxy" refers to -O-(alkyl), wherein alkyl is as defined above. Non-limiting examples include methoxy, ethoxy, propoxy, butoxy, and the like.

The term "cycloalkyl" refers to a saturated or partially unsaturated monocyclic percarbocyclic ring (i.e., monocyclic cycloalkyl) or polycyclic ring system (i.e., polycyclic cycloalkyl), having 3 to 20 (e.g., 3, 4, 5, 6, 7, 8, 9, 10, 11, 12, 13, 14, 15, 16, 17, 18, 19, or 20) ring atoms (i.e., $C_{3-20}$cycloalkyl). The cycloalkyl group is preferably a cycloalkyl group having 3 to 12 ring atoms (i.e., $C_{3-12}$cycloalkyl), more preferably cycloalkyl having 3 to 8 ring atoms (i.e., $C_{3-8}$cycloalkyl), cycloalkyl having 4 to 7 ring atoms (i.e., $C_{4-7}$cycloalkyl), or cycloalkyl having 3 to 6 ring atoms (i.e., $C_{3-6}$cycloalkyl).

Non-limiting examples of monocyclic cycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like.

The polycyclic cycloalkyl comprises spirocycloalkyl, fused cycloalkyl and bridged cycloalkyl.

The term "spirocycloalkyl" refers to a polycyclic system sharing one carbon atom (called the spiro atom) between the rings, which may contain one or more double bonds within the ring, or which may contain one or more heteroatoms selected from nitrogen, oxygen, and sulfur within the ring (the nitrogen may optionally be oxidized, i.e., to form a nitroxide; the sulfur may optionally be oxo-substituted, i.e., to form a sulfoxide or sulfone, but not including -O-O-, -O-S-, or -S-S-), provided that it contains at least one percarbocyclic ring and the point of attachment is on the percarbocyclic ring, having 5 to 20 (e.g., 5, 6, 7, 8, 9, 10, 11, 12, 13, 14, 15, 16, 17, 18, 19, or 20) ring atoms (i.e., 5 to 20 membered spirocycloalkyl). The spirocycloalkyl group is preferably a spirocycloalkyl group having 6 to 14 ring atoms (i.e., 6 to 14 membered spirocycloalkyl group), and more preferably a spirocycloalkyl group having 7 to 10 ring atoms (i.e., 7 to 10 membered spirocycloalkyl group). The spirocycloalkyl group includes a monospirocycloalkyl group and a multispirocycloalkyl group (such as a bispirocycloalkyl group, etc.), preferably a monospirocycloalkyl group or a bispirocycloalkyl group, more preferably 3 membered/4 membered, 3 membered/5 membered, 3 membered/6 membered, 4 membered/4 membered, 4 membered/5 membered, 4 membered/6 membered, 5 membered/3 membered, 5 membered/4 membered, 5 membered/5 membered, 5 membered/6 membered, 5 membered/7 membered, 6 membered/3 membered, 6 membered/4 membered, 6 membered/5 membered, 6 membered/6 membered, 6 membered/7 membered, 7 membered/5 membered or 7 membered/6 membered monospirocycloalkyl group. Non-limiting examples include:

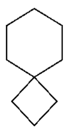 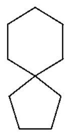 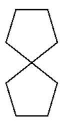 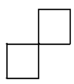 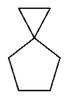 .

The term "fused cycloalkyl" refers to a polycyclic system in which two adjacent carbon atoms are shared between the rings, a monocyclic cycloalkyl group fused to one or more monocyclic cycloalkyl groups, or a monocyclic cycloalkyl group fused to one or more of heterocyclyl, aryl, or heteroaryl groups, where the point of attachment is on the monocyclic cycloalkyl group, which may contain one or more double bonds within the ring, and have 5 to 20 (e.g., 5, 6, 7, 8, 9, 10, 11, 12, 13, 14, 15, 16, 17, 18, 19, or 20) ring atoms (i.e., 5 to 20 membered fused cycloalkyl). The fused cycloalkyl group is preferably a fused cycloalkyl group having 6 to 14 ring atoms (i.e., a 6 to 14 membered fused cycloalkyl group), and more preferably a fused cycloalkyl group having 7 to 10 ring atoms (i.e., a 7 to 10 membered fused cycloalkyl group). The fused cycloalkyl group includes a bicyclic fused cycloalkyl group and a polycyclic fused cycloalkyl group (such as a tricyclic fused cycloalkyl group, a tetracyclic fused cycloalkyl group, etc.), preferably a bicyclic condensed cycloalkyl group or a tricyclic condensed cycloalkyl group, more preferably 3 membered/4 membered, 3 membered/5 membered, 3 membered/6 membered, 4 membered/4 membered, 4 membered/5 membered, 4 membered/6 membered, 5 membered/3 membered, 5 membered/4 membered, 5 membered/5 membered, 5 membered/6 membered, 5 membered/7 membered, 6 membered/3 membered, 6 membered/4 membered, 6 membered/5 membered, 6 membered/6 membered, 6 membered/7

membered, 7 membered/5 membered or 7 membered/6 membered bicyclic condensed cycloalkyl group. Non-limiting examples include:

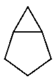 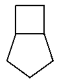 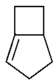 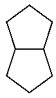 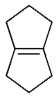 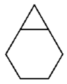 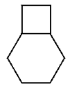 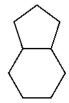 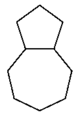.

The term "bridged cycloalkyl" refers to a percarbocyclic polycyclic system that shares two carbon atoms not directly attached between the rings, may contain one or more double bonds within the ring, and has from 5 to 20 (e.g., 5, 6, 7, 8, 9, 10, 11, 12, 13, 14, 15, 16, 17, 18, 19, or 20) carbon atoms (i.e., a 5 to 20 membered bridged cycloalkyl group). The bridged cycloalkyl group is preferably an bridged cycloalkyl group having 6 to 14 carbon atoms (i.e., a 6 to 14 membered bridged cycloalkyl group), and more preferably a bridged cycloalkyl group having 7 to 10 carbon atoms (i.e., a 7 to 10 membered bridged cycloalkyl group). The bridged cycloalkyl group includes a bicyclic bridged cycloalkyl group and a polycyclic bridged cycloalkyl group (e.g., a tricyclic bridged cycloalkyl group, a tetracyclic bridged cycloalkyl group, etc.), and is preferably a bicyclic bridged cycloalkyl group or a tricyclic bridged cycloalkyl group. Non-limiting examples include:

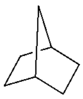 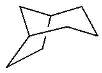 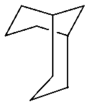 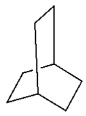 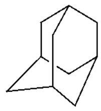.

The term "heterocyclyl" refers to a saturated or partially unsaturated monocyclic heterocycle (i.e., monocyclic heterocyclyl) or polycyclic heterocycle system (i.e., polycyclic heterocyclyl) containing within the ring at least one (e.g., 1, 2, 3, or 4) heteroatoms selected from the group consisting of nitrogen, oxygen, and sulfur (the nitrogen may optionally be oxidized, i.e., to form a nitroxide; the sulfur may optionally be oxo-substituted, i.e., to form a sulfoxide or sulfone, but not including -O-O-, -O-S-, or -S-S-), and having 3 to 20 (e.g., 3, 4, 5, 6, 7, 8, 9, 10, 11, 12, 13, 14, 15, 16, 17, 18, 19, or 20) ring atoms (i.e., a 3 to 20 membered heterocyclyl). The heterocyclyl is preferably a heterocyclyl having 3 to 12 ring atoms (i.e., a 3 to 12 membered heterocyclyl); further preferably a heterocyclyl having 3 to 8 ring atoms (i.e., a 3 to 8 membered heterocyclyl) or a heterocyclyl having 4 to 7 ring atoms (i.e., a 4 to 7 membered heterocyclyl); more preferably a heterocyclyl having 3 to 6 ring atoms (i.e., a 3 to 6 membered heterocyclyl) or preferably a heterocyclyl having 5 or 6 ring atoms (i.e., a 5 or 6 membered heterocyclyl).

Non-limiting examples of said monocyclic heterocyclyl include pyrrolidinyl, tetrahydropyranyl, dihydropyridinyl, tetrahydropyridinyl, piperidinyl, piperazinyl, morpholinyl, thiomorpholinyl, homopiperazinyl, and the like.

The polycyclic heterocyclyl comprises a spiroheterocyclyl, a fused heterocyclyl and a bridged heterocyclyl.

The term "spiroheterocyclyl" refers to a polycyclic heterocyclic ring system in which one atom (referred to as a spiro atom) is shared between the rings, which may contain one or more double bonds within the ring, and which contains at least one (e.g., 1, 2, 3, or 4) heteroatoms within the ring selected from the group consisting of nitrogen, oxygen, and sulfur (the nitrogen may optionally be oxidized, i.e., to form a nitroxide; the sulfur may optionally be oxo-substituted, i.e., to form a sulfoxide or sulfone, but not including -O-O-, -O-S-, or -S-S-), provided that it contains at least one monocyclic heterocyclyl and the point of attachment is on the monocyclic heterocyclyl, having 5 to 20 (e.g., 5, 6, 7, 8, 9, 10, 11, 12, 13, 14, 15, 16, 17, 18, 19, or 20) ring atoms (i.e., 5 to 20 membered spiroheterocyclyl). The spiroheterocyclyl is preferably a spiroheterocyclyl having 6 to 14 ring atoms (i.e., a 6 to 14 membered spiroheterocyclyl), and more preferably a spiroheterocyclyl having 7 to 10 ring atoms (i.e., a 7 to 10 membered spiroheterocyclyl). The spiroheterocyclyl includes a monospiroheterocyclyl and a multispiroheterocyclyl (such as a bispiroheterocyclyl), and is preferably a monospiroheterocyclyl or a bispiroheterocyclyl, and more preferably 3 membered/4 membered, 3 membered/5 membered, 3 membered/6 membered, 4 membered/4 membered, 4 membered/5 membered, 4 membered/6 membered, 5 membered/3 membered, 5 membered/4 membered, 5 membered/5 membered, 5 membered/6 membered, 5 membered/7 membered, 6 membered/3 membered, 6 membered/4 membered, 6 membered/5 membered, 6 membered/6 membered, 6 membered/7 membered, 7 membered/5 membered or 7 membered/6 membered monospiroheterocyclyl. Non-limiting examples include:

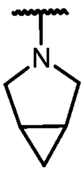 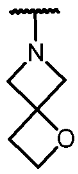 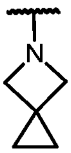 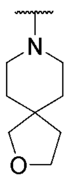 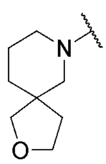 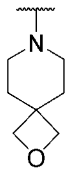 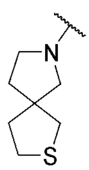 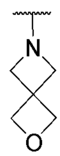 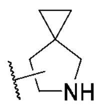,

etc.

The term "fused heterocyclyl" refers to a polycyclic heterocyclic ring system in which adjacent two atoms are shared between the rings, which may contain one or more double bonds within the ring, and which contains at least one (e.g., 1, 2, 3, or 4) heteroatoms selected from nitrogen, oxygen, and sulfur within the ring (the nitrogen may optionally be oxidized, i.e., to form a nitroxide; the sulfur may optionally be oxo-substituted, i.e., to form a sulfoxide or sulfone, but not including -O-O-, -O-S-, or -S-S-), which is a monocyclic heterocyclyl fused to one or more monocyclic heterocyclyl, or a monocyclic heterocyclyl fused to one or more of cycloalkyl, aryl, or heteroaryl, where the point of attachment is on the monocyclic heterocyclyl, and has 5 to 20 (e.g., 5, 6, 7, 8, 9, 10, 11, 12, 13, 14, 15, 16, 17, 18, 19, or 20) ring atoms (i.e., 5 to 20 membered fused heterocyclyl). The fused heterocyclyl is preferably a fused heterocyclyl having 6 to 14 ring atoms (i.e., a 6 to 14 membered fused heterocyclyl), and more preferably a fused heterocyclyl having 7 to 10 ring atoms (i.e., a 7 to 10-membered fused heterocyclyl). The fused heterocyclyl includes a bicyclic and polycyclic fused heterocyclyl (such as a tricyclic fused heterocyclyl, a tetracyclic fused heterocyclyl, etc.), preferably a bicyclic fused heterocyclyl or a tricyclic fused heterocyclyl, and more preferably 3 membered/4 membered, 3 membered/5 membered, 3 membered/6 membered, 4 membered/4 membered, 4 membered/5 membered, 4 membered/6 membered, 5 membered/3 membered, 5 membered/4 membered, 5 membered/5 membered, 5 membered/6 membered, 5 membered/7 membered, 6 membered/3 membered, 6 membered/4 membered, 6 membered/5 membered, 6 membered/6 membered, 6 membered/7 membered, 7 membered/5 membered or 7 membered/6 membered bicyclic fused heterocyclic group. Non-limiting examples include:

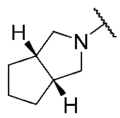 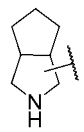 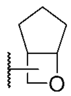 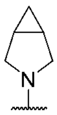 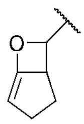 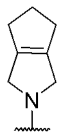 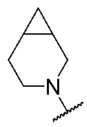

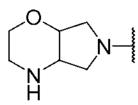 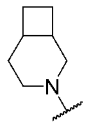 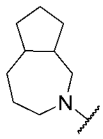 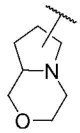 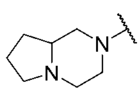 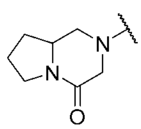

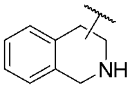 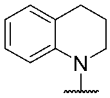 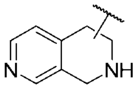 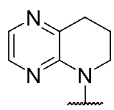 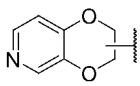 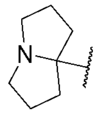 ,

etc.

The term "bridged heterocyclyl" refers to a polycyclic heterocyclic ring system sharing two atoms between the rings which are not directly linked, which may contain one or more double bonds within the ring, and which contains at least one (e.g., 1, 2, 3, or 4) heteroatoms within the ring selected from nitrogen, oxygen, and sulfur (the nitrogen may optionally be oxidized, i.e., to form a nitroxide; the sulfur may optionally be oxo-substituted, i.e., to form a sulfoxide or sulfone, but not including -O-O-, -O-S-, or -S-S-), having 5 to 20 (e.g., 5, 6, 7, 8, 9, 10, 11, 12, 13, 14, 15, 16, 17, 18, 19, or 20) ring atoms (i.e., a 5 to 20 membered bridged heterocyclyl). The bridged heterocyclyl is preferably a bridged heterocyclyl having 6 to 14 ring atoms (i.e., a 6 to 14 membered bridged heterocyclyl), and more preferably a bridged heterocyclyl having 7 to 10 ring atoms (i.e., a 7 to 10 membered bridged heterocyclyl). According to the number of rings, it can be divided into a bicyclic bridged heterocyclyl and a polycyclic bridged heterocyclyl (such as a tricyclic bridged heterocyclyl, a tetracyclic bridged heterocyclyl, etc.), and a bicyclic bridged heterocyclyl or a tricyclic bridged heterocyclyl is preferred. Non-limiting examples include:

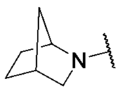 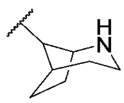 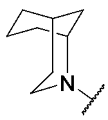 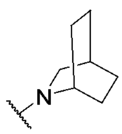 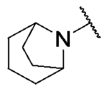 ,

etc.

The term "aryl" refers to a monocyclic percarbocyclic aromatic ring (i.e., a monocyclic aryl group) or a polycyclic aromatic ring system (i.e., a polycyclic aryl group) having 6 to 14 (e.g., 6, 7, 8, 9, 10, 11, 12, 13, or 14) ring atoms (i.e., a 6 to 14 membered aryl group) with a conjugated $\pi$-electron system. The aryl group is preferably an aryl group having 6 to 10 ring

atoms (i.e., a 6 to 10 membered aryl group), more preferably an aryl group having 8 to 10 ring atoms (i.e., an 8 to 10 membered polycyclic aryl group). The monocyclic aryl is, for example, phenyl. Non-limiting examples of said polycyclic aromatic group include naphthyl, anthryl, phenanthryl, and the like.

The term "heteroaryl" refers to a monocyclic heteroaromatic ring (i.e., a monocyclic heteroaryl group) or a polycyclic heteroaromatic ring system (i.e., a polycyclic heteroaryl group) having a conjugated $\pi$-electron system containing within the ring at least one (e.g., 1, 2, 3, or 4) heteroatoms selected from the group consisting of nitrogen, oxygen, and sulfur (the nitrogen may optionally be oxidized, i.e., to form a nitroxide; the sulfur may optionally be oxo-substituted, i.e., to form a sulfoxide or sulfone, but not including -O-O-, -O-S-, or -S-S-) and having 5 to 14 (e.g., 5, 6, 7, 8, 9, 10, 11, 12, 13, or 14) ring atoms (i.e., a 5 to 14 membered heteroaryl group). The heteroaryl group is preferably a heteroaryl group having 5 to 10 ring atoms (i.e., a 5 to 10 membered heteroaryl group), more preferably a heteroaryl group having 5 or 6 ring atoms (i.e., a 5 or 6 membered monocyclic heteroaryl group) or preferably a heteroaryl group having 8 to 10 ring atoms (i.e., a 8 to 10 membered polycyclic heteroaryl group).

Non-limiting examples of the monocyclic heteroaryl include furyl, thienyl, thiazolyl, isothiazolyl, oxazolyl, isoxazolyl, oxadiazolyl, thiadiazolyl, imidazolyl, pyrazolyl, triazolyl, tetrazolyl, furazanyl, pyrrolyl, N-alkylpyrrolyl, pyridyl, pyrimidinyl, pyridonyl, N-alkylpyridone (e.g.,

, 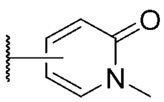 ,

etc.), pyrazinyl, pyridazinyl, etc.

Non-limiting examples of the polycyclic heteroaryl group include indolyl, indazolyl, quinolinyl, isoquinolinyl, quinoxalinyl, phthalazinyl, benzimidazolyl, benzothienyl, benzofuranyl, quinazolinyl, carbazolyl, pyrrolotriazinyl, and the like.

The term "haloalkyl" refers to an alkyl group substituted with one or more halogens, wherein the alkyl group is as defined above.

The term "haloalkoxy" refers to an alkoxy group substituted with one or more halogens, wherein the alkoxy group is as defined above.

The term "hydroxyalkyl" refers to an alkyl group substituted with one or more hydroxyl groups, wherein the alkyl group is as defined above.

The term "halogen" refers to fluorine, chlorine, bromine, or iodine.

The term "hydroxyl" refers to -OH.

The term "mercapto" refers to -SH.

The term "amino" refers to $-NH_2$.

The term "cyano" refers to -CN.

The term "nitro" refers to $-NO_2$.

The term "oxo" refers to "=O".

The term "carbonyl" refers to C=O.

The term "carboxyl" refers to -C(O)OH.

The compound of the present invention may exist in particular stereoisomers forms. The term "stereoisomer" refers to isomers that are identical in structure but differ in the arrangement of atoms in space. It includes cis and trans (or Z and E) isomers, (-)- and (+)-isomers, (R)- and (S)-enantiomers, diastereomers, (D)- and (L)-isomers, tautomers, atropisomers, conformers, and mixtures thereof (e.g., racemates, mixtures of diastereomers). Additional asymmetric atoms may be present in the substituents in the compound of the invention. All of these stereoisomers as well as mixtures thereof are included within the scope of the present invention. Optically active (-)- and (+)-isomers, (R)- and (S)-enantiomers, and (D)- and (L)-isomers can be prepared by chiral synthesis, chiral reagents, or other conventional techniques. An isomer of a certain compound of the present invention can be prepared by asymmetric synthesis or chiral auxiliary, or when the molecule contains a basic functional group (such as an amino group) or an acidic functional group (such as a carboxyl group), forming a salt of diastereomer with a suitable optically active acid or base, followed by diastereomeric resolution by conventional methods well known in the art, to form pure isomer. In addition, the separation of enantiomers and diastereomers is usually accomplished by chromatography.

In the chemical structure of the compound of the present invention, the bond "

" 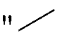 "

represents an unspecified configuration, that is, if a chiral isomer exists in the chemical structure, the bond "

" 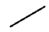 "

" may be"

" 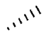 "

" or"

" 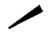 ",

", , or contains "

" 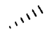 "

"and "

" 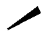 "

" simultaneously.

The compounds of the present disclosure include all suitable isotopic derivatives of the compounds. The term "isotopic derivative" refers to a compound in which at least one atom is replaced by an atom having the same atomic number but a different atomic mass. Examples of isotopes that may be incorporated into the compounds of the present disclosure include stable and radioactive isotopes of hydrogen, carbon, nitrogen, oxygen, phosphorus, sulfur, fluorine, chlorine, bromine, iodine, etc., such as $^{2}H$ (deuterium, D), $^{3}H$ (tritium, T), $^{11}C$, $^{13}C$, $^{14}C$, $^{15}N$, $^{17}O$, $^{18}O$, $^{32}P$, $^{3}3P$, $^{33}S$, $^{34}S$, $^{35}S$, $^{36}S$, $^{18}F$, $^{36}Cl$, $^{82}Br$, $^{123}I$, $^{124}I$, $^{125}I$, $^{129}I$, and $^{131}I$, etc., preferably deuterium. "Optionally" or "optional" means that the subsequently described event or circumstance may, but does not necessarily, occur, including both when the event or circumstance occurs and when it does not. For example, "alkyl optionally substituted with halogen or cyano" includes the case where the alkyl group is substituted with halogen or cyano and the case where the alkyl group is not substituted with halogen or cyano.

"Substituting" or "substituted" mean that one or more hydrogen atoms, preferably from 1 to 6, more preferably from 1 to 3, even more preferably from 1 to 2 hydrogen atoms, in a group are substituted independently by corresponding number of substituents. One skilled in the art is able to determine (experimentally or theoretically) the possible or impossible substitutions without undue effort. For example, an amino or hydroxyl group with a free hydrogen may be unstable when combining with a carbon atom having an unsaturated bond, such as an alkene.

"Pharmaceutical composition" means a composition containing one or more of the compounds described herein or a pharmaceutically acceptable salt thereof in admixture with other chemical components, as well as other components such as pharmaceutically acceptable carriers and excipients. The purpose of the pharmaceutical composition is to promote the administration to the organism, facilitate the absorption of the active ingredient and thus exert the biological activity.

"Pharmaceutically acceptable salt" means a salt of a compound of the invention, which may be selected from the group consisting of an inorganic salt or an organic salt. Such salts are safe and effective when used in mammals, and have the desired biological activity, and may be prepared separately during the final isolation and purification of the compound, or by reacting the appropriate group with a suitable base or acid. Bases commonly used to form pharmaceutically acceptable salts include inorganic bases, such as sodium hydroxide and potassium hydroxide, and organic bases, such as ammonia. Acids commonly used to form pharmaceutically acceptable salts include inorganic acids as well as organic acids.

As used herein, the term "treating" includes treating a disease or disorder in a mammal, especially a human, and includes: (a) inhibiting infection, disease, or disorder, i.e., arresting or delaying the development of infection, disease, or disorder; (b) alleviating infection, disease, or disorder, i.e., causing regression of the disease or disorder, and/or (c) curing infection, disease, or disorder.

As used herein, the term "preventing" includes prophylactic therapy in a mammal, especially a human, to reduce

the likelihood of the occurrence of infection, disease or condition. Patients receiving prophylactic therapy may be selected based on an increased risk of infecting or suffering from a disease or condition as compared to the general population. "Preventing" may include treatment of a subject that has not yet presented with an infection or clinical condition, and prevention of a second occurrence of the same or similar infection or clinical condition.

The term "therapeutically effective amount" for a drug or pharmacologically active agent refers to an amount of the drug or agent that is sufficient to achieve, or at least partially achieve, the desired effect. The determination of a therapeutically effective amount will vary from person to person, depending on the age and general condition of the subject, and depending on the particular active substance, a suitable therapeutically effective amount in an individual case can be determined by one skilled in the art on the basis of routine experimentation.

The term "pharmaceutically acceptable" as used herein means that these compounds, materials, compositions, and/or dosage forms are within the scope of sound medical judgment, suitable for contact with patient tissues without excessive toxicity, irritation, allergic response, or other problems or complications, have a reasonable benefit/risk ratio, and are effective for the intended use.

As used herein, the singular forms "a", "an" and "the" include plural reference, and vice versa, unless the context clearly dictates otherwise.

When the term "about" is applied to a parameter such as pH, concentration, temperature, etc., it indicates that the parameter may vary $\pm$ 10%, and sometimes more preferably within $\pm$ 5%. As will be understood by one skilled in the art, when a parameter is not critical, numbers are generally given for purposes of illustration only and not limitation.

## Detailed description of the invention

The following examples are intended to illustrate but not to limit the present invention, and any modifications, changes, variations, etc., that are within the scope of the present invention are intended to be within the scope of the present invention.

The compounds provided by the present invention may be prepared from readily available starting materials which may be synthesized using or according to methods known in the art, or may be commercially available, using the specific synthetic schemes set forth below. Experimental methods for which specific conditions are not specified in the following examples can be determined by one skilled in the art through routine optimization procedures in accordance with conventional methods and conditions.

Unless otherwise specified, the present invention uses conventional methods such as mass spectrometry and nuclear magnetic resonance to identify compounds, and the steps and conditions can refer to the conventional operation steps and conditions in the art.

In the following examples, the common abbreviations are:

AcOH: Acetic acid, CAS No.: 64-19-7
tBuOK: potassium tert-butoxide, CAS No.: 865-47-4
$Cs_2CO_3$: Cesium carbonate, CAS No.: 534-17-8
BAST: Bis(2-methoxyethyl)aminosulfur trifluoride, CAS No.: 202289-38-1
BEP: 2-Bromo-1-ethylpyridinium tetrafluoroborate, CAS No: 878-23-9
PyBOP: Benzotriazol-1-yl-oxytripyrrolidinophosphonium hexafluorophosphate CAS No.: 128625-52-5
DMTMM: 4-(4,6-Dimethoxytriazin-2-yl)-4-methylmorpholine hydrochloride CAS No.: 3945-69-5
CDI: Carbonyldiimidazole, CAS No.: 208-488-9
CuI: cuprous iodide, CAS No.: 7681-65-4
CuCl: cuprous chloride, CAS No.: 7758-89-6
$CuSO_4$: copper sulfate, CAS No.: 7758-98-7
NaH: sodium hydride, CAS No 7646-69-7
NaOMe: sodium methoxide, CAS No.: 124-41-4
$NaN_3$: sodium azide, CAS No.: 26628-22-8
$NH_4HCO_3$: ammonium bicarbonate, CAS No.: 1066-33-7
$Na_2CO_3$: sodium carbonate, CAS No.: 497-19-8
$K_2CO_3$: potassium carbonate, CAS No.: 584-08-7
$NaBH_4$: sodium borohydride, CAS No.: 16940-66-2
HATU: 2-(7-azabenzotriazole)-N,N,N',N'-tetramethyluronium hexafluorophosphate, CAS No.: 148893-10-1
cataCXium A Pd-G3: [n-Butylbis(1-adamantyl)phosphine](2-amino-1,1'-biphenyl-2-yl)palladium (II) methanesulfonate, CAS: 1651823-59-4
Xantphos: 4,5-bis(diphenylphosphino)-9,9-dimethyloxanthene, CAS No.: 148893-10-1
Dppf Pd G3: (1,1'-Bis(diphenylphosphino)ferrocene)(2'-amino-1,1'-biphenyl-2-yl)palladium methanesulfonate, CAS No.: 1445086-28-1

$Pd(PPh_3)_2Cl_2$: Palladium(II)bis(triphenylphosphine)dichloride, CAS No.: 1445086-28-1
$Pd(dppf)Cl_2$: [1,1'-Bis(diphenylphosphino)ferrocene]palladium dichloride, CAS No.: 72287-26-4
$Pd_2(dba)_3$: Tris(dibenzylideneacetone)dipalladium, CAS No.: 51364-51-3
Pd/C: Palladium-carbon catalyst, CAS No.: 13566-03-5
$Pd(PPh_3)_4$: tetrakis(triphenylphosphine)palladium, CAS No.: 14221-01-3
Pd2$(dba)_3$: Tris(dibenzylideneacetone)dipalladium, CAS No.: 51364-51-3
$Pd(t\text{-}Bu_3P)_2$: bis(tri-tert-butylphosphine)palladium, CAS No.: 53199-31-8
Ruphos: 1'-biphenyl; 2-dicyclohexylphosphino-2',6'-diisopropylbiphenyl; 2-dicyclohexylphosphino-2',6'-diisopropoxy-1,1'-biphenyl, CAS No.: 787618-22-8
RuPhos Palladacycle G3: (2-dicyclohexylphosphino-2',6'-diisopropoxy-1,1'-biphenyl)(2-amino-1,1'-biphenyl-2-yl) palladium(II) methanesulfonate, CAS No.: 1445085-77-7
Xantphos: 4,5-bisdiphenylphosphine-9,9-dimethyloxanthene, CAS No.: 161265-03-8
PE: petroleum ether;
EtOAc: Ethyl acetate, CAS No: 141-78-6
Boc: tert-butoxycarbonyl
DIEA: N,N-diisopropylethylamine, CAS No.: 7087-68-5
DMF: N,N-dimethylformamide, CAS No.: 68-12-2
DMS.$BH_3$: Dimethyl Sulfide Borane, CAS No.: 13292-87-0
DCM: dichloromethane, CAS No.: 75-09-2
Selectfluor: N-fluoro-N'-(chloromethyl)triethylenediamine bis(tetrafluoroborate), CAS No.: 140681-55-6
TCFH, N,N,N',N'-Tetramethylchloroformamidinium-hexafluorophosphate, CAS No.: 94790 -35-9
TFA: trifluoroacetic acid, CAS No.: 76-05-1
THF: Tetrahydrofuran, CAS No.: 109-99-9
THP: 2-Tetrahydropyran
$LiBH_4$: Lithium borohydride, CAS No.: 16949-15-8
L-Selectride: Lithium tri-sec-butylborohydride, CAS No.: 38721-52-7
$Zn(CN)_2$: Zinc cyanide, CAS No: 557-21-1
NMP: N-methylpyrrolidone, CAS No.: 872-50-4
MeOH: Methanol, CAS No: 67-56-1
MeCN: Acetonitrile, CAS No.: 75-05-8
$NaBH_3CN$: Sodium cyanoborohydride, CAS No.: 25895-60-7
NBS: N-Bromosuccinimide, CAS No.: 128-08-5
NCS: N-Chlorosuccinimide, CAS No.: 128-09-6
NBuLi: n-Butyllithium, CAS No.: 109-72-8
SEMCl: 2-(trimethylsilyl)ethoxymethyl chloride, CAS No.: 76513-69-4
TEA: Triethylamine, CAS No.: 121-44-8
$Ti(OEt)_4$: Tetraethyl titanate CAS No.: 3087-36-3
PTsOH: p-toluenesulfonic acid, CAS No.: 104-15-4
$(T\text{-}Bu)_3P{*}HBF_4$: tri-n-butylphosphonium tetrafluoroborate, CAS No.: 113978-91-9
d7-DMF: 7 Deuterium-N,N-dimethylformamide, CAS No.: 4472-41-7
$CDCl_3$: Deuterated chloroform, CAS No.: 865-49-6
d6-DMSO: Deuterated dimethyl sulfoxide, CAS number: 2206-27-1
MS: Mass spectrum
ESI: Electrospray Ionization
$^1$HNMR: Nuclear Magnetic Resonance Hydrogen Spectrum
TLC: Thin Layer Chromatography
Chiral HPLC: Chiral High Performance Liquid Chromatography
Prep-HPLC: Preparative High Pressure Liquid Chromatography
Prep-TLC: Preparative Thin Layer Chromatography
LCMS: Liquid Chromatography-Mass Spectrometry
Rf: Rf value;
min: minute
g: gram
mg: milligram
rt: room temperature
mol: mole
mmol: mmol
mL: milliliter

M: mol/L
Intermediate synthesis

5-(aminomethyl)-4,6-dimethylpyridin-2-amine hydrochloride (Intermediate 1) was prepared according to the following synthetic scheme:

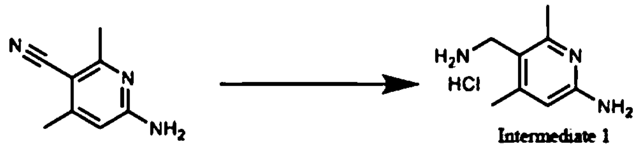

Intermediate 1

To a solution of 5-cyano-4,6-dimethylpyridin-2-amine (50.0 g, 340 mmol, 1.00 eq) in MeOH (100 mL) was added Raney nickel (5.00 g, 58.4 mmol, 1.72 eq) and $NH_3.H_2O$ (114 g, 973.73 mmol, 125.00 mL, 30% purity, 2.87 eq). The mixture was stirred at 80°C under $H_2$ (50 psi) for 20 hour. LC-MS showed that the raw material was exhausted and the product was produced. The mixture was filtered and concentrated in vacuo. The residue was purified by preparative HPLC (column: Phenomenex Luna C18 250*80mm*10um; mobile phase: [water (HCl) -ACN]; gradient: 0% -10% B over 5 min) to give the title compound (Intermediate 1) as a white solid (10.0 g, 53.3 mmol, 15.7% yield, HCl). LCMS(ESI): m/z 152 [M+1]+; $^1$H NMR (400 MHz, DMSO-$d_6$) δ δ 8.54 (br s, 3H), 7.93 (br s, 2H), 6.70 (s, 1H), 3.93 (d, J = 4.2 Hz, 2H), 2.58 (s, 3H), 2.45 (s, 3H).

1-(4-(bromomethyl)benzyl)pyridin-2(1H)-one (Intermediate 2) was prepared according to the following synthetic scheme:

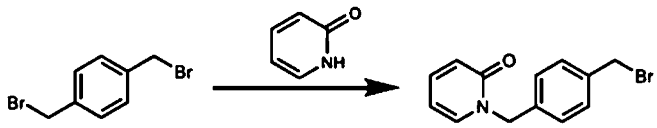

Intermediate 2

To a solution of pyridine-2(1H)-one (25.0 g, 262 mmol, 1.00 eq) in MeCN (500 mL) was added 1,4-bis(bromomethyl)benzene (104.08 g, 394.33 mmol, 1.50 eq) and $K_2CO_3$ (72.7 g, 525 mmol, 2.00 eq). The reaction was stirred at 80°C for 12 h. TLC (plate 1: PE: EtOAc = 1:1, Rf = 0.45) indicated that the starting materials were completely consumed and a major new spot with greater polarity was detected. The reaction mixture was filtered and the filter cake washed with DCM (200 mL). It was then diluted with $H_2O$ (500 mL) and extracted with DCM 1500 mL (500 mL*3). The combined organic layers were washed with brine (500 mL*3), dried over $Na_2SO_4$, filtered and concentrated under reduced pressure to give the crude product. The residue was purified by column chromatography ($SiO_2$, PE/EtOAc = 1/0 to 1/1, plate 2, PE: EtOAc = 1:1, Rf = 0.45). The title compound (Intermediate 2) was obtained as a white solid (8.00 g, 28.8 mmol, 10.9% yield). LCMS(ESI): m/z 278 [M+1]+; $^1$H NMR (400 MHz, $CDCl_3$) δ 7.70-7.69 (d, J = 1.6 Hz, 1H), 7.68-7.53 (m, 1H), 7.40 -7.38 (d, J = 8 Hz, 2H), 7.28-7.26 (d, J = 8 Hz, 2H), 6.59-6.56(d, J = 8.8 Hz, 1H), 6.41-6.38 (m, 1H), 5.19 (s,2H), 4.53 (s, 2H).

(3-fluoro-4-methoxypyridin-2-yl)methylamine hydrochloride (Intermediate 3) was prepared according to the following synthetic scheme:

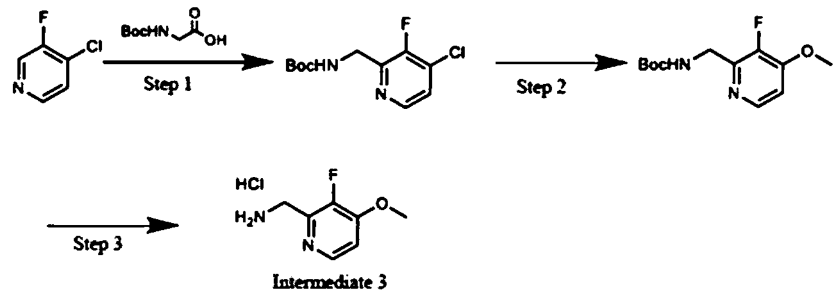


Step 1: Preparation of tert-butyl(4-chloro-3-fluoropyridin-2-yl)methylcarbamate: AgOTf (19.5 g, 76.0 mmol, 0.100 eq) and TFA (17.3 g, 152 mmol, 11.3 mL, 0.200 eq) were added to a solution of 3-fluoro-4-chloropyridine (100 g, 760 mmol, 104 mL, 1.00 eq) and (tert-butoxycarbonyl)glycine (226 g, 1.29 mol, 1.70 eq) in MeCN (1.00 L) and $H_2O$ (250 mL). Then, a solution of $(NH_4)_2S_2O_8$ (312 g, 1.37 mol, 297 mL, 1.80 eq) in $H_2O$ (500 mL) was added dropwise over 0.5 h at 66°C. The mixture was stirred at 66°C for 0.25 h. TLC (PE/EtOAc = 3/1, Rf = 0.420) indicated that the starting material was completely consumed and a new spot with greater polarity were formed. The pH of the reaction mixture was adjusted to 8 by adding aqueous NaOH at 0°C, then diluted with 500 mL $H_2O$ and extracted with EtOAc (1.00 L × 3). The combined organic layers were washed with brine (500 mL × 2), dried over $Na_2SO_4$, filtered and concentrated under reduced pressure to give a residue. The residue was purified by column chromatography (PE: EtOAc = 1: 0-3: 1, PE: EtOAc = 3:1, Rf = 0.420). The title compound (46.0 g, 176 mmol, 23.2% yield) was obtained as a yellow solid. LCMS(ESI): m/z 261 [M+1]+; $^1$H NMR (400 MHz, $CDCl_3$) δ 8.25 (d, J = 5.2 Hz, 1H), 7.29 (t, J = 5.2 Hz, 1H), 5.68 (br s, 1H), 4.54 (br d, J = 2.8 Hz, 2H), 1.46 (s, 9H).

Step 2: Preparation of tert-butyl(4-methoxy-3-fluoropyridin-2-yl)methylcarbamate: To a solution of tert-butyl(4-chloro-3-fluoropyridin-2-yl)methylcarbamate (35.0 g, 134 mmol, 1.00 eq) in MeOH (350 mL) was added NaOMe (5.40 M, 24.7 mL, 1.00 eq) at 0°C. The mixture was stirred at 0°C for 2 hours. The mixture was stirred at 66°C for 34 hours. LC-MS showed about 33% residue of the starting material. Several new peaks were shown on LC-MS, and about 40% of the desired compounds were detected. The reaction mixture was concentrated under reduced pressure to remove MeOH. The residue was diluted with 100 mL $H_2O$ and extracted with EtOAc (200 mL × 3). The combined organic layers were washed with brine (100 mL × 3), dried over $Na_2SO_4$, filtered and concentrated under reduced pressure to give a residue. The residue was purified by column chromatography (PE/EtOAc = 1/0 to 3/1, PE/EtOAc = 3/1, Rf = 0.210). The residue was purified by preparative HPLC (column: Welch Ultimate XB-NH2 250*50*10 um; mobile phase: [hexane-ethanol]; gradient: 1% -30% B over 15 min). The title compound was obtained as a yellow oily liquid (13.5 g, 52.7 mmol, 39.2% yield, 100% purity). LCMS(ESI): m/z 257 [M+1]+.

Step 3: Preparation of (3-fluoro-4-methoxypyridin-2-yl)methylamine hydrochloride (Intermediate 3): To a solution of tert-butyl(4-methoxy-3-fluoropyridin-2-yl)methylcarbamate (13.5 g, 52.7 mmol, 1.00 eq) in MeOH (60.0 mL) was added HCl/MeOH (4 M, 5.00 mL, 3.80 eq) at 0°C. The mixture was stirred at 70°C for 1.8 hours. LC-MS showed that the starting material was completely consumed and a major peak with the desired mass was detected. The reaction mixture was concentrated under reduced pressure to give a residue. The title compound (Intermediate 3) (10.0 g, crude, HCl) was obtained as a pale yellow solid. LCMS(ESI): m/z 157 [M+1]+; $^1$H NMR (400 MHz, DMSO-$d_6$) δ 8.54 (d, J = 6.4 Hz, 1H), 7.56 (t, J = 6.8 Hz, 1H), 4.45 (d, J = 2.40 Hz, 2H), 4.14 (s, 3H).

3-chloro-6-(chloromethyl)quinolone (Intermediate 4) was prepared according to the following synthetic scheme:

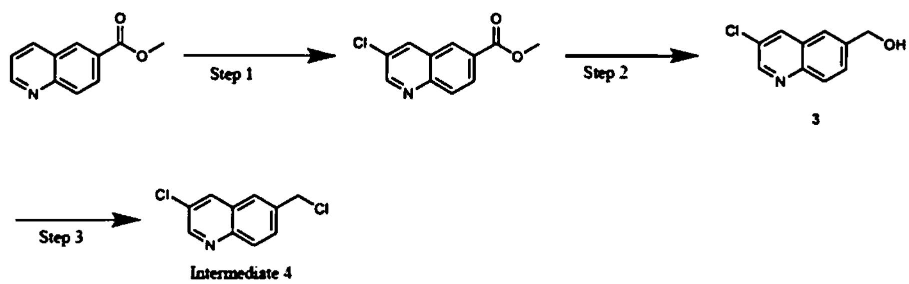


Step 1: Stock solution A: Solution of 6-(methyl formate)quinoline (100 g, 534 mmol, 1.00 eq) in DMF (500 mL). Stock solution B: NCS (214 g, 1.60 mol, 3.00 eq) in DMF (1498 mL). Pump A: 10.30 mL/min, Pump B: 14.54 mL/min,

Temperature = 100°, C1/4 "PFA coil, (100 mL) Residence time: 4 min Flow reaction: 100 g 57 min LCMS showed that the starting material was mostly consumed and the desired MS was detected. The reaction mixture was poured into 2 L $H_2O$ to form some yellow solids, and then filtered and the filter cake was concentrated to give a residue. The residue was slurried with EtOAc (500 mL), filtered, and the filter cake was washed with EtOAc (80.0 mL) to afford the title compound as a yellow solid (58.0 g, 261 mmol, 48.99% yield) LCMS (ESI): m/z 222 [M + 1]+; $^1H$ NMR (400 MHz, $CDCl_3$) δ 9.00 (d, J = 2.4 Hz, 1H), 8.88 (d, J = 2.4 Hz, 1H), 8.67 (d, J = 1.2 Hz, 1H), 8.30 - 8.20 (m, 1H), 8.15 - 8.05 (m, 1H), 3.94 (s, 3H).

Step 2: Stock solution B: NCS (214 g, 1.60 mol, 3.00 eq) in DMF (1498 mL). Pump A: 10.30 mL/min, Pump B: 14.54 mL/min, temperature = 100°C, 1/4 "PFA coil (100 mL), residence time: 4 min, Flow reaction: 100 g 57 min. LCMS showed that the starting material was mostly consumed and the desired MS was detected. The reaction mixture was poured into 2 L $H_2O$ to form some yellow solid, and then filtered and the filter cake was concentrated to give a residue. The residue was slurried with EtOAc (500 mL), filtered, and the filter cake was washed with EtOAc (80.0 mL) to afford the title compound as a yellow solid (58.0 g, 261 mmol, 48.99% yield). LCMS(ESI): m/z 194 [M+1]+; $^1H$ NMR (400 MHz, $CDCl_3$) δ 8.83 (d, J = 2.4 Hz, 1H), 8.55 (d, J = 2.4 Hz, 1H), 8.00 (d, J = 8.8 Hz, 1H), 7.89 (s, 1H), 7.73 (dd, $J_1$= 8.8 Hz, $J_2$ = 1.6 Hz, 1H), 5.48 (brs, 1H), 4.71 (s, 2H).

Step 3: $SOCl_2$ (196 g, 1.65 mol, 120 mL, 17.8 eq) was added to Compound 3 (18.0 g, 92.9 mmol, 1.00 eq), and the mixture was stirred at 20°C for 3 h. LCMS showed that the starting material was consumed and the desired MS was detected. The reaction mixture was concentrated to give a residue which was then slurried with EtOAc (100 mL), filtered and the filter cake was diluted with EtOAc (400 mL), and the combined organic layers were washed with $NaHCO_3$ (sat., 150 mL × 2) and brine (200 mL), dried over $Na_2SO_4$, filtered and concentrated under reduced pressure to give the title compound as a yellow solid (14.0 g, 65.6 mmol, 70.6% yield, 99.4% purity). LCMS (ESI): m/z 212[M+1]+; $^1H$ NMR (400 MHz, $CDCl_3$) δ 8.90 (d, J = 2.4 Hz, 1H), 8.59 (d, J = 2.4 Hz, 1H), 8.14 - 7.97 (m, 2H), 7.84 (dd, $J_1$ = 8.8 Hz, $J_2$ = 1.6 Hz, 1H), 4.98 (s, 2H).

2-(bromomethyl)-6-cyclopropylimidazo[1,2-a]pyridine (Intermediate 5) was prepared according to the following synthetic scheme:

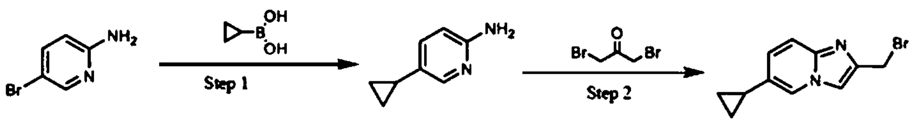


Intermediate 5

Step 1: To a solution of 3-bromo-2-aminopyridine (11 g, 63.58 mmol) and cyclopropylborate (10.92 g, 127.16 mmol) in toluene (200 mL) and $H_2O$ (20 mL) was added $Pd(OAc)_2$ (1.43 g, 6.36 mmol) and SPhos (5.22 g, 12.72 mmol), then $K_3PO_4$(40.49 g, 190.74 mmol) was added. The mixture was stirred overnight at 100°C and diluted with water (1.0 L). The reaction mixture was extracted with DCM (300 mL * 3) and the organic layer was filtered through a pad of celite. The filtrate was concentrated under reduced pressure to dryness. The residue was purified by silica gel chromatography (eluting with EtOAc) to give the title compound as a yellow solid (8.0 g, 93.77% yield). LCMS(ESI): m/z 135.2 $[M+H]^+$.

Step 2: A solution of 3-cyclopropyl-2-aminopyridine (2.0 g, 14.91 mmol) and 2-carbonyl-1,3-dibromopropane (4.83 g, 22.37 mmol) in DME (30 mL) was stirred at 90°C overnight. The mixture was concentrated under reduced pressure and purified by silica gel chromatography (eluting with DCM: MeOH = 30:1) to give the title compound (Intermediate 5) as a yellow solid (2.75 g, 73.47% yield). LCMS (ESI): m/z 252.9[m+H]+.

1-(2-cyano-4-(6,6-difluoro-3-azabicyclo[3.1.0]hexan-3-yl)benzyl)-1H-pyrazole-4-carboxylic acid (Intermediate 6) was prepared according to the following synthetic scheme:

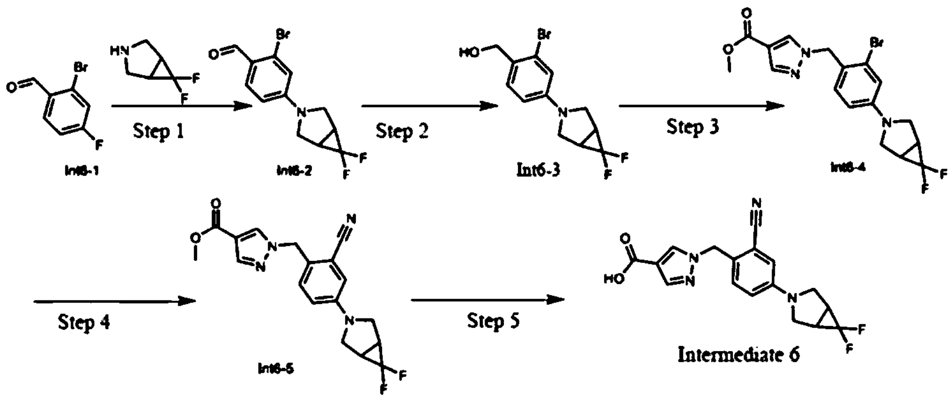


Step 1: To a solution of Int6-1 (0.5 g, 2.46 mmol) in NMP (10 mL) was added compound 6,6-difluoro-3-azabicyclo [3.1.0]hexane (0.38 g, 2.46 mmol), $K_2CO_3$ (0.85 g, 6.15 mmol) and stirred at 120°C for 16 h. After the reaction was monitored for completion by LCMS, the mixture was diluted with water (80 mL) and extracted with EtOAc (80 mL * 3). The combined layers were dried over anhydrous sodium sulfate. The organic phase was filtered and the filtrate was concentrated under reduced pressure. The residue was purified by column chromatography (eluting with polyethylene: ethyl acetate = 100:0 to 85:15) to give the desired compound Int6-2 (0.48 g, 64.51% yield) as a white solid. LCMS (ESI): m/z 304.0 [M+H]+.

Step 2: To a solution of Int6-2 (0.28 g, 2.93 mmol) in MeOH (10 mL) was added $NaBH_4$ (0.042 g, 1.12 mmol) and the mixture was stirred at room temperature for 0.5 h. After the reaction was monitored for completion by LCMS, the reaction mixture was diluted with water (100 mL) and extracted with DCM (100 mL * 3). The organic layer was washed with brine, water, and dried over anhydrous sodium sulfate. The organic phase was filtered and the filtrate was concentrated under reduced pressure to give the desired compound Int6-3 as a white solid (0.275 g, 97.56% yield). LCMS (ESI): m/z 306 [M + H]+.

Step 3: Int6-3 (0.24 g, 0.79 mmol), 5 (0.15 g, 1.19 mmol) and pTsOH (0.45 g, 0.24 mmol) were added to MeCN (10 mL) and the mixture was stirred at 65°C for 16 h. The reaction was monitored by LCMS after completion and the reaction mixture was diluted with water (80 mL) and extracted with EtOAc (80 mL * 3). The complex organic layer was dried over anhydrous sodium sulfate, the organic phase was filtered, and the filtrate was concentrated under reduced pressure. The residue was eluted by column chromatography (PE: EA = 2:1) to give the desired compound Int6-4 (0.125 g, 38.43% yield) as a white solid. LCMS (ESI): m/z 414.2[M + H]+.

Step 4: Compound Int6-4 (0.115 g, 0.28 mmol), $Zn(CN)_2$ (0.066 g, 0.56 mmol), zinc powder (0.010 g, 0.15 mmol), $(t\text{-}Bu)_3P$ * $HBF_4$ (0.016 g, 0.056 mmol) and $Pd_2(dba)_3$ (0.026 g, 0.028 mmol) were added to NMP (10 mL). The reaction mixture was flushed with nitrogen for 5 minutes. The mixture was stirred at 80°C for 2 h. After the reaction was monitored for completion by LCMS, the mixture was diluted with water (100 mL) and extracted with ethyl acetate (100 mL * 3). The combined organic layers were dried over anhydrous sodium sulfate. The organic phase was filtered and the filtrate was concentrated under reduced pressure. The residue was purified by column chromatography (eluting with dimethylchloromethane: methanol = 20:1) to give the desired compound Int6-5 (0.095 g, 95.03% yield) as a white solid. LCMS (ESI): m/z 359.3 [M+H]+.

Step 5: Compound Int6-5 (95 mg, 0.27 mmol) in methanol (12 mL) was mixed with 2 M sodium hydroxide solution, stirring at 30°C for 16 h. After the reaction was monitored for completion by LCMS, the reaction mixture was acidified to pH = 6 with 1M HCl and extracted with DCM (100 mL * 3). The organic layer was washed with water, brine, and dried over anhydrous sodium sulfate. The organic phase was filtered and the filtrate was concentrated under reduced pressure to afford the desired Intermediate 6 (82 mg, 89.83% yield) as a white solid. LCMS (ESI): m/z 345.2[M + H]+.

2-(aminomethyl)-3-fluoro-N-methylpyridin-4-amine hydrochloride (Intermediate 7) was prepared according to the following synthetic scheme:

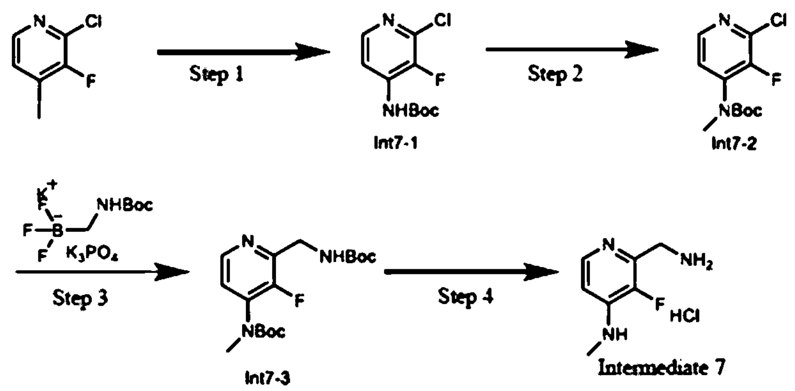


Int7-1 Int7-2 Int7-3 Intermediate 7

Step 1: Preparation of tert-butyl(2-chloro-3-fluoropyridin-4-yl)carbamate: Under nitrogen, 2-chloro-3-fluoro-4-iodopyridine (500 mg, 1.90 mmol), tert-butyl carbamate (250 mg, 2.1 mmol), Tris(dibenzylideneacetone)dipalladium (87 mg, 0.10 mmol), 4,5-bis(diphenylphosphino)-9,9-dimethylxanthene (58 mg, 0.1 mmol) and cesium carbonate (1230 mg, 3.80 mmol) were added to dioxane (10 mL) successively and heated to 100°C for 3 hours. After filtration, the filtrate was collected and concentrated under reduced pressure to give a residue, which was purified by column chromatography (EA: PE = 1:10) to give the title compound (450 mg, yield: 96%) as a yellow oily liquid. MS (ESI) M/Z: 247.1 [M+H]+.

Step 2: Preparation of tert-butyl(2-chloro-3-fluoropyridin-4-yl)(methyl)carbamate: Int7-1 (350 mg, 1.40 mmol) was added to N,N-dimethylformamide (3 mL) under nitrogen, the mixture was cooled to 0°C, and sodium hydride (68 mg, 1.70 mmol) was added. After 30 minutes of reaction at 0°C, a solution of methyl iodide (397 mg, 2.8 mmol) dissolved in N,N-dimethylformamide (2 mL) was slowly added dropwise, and the reaction was allowed to return to room temperature for 1 hour. Water (25 mL) was added, the mixture was extracted with ethyl acetate (25 mL × 3), the organic phases were combined, washed separately with water (50 mL) and saturated sodium chloride (50 mL), dried over anhydrous sodium sulfate, filtered, the filtrate was collected and concentrated under reduced pressure to give a residue. The residue was purified by column chromatography (EA: PE = 1:5) to give the title compound (285 mg, yield: 78%) as a yellow solid. MS (ESI) M/Z: 261.1 $[M+H]^+$.

Step 3: Preparation of tert-butyl(2-(((tert-butoxycarbonyl)amino)methyl)-3-fluoropyridin-4-yl)(methyl)carbamate: Under nitrogen, Int7-2 (280 mg, 1.10 mmol), potassium[(tert-butoxycarbonylamino)methyl]trifluoroborate (521 mg, 2.20 mmol), [n-butylbis(1-adamantyl)phosphine](2-amino-1,1'-biphenyl-2-yl)palladium (II) methanesulfonate (44 mg, 0.06 mmol) and potassium phosphate (466 mg, 2.20 mmol) were added successively to a mixed solution of dioxane (4 mL) and water (1 mL). The reaction was heated to 100°C for 16 hour. After filtration, the filtrate was collected and concentrated under reduced pressure to give a residue which was purified by column chromatography (EA: PE = 1:5) to give the compound Int7-3 (230 mg, yield: 59%) as a yellow solid. MS (ESI) M/Z: 356.2 $[M+H]^+$.

Step 4: Preparation of 2-(aminomethyl)-3-fluoro-N-methylpyridin-4-amine hydrochloride: Int7-3 (230 mg, 0.60 mmol) was added to a solution of dioxane hydrochloride (4.0 M, 5 mL), and the mixture was reacted for 1 hour at room temperature. The mixture was concentrated under reduced pressure to give Intermediate 7 (120 mg, yield: 100%) as a white solid. MS (ESI) M/Z: 156.2 $[M+H]^+$.

3-(3,3-difluorocyclobutyl)-1-(4-((5-fluoro-2-oxopyridin-1(2H)yl)methyl)benzyl)-1H-pyrazole-4-carboxylic acid (Intermediate 8) was prepared according to the following synthetic scheme:

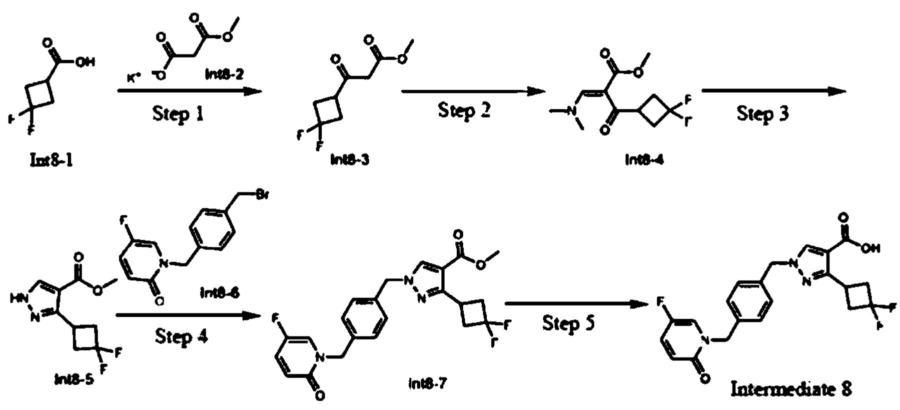


Int8-1 Int8-3 Int8-4 Int8-5 Int8-7 Intermediate 8

Step 1: Preparation of methyl 3-(3,3-difluorocyclobutyl)-3-oxopropionate: To a solution of Int8-1 (600 mg, 4.41 mmol) in THF (20 mL) was added CDI (720 mg, 4.41 mmol) and the mixture was stirred at room temperature for 2 hours. Compound Int8-2 (830 mg, 5.29 mmol) and $MgCl_2$ (500 mg, 5.29 mmol) were then added and stirred overnight at room temperature. After completion of the reaction was monitored by LCMS, the mixture was diluted with water (100 mL) and extracted with EA (100 mL). The organic phase was dried over anhydrous sodium sulfate, filtered, and concentrated under reduced pressure to give the crude product. The residue was purified by preparative TLC (PE: EA = 3:1) to give the title compound (610 mg, 72.01% yield) as a pale yellow oil. MS (ESI) m/z: 193.0 [M+H]+. $^1$H NMR (400 MHz, $CDCl_3$) δ 3.77 (s, 3H), 3.52 (s, 2H), 3.26 (pd, J = 8.8, 2.6 Hz, 1H), 2.89 - 2.72 (m, 4H).

Step 2: Preparation of methyl (E)-2-(3,3-difluorocyclobutane-1-carbonyl)-3-(dimethylamino)propenoic acid: To a solution of Int8-3 (610 mg, 3.17 mmol) in DMF (10 mL) was added DMF-DMA (566 mg, 4.75 mmol), and the mixture was stirred at 70°C for 1 hour. After monitoring the completion of the reaction by LCMS, it was concentrated under reduced pressure to give the title compound as a yellow oil (784 mg, 100% yield). MS (ESI) m/z: 248.2 [M+H]+.

Step 3: Preparation of methyl 3-(3,3-difluorocyclobutyl)-1H-pyrazole-4-carboxylate: To a solution of Int8-4 (784 mg, 3.17 mmol) in t-BuOH (10 mL) was added hydrazine hydroxide (238 mg, 4.75 mmol) and acetic acid (285 mg, 4.75% mmol), then the mixture was stirred at 60°C for 1 h. After monitoring the completion of the reaction by LCMS, the mixture was diluted with $NaHCO_3$ solution (50 mL) and extracted with EA (50 mL). The organic phase was dried over $Na_2SO_4$, filtered, and concentrated under reduced pressure to obtain the crude product. The residue was purified by silica gel chromatography (eluting with PE: EA = 100:0 to 50:50) to give the title compound (423 mg, 61.71% yield) as a white solid. MS (ESI) m/z: 217.1 [M+H]+.

$^1$H NMR (400 MHz, DMSO) δ 13.24 (s, 1H), 8.28 (s, 1H), 3.72 (d, J = 10.8 Hz, 4H), 3.03 - 2.70 (m, 4H).

Step 4: Preparation of methyl 3-(3,3-difluorocyclobutyl)-1-(4-((5-fluoro-2-oxopyridin-1(2H)yl)methyl)benzyl)-1H-pyrazole-4-carboxylate: To a solution of Int8-5 (150 mg, 0.69 mmol) in DMF (8 mL) was added Int8-6 (204 mg, 0.69 mmol) and $K_2CO_3$ (238 mg, 1.72 mmol), and the mixture was stirred overnight at room temperature. After monitoring the completion of the reaction by LCMS, the mixture was diluted with water (50 mL) and extracted with EA (50 mL * 3). The combined organic layers were dried over $Na_2SO_4$ and concentrated under reduced pressure. The residue was purified by preparative TLC (PE: EA = 0:1) to give the title compound (245 mg, 81.85% yield) as a white solid. MS (ESI) m/z: 432.2 [M+H]+.

$^1$H NMR (400 MHz, DMSO) δ 8.40 (s, 1H), 8.11 - 7.99 (m, 1H), 7.57 (ddd, J = 10.3, 7.2, 3.4 Hz, 1H), 7.27 (q, J = 8.3 Hz, 4H), 6.44 (dd, J = 10.0, 5.4 Hz, 1H), 5.30 (s, 2H), 5.01 (s, 2H), 3.72 (s, 3H), 3.67 - 3.60 (m, 1H), 2.97 - 2.85 (m, 2H), 2.84 - 2.71 (m, 2H).

Step 5: Preparation of methyl 3-(3,3-difluorocyclobutyl)-1-(4-((5-fluoro-2-oxopyridin-1(2H)yl)methyl)benzyl)-1H-pyrazole-4-carboxylate: To a solution of Int8-7 (40 mg, 0.093 mmol) in MeOH (2 mL) was added NaOH (2 M, 1 mL, 2 mmol), and the mixture was stirred overnight at room temperature. The reaction mixture was quenched by the addition of HCl solution (2 M, 1 mL, 2 mmol) and concentrated under reduced pressure to afford the title compound (38 mg, 100% yield). MS (ESI) M/Z: 503.21 [M+H]+. $^1$H NMR (400 MHz, DMSO-d6) δ 8.30 (d, J = 6.0 Hz, 1H), 8.28 (s, 1H), 7.90 (d, J = 5.6 Hz, 1H), 7.77 (dd, J = 6.8, 2.0 Hz, 1H), 7.45 - 7.36 (m, 1H), 7.30 - 7.21 (m, 4H), 6.58 - 6.49 (m, 2H), 6.42 - 6.37 (m, 1H), 6.26 - 6.19 (m, 1H), 5.30 (s, 2H), 5.07 (s, 2H), 4.79 (dd, J = 8.4, 5.6 Hz, 2H), 4.68 (dd, J = 6.8, 5.6 Hz, 2H), 4.55 - 4.46 (m, 1H), 4.37 (dd, J = 5.6, 2.0 Hz, 2H), 2.73 (d, J = 4.8 Hz, 3H).

1-ethyl-1,4,5,6-tetrahydrocyclopentadienyl[d]imidazole-4-amine (Intermediate 9) was prepared according to the following synthetic scheme:

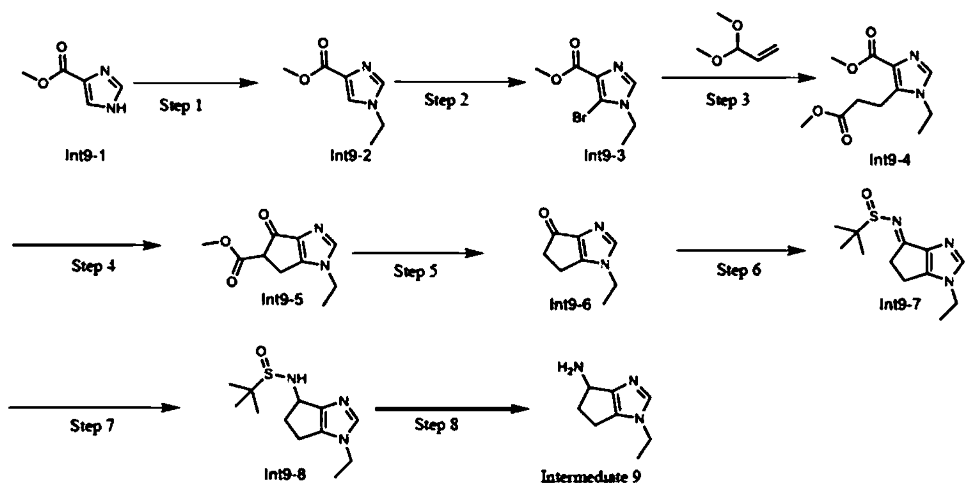


Step 1: To a solution of Int9-1 (15.0 g, 118.94 mmol) in DMF (180 mL) was added $Cs_2CO_3$ (58.13 g, 178.41 mmol) and ethyl iodide (27.83 g, 178.4 mmol), and the mixture was stirred at 70°C for 16 hours. After monitoring the completion of the reaction by LCMS, the reaction mixture was cooled to room temperature and filtered through a pad of celite. The filtrate was concentrated under reduced pressure and purified by silica gel chromatography (eluting with PE: EtOAc = 100:0 to 0:100) to give Int9-2 (6.2 g, 33.81% yield). MS(ESI)M/Z: 155.2[M+H]+.

Step 2: To a solution of Int9-2 (6.2 g, 40.22 mmol) in acetic acid (25 mL) was added NBS (7.87 g, 44.24 mmol) and the mixture was stirred at 70°C for 6 h. After monitoring the completion of the reaction by LCMS, the reaction mixture was concentrated under reduced pressure. The pH of the residue was adjusted to 7-8 with $Na_2CO_3$ saturated solution. The mixture was diluted with $H_2O$ (50 mL) and extracted with DCM (300 mL * 3). The combined organic layers were dried over anhydrous $Na_2SO_4$. The organic phase was filtered and the filtrate was concentrated under reduced pressure. The residue was purified by silica gel chromatography (eluting with PE: EtOAc = 100:0 to 0:100) to give the desired compound Int9-3 (3.5 g, 37.34% yield). MS(ESI)M/Z: 235.0[M+H]+.

Step 3: To a solution of Int9-3 (3.5 g, 15.02 mmol) in N,N-dimethylacetamide (25 mL) and water (5 mL) was added 3,3-dimethoxy-1-propene (2.15 g, 21.03 mmol) and N,N-dicyclohexylmethylamine (4.11 g, 21.03 mmol). The mixture was purged with nitrogen for 5 minutes. Then $PdCl_2[P(O\text{-}Tol)_3]_2$ (0.71 g, 0.90 mmol) was added and the mixture was stirred at 120°C for 4 h. After monitoring the completion of the reaction by LCMS, the reaction mixture was cooled to room temperature and filtered through a pad of celite. The reaction mixture was poured into 200 mL water and extracted with EA (200 mL * 3). The combined organic layers were dried over anhydrous $Na_2SO_4$. The residue was purified by reverse phase HPLC to give Int9-4 (1.4 g, 38.8% yield).

MS(ESI)M/Z: 241.2[M+H]+.

Step 4: Under nitrogen atmosphere, compound Int9-4 (1.1 g, 4.99 mmol) was dissolved in THF (20 mL) and treated with KHMDS (10 mL 1 M THF solution, 10 mmol) at 0°C. The mixture was stirred for 1 hour and the reaction was monitored by LCMS after completion and then poured into a cooled mixture of EtOAc (100 mL) and acetic acid (15 mL). After stirring for 30 minutes, the mixture was filtered. The reaction mixture was diluted with $H_2O$ (200 mL) and extracted with EtOAc (300 mL * 3). The combined organic layers were dried over anhydrous $Na_2SO_4$. The organic phase was filtered and the filtrate was concentrated under reduced pressure. The residue was purified by silica gel chromatography (eluting with DCM: MeOH = 100:0 to 92:8) to give Int9-5 (586 mg, 55.81% yield). MS (ESI) M/Z: 209.1 $[M+H]^+$.

Step 5: To a solution of Int9-5 (586 mg, 2.81 mmol) in water (1 mL), AcOH (5 mL), and HCl (1.5 mL), the mixture was then heated at 70°C for 3 hours. After completion of the reaction, it was monitored by LCMS. The pH of the reaction mixture was adjusted to 7-8 with $Na_2CO_3$. The reaction mixture was diluted with $H_2O$ (20 mL) and extracted with DCM (100 mL * 3). The combined layers were dried over anhydrous $Na_2SO_4$. The organic phase was concentrated to give the desired compound Int9-6 (410 mg, 97% yield). MS(ESI)M/Z: 151.0[M+H]+.

Step 6: A mixture of Int9-6 (310 mg, 2.06 mmol) and $Ti(OEt)_4$ (1.41 g, 6.18 mmol) was dissolved in toluene (20 mL), the mixture was stirred for 15 min, and then treated with t-butylsulfinamide (620 mg, 5.15 mmol). The mixture was heated at 100°C for 8 hours. Reaction completion was monitored by LCMS. After cooling to room temperature, the mixture was treated with saturated aqueous NaCl solution (10 mL). The mixture was stirred for 1 hour and then filtered over celite. The filter cake was stirred twice in THF (20 mL) for 10 minutes and filtered over celite. The combined organic phases were concentrated and the residue was purified by silica gel chromatography (DCM: MeOH = 95:5) to give the compound Int9-7 (330 mg, 63.10% yield). MS(ESI)M/Z: 254.2[M+H]+.

Step 7: Under nitrogen atmosphere, Int9-7 (330 mg, 1.3 mmol) was dissolved in THF (15 mL) and treated with L-Selectride (1.9 mL 1 M THF solution, 1.9 mmol) at 0°C. The mixture was stirred for 1.5 h and the reaction was monitored by LCMS after completion. The reaction was then treated dropwise with MeOH (2 mL). The solvent was evaporated in vacuo

and the residue was diluted with $H_2O$ (50 mL) and extracted with DCM (80 mL * 3). The combined layers were dried over anhydrous $Na_2SO_4$. The organic phase was concentrated to give the desired compound Int9-8 (340 mg, 100% yield) and used without further purification. MS(ESI)M/Z: 256.1[M+H]+.

Step 8: A mixture of Int9-8 (340 mg, 1.33 mmol) in isopropanol (10 mL) was treated with a solution of 2 M HCl/1,4-dioxane in isopropanol (1 mL) and stirred for 2 h. After monitoring the completion of the reaction by LCMS, the mixture was concentrated and the residue was purified by reverse phase to give Intermediate 9 (122 mg, 60.6% yield) as an oil. MS(ESI)M/Z: 152.2[M+H]+.

(6,8-dimethylimidazo[1,5-a]pyridin-7-yl)methylamine (Intermediate 10) was prepared according to the following synthetic scheme:

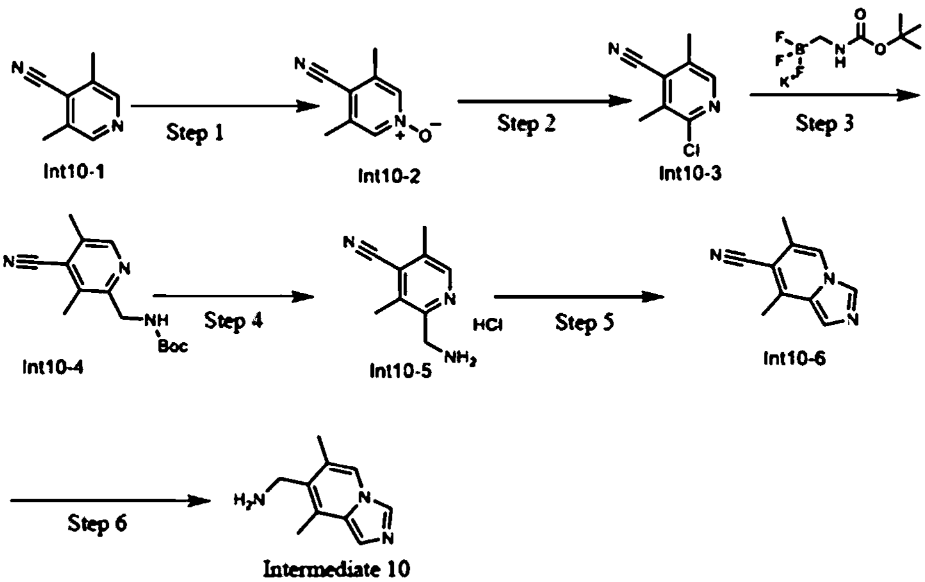

Int10-1
Int10-2
Int10-3
Int10-4
Int10-5
Int10-6
Intermediate 10

Step 1: To a solution of Int10-1 (1.00 g, 7.57 mmol, 1.00 eq) in DCM (10.0 mL) at 0°C was added m-CPBA (3.07 g, 15.1 mmol, 85% purity, 2.00 eq), followed by stirring at 20°C for 2 h. The reaction mixture was diluted with $H_2O$ (20.0 mL), extracted with EtOAc (30.0 mL × 3) and washed with sat. aq. $NaHCO_3$ (30.0 mL × 3), dried over $Na_2SO_4$, filtered and concentrated under reduced pressure to give a residue. The crude product was milled with PE/DCM = 5/1 (50.0/10.0 mL) at 20°C for 30 min. Compound Int10-2 was obtained as a white solid (1.00 g, 6.75 mmol, 89.2% yield). MS(ESI)M/Z: 149 [M+H]+.

Step 2: To a solution of Int10-2 (1.00 g, 6.75 mmol, 1.00 eq) in $POCl_3$ (16.4 g, 107 mmol, 10.0 mL), the mixture was stirred at 100°C for 2 h. The reaction mixture was filtered and concentrated under reduced pressure to give a residue. The residue was purified by column chromatography ($SiO_2$, PE/EtOAc = 100/1 to 5/1). Compound Int10-3 was obtained as a colorless oil (0.700 g, 4.20 mmol, 62.2% yield). MS(ESI)M/Z: 167 [M+H]+.

Step 3: Prepration of Int10-4: To a solution of Int10-3 (400 mg, 2.40 mmol, 1.00 eq), potassium((tert-butoxycarbonyl)amino)methyl)trifluoroborate (854 mg, 3.60 mmol, 1.50 eq) in dioxane (10.0 mL) and $H_2O$ (2.00 mL) was added $K_3PO_4$ (1.53 g, 7.20 mmol, 3 eq) and CataCXiumAPdG3 (175 mg, 240 μmol, 0.100 eq) and the mixture was stirred at 110°C for 3 h. LC-MS (EW47914-123-P1A) showed that the desired mass was detected. The reaction mixture was filtered and concentrated under reduced pressure to give a residue. The residue was purified by preparative HPLC to afford the compound Int10-4 (100 mg, 383 μmol, 15.9% yield) as a white solid. MS(ESI)M/Z: 206 [M+H]+.

Step 4: HCl/dioxane (2 M, 2.00 mL, 7.47 eq) was added to a solution of Int10-4 (140 mg, 536 μmol, 1.00 eq) in DCM (2.00 mL) at 0°C. The combined mixture was stirred at 20°C for 5 hours. The reaction mixture was concentrated under reduced pressure to give a residue. Compound Int10-5 was obtained as a white solid (100 mg, 506 μmol, 94.4% yield, HCl). MS(ESI)M/Z: 162 [M+H]+.

Step 5: To a solution of Int10-5 (100 mg, 506 μmol, 1.00 eq, HCl) in HCOOH (24.8 mg, 506 μmol, 2.00 mL, 98% purity, 1.00 eq) and $Ac_2O$ (1.00 mL), the mixture was stirred at 50°C for 1 h. The reaction mixture was filtered and concentrated under reduced pressure to give a residue, which was then diluted with sat. aq. $NaHCO_3$ (10 mL), extracted with EtOAc (20.0 mL × 3), dried over $Na_2SO_4$, filtered and concentrated under reduced pressure to give a residue. A yellow solid compound Int10-6 (85.0 mg, 496 μmol, 98.1% yield) was obtained. MS(ESI)M/Z: 172 [M+H]+.

Step 6: Raney nickel (10.0 mg, 117 μmol, 1.00 eq) was added to the flask under an argon atmosphere, and methanol (2.00 mL) was added to the flask under an argon atmosphere. Compound Int10-6 (20.0 mg, 117 μmol, 1 eq) and $NH_3{\bullet}H_2O$ (2.12 g, 18.2 mmol, 2.33 mL, 30% purity, 156 eq) in MeOH (2 mL) were added to the flask under an argon atmosphere, the suspension was then degassed and purged three times with $H_2$, and the reaction mixture was stirred under $H_2$ (50 Psi) at 25°C for 5 h. LC-MS showed that Int10-6 was completely consumed and the desired mass was detected. The reaction mixture was filtered and concentrated under reduced pressure to give a residue. A yellow solid intermediate 10 (20.0 mg, 114 μmol, 97.7% yield) was obtained. MS(ESI)M/Z: 176 [M+H]+.

(6,8-dimethylimidazo[1,5-a]pyridin-7-yl)methylamine (Intermediate 11) was prepared according to the following synthetic scheme:

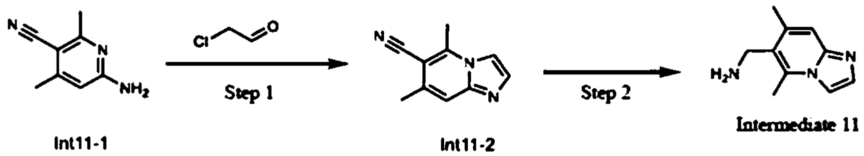


Int11-1 Int11-2 Intermediate 11

Step 1: To a solution of Int11-1 (500 mg, 3.40 mmol) in EtOH (10 mL) was added compound 2-chloroacetaldehyde (733.98 mg, 3.74 mmol) at 25°C. The mixture was stirred at 80°C for 16 hours. The reaction mixture was concentrated to give a crude product. The crude product was triturated with EA (5 mL) at 25°C for 30 min. Compound Int11-2 was obtained as a yellow solid (560 mg, 96% yield). MS(ESI) m/z: 172.2[m+H]+.

Step 2: Raney nickel (41.90 mg, 0.71 mmol) was added to the flask under Ar atmosphere. MeOH (15 mL) was then added to the flask under Ar atmosphere. Compound Int11-2 (300 mg, 1.75 mmol) and ammonium hydroxide (3 mL) in MeOH (10 mL) were added to the flask under Ar atmosphere. The suspension was degassed and purged 3 times with $H_2$. The mixture was stirred under $H_2$ at 25°C overnight. The reaction mixture was filtered. The filtrate was concentrated to give Intermediate 11 as a yellow solid (275 mg, 89.56% yield). MS(ESI)m/z: 176.2[m+H]+.

(3-fluoro-4-(methylthio)pyridin-2-yl)methylamine (Intermediate 12) was prepared according to the following synthetic scheme:

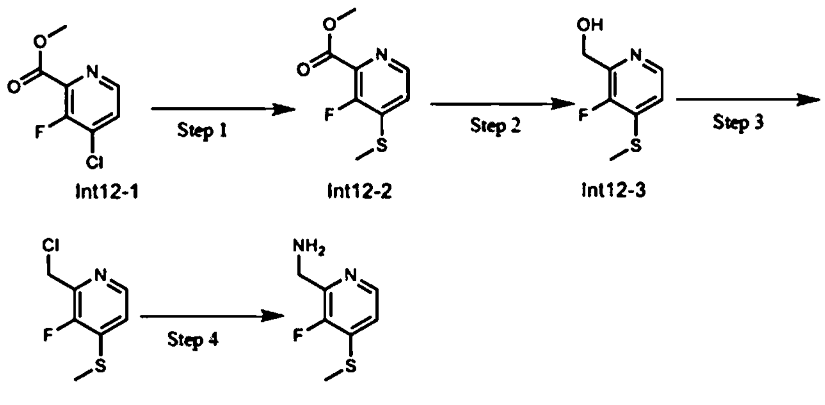


Int12-1 Int12-2 Int12-3 Int12-4 Intermediate 12

Step 1: Int12-1 (400 mg, 2.12 mmol), sodium methyl mercaptide (163 mg, 2.33 mmol) were added to DMF (5 mL) and reacted at 50°C for 2 h. The mixture was cooled to room temperature, adding water (20 mL), extracted with ethyl acetate (30 mL × 3), the organic phases were combined, washed separately with water (50 mL × 1) and saturated aqueous sodium chloride solution (50 mL × 1), dried over anhydrous sodium sulfate, and concentrated under reduced pressure to give a residue. The residue was purified by normal phase chromatography (petroleum ether/ethyl acetate = 0-100%) to give the compound Int12-2 (250 mg, yield: 59%) as a white solid. MS (ESI) M/Z: 202.0 $[M+H]^+$.

Step 2: Int12-2 (250 mg, 1.2 mmol) was added to methanol (20 mL), sodium borohydride (900 mg, 2.4 mmol) was added at 0°C, and the mixture was reacted overnight at room temperature. Water (80 mL) was added, the mixture was extracted with ethyl acetate (30 mL × 3), the organic phases were combined, washed with water (50 mL × 1) and saturated

aqueous sodium chloride solution (50 mL × 1) respectively, dried over anhydrous sodium sulfate, and concentrated under reduced pressure to obtain crude Int12-3 (200 mg) which was used directly in the next step. MS (ESI) M/Z: 174.0 $[M+H]^+$.

Step 3: Crude Int12-3 was added to dichloromethane (10 mL), thionyl chloride (280 mg, 2.4 mmol) was added dropwise and the mixture was reacted for 1 hour at room temperature. The mixture was concentrated under reduced pressure to obtain crude Int12-4 (190 mg), which was used directly in the next step. MS (ESI) M/Z: 192.0 $[M+H]^+$.

Step 4: Crude Int12-4 was added to aqueous ammonia (20 mL) and the mixture was reacted for 2 hours at room temperature. The mixture was concentrated under reduced pressure to obtain crude 399-4 (150 mg), which was used directly in the next step. MS (ESI) M/Z: 173.0 $[M+H]^+$.

(7-fluoro-1-methyl-1H-pyrrolo[3,2-c]pyridin-6-yl)methylamine hydrochloride (Intermediate 13) was prepared according to the following synthetic scheme:

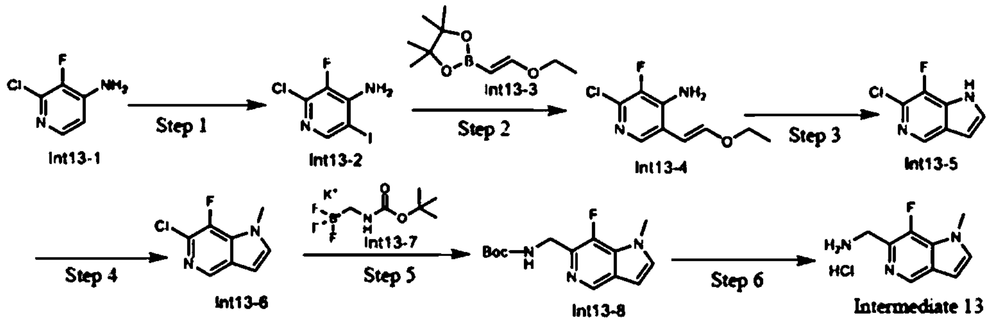


Step 1: To a solution of Int13-1 (1.0 g, 6.82 mmol) in acetonitrile (8 mL) was added TsOH (65 mg, 0.34 mmol) and NIS (1.69 g, 7.5 mmol) and the mixture was stirred at 70°C overnight. After the reaction was monitored for completion by LCMS, the reaction mixture was diluted with water (150 mL) and extracted with EA (150 mL * 3). The combined organic phases were concentrated and the residue was purified by flash chromatography (PE: EA = 100:0 to 70:30) to afford the desired compound Int13-2 (1.68 g, 90.37% yield) as a pale yellow solid. MS (ESI) m/z: 273.0 $[M+H]^+$.

Step 2: To a solution of Int13-2 (1.38 g, 5.07 mmol) in DCE (24 mL) and $H_2O$ (8 mL) was added Int13-3 (2.01 g, 10.14 mmol) and $Na_2CO_3$ (1.61 g, 15.21 mmol), the mixture was purged with nitrogen for 5 minutes. $Pd(PPh_3)_4$ (590 mg, 0.51 mmol) was added and the mixture was stirred at 100°C overnight. After monitoring the completion of the reaction by LCMS, the reaction mixture was cooled to room temperature and filtered through a pad of celite. The reaction mixture was poured into $H_2O$ (200 mL) and extracted with DCM (200 mL * 3). The residue was purified by flash chromatography (PE: EA= 100:0 to 83:17) to give the desired compound Int13-4 (800 mg, 72.91% yield). MS(ESI)m/z: 217.0[m+H]+.

Step 3: To a solution of Int13-4 (800 mg, 3.69 mmol) in EtOH (15 mL) was added hydrogen chloride (2.5 mL), and the mixture was stirred at 80°C for 2 h. After monitoring the completion of the reaction by LCMS, the reaction mixture was concentrated under reduced pressure. The residue was diluted with $H_2O$ (80 mL), basified to pH = 8 to 9 with saturated $NaHCO_3$ solution, and extracted with DCM (200 mL * 3 mL). The combined organic phases were concentrated under reduced pressure to afford the desired compound Int13-5 (524 mg, 83.19% yield) as a white solid, which was used without further purification.

MS(ESI)m/z: 171.0[m+H]+.

Step 4: A solution of Int13-5 (270 mg, 1.58 mmol) in DMF (9 mL) was cooled to 0°C, NaH (189 mg, 4.74 mmol) was added, and the solution was stirred at 0°C for 0.5 h. After addition of methyl iodide (269 mg, 1.90 mmol), the mixture was brought to room temperature and the solution was stirred for 1 h. After monitoring the completion of the reaction by LCMS, the reaction mixture was quenched by the addition of saturated ammonium chloride (15 mL), diluted with water (150 mL) and extracted with EA (150 mL * 3). The combined organics were concentrated. The residue was purified by flash chromatography (PE: EA = 100:0 to 80:20) to give the desired compound Int13-6 (275 mg, 94.11% yield).

MS(ESI)m/z: 185.0[m+H]+.

Step 5: To a solution of Int13-6 (180 mg, 0.98 mmol) in 1,4-dioxane (10 mL) and $H_2O$ (2 mL) was added Int13-7 (348 mg, 1.47 mmol), $K_3PO_4$ (624 mg, 2.94 mmol) and the mixture was purged with nitrogen for 5 min. $CataCXiumAPdG_3$ (71 mg, 0.098 mmol) was added and the mixture was stirred at 100°C overnight. After completion of the reaction was monitored by LCMS, the mixture was diluted with water (120 mL) and extracted with EA (120 mL * 3). The combined organic layers were dried over $Na_2SO_4$, concentrated under reduced pressure. The residue was purified by flash chromatography (PE: EA= 100:0 to 50:50) to afford the desired compound Int13-8 (200 mg, 73.43% yield) as a white solid. MS(ESI)m/z: 280.2[m+H]+.

Step 6: A solution of Int13-8 (200 mg, 0.72 mmol) in 4M HCl in dioxane (10 mL) was stirred at room temperature for

2 hours. After completion of the reaction, it was monitored by LCMS and concentrated under reduced pressure to give the desired compound Intermediate 13 (154 mg HCl salt, 100% yield).

MS(ESI)m/z: 180.2[m+H]+.

(7-fluoro-1H-pyrrolo[3,2-c]pyridin-6-yl)methylamine hydrochloride (Intermediate 14) was prepared according to the following synthetic scheme:

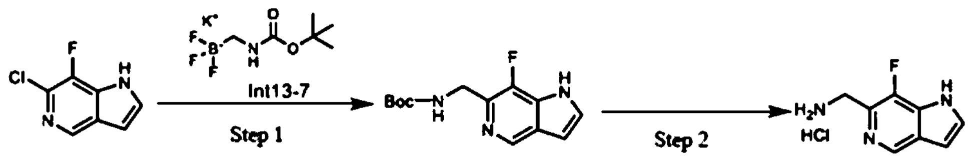


Int13-5 Int14-1 Intermediate 14

Step 1: To a solution of Int13-5 (180 mg, 1.06 mmol) in 1,4-dioxane (10 mL) and $H_2O$ (2 mL) was added Int13-7 (377 mg, 1.59 mmol) and $K_3PO_4$ (675 mg, 3.18 mmol), the mixture was purged with nitrogen for 5 min. CataCXiumAPdG$_3$ (62 mg, 0.085 mmol) was added and the mixture was stirred at 105°C overnight. After completion of the reaction was monitored by LCMS, the mixture was diluted with water (100 mL) and extracted with EA (100 mL * 3). The combined organic layers were dried over $Na_2SO_4$, concentrated under reduced pressure. The residue was purified by flash chromatography (PE: EA = 100:0:0:100) to afford the desired compound Int14-1 (172 mg, yield 61.44%) as a pale yellow solid. MS(ESI)m/z: 266.2[m+H]+.

Step 2: A solution of Int14-1 (172 mg, 0.65 mmol) in 4 M HCl in dioxane (10 mL) was stirred at room temperature for 4 hours. After completion of the reaction, it was monitored by LCMS and concentrated under reduced pressure to afford the desired compound Intermediate 14 (130 mg HCl salt, 100% yield). MS(ESI)m/z: 166.0[m+H]+.

(7-fluoro-1-methyl-2,3-dihydro-1H-pyrrolo[3,2-c]pyridin-6-yl)methylamine hydrochloride (Intermediate 15) was prepared according to the following synthetic scheme:

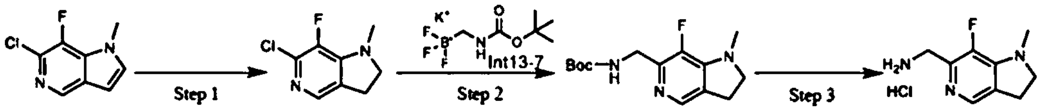


Int13-6 Int15-1 Int15-2 Intermediate 15

Step 1: To a solution of Int13-6 (200 mg, 1.08 mmol) in THF (5 mL) was added DMS-$BH_3$ (1.08 mL, 10.8 mmol, 10 M/L), and the mixture was purged with nitrogen for 3 min and stirred at 65°C overnight. The reaction mixture was cooled to room temperature, MeOH (8 mL) was added, stirred at 60°C for 0.5 h, and concentrated under reduced pressure. The residue was then diluted with water (120 mL) and extracted with EA (120 mL * 3). The combined organics were concentrated. The residue was purified by flash chromatography (PE: EA = 100:0:0:100) to give Int15-1 (71 mg, 35.12% yield). MS(ESI)m/z: 187.0[m+H]+.

Step 2: To a solution of Int15-1 (71 mg, 0.38 mmol) in 1,4-dioxane (10 mL) and $H_2O$ (2 mL) was added Int13-7 (180 mg, 0.76 mmol), $K_3PO_4$ (242 mg, 1.14 mmol) and the mixture was purged with nitrogen for 5 min. CataCXiumAPdG$_3$ (27 mg, 0.038 mmol) was added and the mixture was stirred at 100°C overnight. After completion of the reaction was monitored by LCMS, the mixture was diluted with water (120 mL) and extracted with EA (120 mL * 3). The combined organic layers were dried over $Na_2SO_4$, and concentrated under reduced pressure. The residue was purified by flash chromatography (PE: EA = 100:0 to 0:100) to afford the desired compound Int15-2 (60 mg, 56.05% yield) as a white solid. MS(ESI)m/z: 282.2[m+H]+.

Step 3: A solution of Int15-2 (60 mg, 0.21 mmol) in 4.0 M HCl in dioxane (5 mL) was stirred at room temperature for 2 hours. After completion of the reaction, it was monitored by LCMS and concentrated under reduced pressure to give Intermediate 15 (47 mg HCl salt, 100% yield). MS(ESI)m/z: 182.0[m+H]+.

((7-fluoro-2,3-dihydro-1H-pyrrolo[3,2-c]pyridin-6-yl)methylamine hydrochloride (Intermediate 16) was prepared according to the following synthetic scheme:

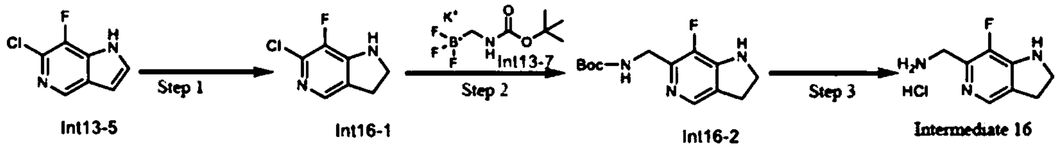


Int13-5 Int16-1 Int16-2 Intermediate 16

Step 1: To a solution of Int13-5 (600 mg, 3.52 mmol) in THF (8 mL) was added DMS-$BH_3$ (3.52 mL, 35.2 mmol, 10 M/L), and the mixture was purged with nitrogen for 3 min and stirred overnight at 65°C. The reaction mixture was cooled to room temperature, MeOH (10 mL) was added, stirred at 60°C for 0.5 h, and concentrated under reduced pressure. The residue was then diluted with water (200 mL) and extracted with EA (300 mL * 3). The combined organics were concentrated. The residue was purified by flash chromatography (PE: EA = 100:0 to 0:100) to give the desired compound Int16-1 (405 mg, 66.71% yield). MS(ESI)m/z: 173.0[m+H]+.

Step 2: To a solution of Int16-1 (200 mg, 1.16 mmol) in 1,4-dioxane (10 mL) and $H_2O$ (2 mL) was added Int13-7 (550 mg, 2.32 mmol) and $K_3PO_4$ (738 mg, 3.48 mmol), the mixture was purged with nitrogen for 5 min. CataCXiumAPdG$_3$ (84 mg, 0.12 mmol) was added and the mixture was stirred at 100°C overnight. After completion of the reaction was monitored by LCMS, the mixture was diluted with water (200 mL) and extracted with EA (200 mL * 3). The combined organic layers were dried over $Na_2SO_4$, concentrated under reduced pressure. The residue was purified by flash chromatography (PE: EA= 100:0 to 0:100) to afford the desired compound Int16-2 (178 mg, 57.47% yield) as a white solid. MS(ESI)m/z: 268.0[m+H]+.

Step 3: A solution of Int16-2 (178 mg, 0.67 mmol) in 4 M HCl in dioxane (8 mL) was stirred at room temperature for 2 hours. After completion of the reaction, it was monitored by LCMS and concentrated under reduced pressure to afford the desired compound Intermediate 16 (136 mg, HCl salt, 100% yield). MS(ESI)m/z: 168.0[m+H]+.

1-(4-(cyclopropylmethoxy)benzyl)-1H-pyrazole-4-carboxylic acid (Intermediate 17) was prepared according to the following synthetic scheme:

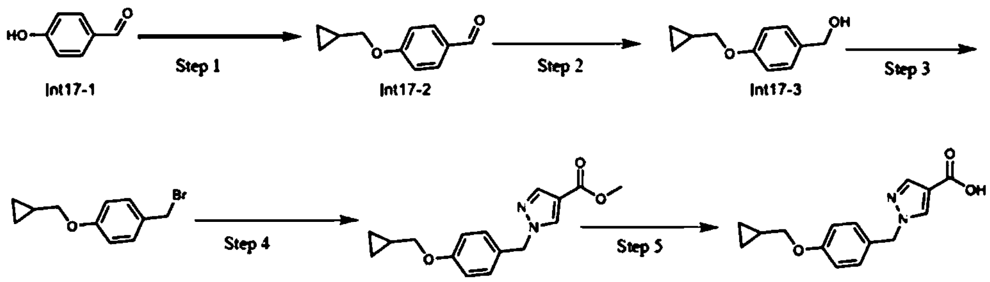


Int17-1 Int17-2 Int17-3 Int17-4 Int17-5 Intermediate 17

Step 1: (bromomethyl)cyclopropane (500 mg, 3.75 mmol), $K_2CO_3$ (1.037 g, 7.51 mol) and Int17-1 (550 mg, 4.5 mol) were added to acetone (5 mL) at room temperature, heating to 60°C and stirring for 12 h. The mixture was concentrated at room temperature under reduced pressure and purified by column chromatography (ethyl acetate: petroleum ether = 0-100%) to give compound Int17-2 (440 mg, yield: 72.7%) as a white solid. MS (ESI) M/Z: 177.1[M+1]+.

Step 2: Int17-2 (480 mg, 2.47 mmol) was added to MeOH (3 mL) under nitrogen, the mixture was cooled to 0°C, replaced with $N_2$ three times, $NaBH_4$ (309 mg, 7.41 mmol) was added, stirring at 0°C for 2 h, 1m/L aqueous solution of $NH_4Cl$ (3 mL) was added, the mixture was filtered through celite, dried over anhydrous sodium sulfate, and concentrated under reduced pressure to give Int17-3 (440 mg, crude) as a yellow oily liquid. MS (ESI) M/Z: 179.1 $[M+H]^+$.

Step 3: Int17-3 (440 mg, 2.47 mmol) was added to dichloromethane (3 mL) at room temperature, the mixture was cooled to 0°C, $PBr_3$ (1.34 g, 4.96 mmol) was added, and the mixture was stirred at room temperature for 2 hours. The pH of the reaction was adjusted to basic with saturated aqueous sodium bicarbonate, extracted with DCM (10 mLx3), the organic phases were combined, dried over anhydrous sodium sulfate, and concentrated under reduced pressure to give compound Int17-4 (661 mg, crude) as a white solid.

MS (ESI) M/Z: 241.1$[M+1]^+$.

Step 4: At room temperature, Int17-4 (661 mg, 2.75 mmol) was added to DMF (5 mL), and $K_2CO_3$ (1.51 g, 11 mmol) and methyl pyrrole-3-carboxylate (381.15 mg, 3.025 mol) were added portionwise. The mixture was stirred at room temperature for 12 h. After addition of water (30 mL) and extraction with DCM (30 mL × 3), the organic phases were combined, dried over anhydrous $Na_2SO_4$, filtered and concentrated under reduced pressure to give a residue which was purified by flash chromatography (PE: EA = 3:1, Rf = 0.4) to give Int17-5 (334 mg, yield: 42.4%) as a white solid. MS (ESI) M/Z: 287.1$[M+1]^+$.

Step 5: Int17-5 (60 mg, 0.21 mmol) was added to MeOH (3 mL) at room temperature. NaOH aqueous solution (1M) (1 mL) was added, stirring at room temperature for 12 h. The pH of the reaction mixture was adjusted to acidic with aqueous 1 N hydrogen chloride, extracted with DCM (10 mL × 3), the organic phases were combined, dried over anhydrous sodium sulfate, and concentrated under reduced pressure to give Intermediate 17 (60 mg) as a white solid. MS (ESI) M/Z: 273.3 $[M+1]^+$.

(7-fluoro-2,3-dihydrofluoro[3,2-c]pyridin-6-yl)methylamine hydrochloride (Intermediate 18) was prepared according to the following synthetic scheme:

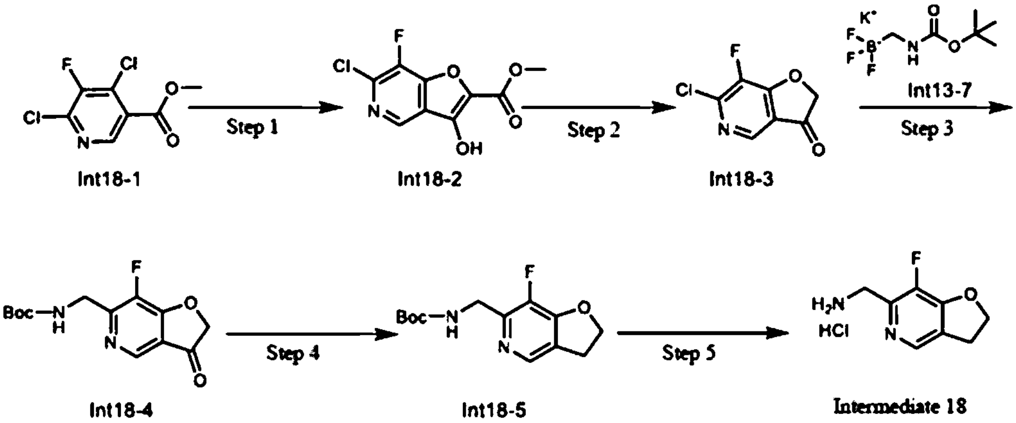


Int18-1 Int18-2 Int18-3 Int18-4 Int18-5 Intermediate 18

Step 1: NaH (1.96 g, 40.2 mol, 60% purity) was added portionwise to a solution of methyl glycolate (2.4 g, 40.2 mol) in ethylene glycol dimethyl ether (50 mL) under an ice bath. The reaction was allowed to warm to room temperature and stirred for 30 minutes. Int18-1 (9 g, 40.2 mmol) was then dissolved in ethylene glycol dimethyl (10 mL) and added dropwise to the above reaction, reacting for 2 hours at 35°C. After completion of the reaction, the reaction mixture was diluted with ethyl acetate and quenched with saturated sodium bicarbonate solution. The organic phase was extracted, washed with saturated saline, dried over anhydrous sodium sulfate and concentrated. The residue was purified by flash chromatography (PE: EA = 5:1 to 1:2) to afford the desired compound Int18-2 (6.4 g, 65% yield) as a white solid. MS(ESI) m/z: 246.0[M+H]+.

Step 2: Int18-2 (6.4 g, 26 mmol) was dissolved in a mixed solvent of tetrahydrofuran (60 mL) and water (16 mL) and lithium hydroxide monohydrate (4.45 g, 106.8 mmol) was added. The mixture was heated to 40°C for 2 days. After the completion of the reaction, the reaction solution was cooled to room temperature, filtered and the filter cake was washed with tetrahydrofuran. The filter cake was then dissolved in a mixed solvent of ethyl acetate and water, and the system was acidified to pH 6-7 with 1M hydrochloric acid in an ice-water bath. The mixture was layered, and the aqueous phase was extracted with ethyl acetate, the organic phases were combined, dried over anhydrous sodium sulfate, filtered, and the filtrate was concentrated by rotary evaporation to give compound Int18-3 (2.9 g, 15.6 mmol, 60%). MS m/z (ESI): 188.0 [M+H]+.

Step 3: To a solution of Int18-3 (0.96 g, 5.2 mmol) in 1,4-dioxane (10 mL) and $H_2O$ (2 mL) was added Int13-7 (2.45 g, 10.4 mmol) and $K_3PO_4$ (3.30 g, 15.6 mmol), and the mixture was purged with nitrogen for 5 min. CataCXiumAPdG$_3$ (376 mg, 0.53 mmol) was added and the mixture was stirred at 100°C overnight. After completion of the reaction was monitored by LCMS, the mixture was diluted with water (200 mL) and extracted with EA (200 mL * 3). The combined organic layers were dried over $Na_2SO_4$, concentrated under reduced pressure. The residue was purified by flash chromatography (PE: EA = 5:1 to 1:2) to afford the desired compound Int18-4 (733 mg, 50% yield) as a white solid. MS(ESI)m/z: 283.1[M+H]+.

Step 4: A solution of Int18-4 (733 mg, 2.6 mmol) in absolute ethanol (40 mL) was hydrogenated with 20% palladium hydroxide (150 mg) at 3 atmospheres overnight, the catalyst was removed by filtration through a pad of celite, and the filtrate was concentrated to give compound Int18-5 (629 mg, 90%), MS(ESI)m/z: 269.1[M+H]+.

Step 5: A solution of Int18-5 (629 mg, 2.34 mmol) in 4 M HCl in dioxane (8 mL) was stirred at room temperature for

2 hours. After completion of the reaction, it was monitored by LCMS and concentrated under reduced pressure to afford the desired compound Intermediate 18 (440 mg, HCl salt, 92% yield). MS(ESI)m/z: 169.0[M+H]+.

Example 1. N-((6-amino-2,4-dimethylpyridin-3-yl)methyl)-1-(4-((2-oxopyridin-1(2H)-yl)methyl)benzyl)-1H-1,2,3-triazole-4-carboxamide (Compound 1) was prepared according to the following synthetic scheme:

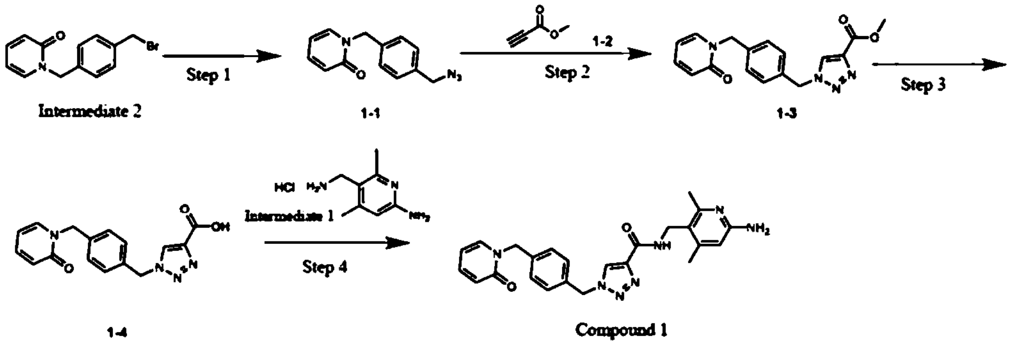


Step 1: To a solution of Intermediate 2 (500 mg, 1.80 mmol, 1.00 eq) in DMF (5.00 mL) was added $NaN_3$ (175 mg, 2.70 mmol, 1.50 eq). The mixture was stirred at 25°C for 1 hour. TLC (plate 1, petroleum ether: ethyl acetate = 1:1) indicated that reactant 1 was completely consumed and a new spot was formed. The reaction mixture was partitioned between $H_2O$ (30.0 mL) and EtOAc (50.0 mL). The organic phase was separated, washed with brine (30.0 mL), dried over $Na_2SO_4$, filtered and concentrated under reduced pressure to give a residue. Compound 1-1 was obtained as a colorless oil (350 mg, 1.46 mmol, 81.0% yield). LCMS(ESI): m/z 241.

Step 2: A solution of Compound 1-1 (350 mg, 1.46 mmol, 1.00 eq), Compound 1-2 (122 mg, 1.46 mmol, 121 μL, 1.00 eq), $CuSO_4$ (72.7 mg, 291 μmol, 0.200 eq) and vitamin C sodium salt (288 mg, 1.46 mmol, 1.00 eq) in t-BuOH (20.0 mL) and $H_2O$ (5.00 mL) were degassed and purged 3 times with $N_2$, and the mixture was then stirred at 25°C under $N_2$ atmosphere for 2 h. TLC (plate 1, dichloromethane: methanol = 10:1) indicated that the starting material was consumed and a new spot were detected. The residue was diluted with $H_2O$ (30.0 mL) and extracted with EtOAc (50.0 mL). The combined organic layers were washed with brine (30.0 mL), dried over $Na_2SO_4$, filtered and concentrated under reduced pressure to give a residue. The residue (dichloromethane: methanol = 10:1) was purified by preparative TLC ($SiO_2$, dichloromethane: MeOH = 10:1). Compound 1-3 was obtained as a white solid (100 mg, 308 μmol, 21.1% yield). LCMS(ESI): m/z 325; $^1H$ NMR (400 MHz, DMSO-$d_6$) δ 7.90 (s, 1H), 7.29-7.27 (m, 2H), 7.24 - 7.18 (m, 2H), 6.56 - 6.53 (d, J = 9.2 Hz, 1H), 6.13 - 6.09 (t, J = 6.8 Hz, 2H), 5.48 (s, 2H), 5.07 (s, 2H), 3.85 (s, 3H).

Step 3: NaOH (24.6 mg, 616 μmol, 2.00 eq) was added to a solution of Compound 1-3 (100 mg, 308 μmol, 1.00 eq) in MeOH (1.00 mL) and $H_2O$ (0.500 mL) at 0°C. The mixture was stirred at 25°C for 1 hour. LC-MS showed that the desired mass was detected. The reaction mixture was filtered and concentrated under reduced pressure to give a residue. The residue was dissolved in water (5.00 mL) and acidified to pH = 3 with 2 M HCl. The solids were filtered and collected to give a residue. Compound 1-4 was obtained as a white solid (90.0 mg, 290 μmol, 94.0% yield). LCMS(ESI): m/z 311.

Step 4: A mixture of Compound 1-4 (50.0 mg, 161 μmol, 1.00 eq), Intermediate 1 (30.2 mg, 161 μmol, 1.00 eq, HCl), HATU (91.9 mg, 241 μmol, 1.50 eq), and TEA (48.9 mg, 483 μmol, 67.2 μL, 3.00 eq) in DMF (1.00 mL) was degassed and purged 3 times with $N_2$, and then the mixture was stirred for 2 h at 25°C under $N_2$ atmosphere. TLC (plate 1, dichloromethane: methanol = 10:1) showed that Compound 1-4 was consumed and a new spot was detected. The residue was diluted with $H_2O$ (30.0 mL) and extracted with EtOAc (50 mL). The combined organic layers were washed with brine (30.0 mL), dried over $Na_2SO_4$, filtered and concentrated under reduced pressure to give a residue. The residue (plate 1, dichloromethane: methanol = 10:1) was purified by preparative TLC ($SiO_2$, dichloromethane: methane = 10:1) to give the title Compound 1 (21.92 mg, 49.3 μmol, yield 30.6%, purity 98.6%) as a white solid. LCMS(ESI):m/z 444; $^1H$ NMR (400 MHz, DMSO-d $_6$) δ 7.90 (s, 1H), 7.31 - 7.27 (m, 2H), 7.20 (s, 2H), 6.91 (s, 1H), 6.58 - 6.56 (d, J = 9.2 Hz, 2H), 6.18 - 6.10 (m, 2H), 5.47 (s, 1H), 5.09 (s, 1H), 4.49 - 4.48 (d, J = 4.8 Hz, 2H), 4.39 (s, 2H), 2.40 (s, 3H), 2.22 (s, 3H).

Example 2. N-((6-amino-2,4-dimethylpyridin-3-yl)methyl)-3-(methoxymethyl)-1-(4-((2-oxopyridin-1(2H)-yl)methyl)benzyl)-1H-pyrazole-4-carboxamide (Compound 2) was prepared according to the following synthetic scheme:

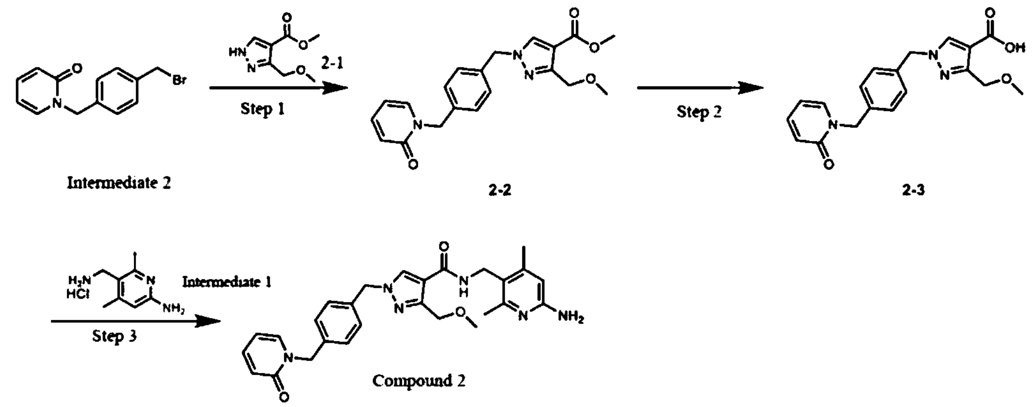


Intermediate 2, 2-2, 2-3, Compound 2

Step 1: To a solution of Intermediate 2 (500 mg, 1.80 mmol, 1.00 eq) in DMF (20.0 mL) was added Compound 2-1 (305 mg, 1.80 mmol, 1.00 eq) and $K_2CO_3$ (496 mg, 3.60 mmol, 2.00 eq). The mixture was stirred at 60°C for 12 hours. TLC (plate 1: DCM: MeOH = 10:1, Rf = 0.34) indicated that Compound 2-1 was completely consumed. The reaction mixture was diluted with $H_2O$ (10 mL) and extracted with DCM (10 mL * 3). The combined organic layers were dried over $Na_2SO_4$, filtered and concentrated under reduced pressure to give a residue. The residue was purified by column chromatography ($SiO_2$, DCM/MeOH = 1/0-10/1, Plate 2: DCM: MeOH = 10:1, Rf = 0.34). Compound 2-2 was obtained as a white solid (300 mg, 816 μmol, 45.4% yield), LCMS (ESI): m/z 368 [M + 1]+; $^1$H NMR (400 MHz, DMSO-$d_6$) δ 8.41 (s, 1H), 7.76-7.74 (d, J = 8 Hz, 1H), 7.73-7.72 (m, 1H), 7.42-7.40 (m, 4H), 6.40-6.38 (m, 1H), 6.23-6.20 (t, J = 12 Hz, 1H), 5.29 (s, 2H), 5.06 (s,2H), 4.48 (s, 2H), 3.71 (s, 3H), 3.22 (s, 3H).

Step 2: To a solution of Compound 2-2 (300 mg, 816 μmol, 1.00 eq) in MeOH (2.00 mL) and $H_2O$ (2.00 mL) was added NaOH (163 mg, 4.08 mmol, 5.00 eq). The mixture was stirred at 25°C for 1 hour. TLC (plate 1: DCM: MeOH = 10:1, Rf = 0.15) showed that Compound 2-2 was completely consumed. The mixture was concentrated under reduced pressure to remove MeOH, and then the pH was adjusted to 6-7 with 1 M HCl. Then the mixture was concentrated under reduce pressure to obtain a crude product. Compound 2-3 was obtained as a white solid (280 mg, crude). LCMS(ESI): m/z 352 [M-1]-.

Step 3: To a solution of Compound 2-3 (50.0 mg, 141 μmol, 1.00 eq) in DMF (2.00 mL) was added Intermediate 1 (26.6 mg, 141 μmol, 1.00 eq, HCl), HATU (80.7 mg, 212 μmol, 1.50 eq) and TEA (71.6 mg, 707 μmol, 98.5 μL, 5.00 eq). The mixture was stirred at 25°C under $N_2$ for 2 h. LC-MS showed that the desired mass was detected. $NaHCO_3$ (5.00 mL) was added to the mixture and stirred for 0.5 h. The mixture was then diluted with 10.0 mL $H_2O$ and extracted with 30.0 mL DCM (10.0 mL * 3). The combined organic layers were washed with 20.0 mL (10.0 mL * 2) of brine, dried over $Na_2SO_4$, filtered and concentrated under reduced pressure to give a residue. The residue was purified by preparative TLC ($SiO_2$, DCM: MeOH = 10:1, Rf = 0.38). The title Compound 2 (60.0 mg, 121 μmol, 85.6% yield, 98.2% purity) was obtained as a white solid. LCMS (ESI): m/z 487 [M + 1]+. $^1$H NMR δ (400 MHz, $CD_3OD$-$d_4$) δ 8.11 (s, 1H), 7.99-7.97 (m, 1H), 7.80-7.79 (m, 1H), 7.34-7.26 (m, 4H), 6.84-6.71 (m, 3H), 5.34-5.31 (m, 4H), 4.59 (s, 2H), 4.45 (s, 2H), 3.31-3.30 (m, 3H), 2.59 (s, 3H), 2.46 (s, 3H).

Example 3. N-((6-amino-2,4-dimethylpyridin-3-yl)methyl)-5-((3-chloroquinolin-6-yl)methyl)thiophene-2-carboxamide (Compound 3) was prepared according to the following synthetic scheme:

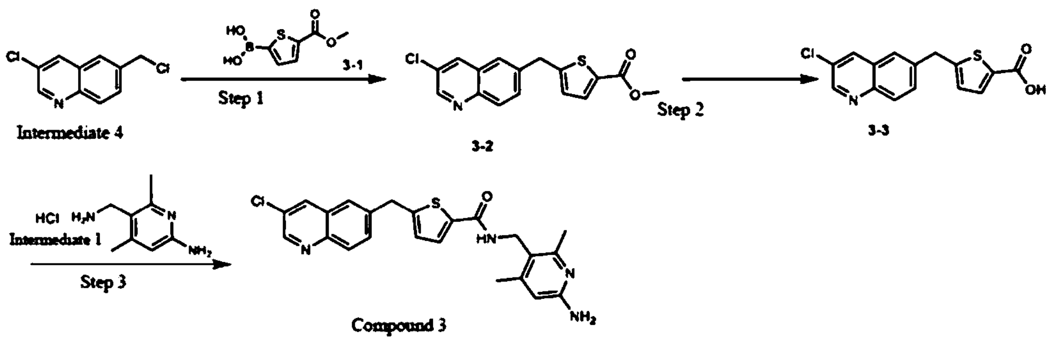


Intermediate 4, 3-2, 3-3, Compound 3

Step 1: To a solution of Intermediate 4 (500 mg, 2.36 mmol, 1.00 eq) in DMF (25.0 mL) was added Compound 3-1

(526 mg, 2.83 mmol, 1.20 eq), $Na_2CO_3$ (499.78 mg, 4.72 mmol, 2.00 eq) and Pd(dppf)$Cl_2$ (172 mg, 236 μmol, 0.100 eq), then the mixture was degassed and purged 3 times with $N_2$, and the mixture was heated to 60°C and stirred for 12 h. LCMS showed that Intermediate 4 was consumed and the desired MS was detected. The reaction mixture was diluted with $H_2O$ (25.0 mL) and then extracted with EtOAc (20.0 mL * 2). The combined organic layers were washed with water (30.0 mL * 1) and brine (30.0 mL * 1), dried over $Na_2SO_4$, filtered and concentrated under reduced pressure to give a residue. The residue was purified by preparative HPLC (column: Phenomenex luna C18 150 * 40 mm * 15 um; mobile phase: [water (FA) -ACN]; gradient: 49% -79% B over 15 min) to give the desired Compound 3-2 (360 mg, 1.13 mmol, 48.0% yield) as a yellow solid. LCMS(ESI): m/z 318 [M+1]+; $^1$H NMR(400MHz, DMSO-$d_6$) δ 8.85 (d, J = 2.4 Hz, 1H), 8.53 (d, J = 2.4 Hz, 1H), 8.01 (d, J = 8.8 Hz, 1H), 7.86 (s, 1H), 7.77 - 7.62 (m, 2H), 7.07 (d, J = 3.6 Hz, 1H), 4.43 (s, 2H), 3.76 (s, 3H).

Step 2: To a solution of Compound 3-2 (460 mg, 1.45 mmol, 1.00 eq) in MeOH (2.00 mL) at 0°C was added a solution of NaOH (4 M, 724 μL, 2.00 eq) in $H_2O$ (2.00 mL), the mixture was then stirred at 50°C for 2 h. LCMS showed that Compound 3-2 was consumed and the desired MS was detected. The reaction mixture was concentrated to give a residue and diluted with $H_2O$ (3.00 mL) and the pH was adjusted to 6 with 1M HCl. The mixture was lyophilized to give Compound 3-3 (600 mg, crude) as a white solid. LCMS(ESI): m/z 304 [M+1]+; $^1$H NMR(400MHz, DMSO-$d_6$) δ 8.85 (d, J = 2.4 Hz, 1H), 8.55 (d, J = 2.0 Hz, 1H), 8.01 (d, J = 8.8 Hz, 1H), 7.86 (s, 1H), 7.72 (dd, $J_1$ = 8.8 Hz, $J_2$ = 1.6 Hz, 1H), 7.49 (d, J = 3.6 Hz, 1H), 6.98 (d, J = 3.6 Hz, 1H), 4.39 (s, 2H).

Step 3: To a solution of Compound 3-3 (90.0 mg, 296 μmol, 1.00 eq) in DMF (5.00 mL) was added HATU (338 mg, 889 μmol, 3.00 eq), DIEA (191 mg, 1.48 mmol, 258 μL, 5.00 eq) and Intermediate 1 (66.7 mg, 356 μmol, 1.20 eq, HCl), then the mixture was stirred at 25°C for 1 h. LCMS showed that Compound 3-3 was consumed. The reaction mixture was diluted with $H_2O$ (10.0 mL) and then extracted with EtOAc (20.0 mL * 3). The combined organic layers were washed with water (30.0 mL * 1) and brine (30.0 mL * 1), dried over $Na_2SO_4$, filtered and concentrated under reduced pressure to give a residue. The residue was purified by preparative HPLC (column: Waters Xbridge C18 150 * 50 mm * 10 μm; mobile phase: [water ($NH_4HCO_3$) -ACN]; gradient: 28%-58% B over 10 min) to give the title Compound 3 (30.0 mg, 67.4 μmol, 22.8% yield, 98.2% purity) as a yellow solid. LCMS(ESI): m/z 437 [M+1]+; $^1$H NMR(400MHz, DMSO-$d_6$) δ8.80 (d, J = 2.0 Hz, 1H), 8.09 (d, J = 1.6 Hz, 1H), 8.04 (d, J = 4.8 Hz, 1H), 7.64 - 7.54 (m, 2H), 7.38 (d, J = 2.8 Hz, 1H), 6.83 (d, J = 3.6 Hz, 1H), 6.31 (s, 1H), 4.51 (d, J = 4.8 Hz, 2H), 4.33 (s, 2H), 2.51 (s, 3H), 2.31 (s, 3H).

Example 4. N-((6-amino-2,4-dimethylpyridin-3-yl)methyl)-1-(4-((2-oxopyridin-1(2H)-yl)methyl)benzyl)-4-(tetrahydrofuran-2-yl)-1H-pyrazole-3-carboxamide (Compound 4) was prepared according to the following synthetic scheme:

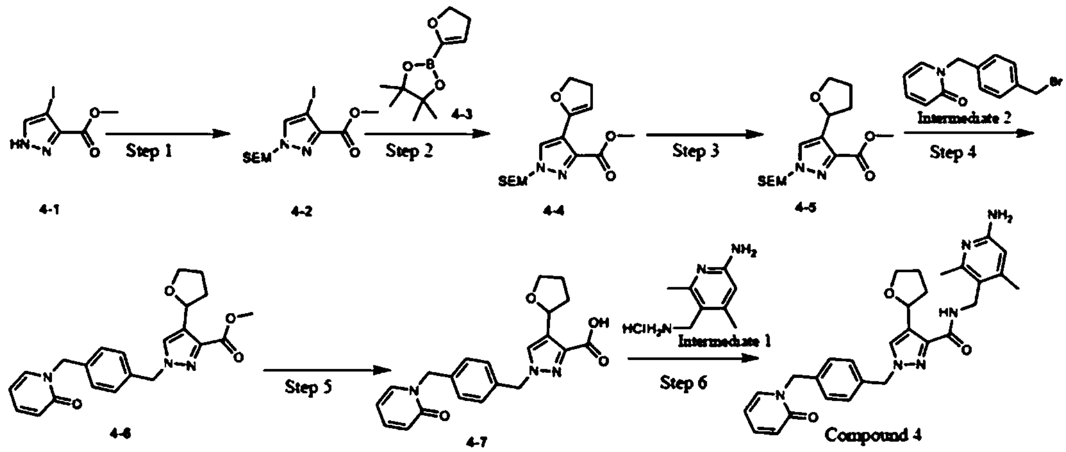


Step 1: To a solution of Compound 4-1 (5.00 g, 19.8 mmol, 1.00 eq) in THF (40.0 mL) at 0°C was added NaH (1.03 g, 25.8 mmol, 60.0% purity, 1.30 eq). The mixture was stirred at 0°C for 0.5 h. SEMCl (3.97 g, 23.8 mmol, 4.21 mL, 1.20 eq) was then added to the mixture at 0°C. The reaction mixture was stirred at 25°C for 1 hour. LC-MS showed that Compound 4-1 was completely consumed and a main peak with the desired m/z was detected. The reaction mixture was quenched by addition of saturated $NH_4Cl$ aqueous solution (20.0 mL) at 0°C, then diluted with $H_2O$ (20.0 mL) and extracted with ethyl acetate (50.0 mL × 3). The combined organic layers were dried over $Na_2SO_4$, filtered and concentrated under reduced pressure to give a residue. The residue was purified by column chromatography ($SiO_2$, petroleum ether: ethyl acetate = 100:1 to 3:1, petroleum ether: ethyl acetate = 3:1). Compound 4-2 was obtained as a yellow oil (6.00 g, 15.7 mmol, 79.1% yield). LCMS(ESI): m/z 383 [M+1]+; $^1$H NMR(400MHz, DMSO-$d_6$) δ 7.41 (s, 1H), 5.48 (s, 2H), 3.95 (s, 3H), 3.56 (t, J = 8.4 Hz, 1H), 0.90 (t, J = 8.4 Hz, 1H), -0.01 (s, 9H).

Step 2: A mixture of Compound 4-2 (250 mg, 654 μmol, 1.00 eq), Compound 4-3 (256 mg, 1.31 mmol, 2.00 eq), Ad2nBuP-Pd G3 (47.6 mg, 65.4 μmol, 0.100 eq) and $K_3PO_4$ (278 mg, 1.31 mmol, 2.00 eq) in 1,4-dioxane (2.00 mL) and $H_2O$ (0.400 mL) was placed in a microwave tube. The sealed tube was heated under microwave at 110°C for 2 hours. LC-MS showed that Compound 4-2 was completely consumed and a peak with the desired mass was detected. To the reaction mixture was added $H_2O$ (100 mL) and the mixture was extracted with ethyl acetate (80.0 mL × 3). The combined organic layers were dried over anhydrous $Na_2SO_4$, filtered and concentrated under reduced pressure to give a residue. The residue was subjected to preparative HPLC (TFA conditions; column: Phenomenex moon C18 150 * 40 mm * 15 um; mobile phase: [water (TFA) -ACN]; gradient: 35% -65% B over 15 min). The residue (petroleum ether: ethyl acetate = 1:1) was purified by preparative TLC ($SiO_2$, petroleum ether: ethyl acetate = 1:1). Compound 4-4 was obtained as a yellow oily liquid (75.0 mg, 8.55% yield). LCMS(ESI): m/z 325 [M+1]+.

Step 3: To a solution of Pd/C (50.0 mg, 10.0% purity) in MeOH (2.00 mL) Compound 4-4 (75.0 mg, 231 μmol, 1.00 eq) was added under $N_2$ atmosphere. The suspension was degassed and purged 3 times with $H_2$. The mixture was stirred at 25°C for 12 hours under $H_2$ (15 psi). LC-MS showed that Compound 4-4 was completely consumed and a main peak with the desired mass was detected. The reaction mixture was filtered and concentrated under reduced pressure to give a residue. Compound 4-5 was obtained as a colorless oil (73.0 mg, 207 μmol, 89.5% yield, 92.5% purity). LCMS(ESI): m/z 327 [M+1]+.

Step 4: To a solution of Compound 4-5 (73.0 mg, 224 μmol, 1.00 eq) in 1,4-dioxane (0.500 mL) was added HCl/1,4-dioxane (4.00 M, 280 μL, 5.00 eq). The mixture was stirred at 70°C for 12 hours. LC-MS showed that Compound 4-5 was completely consumed and a main peak with the desired mass was detected. The reaction mixture was filtered and concentrated under reduced pressure to give a residue. The residue was subjected to preparative HPLC (HCl conditions; column: Phenomenex luna C18 150 * 25 mm * 10 um; mobile phase: [water (HCl) -ACN]; gradient: 8%-38% B over 9 min). Compound 4-6 was obtained as a yellow oil (11.0 mg, 56.1 μmol, 25.1% yield). LCMS(ESI): m/z 394 [M+1]+; $^1$HNMR (400MHz, $CDCl_3$) δ 7.37 - 7.20 (m, 7H), 6.62 (d, J = 8.8 Hz, 1H), 6.17 (t, J = 6.4 Hz, 1H), 5.38 - 5.22 (m, 3H), 5.13 (s, 2H), 4.03 - 3.95 (m, 1H), 3.92 (s, 3H), 3.87 -3.77 (m, 1H), 2.58 - 2.36 (m, 1H), 2.07 - 1.85 (m, 2H), 1.68 - 1.62 (m, 1H).

Step 5: To a solution of Compound 4-6 (10.0 mg, 25.4 μmol, 1.00 eq) in MeOH (0.500 mL) was added NaOH (2.03 mg, 50.8 μmol, 2.00 eq) and $H_2O$ (0.100 mL). The mixture was stirred at 25°C for 3 hours. LC-MS showed that Compound 4-6 was completely consumed and a main peak with the desired mass was detected. The pH of the reaction mixture was adjusted to 3 with 1.00 M HCl. The reaction mixture was then concentrated under reduced pressure to give a residue. Compound 4-7 was obtained as a yellow solid (9.00 mg, 23.7 μmol, 93.3% yield). LCMS(ESI): m/z 380 [M+1]+.

Step 6: To a solution of Compound 4-7 (9.00 mg, 23.7 μmol, 1.00 eq) and Intermediate 1 (4.30 mg, 28.5 μmol, 1.20 eq) in DMF (1.00 mL) was added HATU (13.5 mg, 35.6 μmol, 1.50 eq) and TEA (7.20 mg, 71.2 μmol, 9.91 μL, 3.00 eq). The mixture was stirred at 25°C for 1 hour. LC-MS showed that Compound 4-7 was completely consumed and a main peak with the desired mass was detected. The reaction mixture was partitioned between ethyl acetate (80.0 mL) and $H_2O$ (80.0 mL). The organic phase was separated, washed with 60.0 mL (20.0 mL * 3) $NaHCO_3$, dried over $Na_2SO_4$, filtered and concentrated under reduced pressure to give a residue. The residue was subjected to preparative HPLC (neutral conditions; column: Waters xbridge 150 * 25mm 10 um; mobile phase: [water ($NH_4HCO_3$) -ACN]; gradient: 18%-38% B over 8 min). The title Compound 4 was obtained as a yellow solid (2.13 mg, 4.13 μmol, 17.4% yield). LCMS(ESI): m/z 513 [M+1]+; 1HNMR (400MHz, $CDCl_3$) δ 7.39 - 7.29 (m, 3H), 7.26 - 7.23 (m, 2H), 7.19 - 7.12 (m, 2H), 6.88 (t, J = 4.8 Hz, 1H), 6.61 (d, J = 9.2 Hz, 1H), 6.30 (s, 1H), 6.17 (t, J = 6.4 Hz, 1H), 5.30 (t, J = 6.8 Hz, 1H), 5.21 - 5.10 (m, 6H), 4.50 (d, J = 4.8 Hz, 2H), 3.99 - 3.92 (m, 1H), 3.85 - 3.77 (m, 1H), 2.51 (s, 3H), 2.49 - 2.42 (m, 1H), 2.32 (s, 3H), 1.97 - 1.91 (m, 2H), 1.73 - 1.67 (m, 1H).

Example 5. N-((6-amino-2,4-dimethylpyridin-3-yl)methyl)-1-((3-chloroquinolin-6-yl)methyl)-3-(methoxymethyl)-1H-pyrazole-4-carboxamide (Compound 5) was prepared according to the following synthetic scheme:

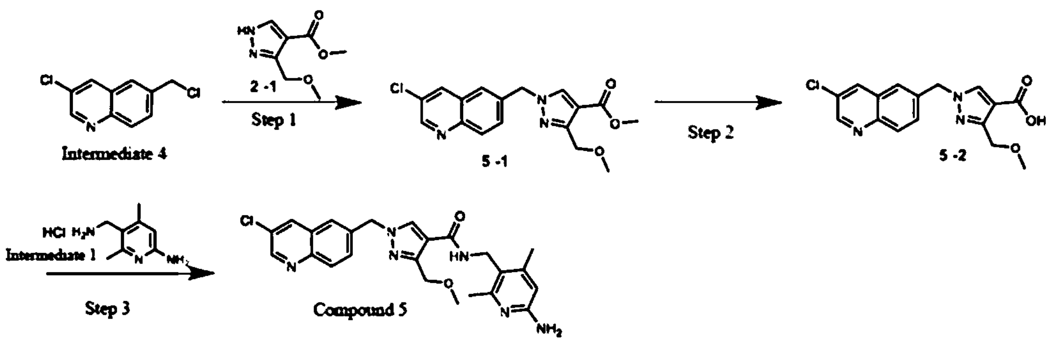

Step 1: To a solution of Intermediate 4 (250 mg, 1.18 mmol, 1.00 eq) and Compound 2-1 (261 mg, 1.53 mmol, 1.30 eq) in DMF (5.00 mL) was added $Cs_2CO_3$ (384 mg, 1.18 mmol, 1.00 eq), and the reaction mixture was stirred at 80°C for 2 hours. LC-MS showed that Compound 4 was consumed and a peak with the desired mass was detected. The reaction mixture was filtered with MeOH (10.0 mL) and the filtrate was concentrated under reduced pressure to give a residue. The residue was purified by preparative TLC ($SiO_2$, petroleum ether: ethyl acetate = 1:1, Rf = 0.30). Compound 5-1 was obtained as a white solid (230 mg, 665 μmol, 56.4% yield). LCMS(ESI): m/z 346 [M+1]+; $^1$HNMR (400MHz, $CDCl_3$) δ 8.84 (d, J = 2.4 Hz, 1H), 8.12 - 8.07 (m, 2H), 7.89 (s, 1H), 7.62 - 7.55 (m, 2H), 5.49 (s, 2H), 4.76 (s, 2H), 3.81 (s, 3H), 3.49 (s, 3H).

Step 2: To a solution of Compound 5-1 (230 mg, 665 μmol, 1.00 eq) in THF (2.00 mL) and MeOH (2.00 mL) was added a solution of NaOH (186 mg, 4.66 mmol, 7.00 eq) in $H_2O$ (1.00 mL). The mixture was stirred at 50°C for 3 hours. LC-MS showed that Compound 5-1 was completely consumed and a main peak with the desired mass was detected. The reaction mixture was concentrated to remove MeOH. Then, the pH of the mixture was adjusted to 7 with 1.00 M HCl, and then the mixture was concentrated under vacuum to obtain a residue. Compound 5-2 was obtained as a white solid (220 mg, 663 μmol, 99.7% yield). LCMS(ESI): m/z 332 [M+1]+; $^1$HNMR (400MHz, $CDCl_3$) δ 8.86 (d, J = 2.4 Hz, 1H), 8.57 (d, J = 2.4 Hz, 1H), 8.06 - 7.98 (m, 2H), 7.81 (d, J = 1.2 Hz, 1H), 7.64 (dd, $J_1$ = 8.8 Hz, $J_2$ = 2.0 Hz, 1H), 5.48 (s, 2H), 4.61 (s, 2H), 3.21 (s, 3H).

Step 3: To a solution of Compound 5-2 (110 mg, 332 μmol, 1.00 eq) and Intermediate 1 (60.2 mg, 398 μmol, 1.20 eq) in DMF (2.00 mL) was added HATU (189 mg, 497 μmol) and TEA (101 mg, 995 μmol). The mixture was stirred at 25°C for 1 hour. LC-MS showed that Compound 5-2 was consumed and a peak with the desired mass was detected. The reaction mixture was diluted with 50.0 mL saturated aqueous $NaHCO_3$, was extracted with ethyl acetate (50.0 mL × 3) and the combined organic phases were dried over $Na_2SO_4$ and concentrated to give a residue. The residue was subjected to preparative HPLC (column: Waters xbridge 150 * 25mm 10 um; mobile phase: [water ($NH_4HCO_3$) - ACN]; gradient: 25%-45% B over 8 min). The title Compound 5 (41.79 mg, 87.6 μmol, 26.4% yield, 97.5% purity) was obtained as a white solid. LCMS(ESI): m/z 465[M+1]+; $^1$HNMR (400MHz, $CDCl_3$) δ 8.88 (d, J = 2.4 Hz, 1H), 8.58 (d, J = 2.0 Hz, 1H), 8.34 (s, 1H), 8.03 (d, J = 8.4 Hz, 1H), 7.82 (s, 1H), 7.75 (s, 1H), 7.65 (dd, $J_1$ = 8.8 Hz, $J_2$ = 1.8 Hz, 1H), 6.11 (s, 1H), 5.64 (s, 2H), 5.51 (s, 2H), 4.49 (s, 2H), 4.26 (d, J = 4.4 Hz, 2H), 3.13 (s, 3H), 2.29 (s, 3H), 2.16 (s, 3H).

Example 6. N-((6-amino-2,4-dimethylpyridin-3-yl)methyl)-1-(6-cyclopropylimidazo[1,2-a]pyridin-2-yl)methyl-1H-1,2,3-triazole-4-carboxamide (Compound 6) was prepared according to the following synthetic scheme:

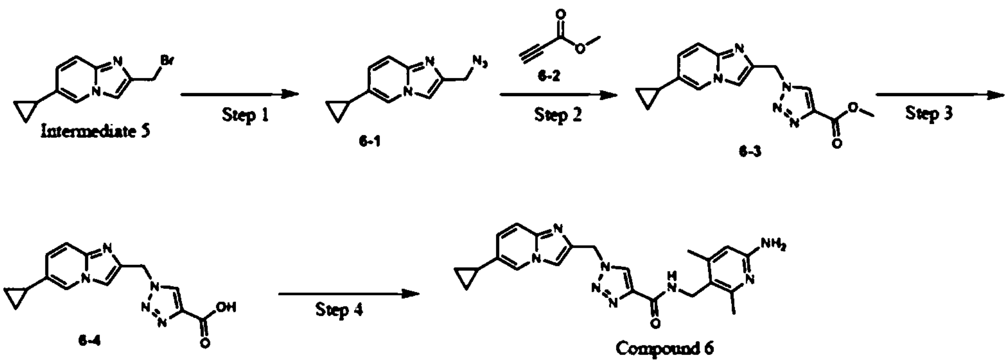


Step 1: A solution of Intermediate 5 (1.68 g, 6.69 mmol) and $NaN_3$ (0.76 g, 11.71 mmol) in DMF (20 mL) was stirred overnight at room temperature under nitrogen. The mixture was diluted with water (400 mL) and extracted with DCM (100 mL * 3). The organic layer was then filtered through a pad of celite and the filtrate was concentrated under reduced pressure. The residue was purified by silica gel chromatography (eluting with EtOAc) to afford the desired Compound 6-1 (0.556 g, 38.97% yield) as a yellow solid. LCMS(ESI): m/z 214.0[m+H]+.

Step 2: To a solution of Compounds 6-1 (0.556 g, 2.61 mmol) and 6-2 (0.24 g, 2.87 mmol) in tert-butanol (4 mL) and water (4 mL) was added vitamin C sodium salt (52 mg, 0.26 mmol) and $CuSO_4$ (42 mg, 0.26 mmol). After stirring the mixture at room temperature for 3 hours, the mixture was diluted with water (200 mL) and extracted with DCM (50 mL * 3). The combined organic layer was concentrated under reduced pressure to obtain a residue. The residue was purified by silica gel column chromatography (eluting with DCM: MeOH = 10:1) to give the target Compound 6-3 (0.65 g, 87.38% yield) as a yellow solid. LCMS(ESI): m/z: 298.0[m+H]+.

Step 3: NaOH (0.068 g, 1.7 mmol) was added to a solution of Compound 6-3 (0.1 g, 0.34 mmol) in MeOH (1.5 mL) and water (1.5 mL). After the mixture was stirred at room temperature for 1 h, the reaction was monitored by LCMS. The pH was adjusted to 4-5 with hydrochloric acid solution (1.0 M), and the mixture was concentrated under reduced pressure to

give the desired Compound 6-4 (0.095 g, 99.70% yield) as a yellow solid. LCMS(ESI): 284.0[M + H]+.

Step 4: To DMF (3 mL) was added Compound 6-4 (0.095 g, 0.34 mmol), Compound 2 (0.051 g, 0.34 mmol), DIEA (0.066 g, 0.51 mmol) and HATU (0.13 g, 0.34 mmol). The mixture was stirred overnight at room temperature and diluted with water (100 mL). The mixture was extracted with DCM (50 mL * 3). The combined organic layer was concentrated under reduced pressure and the residue was purified by chromatography on silica gel (DCM: MeOH = 10:1) to afford the title Compound 6 (5.69 mg, 4.07% yield) as a white solid. LCMS(ESI): 417.2[M + H]+; $^1$H NMR (400 MHz, DMSO-d6) δ 8.54 (s, 1H), 8.41 (s, 1H), 8.35 (s, 1H), 7.83 (s, 1H), 7.40 (d, J = 9.4 Hz, 1H), 7.01 (d, J = 8.2 Hz, 1H), 6.21 (s, 1H), 5.72 (s, 2H), 4.33 (d, J = 4.9 Hz, 2H), 3.30 - 3.27 (m, 2H), 2.34 (s, 3H), 2.21 (s, 3H), 1.97 - 1.88 (m, 1H), 0.92 (q, J = 6.0 Hz, 2H), 0.67 (d, J = 5.1 Hz, 2H).

Example 7. N-((6-amino-2,4-dimethylpyridin-3-yl)methyl)-1-(2-cyano-4-(6,6-difluoro-3-azabicyclo[3.1.0]hexan-3-yl) benzyl)-1H-pyrazole-4-carboxamide (Compound 7) was prepared according to the following synthetic scheme:

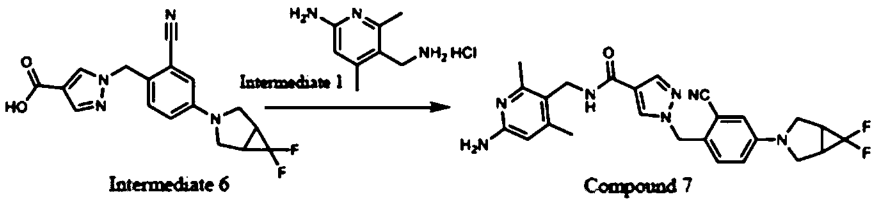


Intermediate 6

Compound 7

To a mixture of Intermediate 6 (0.040 g, 0.12 mmol) and Intermediate 1 (0.022 g, 0.14 mmol) in DMF (3 mL) was added DIEA (0.047 g, 0.36 mmol), HATU (0.046 g, 0.12 mmol). After the mixture was stirred at room temperature for 2 hours, the reaction was monitored for completion by LCMS. The reaction mixture was diluted with water (100 mL) and extracted with DCM (100 mL * 3). The combined organic layer was washed with brine, water and dried over anhydrous $Na_2SO_4$. The organic layer was then concentrated under reduced pressure and the residue was purified by preparative TLC (DCM: MeOH = 15:1) to give the title Compound 7 (37.41 mg, 67.44% yield) as a white solid. LCMS(ESI)M/Z: 478.4 [m+H]+; $^1$H NMR (400 MHz, DMSO) δ 8.17 (s, 1H), 8.00 (s, 1H), 7.86 (s, 1H), 7.29 (d, J = 8.8 Hz, 1H), 6.94 (d, J = 2.4 Hz, 1H), 6.83 (dd, J = 8.7, 2.7 Hz, 1H), 6.23 (d, J = 26.4 Hz, 3H), 5.33 (s, 2H), 4.25 (d, J = 4.8 Hz, 2H), 3.63 (d, J = 10.4 Hz, 2H), 3.56 (d, J = 8.4 Hz, 2H), 2.71 (d, J = 10.5 Hz, 2H), 2.34 (s, 3H), 2.21 (s, 3H).

Example 8. N-(4-Carbamoylbenzyl)-1-(2-cyano-4-(6,6-difluoro-3-azabicyclo[3.1.0]hexan-3-yl)benzyl)-1H-pyrazole-4-carboxamide (Compound 8) was prepared according to the following synthetic scheme:

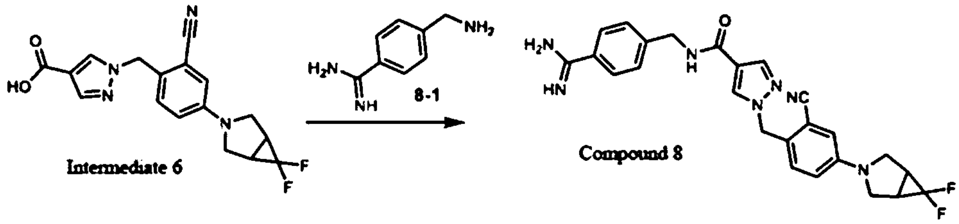


Intermediate 6

Compound 8

To a mixture of Intermediate 6 (82 mg, 0.24 mmol) and Intermediate 8-1 (80 mg, 0.36 mmol) in DMF (3 mL) was added DIEA (93 mg, 0.72 mmol) and HATU (91 mg, 0.24 mol) and the mixture was stirred at room temperature for 2 hours. After monitoring the completion of the reaction by LCMS, the reaction mixture was diluted with water (100 mL) and extracted with DCM (100 mL * 3). The organic layer was washed with brine and water, and dried over $Na_2SO_4$. The organic phase was filtered and the filtrate was concentrated under reduced pressure. The residue was purified by preparative HPLC (gradient elution, water/acetonitrile 100/0 to 50/50, HCl) to give the title Compound 8 (27.61 mg HCl salt, 24.38% yield). LCMS(ESI)M/Z: 476.2[M+H]+; $^1$H NMR(400 MHz, DMSO) δ 9.33 (s, 2H), 9.12 (s, 2H), 8.84 (t, J = 5.9 Hz, 1H), 8.21 (s, 1H), 7.92 (s, 1H), 7.78 (d, J = 8.0 Hz, 2H), 7.49 (d, J = 8.0 Hz, 2H), 7.33 (d, J = 8.4 Hz, 1H), 6.95 (d, J = 2.4 Hz, 1H), 6.84 (dd, J = 8.7, 2.4 Hz, 1H), 5.37 (s, 2H), 4.47 (d, J = 5.6 Hz, 2H), 3.64 (d, J = 10.4 Hz, 2H), 3.57 (d, J = 9.2 Hz, 2H), 2.72 (d, J = 10.8 Hz, 2H).

Example 9. N-((6-amino-2,4-dimethylpyridin-3-yl)methyl)-1-(3-chloroquinolin-6-yl)methyl-1H-1,2,3-triazole-4-carboxamide (Compound 9) was prepared according to the following synthetic scheme:

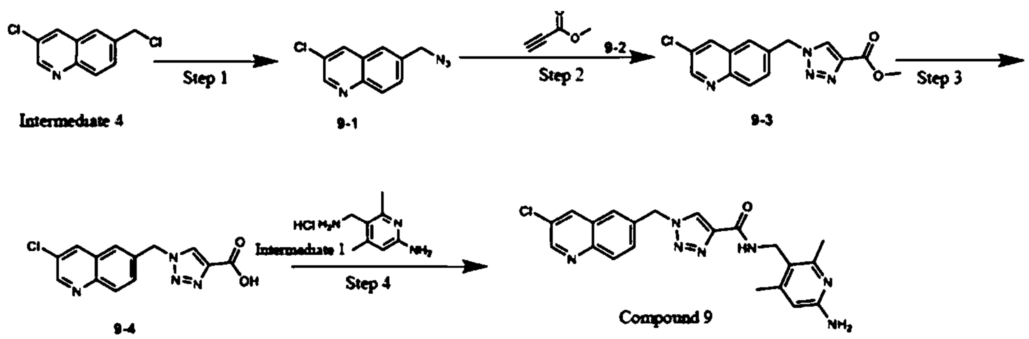

Intermediate 4, 9-1, 9-3, 9-4, Compound 9

Step 1: To a solution of Intermediate 4 (300 mg, 1.41 mmol, 1.00 eq) in DMF (5.00 mL) was added $NaN_3$ (100 mg, 1.54 mmol, 1.09 eq), then the mixture was stirred at 20°C for 12 h. The LCMS showed that Intermediate 4 was consumed and the desired mass was detected. The reaction was diluted with $NaHCO_3$ (sat., 10.0 mL) and then extracted with EtOAc (15.0 mL × 2), and the combined organic layers were washed with water (20.0 mL × 1) and brine (30.0 mL × 1), dried over $Na_2SO_4$, filtered and concentrated under reduced pressure to give Compound 9-1 (260 mg, 1.19 mmol, 84.1% yield) as a yellow solid. LCMS(ESI): m/z 219 [M+1]+; $^1$H NMR (400 MHz, $CDCl_3$) δ 8.85 (d, J = 2.4 Hz, 1H), 8.26 - 8.02 (m, 2H), 7.83 - 7.59 (m, 2H), 4.57 (s, 2H).

Step 2: To a mixture of Compound 9-1 (270 mg, 1.23 mmol, 1.00 eq) and Compound 9-2 (103.82 mg, 1.23 mmol, 103 μL, 1.00 eq) in $H_2O$ (5.00 mL) and t-BuOH (5.00 mL) added Vitamin C sodium salt (1 M, 1.23 mL, 1.00 eq) and $CuSO_4$ (2 M, 123 μL, 0.20 eq), and the reaction mixture was stirred at 20°C for 12 hours. LCMS showed that Compound 9-1 was consumed and the desired mass was detected. The reaction mixture was filtered and the filter cake was concentrated to afford the desired Compound 9-3 (330 mg, 1.09 mmol, 88.3% yield) as a yellow solid. LCMS(ESI): m/z 303 [M+1]+; $^1$H NMR (400 MHz, DMSO-$d_6$) δ 8.97 (s, 1H), 8.90 (d, J = 2.4 Hz, 1H), 8.61 (d, J = 2.0 Hz, 1H), 8.07 (d, J = 8.8 Hz, 1H), 7.87 (s, 1H), 7.76 (dd, $J_1$ = 8.8 Hz, $J_2$ = 1.6 Hz, 1H), 5.90 (s, 2H), 3.83 (s, 3H).

Step 3: To a solution of Compound 9-3 (300 mg, 991 μmol, 1.00 eq) in MeOH (5.00 mL) was added a solution of NaOH (79.3 mg, 1.98 mmol, 2.00 eq) in $H_2O$ (5 mL) at 0°C, and the mixture was then stirred at 50°C for 1 h. LCMS showed that Compound 9-3 was consumed and the desired MS was detected. The reaction mixture was concentrated to remove MeOH, then the pH was adjusted to 6 with 1 M HCl, and then the mixture was concentrated to give the desired Compound 9-4 (400 mg, crude) as a yellow solid. LCMS(ESI): m/z 289 [M+1]+; $^1$H NMR (400 MHz, DMSO-$d_6$) δ 9.10 - 8.76 (m, 2H), 8.64 - 8.53 (m, 1H), 8.12 - 7.73 (m, 3H), 6.25 - 5.66 (m, 2H).

Step 4: To a mixture of Compound 9-4 (100 mg, 346 μmol, 1.00 eq) and Intermediate 1 (78.0 mg, 416 μmol, 1.20 eq, HCl) in DMF (8 mL) was added HATU (395 mg, 1.04 mmol, 3.00 eq) and DIEA (224 mg, 1.73 mmol, 302 μL, 5.00 eq), and then the mixture was stirred at 20°C for 1h. LCMS showed that Compound 9-4 was consumed and the desrired mass was detected. The reaction was diluted with $H_2O$ (5.0 mL) and then extracted with EtOAc (10.0 mL × 2), the combined organic layers were washed with water (20.0 mL × 2) and brine (30.0 mL × 1), dried over $Na_2SO_4$, filtered and concentrated under reduced pressure to obtain a residue. The residue was purified by Prep-HPLC (column: Waters Xbridge C18 150 * 50 mm * 10 um; mobile phase: [water ($NH_4HCO_3$) -MeCN]; gradient: 15% -45% B over 10 min) to give the title Compound 9 (0.0250 g, 58.9 μmol, 17.0% yield, 99.4% purity) as a yellow solid. LCMS(ESI): m/z 422 [M+1]+; $^1$H NMR(400 MHz, $CDCl_3$) δ 8.87 (d, J = 2.0 Hz, 1H), 8.22 - 8.10 (m, 2H), 8.06 (s, 1H), 7.69 - 7.62 (m, 1H), 7.59 (dd, J = 2.0, 8.8 Hz, 1H), 7.09 - 6.98 (m, 1H), 6.30 (s, 1H), 5.76 (s, 2H), 4.55 (d, J = 5.2 Hz, 2H), 2.53 (s, 3H), 2.33 (s, 3H).

Example 10. N-((6-amino-2,4-dimethylpyridin-3-yl)methyl)-1-((3-chloroquinolin-6-yl)methyl)-1H-pyrazole-4-carboxamide (Compound 10) was prepared according to the following synthetic scheme:

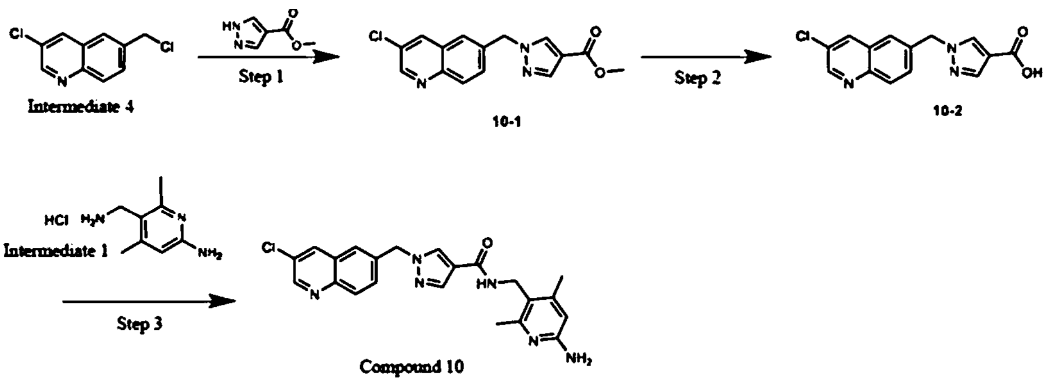


Step 1: To a solution of Intermediate 4 (200 mg, 0.94 mmol) and methyl 1H-pyrazole-4-carboxylate (143 mg, 1.12 mmol) in DMF (3 mL) was added $K_2CO_3$ (300 mg, 2.16 mmol) and the mixture was stirred at room temperature for 2 h. After the reaction was monitored for completion by LCMS, the reaction mixture was diluted with $H_2O$ (30 mL), extracted with EtOAc (50 mL * 3) and dried over anhydrous $Na_2SO_4$. The organic phase was filtered and the filtrate was concentrated under reduced pressure. The residue was purified by Prep-TLC (EtOAc) to give Compound 10-1 (180 mg, 63% yield) as a white solid. LCMS (ESI): m/z 302 [M + H]+.

Step 2: To a solution of Compound 10-1 (180 mg, 0.60 mmol) in MeOH (4 mL) and $H_2O$ (1 mL) was added NaOH (96 mg, 2.39 mmol) at 0°C, and the mixture was stirred at room temperature for 2 h. After LCMS monitored the completion of the reaction, the pH of the reaction mixture was adjusted to 4-5 with 1.0 M hydrochloric acid solution. The reaction mixture was diluted with $H_2O$ (20 mL) and extracted with DCM: MeOH = 10.1 (20 mL * 3) and dried over anhydrous $Na_2SO_4$. The organic phase was filtered and the filtrate was concentrated under reduced pressure to give the desired Compound 10-2 (146 mg, 85% yield) as a white solid. LCMS(ESI): m/z 288[M + H]+.

Step 3: To a mixture of Compound 10-2 (120 mg, 416 μmol, 1.00 eq) and Intermediate 1 (93 mg, 500 μmol, 1.20 eq, HCl) in DMF (10 mL) was added HATU (474 mg, 1.2 mmol, 3.00 eq) and DIEA (269 mg, 2.07 mmol, 363 μL, 5.00 eq), and then the mixture was stirred at 20°C for 1h. LCMS showed that Compound 10-2 was consumed and the desired mass was detected. The reaction was diluted with $H_2O$ (5.0 mL) and then extracted with EtOAc (10.0 mL × 2), the combined organic layers were washed with water (20.0 mL × 2) and brine (30.0 mL × 1), dried over $Na_2SO_4$, filtered and concentrated under reduced pressure to obtain a residue. The residue was purified by Prep-HPLC (column: Waters Xbridge C18 150 * 50 mm * 10 um; mobile phase: [water ($NH_4HCO_3$) -MeCN]; gradient: 15% -45% B over 10 min) to give the title Compound 10 (30 mg, 20% yield) as a yellow solid. LCMS(ESI): m/z 421 [M+1]+; $^1$H NMR(400 MHz, $CDCl_3$) δ 8.88 (d, J = 2.0 Hz, 1H), 8.24 - 8.12 (m, 2H), 8.08 (s, 1H), 7.94 (s, 1H), 7.68 - 7.63 (m, 1H), 7.59 (dd, J = 2.0, 8.8 Hz, 1H), 7.09 - 6.98 (m, 1H), 6.32 (s, 1H), 5.76 (s, 2H), 4.56 (d, J = 5.2 Hz, 2H), 2.55 (s, 3H), 2.31 (s, 3H).

Example 11. N-((6-amino-2,4-dimethylpyridin-3-yl)methyl)-1-(4-((2-oxopyridin-1(2H)-yl)methyl)benzyl)-1H-1,2,4-triazole-3-carboxamide (Compound 11) was prepared according to the following synthetic scheme:

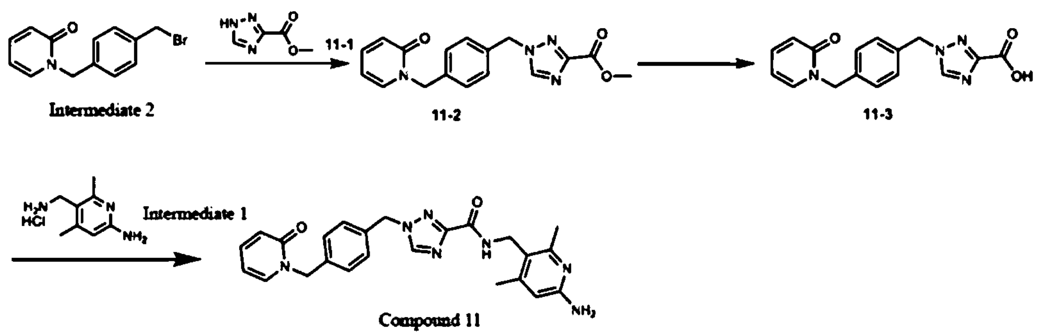


Step 1: To a solution of Intermediate 2 (400 mg, 1.89 mmol, 1.00 eq) in DMF (10.0 mL) was added $K_2CO_3$ (521 mg, 3.77 mmol, 2.00 eq) and Compound 11-1 (240 mg, 1.89%mmol, 1.00eq). The mixture was stirred at 50°C for 12 hours. LCMS showed that Compound 11-1 was consumed and the desired MS was detected. The reaction mixture was diluted with $H_2O$ (10.0 mL) and then extracted with EtOAc (20.0 mL * 2) and the combined organic layers were washed with water (30.0 mL * 1) and brine (30.0 mL * 1), dried over $Na_2SO_4$, filtered and concentrated under reduced pressure to give a

residue. The residue was purified by preparative TLC (petroleum ether/ethyl acetate = 0:1, Rf1 = 0.14, Rf2 = 0.48) to give Compound 11-2 (120 mg, 21.0% yield) as a white solid. LCMS(ESI): m/z 325[M+1]+.

Step 2: To a solution of Compound 11-2 (100 mg, 330 μmol, 1.00 eq) in MeOH (3.00 mL) was added NaOH (4 M, 165 μL, 2.00 eq). The mixture was stirred at 20°C for 2 h. LCMS showed that Compound 11-2 was consumed and the desired MS was detected. The reaction mixture was diluted with THF (10.0 mL) and then concentrated under reduced pressure to give Compound 11-3 (120 mg, crude) as a white solid. LCMS(ESI): m/z 309[M-1]-.

Step 3: To a solution of Compound 11-3 (48.0 mg, 144 μmol, 1.00 eq) in DMF (3.00 mL) was added Intermediate 1 (26.2 mg, 173 μmol, 1.20 eq), HATU (82.3 mg, 217 μmol, 1.50 eq) and TEA (43.8 mg, 433 μmol, 60.3 μL, 3.00 eq). The mixture was stirred at 25°C for 2 hours. TLC (DCM: MeOH = 10:1) indicated that Compound 11-3 was completely consumed. The reaction mixture was partitioned between DCM (40.0 mL) and $H_2O$ (40.0 mg). The organic phase was separated, dried over $Na_2SO_4$, filtered and concentrated under reduced pressure to give a residue. The residue (plate 2, DCM: MeOH = 10:1) was purified by preparative TLC ($SiO_2$, DCM: MeOH = 10:1) to afford the title Compound 11 (21.7 mg, 48.7 μmol, 33.8% yield, 99.6% purity) as a white solid. LCMS(ESI): m/z 444 [M+1]+; $^1H$ NMR δ 7.98 (s, 1H), 7.36 - 7.32 (m, 2H), 7.30 - 7.28 (m, 3H), 7.26 - 7.25 (m, 1H), 6.93 (s, 1H), 6.61 (d, J = 8.0 Hz, 1H), 6.25 (s, 1H), 6.19 - 6.16 (m, 1H), 5.36 (s, 2H), 5.14 (s, 2H), 4.58 - 4.57 (m, 4H), 2.47 (s, 3H), 2.29 (s, 3H).

Example 12. N-(4-carbamoylbenzyl)-1-(4-((2-oxopyridin-1(2H)-yl)methyl)benzyl)-1H-pyrazole-4-carboxamide (Compound 12) was prepared according to the following synthetic scheme:

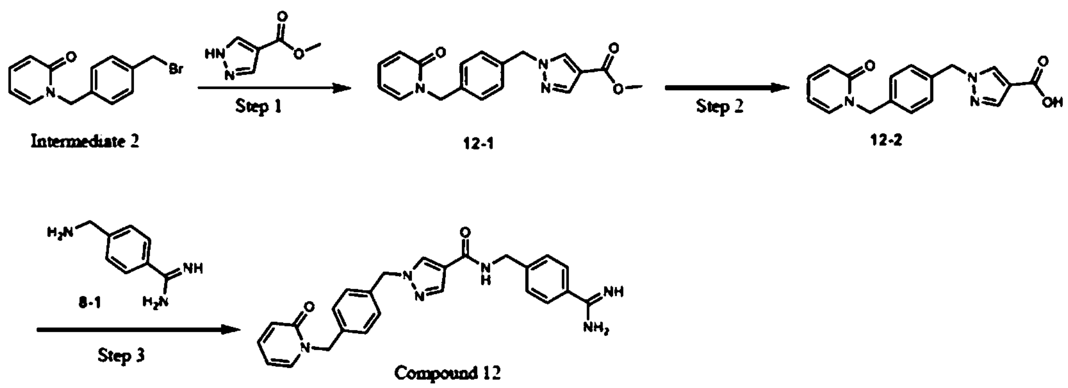


Step 1: To a solution of Intermediate 2 (200 mg, 0.72 mmol) and methyl 1H-pyrazole-4-carboxylate (110 mg, 0.86 mmol) in DMF (3 mL) was added $K_2CO_3$ (300 mg, 2.16 mmol) and the mixture was stirred at room temperature for 2 h. After the reaction was monitored for completion by LCMS, the reaction mixture was diluted with $H_2O$ (30 mL) and extracted with EtOAc (50 mL * 3) and dried over anhydrous $Na_2SO_4$. The organic phase was filtered and the filtrate was concentrated under reduced pressure. The residue was purified by Prep-TLC (EtOAc) to give Compound 12-1 (120 mg, 51.7% yield) as a white solid. LCMS (ESI): m/Z 324.2 [M + H]+.

Step 2: To a solution of Compound 12-1 (120 mg, 0.37 mmol) in MeOH (4 mL) and $H_2O$ (1 mL) was added NaOH (89 mg, 2.22 mmol) at 0°C, and the mixture was stirred at room temperature for 2 h. After LCMS monitored the completion of the reaction, the pH of the reaction mixture was adjusted to 4-5 with 1.0 M hydrochloric acid solution. The reaction mixture was diluted with $H_2O$ (20 mL) and extracted with DCM: MeOH = 10.1 (20 mL * 3) and dried over anhydrous $Na_2SO_4$. The organic phase was filtered and the filtrate was concentrated under reduced pressure to give the desired Compound 12-2 (100 mg, 87.7% yield) as a white solid. LCMS(ESI): m/z 310.1[ M + H ]+.

Step 3: To a mixture of Compound 12-2 (100 mg, 0.32 mmol) and Compound 8-1 (48 mg, 0.32 mmol) in DMF (3 mL) was added HATU (120 mg, 0.32 mmol) and TEA (160 mg, 1.60 mmol). The mixture was stirred at room temperature for 2 h. After monitoring the completion of the reaction by LCMS, the reaction mixture was diluted with $H_2O$ (20 mL) and extracted with DCM: MeOH = 10:1 (20 mL * 3) and dried over anhydrous sodium sulfate. The organic phase was filtered and the filtrate was concentrated under reduced pressure. The residue was purified by Prep-TLC (DCM: MeOH = 7:1) to afford the title Compound 12 (20.06 mg, 14.1% yield) as a white solid. LCMS(ESI): m/z 441.2[M + H]+; $^1H$ NMR (400 MHz, DMSO) δ 9.32 (s, 2H), 9.11- 9.01 (m, 2H), 8.83 (m, 1H), 8.28 (s, 1H), 7.92 (s, 1H), 7.78 - 7.77 (m, 3H), 7.51-7.49 (m, 2H), 7.44 - 7.40 (m, 1H), 7.26 - 7.23 (m, 4H), 6.42-6.39 (m, 1H), 6.23 - 6.22 (m, 1H), 5.32 (s, 2H), 5.08 (s, 2H), 4.54 - 4.47 (m, 2H).

Example 13. N-((6-amino-2,4-dimethylpyridin-3-yl)methyl)-4-(4-((2-oxopyridin-1(2H)-yl)methylbenzyl)pyridine carboxamide (Compound 13) was prepared according to the following synthetic scheme:

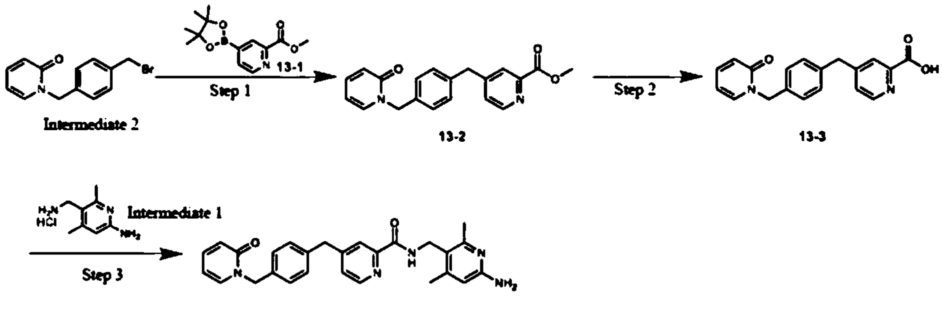


Intermediate 2, 13-2, 13-3, Compound 13

Step 1: Intermediate 2 (200 mg, 719 μmol, 1.00 eq), Compound 13-1 (227 mg, 862 μmol, 1.20 eq), $Pd(t\text{-}Bu_3P)_2$ (36.8 mg, 71.9 μmol, 0.10 eq) and DIEA (371 mg, 2.88 mmol, 500 μL, 4.00 eq) in 1,4-dioxane (2 mL) and $H_2O$ (0.4 mL) were placed in a microwave tube. The sealed tube was heated under microwave at 110°C for 1 hour. LC-MS showed that desired mas of product was detected. The reaction mixture was concentrated under reduced pressure to remove 1,4-dioxane and $H_2O$. The crude product 13-2 (70 mg) was purified by preparative TLC. LCMS(ESI): m/z 335 [M+1]+.

Step 2: To a solution of Compound 13-2 (70.0 mg, 209 μmol, 1.00 eq) in MeOH (1.00 mL) and $H_2O$ (1.00 mL) was added NaOH (41.9 mg, 1.05 mmol, 5.00 eq). The mixture was stirred at 25°C for 2 hours. LC-MS showed that the desired mass was detected. The mixture was concentrated under reduced pressure to remove MeOH, and then the pH was adjusted to 6-7 with 1 M HCl. And then the mixture was concentrated under reduce pressure to obtain a crude product. Compound 13-3 was obtained as a white solid (60.0 mg, 187 μmol, 89.5% yield). LCMS(ESI): m/z 321 [M+1]+.

Step 3: To a solution of Compound 13-3 (60.0 mg, 187 μmol, 1.00 eq) in DMF (2.00 mL) was added Intermediate 1 (35.2 mg, 187 μmol, 1.00 eq, HCl), HATU (106 mg, 280 μmol, 1.50 eq) and TEA (94.7 mg, 936 μmol, 130 μL, 5.00 eq). The mixture was stirred at 25°C under $N_2$ atmosphere for 12 h. LC-MS showed that the desired mass was detected. $NaHCO_3$ (5.00 mL) was added to the mixture and stirred for 0.5 h. It was then diluted with 10.0 mL $H_2O$ and extracted with 30.0 mL DCM (10.0 mL * 3). The combined organic layers were washed with 20.0 mL (10.0 mL * 2) brine, dried over $Na_2SO_4$, filtered and concentrated under reduced pressure to give a residue. By preparative HPLC (column: Waters xbridge 150 * 25mm * 10 um; mobile phase: [water ($NH_4HCO_3$-MeCN]; gradient: 20% -50% B over 10 min) gave the title Compound 13 (21.0 mg, 44.5 μmol, 23.7% yield, 96.0% purity) as a white solid. LCMS(ESI): m/z 454 [M+1]+; $^1H$ NMR δ (400 MHz, $CDCl_3$ δ 8.36-8.35 (d, J = 4.8 Hz, 1H), 8.05 (s, 1H), 7.86 (s, 1H), 7.32-7.27 (m, 1H), 7.26-7.25 (m, 2H), 7.23-7.14 (m, 3H), 6.62-6.60 (d, J = 8 Hz, 1H), 6.22 (s, 1H), 6.17-6.14 (m, 1H), 5.11 (s, 2H), 4.56-4.55 (d, J = 5.2 Hz, 2H), 4.37 (s, 2H) , 2.46 (s, 3H) , 2.28 (s, 3H).

Example 14. 1-(4-((1H-pyrazol-1-yl)methyl)benzyl)-N-((6-amino-2,4-dimethylpyridin-3-yl)methyl)-3-(methoxymethyl)-1H-pyrazol-4-carboxamide (Compound 14)

Compound 14 was prepared according to the following synthetic scheme:

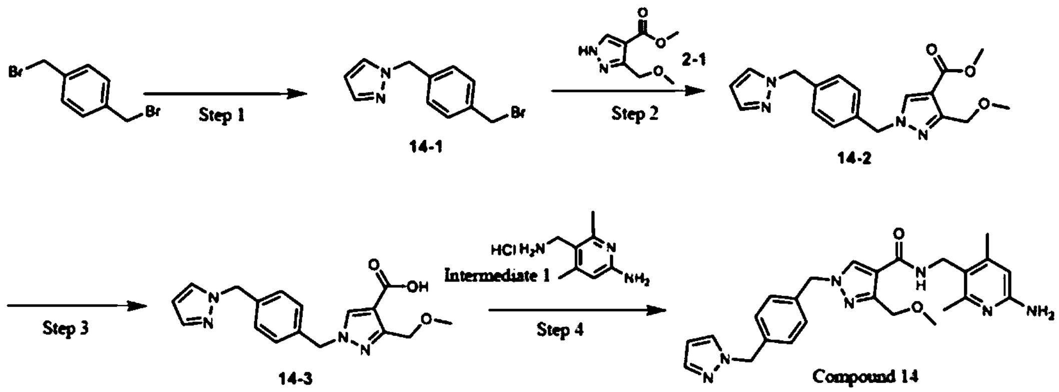


Step 1: To a solution of pyrrole (0.25 g, 3.67 mmol, 1.00 eq) in THF (10.0 mL) was added NaH (293 mg, 7.34 mmol, 60.0% purity, 2.00 eq) and 1,4-bis(bromomethyl)benzene (1.00 g, 3.79 mmol, 1.03 eq) at 0°C. The reaction mixture was stirred at 25°C for 2 hours. TLC (petroleum ether: ethyl acetate = 3:1) showed that the pyrrole was consumed. The residue was diluted with $H_2O$ (30.0 mL) and extracted with EtOAc (50.0 mL). The combined organic layers were washed with brine (30.0 mL), dried over $Na_2SO_4$, filtered and concentrated under reduced pressure to give a residue. The residue (plate 1, petroleum ether: ethyl acetate = 3:1) was purified by column chromatography ($SiO_2$, petroleum ether: ethyl acetate = 20:1 to 3:1). Compound 14-1 was obtained as a white solid (300 mg, 1.19 mmol, 32.5% yield). LCMS(ESI): m/z 251[M+1]+.

Step 2: To a solution of Compound 14-1 (200 mg, 796 μmol, 1.00 eq) in DMF (4.00 mL) was added $K_2CO_3$ (220 mg, 1.59 mmol, 2.00 eq) and Compound 2-1 (136 mg, 796 umol, 1.00 eq). The mixture was stirred at 25°C for 2 hours. TLC (plate 1, dichloromethane: methanol = 10:1) indicated that reactant 1 was completely consumed and two new spots were formed. The residue was diluted with $H_2O$ (30.0 mL) and extracted with EtOAc (50.0 mL). The combined organic layers were washed with brine (30.0 mL), dried over $Na_2SO_4$, filtered and concentrated under reduced pressure to give a residue. The residue (dichloromethane: methanol = 10:1) was purified by preparative TLC ($SiO_2$, dichloromethane: methane = 10:1). Compound 14-2 was obtained as a white solid (90.0 mg, 264 μmol, 33.2% yield). LCMS(ESI): m/z 341 [M+1]+; $^1H$ NMR (400 MHz, DMSO-$d_6$) δ 8.41 (s, 1H), 7.80 (s,1H), 7.44 (s,1H), 7.25 - 7.22 (m, 2H), 7.19 - 7.17 (m, 2H), 6.25 (s,1H), 5.33 - 5.30 (m, 4H), 4.49 (s, 2H), 3.72 (s, 3H), 3.34 (s, 1H).

Step 3: A solution of Compound 14-2 (50.0 mg, 147 μmol, 1.00 eq) in MeOH (1.00 mL) and $H_2O$ (0.20 mL) was added NaOH (23.5 mg, 588 μmol, 4.00 eq) at 0°C. The mixture was stirred at 25°C for 4 hours. LC-MS showed that the desired mass was detected. The reaction mixture was concentrated under reduced pressure to give a residue. The residue was diluted with water (10.0 mL) and adjusted to pH = 3 with HCl (1.00 M) and then concentrated under reduced pressure to give a residue. Compound 14-3 was obtained as a white solid (45.0 mg, 138 μmol, 93.9% yield). LCMS (ESI): m/z 327 [M+1]+.

Step 4: To a solution of Compound 14-3 (45.0 mg, 138 μmol, 1.00 eq) in DMF (2.00 mL) was added Intermediate 1 (25.0 mg, 165 μmol, 1.20 eq), TEA (41.9 mg, 414 μmol, 57.6 μL, 3.00 eq) and HATU (78.6 mg, 207 μmol, 1.50 eq). The mixture was stirred at 25°C for 2 hours. LC-MS showed that the desired mass was detected. The reaction mixture was partitioned between EtOAc (20.0 mL) and $H_2O$ (20.0 mL). The organic phase was separated, dried over $Na_2SO_4$, filtered and concentrated under reduced pressure to give a residue. The residue was purified by preparative HPLC (column: Waters Xbridge 150 * 25mm * 5 um; mobile phase: [water ($NH_4HCO_3$)-ACN]; gradient: 16%-46% B). The title Compound 14 was obtained as a white solid (23.29 mg, 50.03 μmol, 36.28% yield, 98.71% purity). LCMS(ESI): m/z 460 [M+1]+; $^1H$ NMR (400 MHz, $CDCl_3$)δ 7.90 (s, 1H), 7.88 (s, 1H), 7.55 - 5.54 (m, 1H), 7.39 - 7.38 (m, 1H), 7.21 - 7.17 (m, 4H), 6.29 - 6.25 (m, 2H), 5.32 (s, 2H), 5.21 (s, 2H), 4.53 - 4.45 (m, 6H), 3.13 (s, 3H), 2.48 (s, 3H), 2.30 (s, 3H).

Using the appropriate starting materials, the compounds shown in the following table were prepared using procedures analogous to those described in Examples 2, 3, 5, and 6.

Table 1.

| NO. | Structural formula | Mass spectrum ESI (+) m/z [M + 1]+ |
|---|---|---|
| 22 | 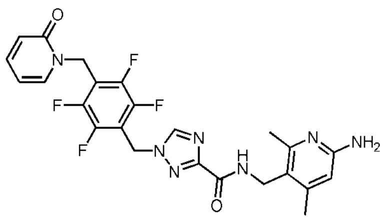 | 516.2 |

(continued)

| NO. | Structural formula | Mass spectrum ESI (+) m/z [M + 1]+ |
|---|---|---|
| 23 | 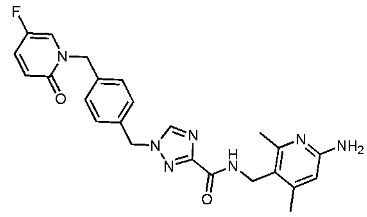 | 462.2 |
| 24 | 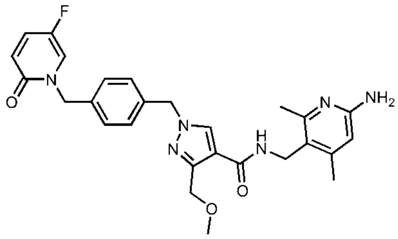 | 505.2 |
| 26 | 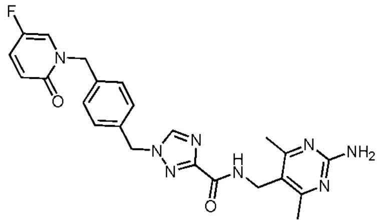 | 463.2 |
| 27 | 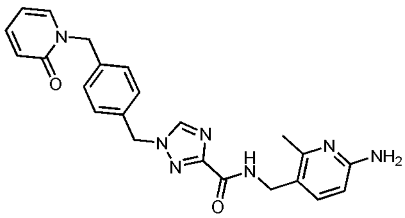 | 430.3 |
| 28 | 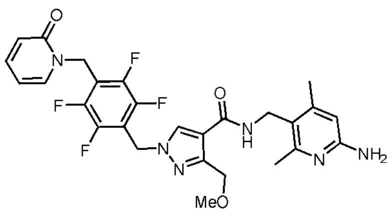 | 559.2 |
| 29 | 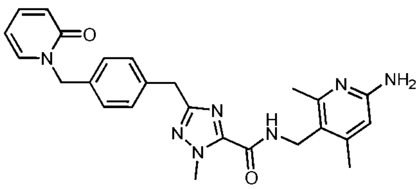 | 458.2 |
| 30 | 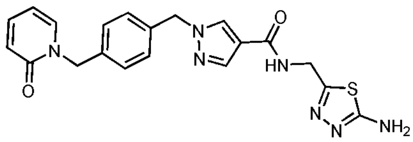 | 422.2 |

(continued)

| NO. | Structural formula | Mass spectrum ESI (+) m/z [M + 1]+ |
|---|---|---|
| 31 | 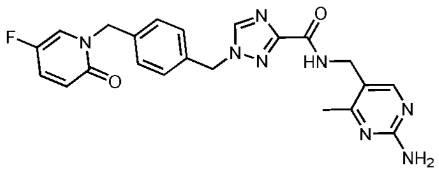 | 449.2 |
| 36 | 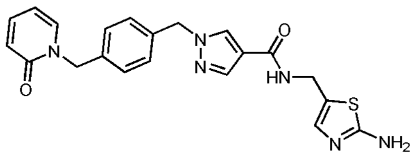 | 421.1 |
| 44 | 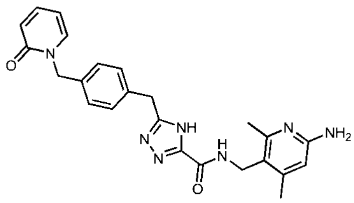 | 444.2 |
| 48 | 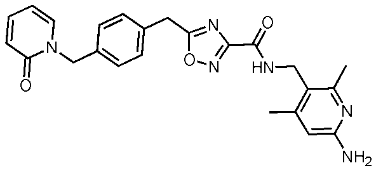 | 445.2 |
| 49 | 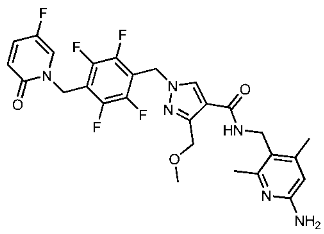 | 577.2 |
| 50 | 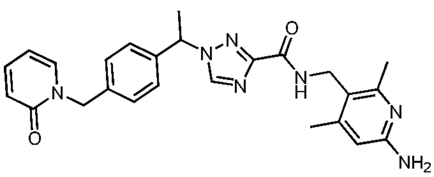 | 458.2 |
| 54 | 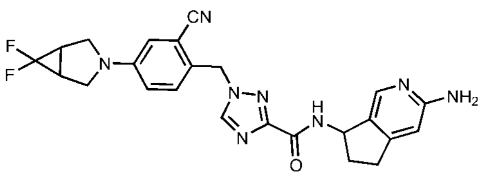 | 477.2 |
| 55 | 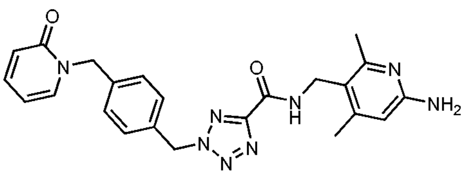 | 445.2 |

(continued)

| NO. | Structural formula | Mass spectrum ESI (+) m/z [M + 1]+ |
|---|---|---|
| 56 | 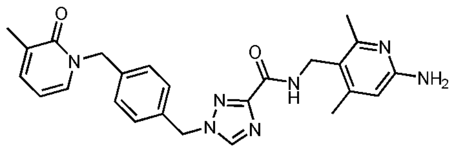 | 458.5 |
| 60 | 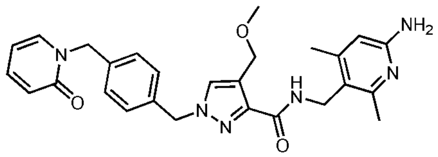 | 487.2 |
| 61 | 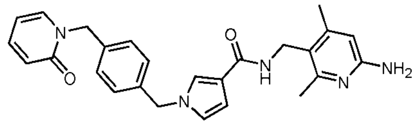 | 442.2 |
| 62 | 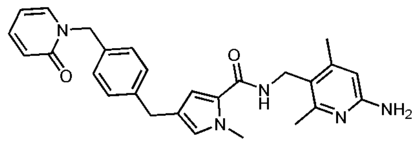 | 456.2 |
| 63 | 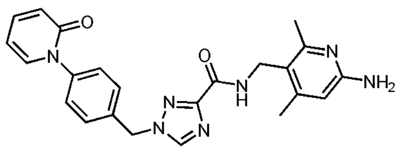 | 430.2 |
| 64 | 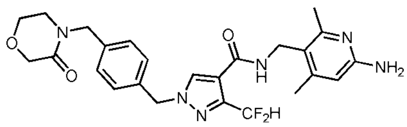 | 499.2 |
| 67 | 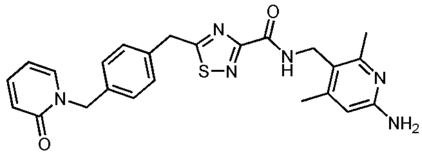 | 461.2 |
| 68 | 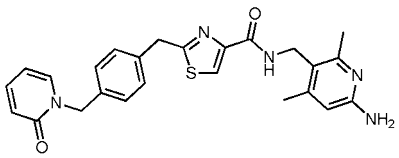 | 460.2 |
| 69 | 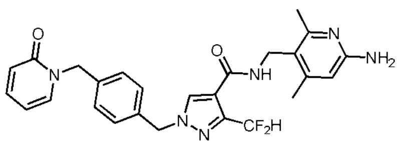 | 493.2 |
| 71 | 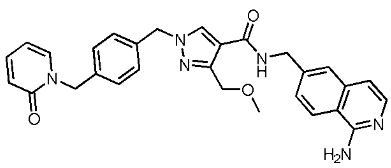 | 509.2 |

(continued)

| NO. | Structural formula | Mass spectrum ESI (+) m/z [M + 1]+ |
|---|---|---|
| 73 | 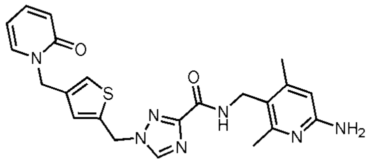 | 450.2 |
| 74 | 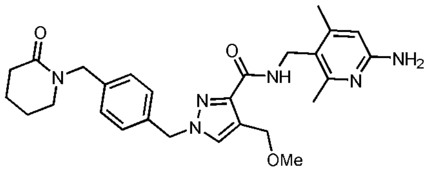 | 491.3 |
| 75 | 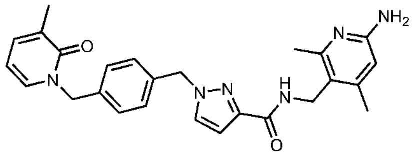 | 457.2 |
| 76 | 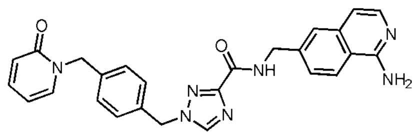 | 466.2 |
| 78 | 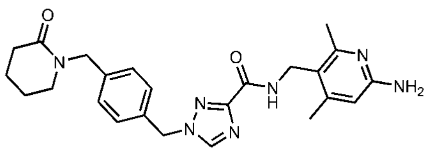 | 448.2 |
| 80 | 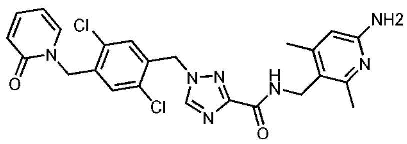 | 512.1 |
| 81 | 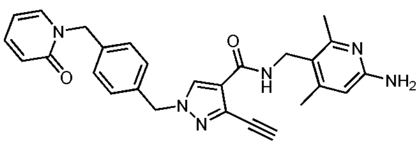 | 467.2 |
| 84 | 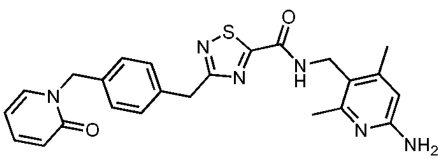 | 461.2 |
| 90 | 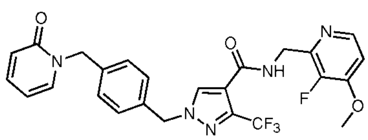 | 516.2 |
| 94 | 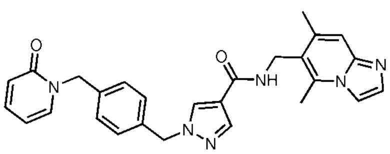 | 467.2 |

(continued)

| NO. | Structural formula | Mass spectrum ESI (+) m/z [M + 1]+ |
|---|---|---|
| 97 | 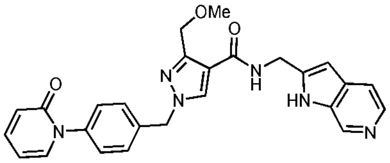 | 469.2 |
| 100 | 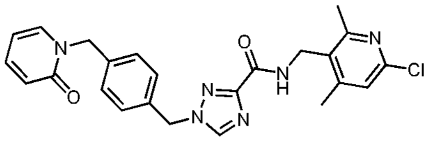 | 463.2 |
| 108 | 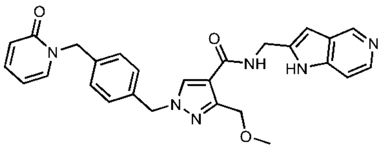 | 483.2 |
| 114 | 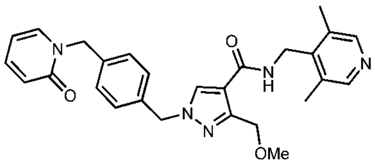 | 472.2 |
| 116 | 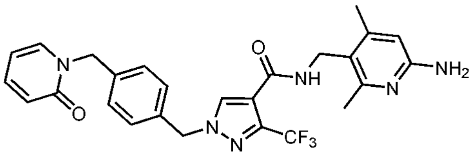 | 511.2 |
| 182 | 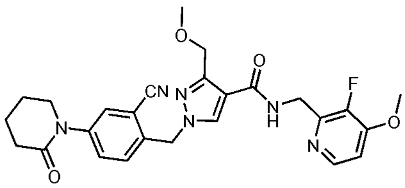 | 507.2 |
| 187 | 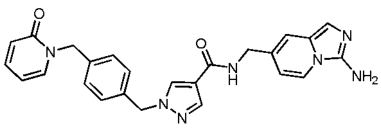 | 454.2 |
| 189 | 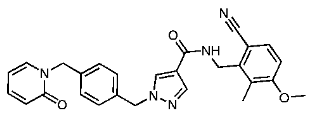 | 468.2 |
| 190 | 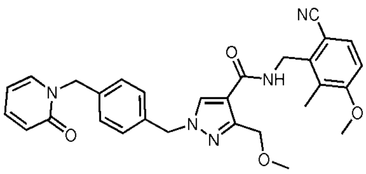 | 512.2 |
| 197 | 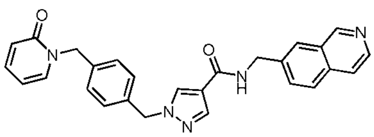 | 450.2 |

(continued)

| NO. | Structural formula | Mass spectrum ESI (+) m/z [M + 1]+ |
|---|---|---|
| 202 | 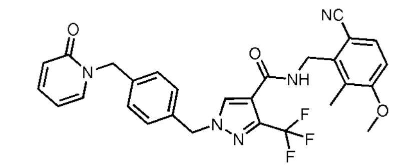 | 536.2 |
| 207 | 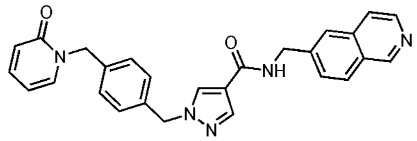 | 450.2 |
| 214 | 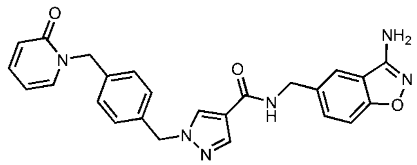 | 455.2 |
| 215 | 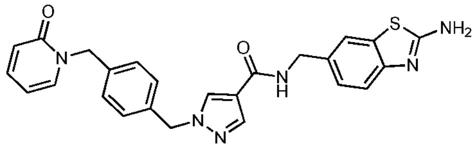 | 471.1 |
| 250 | 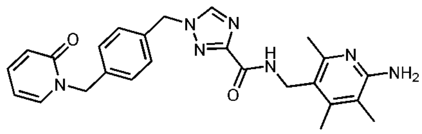 | 458.2 |
| 253 | 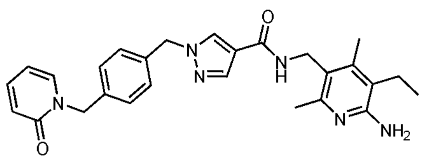 | 471.2 |
| 256 | 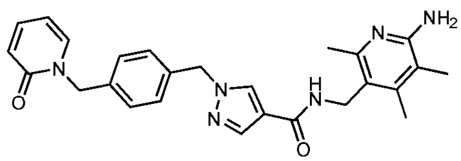 | 457.2 |
| 257 | 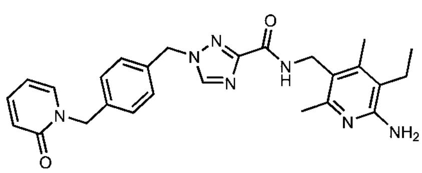 | 472.2 |
| 280 | 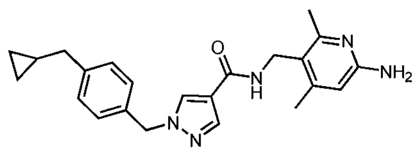 | 390.2 |

Example 15. N-(4-carbamoylbenzyl)-1-((6-cyclopropylimidazo[1,2-a]pyridin-2-yl)methyl)-1H-pyrazole-4-carboxamide (Compound 15) was prepared according to the following synthetic scheme:

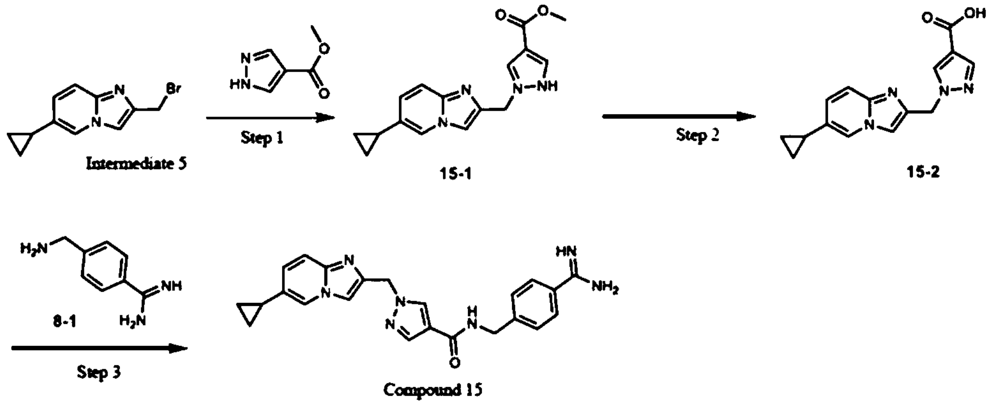


Step 1: To a solution of Intermediate 5 (0.2 g, 0.8 mmol) in DMF (3 mL) was added methyl 1H-pyrazole-4-carboxylate (0.12 g, 0.96 mmol) and $K_2CO_3$ (0.33 g, 2.4 mmol) and the mixture was stirred at room temperature overnight. After completion of the reaction was monitored by LCMS, the mixture was diluted with water (100 mL) and extracted with DCM (100 mL * 3). The composite organic layer was filtered through celite, and the filtrate was concentrated under reduced pressure. The residue was purified by silica gel (EtOAc elution) chromatography to afford the desired Compound 15-1 (0.123 g, 52.12% yield) as a yellow solid. LCMS (ESI): m/z 297.6[M + H]+.

Step 2: NaOH (0.17 g, 4.2 mmol) was added to Compound 15-1 (0.123 g, 0.42 mmol) in MeOH (3 mL) and water (1.5 mL), stirring at room temperature for 1 h. After LCMS monitored the completion of the reaction, the pH of the reaction mixture was adjusted to 4-5 with 1.0 M hydrochloric acid solution. The mixture was extracted with DCM (100 mL * 3). The combined organic layer was filtered through celite and the filtrate was concentrated under reduced pressure to give the desired Compound 15-2 (0.1 g, 85.34% yield) as a yellow solid. LCMS (ESI): m/z 283.5[M + H]+.

Step 3: To Compound 15-2 (0.1 g, 0.35 mmol) in DMF (3 mL) was added Compound 8-1 (0.052 g, 0.35 mmol), DIEA (0.068 g, 0.52 mmol) and HATU (0.13 g, 0.35 mmol), and the mixture was stirred at room temperature for 2 h. After the reaction was monitored for completion by LCMS, the reaction mixture was diluted with water (100 mL) and extracted with DCM (50 mL * 3). The composite organic layer was filtered through celite, and the filtrate was concentrated under reduced pressure. The residue was purified by chromatography on silica gel (eluting with DCM: MeOH = 10:1) to give the title Compound 15 (28.69 mg, 19.59% yield) as a white solid. LCMS(ESI): m/z 414.1[M + H]+; $^1$H NMR (400 MHz, DMSO-d6) δ 9.11 (d, J = 129.3 Hz, 3H), 8.77 (s, 1H), 8.35 (s, 1H), 8.23 (s, 1H), 7.89 (s, 1H), 7.80 - 7.69 (m, 3H), 7.49 (d, J = 8.0 Hz, 2H), 7.41 (d, J = 9.1 Hz, 1H), 7.02 (d, J = 9.1 Hz, 1H), 5.42 (s, 2H), 4.47 (d, J = 5.6 Hz, 2H), 1.93 (s, 1H), 0.92 (d, J = 6.9 Hz, 2H), 0.67 (d, J = 4.6 Hz, 2H).

Example 16. N-(4-carbamoylbenzyl)-1-(4-(6,6-difluoro-3-azabicyclo[3.1.0]hexan-3-yl)-2-ethylbenzyl)-1H-pyrazole-4-carboxamide (Compound 16) was prepared according to the following synthetic scheme:

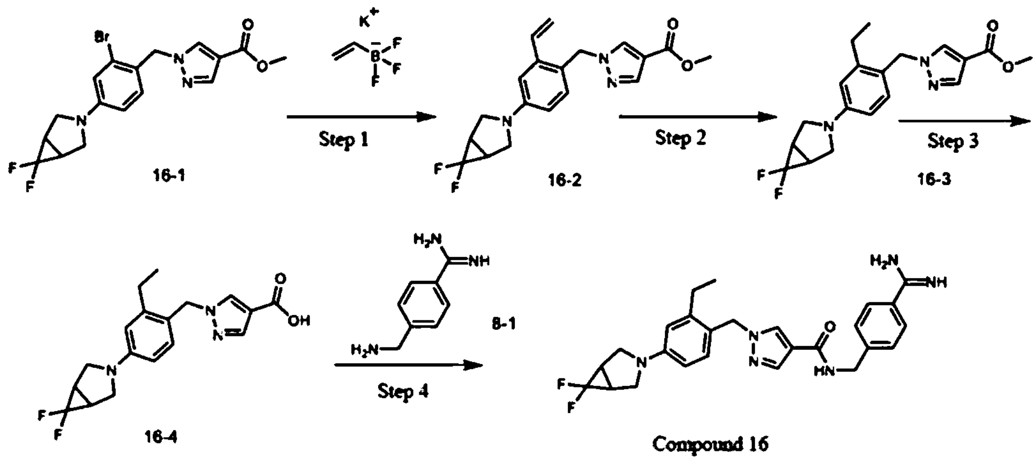


Step 1: To a solution of Compound 16-1 (160 mg, 0.39 mmol) in dioxane (10 mL) was added potassium trifluorovinylborate (79 mg, 0.58 mmol), $Na_2CO_3$ (124 mg, 1.17 mmol), $H_2O$ (2 mL) and $Pd(dppf)Cl_2$ (29 mg, 0.039 mmol).

The reaction mixture was flushed with nitrogen for 5 minutes. The mixture was stirred at 100°C for 2 h. After monitoring the completion of the reaction by LCMS, the mixture was diluted with water (50 mL) and extracted with EA (50 mL * 3). The combined organic layers were dried over anhydrous $Na_2SO_4$. The organic phase was filtered and the filtrate was concentrated under reduced pressure. The residue was purified by column chromatography (eluting with PE: EA = 3:1) to give the desired Compound 16-2 (106 mg, 75.99% yield) as a colorless oily liquid. MS (ESI) M/Z: 360.2 $[M+H]^+$.

Step 2: To a solution of Compound 16-2 (106 mg, 0.29 mmol) in MeOH (10 mL) was added Pd/C (31 mg, 0.29% mmol). The reaction mixture was flushed with $H_2$ for 5 min and protected with $H_2$. The mixture was stirred at room temperature for 2 hours. After monitoring the completion of the reaction by LCMS, the mixture was filtered and the filtrate was concentrated under reduced pressure. The residue gave the desired Compound 16-3 (100 mg, 93.81% yield) as a colorless oily liquid. MS (ESI) M/Z: 384.2 $[M+Na]^+$.

Step 3: A mixture of Compound 16-3 (100 mg, 0.28 mmol) in MeOH (2 mL) was treated with 2 M aqueous sodium hydroxide (1 mL) and the reaction mixture was stirred at room temperature for 16 h. After monitoring the completion of the reaction by LCMS, the reaction mixture was acidified to pH = 5-6 with 1 M HCl. The reaction was concentrated under reduced pressure to afford the desired Compound 16-4 (96 mg, 100% yield) as a white solid. MS (ESI) M/Z: 348.0 $[M+H]^+$.

Step 4: To a solution of Compound 16-4 (70 mg, 0.2 mmol) in DMF (2 mL) was added DIEA (78 mg, 0.6 mol) and HATU (92 mg, 0.24 mmol) and the mixture was stirred at room temperature for 10 min. 8-1 (45 mg, 0.3 mmol) was then added to the reaction. The mixture was stirred at room temperature for 1 hour. After monitoring the completion of the reaction by LCMS, the reaction mixture was concentrated under reduced pressure. The residue was purified by preparative TLC (DCM: MeOH = 15: 1) to give Compound 16 (5.54 mg, 5.74% yield) as a white solid. MS (ESI) M/Z: 479.1 $[M+H]^+$; HPLC: 254 nm: 98.558%,214 nm:98.700%; $^1$H NMR(400 MHz, DMSO-$d_6$) δ 9.28 (s, 2H), 8.98 (s, 2H), 8.74 (t, J = 6.0 Hz, 1H), 8.37 (s, 1H), 8.00 (s, 1H), 7.86 (s, 1H), 7.75 (d, J = 8.3 Hz, 2H), 7.47 (d, J = 8.3 Hz, 2H), 7.10 (d, J = 8.9 Hz, 1H), 6.38 (d, J = 6.9 Hz, 2H), 5.22 (s, 2H), 4.45 (d, J = 5.9 Hz, 2H), 3.58 (s, 2H), 3.50 (s, 2H), 2.67 (d, J = 10.7 Hz, 2H), 2.60 - 2.52 (m, 2H), 1.03 (t, J = 7.5 Hz, 3H).

Example 17. (R)-N-(1-ethyl-1,4,5,6-tetrahydrocyclopentadienyl[d]imidazol-4-yl)-3-(methoxymethyl)-1-(4-(2-oxopyridin-1(2H)-yl)methyl)benzyl)-1H-pyrazole-4-carboxamide (Compound 130) and (S)-N-(1-ethyl-1,4,5,6-tetrahydrocyclopentadienyl[d]imidazol-4-yl)-3-(methoxymethyl)-1-(4-(2-oxopyridin-1(2H)-yl)methyl)benzyl)-1H-pyrazole-4-carboxamide (Compound 131) were prepared according to the following synthetic scheme:

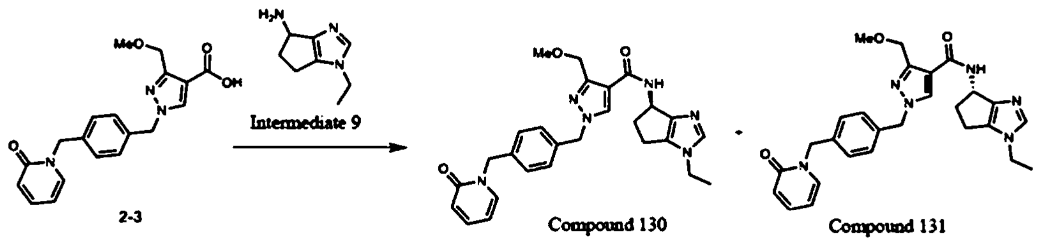


2-3

Compound 130 , Compound 131

To a solution of Compound 2-3 (45 mg, 0.13 mmol) in DMF (3 mL) was added HATU (54 mg, 0.14 mmol) and DIEA (51 mg, 0.39 mmol), and the mixture was stirred at room temperature for 10 min. Intermediate 9 (39 mg, 0.26 mmol) was then added to the reaction. The mixture was stirred at room temperature for 1 hour. After monitoring the completion of the reaction by LCMS, the reaction mixture was diluted with water (50 mL) and extracted with DCM (50 mL * 3). The combined organics were concentrated. The residue was purified by preparative TLC (DCM: MeOH = 10:1) to give the desired compound (32 mg, 51.64% yield) as a white solid. 32 mg of the compound was then subjected to SFC resolution (AD chiral column) to afford the title Compound 130 (retention time 3.92 min, 9.21 mg), MS (ESI) m/z: 487.3 $[M+H]^+$; HPLC: 254 nm: 998.453%, 214 nm: 98.272%; $^1$H NMR (400 MHz, DMSO) δ 8.23 (s, 1H), 8.02 (d, J = 7.8 Hz, 1H), 7.75 (dd, J = 6.7, 1.6 Hz, 1H), 7.50 (s, 1H), 7.40 (ddd, J = 8.9, 6.6, 2.0 Hz, 1H), 7.23 (q, J = 8.1 Hz, 4H), 6.39 (d, J = 9.0 Hz, 1H), 6.22 (t, J = 6.7 Hz, 1H), 5.25 (s, 2H), 5.07 (d, J = 7.4 Hz, 3H), 4.48 (s, 2H), 3.90 (q, J = 7.2 Hz, 2H), 3.15 (s, 3H), 2.85 (dt, J = 16.8, 6.4 Hz, 1H), 2.80 - 2.70 (m, 1H), 2.63 - 2.56 (m, 1H), 2.17 - 2.05 (m, 1H), 1.30 (t, J = 7.3 Hz, 3H) and the title Compound 131 (retention time 5.04 min, 9.46 mg) as a white solid, MS (ESI) m/z: 487.3 $[M + H]^+$; HPLC: 254 nm: 97.604%, 214 nm: 97.761%; $^1$H NMR400 MHz, DMSO) δ 8.23 (s, 1H), 8.03 (d, J = 7.8 Hz, 1H), 7.75 (dd, J = 6.8, 1.8 Hz, 1H), 7.50 (s, 1H), 7.41 (ddd, J = 8.9, 6.6, 2.0 Hz, 1H), 7.24 (q, J = 8.2 Hz, 4H), 6.39 (d, J = 9.2 Hz, 1H), 6.22 (td, J = 6.7, 1.3 Hz, 1H), 5.25 (s, 2H), 5.07 (d, J = 6.5 Hz, 3H), 4.48 (s, 2H), 3.90 (q, J = 7.1 Hz, 2H), 3.15 (s, 3H), 2.91 - 2.81 (m, 1H), 2.78 - 2.70 (m, 1H), 2.63 - 2.56 (m, 1H), 2.12 (ddd, J = 12.8, 8.4, 4.0 Hz, 1H), 1.30 (t, J = 7.3 Hz, 3H).

Using the appropriate starting materials, the compounds shown in the following table were prepared using procedures analogous to those described in Examples 2, 15, 16, and 17.

Table 2.

| NO. | Structural formula | Mass spectrum ESI (+) m/z [M + 1]+ |
|---|---|---|
| 17 | 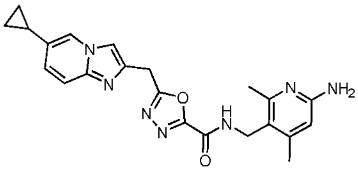 | 418.2 |
| 18 | 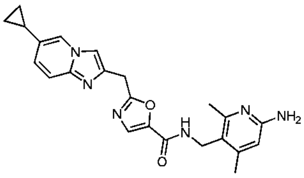 | 417.2 |
| 19 | 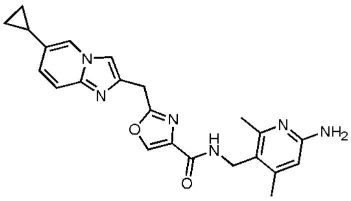 | 417.2 |
| 21 | 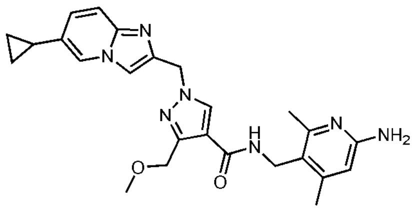 | 460.2 |
| 35 | 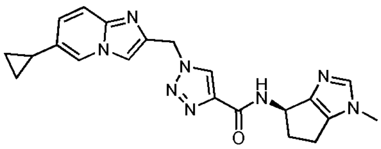 | 403.2 |
| 37 | 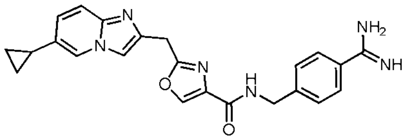 | 415.2 |
| 39 | 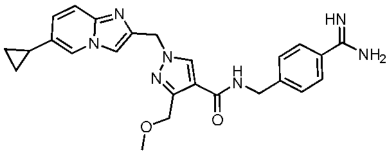 | 458.2 |
| 45 | 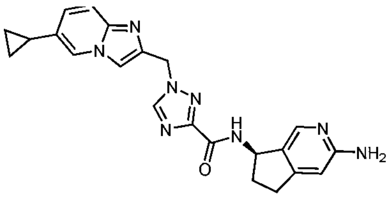 | 415.2 |
| 46 | 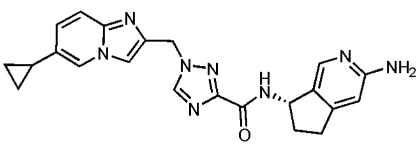 | 415.2 |

(continued)

| NO. | Structural formula | Mass spectrum ESI (+) m/z [M + 1]+ |
|---|---|---|
| 47 | 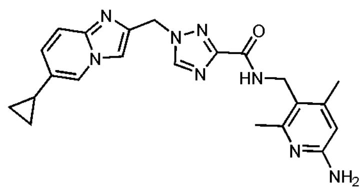 | 417.2 |

Example 18. N-((6,8-dimethylimidazo[1,5-a]pyridin-7-yl)methyl)-3-(methoxymethyl)-1-(4-(2-oxopyridin-1(2H)-yl)benzyl)-1H-pyrazole-4-carboxamide (Compound 147) was prepared according to the following synthetic scheme:

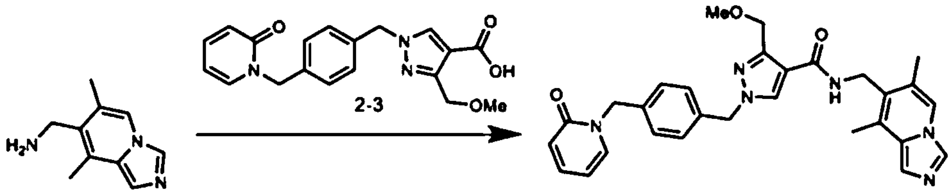


Intermediate 10

Compound 147

A mixture of Compound 2-3 (30 mg, 84.90 μmol, 1 eq), Intermediate 10 (20.0 mg, 114 μmol, 1.34 eq) and EDCI (48.8 mg, 255 μmol, 3.00 eq) in Py (1.00 mL) was stirred at 20°C for 2 h. LC-MS (EW47914-151-PIA1) showed that the desired mass was detected. The reaction mixture was filtered and concentrated under reduced pressure to give a residue. The residue was purified by preparative TLC (DCM/MeOH = 10/1). Compound 147 (7.39 mg, 13.6 μmol, 16.0% yield, 94.4% purity) was obtained as a pale yellow solid, which was confirmed by $^1$H NMR (EW47914-151-P1A), LC-MS (EW47914-51-P1B), and HPLC (EW4791451-P1J). MS (ESI) m/z: 511 $[M+H]^+$; $^1$H NMR: EW47914-151-P1A (400 MHz, $CDCl_3$) δ 8.07 (s, 1H), 7.96 - 7.87 (m, 2H), 7.69 (s, 1H), 7.41 (s, 1H), 7.35 - 7.29 (m, 2H), 7.28 - 7.28 (m, 1H), 7.25 (dd, $J_1$ = 1.8, $J_2$ = 6.8 Hz, 1H), 7.22 - 7.18 (m, 2H), 6.61 (d, J = 9.2 Hz, 1H), 6.18 - 6.14 (m, 1H), 5.21 (s, 2H), 5.13 (s, 2H), 4.52 (d, J = 4.8 Hz, 2H), 4.47 (s, 2H), 3.11 (s, 3H), 2.50 (s, 3H), 2.30 (s, 3H).

Example 19. N-((1H-pyrrolo[3,2-c]pyridin-2-yl)methyl)-1-(2-cyano-4-(6,6-difluoro-3-azabicyclo[3.1.0]hexan-3-yl)benzyl)-3-(methoxymethyl)-1H-pyrazole-4-carboxamide (Compound 157) was prepared according to the following synthetic scheme:

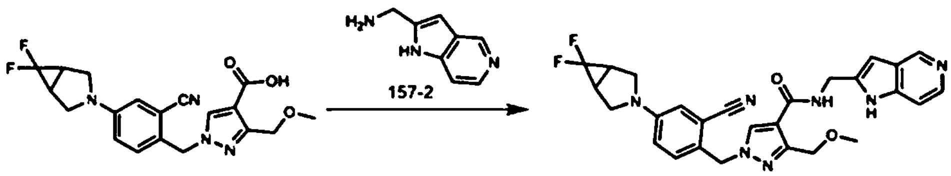


157-1

Compound 157

To a solution of 157-1 (prepared with reference to the synthesis of Intermediate 6) (23 mg, 0.059 mmol) in DMF (2 mL) was added DIEA (23 mg, 0.18 mmol) and HATU (27 mg, 0.071 mmol) and the mixture was stirred at room temperature for 10 min. 157-2 (10 mg, 0.065 mmol) was then added to the reaction. The mixture was stirred at room temperature for 1 hour. After monitoring the completion of the reaction by LCMS, the reaction mixture was concentrated under reduced pressure. The residue was purified by preparative TLC (DCM: MeOH = 12:1) to give the title compound (15.77 mg, 51.45% yield) as a white solid. MS (ESI) m/z: 518.3[m+H]+. $^1$H NMR(400 MHz, DMSO-d6) δ 11.46 (s, 1H), 8.65 (s, 1H), 8.47 (t, J = 5.5 Hz, 1H), 8.20 (s, 1H), 8.03 (d, J = 5.2 Hz, 1H), 7.42 (d, J = 5.3 Hz, 1H), 7.31 (d, J = 8.7 Hz, 1H), 6.96 (d, J = 2.5 Hz, 1H), 6.89 - 6.81 (m, 1H), 6.34 (s, 1H), 5.34 (s, 2H), 4.58 (d, J = 7.6 Hz, 4H), 3.64 (d, J = 10.4 Hz, 2H), 3.57 (d, J = 8.5 Hz, 2H), 3.22 (s, 3H), 2.72 (d, J = 10.7 Hz, 2H).

Example 20. N-((5,7-dimethylimidazo[1,2-a]pyridin-6-yl)methyl)-1-(4-((2-oxopyridin-1(2H)-yl)methyl]benzyl)-3-(trifluoromethyl)-1H-pyrazole-4-carboxamide (Compound 142) was prepared according to the following synthetic scheme:

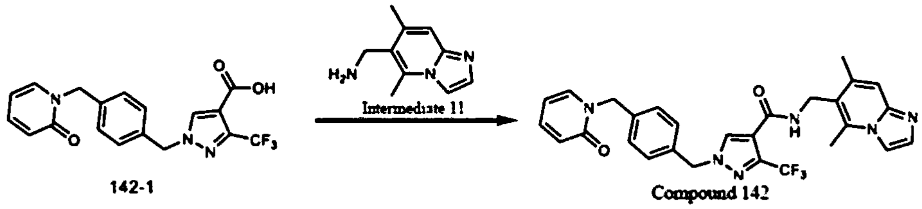


To a solution of 1-(4-((2-oxopyridin-1(2H)yl)methyl)benzyl)-3-(trifluoromethyl)-1H-pyrazole-4-carboxylic acid (prepared with reference to the synthesis of Intermediate 2-3), Compound 142-1 (45.28 mg, 0.12 mmol) in DMF (3 mL) was added HATU (45.63 mg, 0.12 mol) and DIEA (46.53 mg, 0.36 mmol), and the mixture was stirred at room temperature for 0.5 h. Intermediate 11 (31.54 mg, 0.18 mmol) was then added to the reaction. The mixture was stirred at room temperature for 2 hours. After monitoring the completion of the reaction by LCMS, the reaction mixture was concentrated under reduced pressure. The residue was purified by preparative TLC (DCM: MeOH = 15:1) to give the title compound (20.74 mg, 32.33% yield) as a white solid. MS(ESI) m/z: 535.2[m+H]+. $^1$H NMR (400 MHz, DMSO-d6) δ 8.35 (s, 1H), 8.32 (t, J = 4.8 Hz, 1H), 7.79 (s, 1H), 7.74 (dd, J = 6.6, 1.8 Hz, 1H), 7.54 (d, J = 1.2 Hz, 1H), 7.40 (ddd, J = 8.9, 6.6, 2.1 Hz, 1H), 7.32 (s, 1H), 7.25 (s, 4H), 6.38 (d, J = 9.2 Hz, 1H), 6.21 (td, J = 6.7, 1.3 Hz, 1H), 5.36 (s, 2H), 5.05 (s, 2H), 4.48 (d, J = 4.7 Hz, 2H), 2.62 (s, 3H), 2.38 (s, 3H); $^{19}$ F NMR (400 MHz, DMSO-d6) δ -59.52 (s).

Using the appropriate starting materials, the compounds shown in the following table were prepared using procedures analogous to those described in Examples 17, 18, 19, and 20.

Table 3.

| NO. | Structural formula | Mass spectrum ESI (+) m/z [M + 1]$^+$ |
|---|---|---|
| 20 | 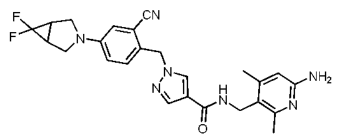 | 478.2 |
| 40 | 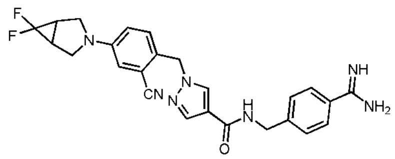 | 476.2 |
| 41 | 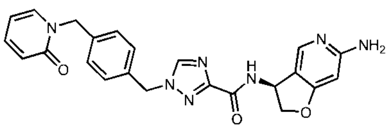 | 444.2 |
| 42 | 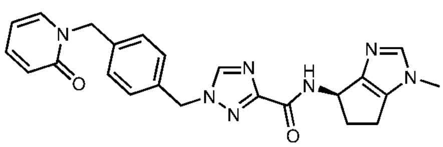 | 430.1 |
| 43 | 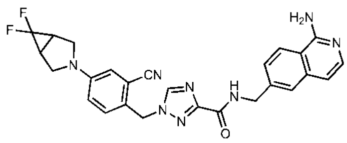 | 501.2 |

(continued)

| NO. | Structural formula | Mass spectrum ESI (+) m/z [M + 1]+ |
|---|---|---|
| 51 | 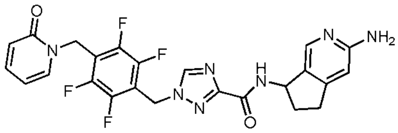 | 514.2 |
| 53 | 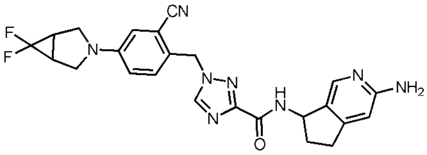 | 477.2 |
| 57 | 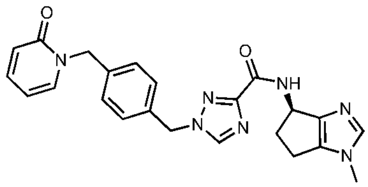 | 430.2 |
| 58 | 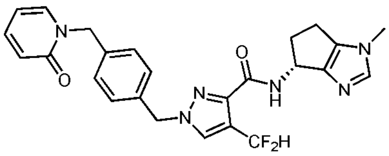 | 479.2 |
| 59 | 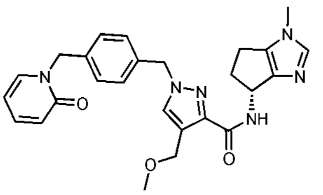 | 473.2 |
| 70 | 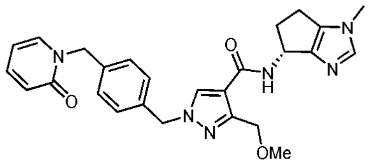 | 473.2 |
| 71 | 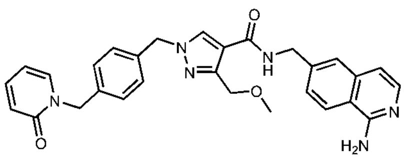 | 509.2 |
| 79 | 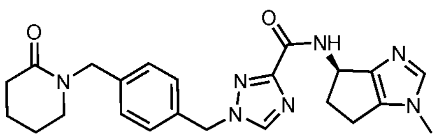 | 434.1 |
| 82 | 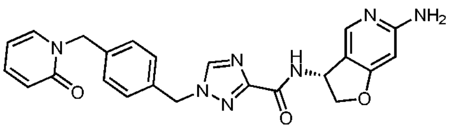 | 444.2 |

(continued)

| NO. | Structural formula | Mass spectrum ESI (+) m/z [M + 1]+ |
|---|---|---|
| 83 | 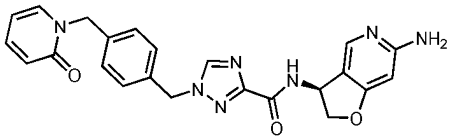 | 444.2 |
| 86 | 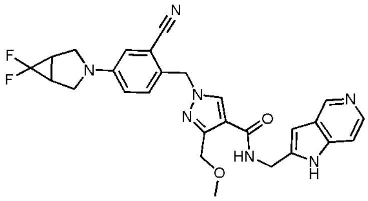 | 518.2 |
| 94 | 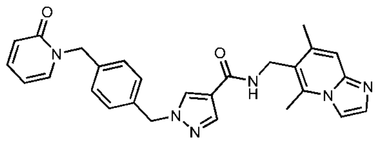 | 467.2 |
| 96 | 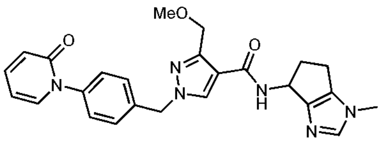 | 459.2 |
| 97 | 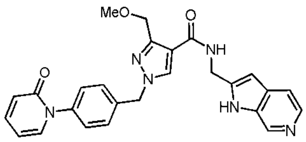 | 467.2 |
| 104 | 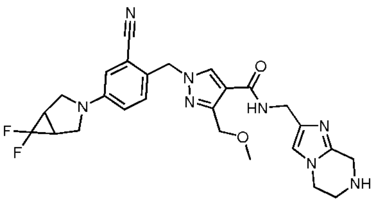 | 523.2 |
| 107 | 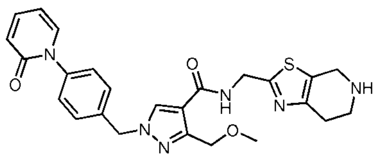 | 491.2 |
| 108 | 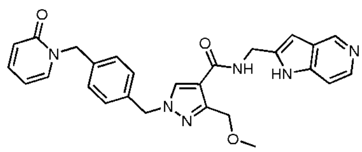 | 482.2 |
| 112 | 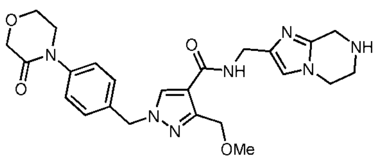 | 480.2 |

(continued)

| NO. | Structural formula | Mass spectrum ESI (+) m/z [M + 1]+ |
|---|---|---|
| 118 | 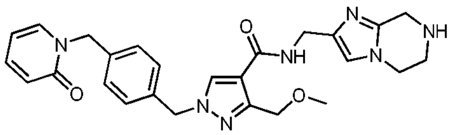 | 488.2 |
| 119 | 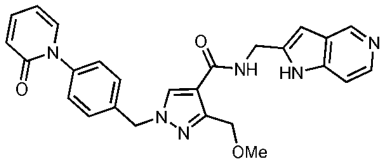 | 467.1 |
| 120 | 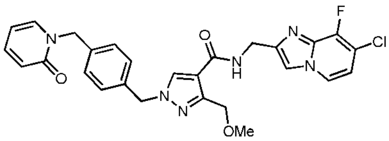 | 535.2 |
| 122 | 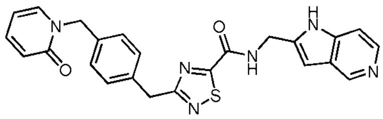 | 457.1 |
| 123 | 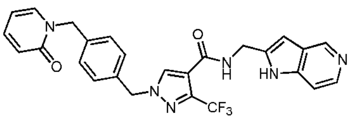 | 507.2 |
| 125 | 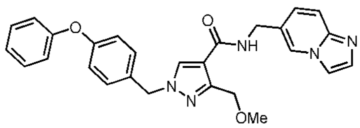 | 468.2 |
| 126 | 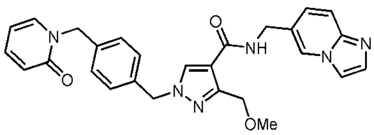 | 483.2 |
| 127 | 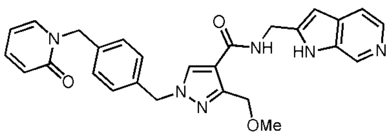 | 483.2 |
| 129 | 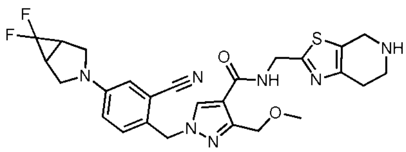 | 540.2 |
| 130 | 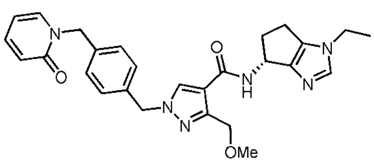 | 487.2 |

(continued)

| NO. | Structural formula | Mass spectrum ESI (+) m/z [M + 1]+ |
|---|---|---|
| 133 | 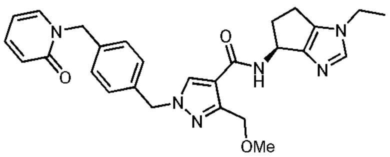 | 487.2 |
| 135 | 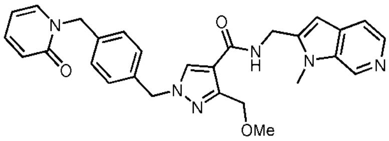 | 497.2 |
| 136 | 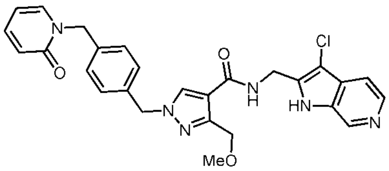 | 517.2 |
| 137 | 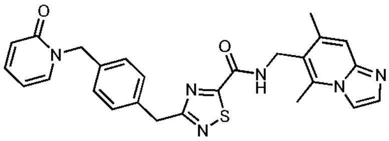 | 485.2 |
| 139 | 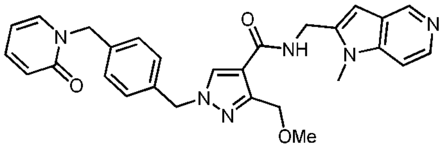 | 497.2 |
| 141 | 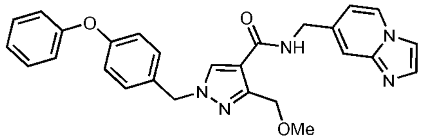 | 468.2 |
| 142 | 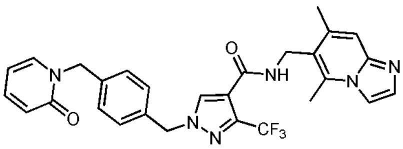 | 535.2 |
| 143 | 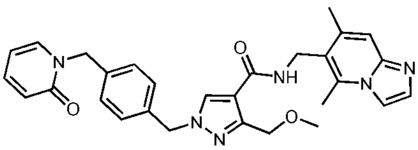 | 511.2 |
| 145 | 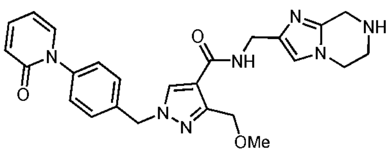 | 474.2 |
| 147 | 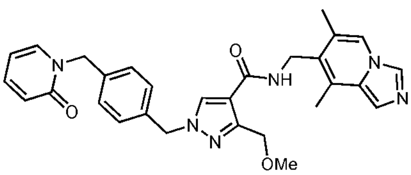 | 511.2 |

(continued)

| NO. | Structural formula | Mass spectrum ESI (+) m/z [M + 1]+ |
|---|---|---|
| 148 | 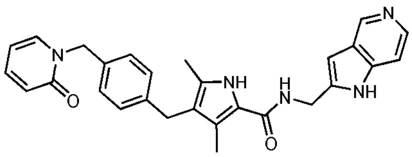 | 466.2 |
| 152 | 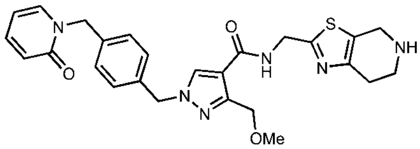 | 505.2 |
| 154 | 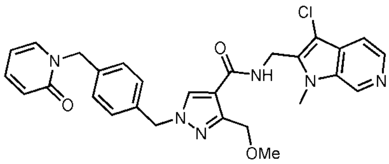 | 531.2 |
| 155 | 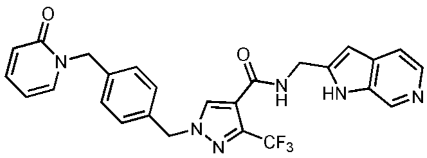 | 507.2 |
| 161 | 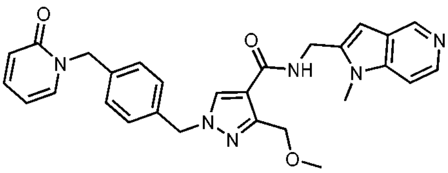 | 497.2 |
| 176 | 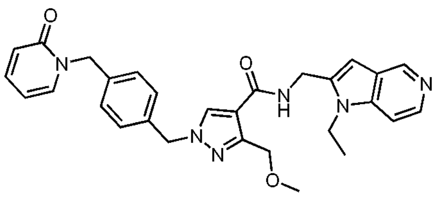 | 511.2 |
| 177 | 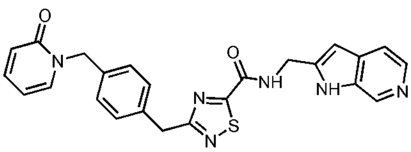 | 457.2 |
| 178 | 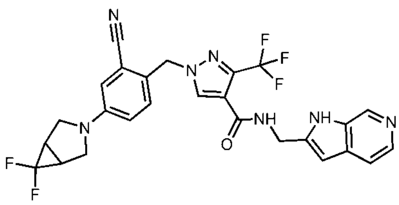 | 542.2 |
| 185 | 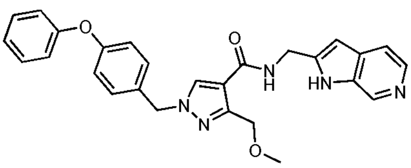 | 468.2 |

(continued)

| NO. | Structural formula | Mass spectrum ESI (+) m/z [M + 1]+ |
|---|---|---|
| 186 | 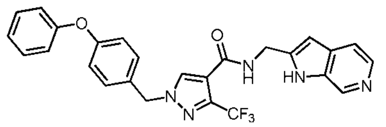 | 492.2 |
| 188 | 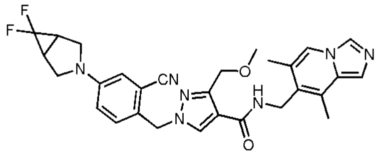 | 546.2 |
| 192 | 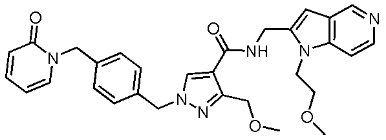 | 541.3 |
| 194 | 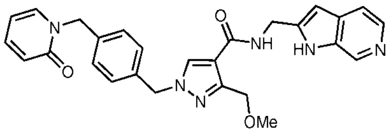 | 483.2 |
| 195 | 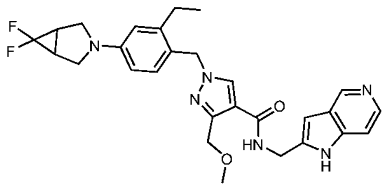 | 521.2 |
| 196 | 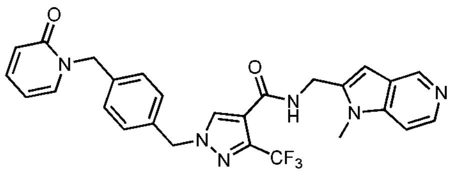 | 521.2 |
| 197 | 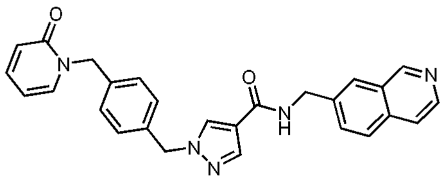 | 450.2 |
| 199 | 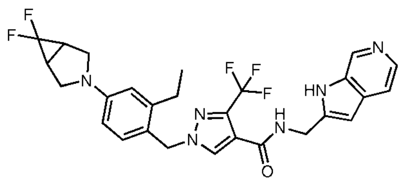 | 545.2 |
| 200 | 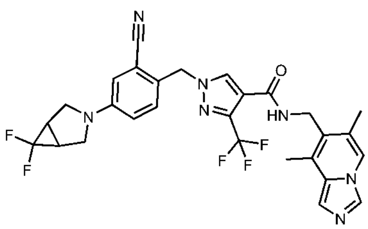 | 570.2 |

(continued)

| NO. | Structural formula | Mass spectrum ESI (+) m/z [M + 1]+ |
|---|---|---|
| 203 | 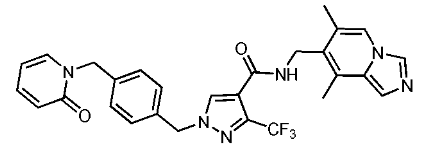 | 535.2 |
| 204 | 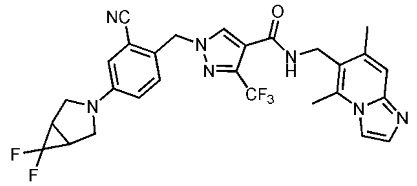 | 570.2 |
| 206 | 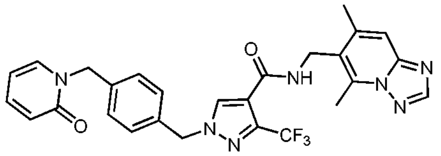 | 536.2 |
| 207 | 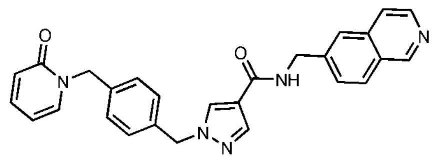 | 450.2 |

Example 21. N-((6-amino-2,4-dimethylpyridin-3-yl)methyl)-3-(difluoromethyl)-1-(4-(2-oxopyridin-1(2H)-yl)methylbenzyl)-1H-pyrazole-4-carboxamide (Compound 69) was prepared according to the following synthetic scheme:

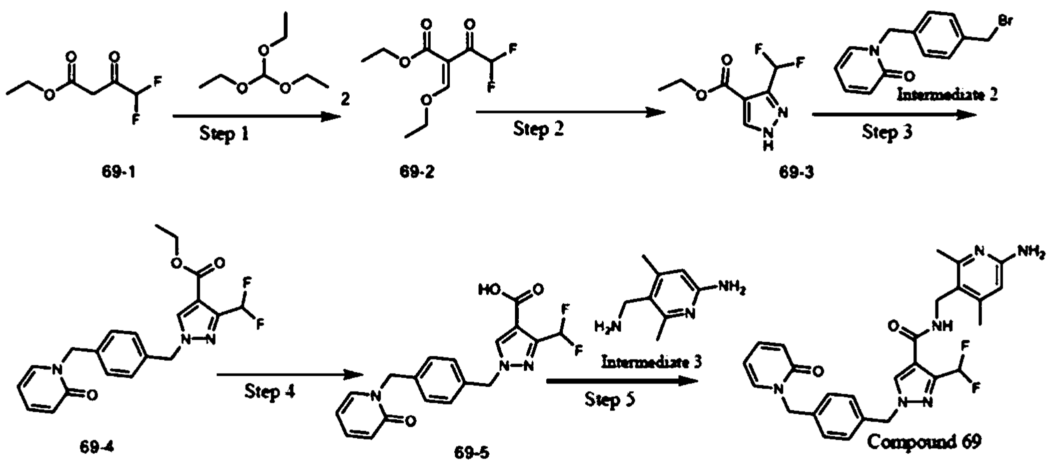


Step 1: Preparation of ethyl (Z)-2-(ethoxymethylene)-4,4-difluoro-3-oxobutanoate: To a solution of Compound 69-1 (2.0 g, 12.04 mmol) in acetic anhydride (20 mL) was added triethyl orthoformate 2 (3.57 g, 24.08 mmol) and the mixture was stirred at 100°C for 4 hours. After monitoring the completion of the reaction by LCMS, the reaction solution was concentrated to afford the desired title compound (2.67 g, 100% yield) as a yellow oil, which was used directly in the next reaction. MS (ESI) M/Z: 223.1[M+H]+.

Step 2: Preparation of ethyl 3-(difluoromethyl)-1H-pyrazole-4-carboxylate: To a solution of Compound 69-2 (2.67 g, 12.02 mmol) in EtOH (30 mL) was added hydrazine hydrate (0.7 mL, 14.42 mmol) and the mixture was stirred overnight at room temperature. After completion of the reaction, it was monitored with LCMS, the solvent was evaporated in vacuo, and the residue was purified by chromatography on a silica gel column (DCM: MeOH = 100:0 to 96:4) to give the title compound (1.74 g, yield 76.15%) as a white solid. MS (ESI) M/Z: 191.0[M+H]+.

Step 3: Preparation of ethyl 3-(difluoromethyl)-1-(4-((2-oxopyridin-1(2H)yl)methyl)benzyl)-1H-pyrazole-4-carboxylate: To a solution of Compound 69-3 (380 mg, 1.19 mmol) in DMF (10 mL) was added Intermediate 2 (500 mg, 1.18 mmol). The combined organic phases were concentrated and the residue was purified by preparative TLC (PE: EA = 1:1) to give the title compound (400 mg, 57.38% yield). MS (ESI) M/Z: 388.1[M+H]+; $^1H$ NMR(400 MHz, DMSO) δ 8.60 (s, 1H), 7.77 (dd, J = 6.7, 2.0 Hz, 1H), 7.41 (ddt, J = 8.7, 6.5, 2.1 Hz, 1H), 7.37 - 7.27 (m, 4H), 7.11 (dd, J = 53.6, 2.2 Hz, 1H), 6.41 (d, J = 9.1 Hz, 1H), 6.23 (td, J = 6.6, 1.3 Hz, 1H), 5.40 (s, 2H), 5.08 (d, J = 2.0 Hz, 2H), 4.23 (qd, J = 7.1, 2.2 Hz, 2H), 1.26 (td, J = 7.1, 2.2 Hz, 3H).

Step 4: Preparation of 3-(difluoromethyl)-1-(4-((2-oxopyridin-1(2H)yl)methyl)benzyl)-1H-pyrazole-4-carboxylic acid: To a solution of Compound 69-4 (50 mg, 0.13 mmol) in MeOH (2 mL) was added NaOH (2 M, 1 mL, 2 mmol), and the mixture was stirred at room temperature overnight. The reaction mixture was quenched by the addition of HCl solution (2 M, 1 mL, 2 mmol) and concentrated under reduced pressure to afford the title compound (162 mg, 100% yield) as a white solid. MS (ESI) M/Z: 360.2[M+H]+.

Step 5: Preparation of 1-(4-(cyclopropylmethyl)benzyl)-N-((3-fluoro-4-methoxypyridin-2-yl)methyl)-3-(methoxymethyl)-1H-pyrazole-4-carboxamide: To a solution of Compound 69-5 (45 mg, 0.13 mmol) in DMF (2 mL) was added HATU (59 mg, 0.16 mmol) and DIEA (50 mg, 0.39 mmol), and the mixture was stirred at room temperature for 10 min. Intermediate 3 (20 mg, 0.13 mmol) was then added to the reaction. The mixture was stirred at room temperature for 1 hour. After monitoring the completion of the reaction by LCMS, the reaction mixture was concentrated under reduced pressure. The residue was purified by preparative TLC (DCM: MeOH = 10:1) to give the title compound (43.54 mg, 70.59% yield) as a white solid. MS (ESI) M/Z: 493.4[M+H]+; HPLC: 254 nm: 95.822%, 214 nm: 97.754%; $^1H$ NMR(400 MHz, DMSO) δ 8.35 (s, 1H), 8.14 (s, 1H), 7.76 (dd, J = 6.8, 1.8 Hz, 1H), 7.50 - 7.14 (m, 6H), 6.39 (d, J = 9.1 Hz, 1H), 6.20 (dd, J = 27.4, 20.7 Hz, 4H), 5.35 (s, 2H), 5.07 (s, 2H), 4.25 (d, J = 4.6 Hz, 2H), 2.33 (s, 3H), 2.19 (s, 3H).

Example 22. 1-(4-(cyclopropylmethyl)benzyl)-N-((3-fluoro-4-methoxypyridin-2-yl)methyl)-3-(methoxymethyl)-1H-pyrazole-4-carboxamide (Compound 91) was prepared according to the following synthetic scheme:

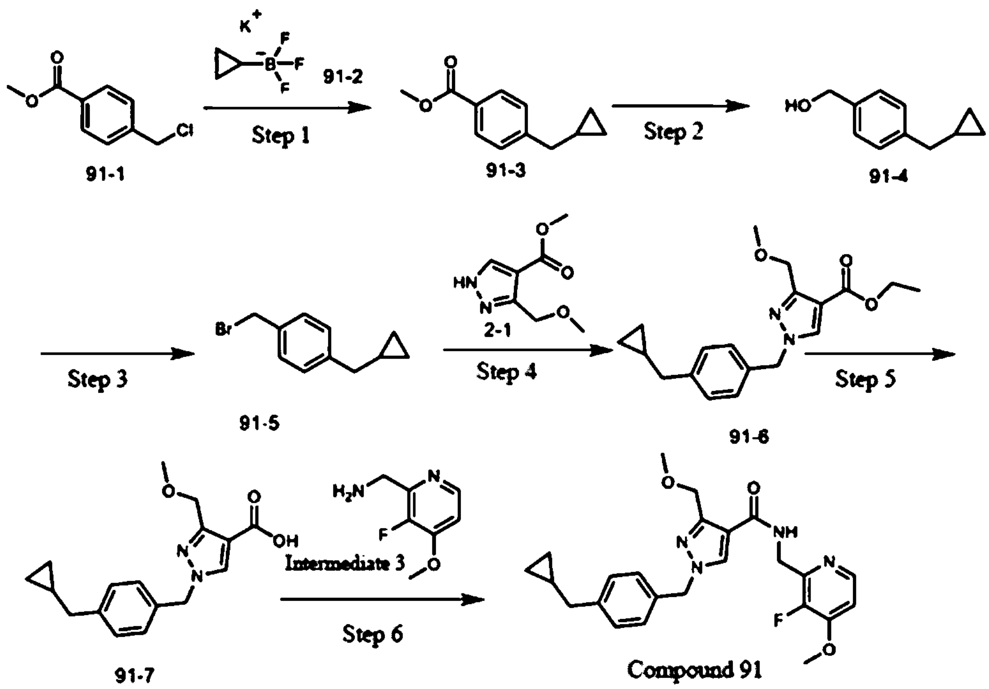


Step 1: Preparation of methyl 4-(cyclopropylmethyl)benzoate: To a solution of 4-methyl formate benzyl chloride (2 g, 10.83 mmol) in toluene (20 mL) and water (1 mL) was added potassium cyclopropyltrifluoroborate (1.92 g, 13.00 mmol), Ru phos (0.51 g, 1.08 mmol), $Pd_2(dba)_3$ (0.99 g, 1.08 mmol) and $K_2CO_3$ (2.99 g, 21.66 mmol) under $N_2$ and the mixture was stirred at 120°C for 4 h. The mixture was diluted with water (50 mL) and extracted with EA (50 mL * 3). The combined organic layers were filtered through a pad of celite and the filtrate was concentrated under reduced pressure. The residue was purified by silica gel chromatography (PE: EA = 20:1) to give the title compound (1.15 g, 55.80% yield) as a colorless oily liquid. MS (ESI) M/Z: 232.1[M+ACN+H]+.

Step 2: Preparation of (4-(cyclopropylmethyl)phenyl)methanol: Compound 91-3 (100 mg, 0.53 mmol) was

dissolved in THF (3 mL). After stirring at 0°C for 15 min, $LiAlH_4$ (30 mg, 0.80 mmol) was added to the reaction solution and the mixture was stirred at 0°C for 30 min. After monitoring the completion of the reaction by LCMS, water (2.0 mL) was added to the reaction solution and a large amount of white solids were precipitated. The reaction solution was dried by adding anhydrous sodium sulfate, filtered, and the filtrate was concentrated under reduced pressure. The obtained residue was purified by silica gel column chromatography (PE: EA = 10:1) to obtain the title compound (56 mg, yield 65.67%) as a colorless oily liquid.

MS (ESI) M/Z: 145.1[M-18+H]+.

Step 3: Preparation of 1-(bromomethyl)-4-(cyclopropylmethyl)benzene: To a solution of Compound 91-4 (50 mg, 0.31 mmol) in DCM (10 mL) was slowly added $PBr_3$ (125.87 mg, 0.46 mmol) at 0°C. The reaction mixture was stirred at room temperature for 1 hour. TLC showed that the reaction was complete. The mixture was diluted with water (20 mL) and extracted with DCM (20 mL * 3). The organic layer was washed 3 times with brine, dried over $Na_2SO_4$ and concentrated. The crude product was purified by column chromatography (PE: EA= 30:1) to give the title compound (45 mg, yield 64.86%) as a colorless oily liquid. $^1H$ NMR (400 MHz, DMSO-d6) δ 7.17 (d, J = 8.0 Hz, 2H), 7.03 (dd, J = 15.1, 6.3 Hz, 2H), 4.50 (s, 2H), 2.29 (s, 2H), 0.80 - 0.69 (m, 1H), 0.32 - 0.22 (m, 2H), 0.03 - -0.04 (m, 2H).

Step 4: Preparation of ethyl 1-(4-(cyclopropylmethyl)benzyl)-3-(methoxymethyl)-1H-pyrazole-4-carboxylate: To a solution of Compound 3 (35 mg, 0.16 mmol) in DMF (2 mL) was added 91-5 (32 mg, 0.18 mmol) and $K_2CO_3$ (44 mg, 0.32 mmol), and the mixture was stirred at room temperature for 1 h. After monitoring the completion of the reaction by LCMS, the mixture was diluted with water (50 mL), extracted with EA (50 mL * 3), and dried over $Na_2SO_4$. The combined organic layers were concentrated under reduced pressure. The residue was purified by preparative TLC (PE: EA = 5:1) to give the title compound (39 mg, 76.38% yield) as a colorless oily liquid. MS (ESI) M/Z: 329.2[M+H]+.

Step 5: Preparation of 1-(4-(cyclopropylmethyl)benzyl)-3-(methoxymethyl)-1H-pyrazole-4-carboxylic acid: To a solution of Compound 91-6 (40 mg, 0.12 mmol) in MeOH (2 mL) was added NaOH (2 M, 1 mL, 2 mmol) and the mixture was stirred at room temperature for 1 hour. After monitoring the completion of the reaction by LCMS, the reaction mixture was quenched by the addition of HCl solution (2 M, 1.05 mL, 2.1 mmol) and concentrated under reduced pressure to afford the title compound (70 mg, 100% yield) as a white solid. MS (ESI) m/z: 301.2[m+H]+.

Step 6: Preparation of 1-(4-(cyclopropylmethyl)benzyl)-N-((3-fluoro-4-methoxypyridin-2-yl)methyl)-3-(methoxymethyl)-1H-pyrazole-4-carboxamide:
N-(4-carbamoylbenzyl)-1-(4-(cyclopropylmethyl)benzyl)-1H-pyrazole-4-carboxamide (Compound 105) was prepared according to the following synthetic scheme: To a solution of Compound 91-7 (35 mg, 0.12 mmol) in DMF (1 mL) was added DIEA (47 mg, 0.36 mmol) and HATU (55 mg, 0.14 mmol), and the mixture was stirred at room temperature for 10 min. Intermediate 3 (21 mg, 0.13 mmol) was then added to the reaction. The mixture was stirred at room temperature for 1 hour. After monitoring the completion of the reaction by LCMS, the reaction mixture was concentrated under reduced pressure. The residue was purified by preparative TLC (DCM: MeOH = 15: 1) to give the title compound (15.76 mg, 30.84% yield) as a brown solid. MS (ESI) m/z: 439.4[m+H]+; HPLC: 254 nm: 98.800%, 214 nm: 98.780%; $^1H$ NMR(400 MHz, DMSO-d6) δ 8.43 (t, J = 5.3 Hz, 1H), 8.29 - 8.20 (m, 2H), 7.24 (d, J = 8.1 Hz, 2H), 7.18 (dd, J = 6.8, 4.4 Hz, 3H), 5.27 (s, 2H), 4.57 - 4.46 (m, 4H), 3.92 (s, 3H), 3.27 (s, 3H), 2.47 (d, J = 7.0 Hz, 2H), 0.97 - 0.88 (m, 1H), 0.49 - 0.40 (m, 2H), 0.17 (q, J = 4.9 Hz, 2H).

Example 23. N-((3-fluoro-4-(methylthio)pyridin-2-yl)methyl)-3-(methoxymethyl)-1-(4-(2-oxopyridin-1(2H)-yl)methyl) benzyl)-1H-pyrazole-4-carboxamide (Compound 132) was prepared according to the following synthetic scheme:

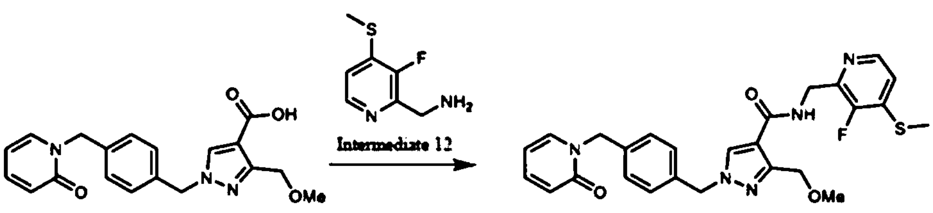


2-3

Compound 132

Crude 2-3 (35 mg, 0.098 mmol), crude Intermediate 12 (17 mg, 0.098 mmol), HATU (37 mg, 0.098 mmol) and TEA (0.046 mL, 0.33 mmol) were sequentially added to DMF (2 mL). The reaction was carried out at room temperature for 1 hour. Water (20 mL) was added, the mixture was extracted with dichloromethane (30 mL × 3), and the combined organic phases were washed separately with water (50 mL × 1) and saturated aqueous sodium chloride solution (50 mL × 1), dried over anhydrous sodium sulfate, and concentrated under reduced pressure to give a residue, which was isolated on preparative plates (DCM: MeOH = 10:1) to give Compound 132 (5 mg, yield: 12.0%) as a white solid.

MS (ESI) M/Z: 508.2 $[M+H]^+$.

$^1H$ NMR (400 MHz, Chloroform-d) δ 8.60 - 8.52 (m, 1H), 8.22 (d, *J* = 5.2 Hz, 1H), 7.90 (s, 1H), 7.33 - 7.25 (m, 4H), 7.24 - 7.17 (m, 4H), 7.01 (t, *J* = 5.5 Hz, 1H), 6.63 - 6.57 (m, 1H), 6.17 - 6.10 (m, 1H), 5.21 (s, 2H), 5.12 (s, 2H), 4.74 (dd, *J* = 4.9, 2.1 Hz, 2H), 4.61 (s, 2H), 3.42 (s, 3H), 2.48 (s, 3H).

Example 24. N-((3-fluoro-4-methoxypyridin-2-yl)methyl)-3-(methoxymethyl)-1-(4-((2-oxo-2,3-dihydro-1H-pyrrolo[3,2-b]pyridin-1-yl)methyl]benzyl)-1H-pyrazole-4-carboxamide (Compound 113) was prepared according to the following synthetic scheme:

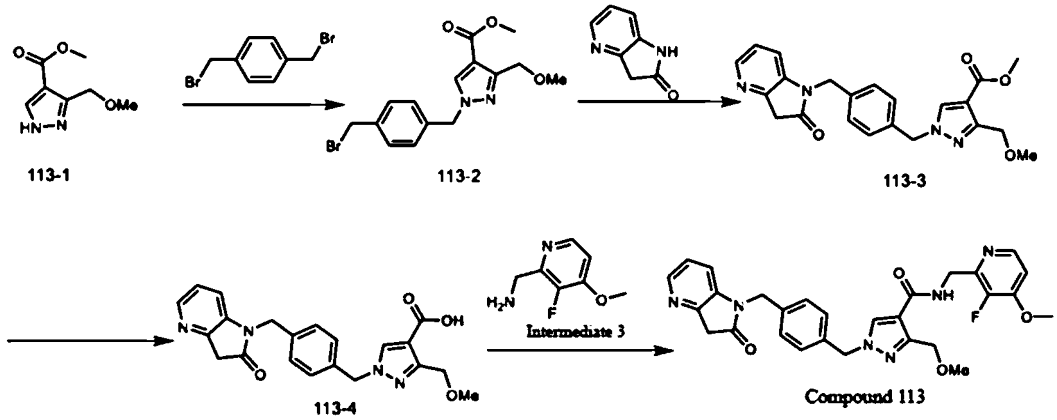

113-1
113-2
113-3
113-4
Compound 113

Step 1: Preparation of methyl 1-(4-(bromomethyl)benzyl)-3-(methoxymethyl)-1H-pyrazole-4-carboxylate: Compound methyl 3-(methoxymethyl)-1H-pyrazole-4-carboxylate (200 mg, 1.18 mmol) and potassium carbonate (324 mg, 2.35 mmol) were added successively to acetonitrile (5 mL) and the mixture was stirred at room temperature for 5 min. 1,4-bis(bromomethyl)benzene (261 mg, 1.44 mmol) was added and the mixture was stirred at 80°C for 16 h. Water (30 mL) was added, the mixture was extracted with dichloromethane (30 mL × 3), and the combined organic phases were washed separately with water (20 mL) and saturated aqueous sodium chloride solution (20 mL), dried over anhydrous sodium sulfate, filtered, and concentrated under reduced pressure to give a residue. The residue was purified by column chromatography (EA: PE = 0-100%) to give the title compound (110 mg, yield: 25%) as a white solid. MS (ESI) M/Z: 353.0 $[M+H]^+$.

Step 2: Preparation of methyl 3-(methoxymethyl)-1-(4-((2-oxo-2,3-dihydro-1H-pyrrolo[3,2-b]pyridin-1-yl)methyl)benzyl)-1H-pyrazole-4-carboxylate: At room temperature, 113-2 (60 mg, 0.17 mmol) was added to acetonitrile (3 mL), $K_2CO_3$ (30 mg, 0.217 mol) and 1,3-dihydro-2H-pyrrolo[3,2-b]pyridin-2-one (25 mg, 0.19 mol) were added in portions and the mixture was stirred from room temperature to 80°C for 12 h. After addition of water (10 mL) and extraction with DCM (10 mL × 3), the organic phases were combined, dried over anhydrous $Na_2SO_4$, filtered and concentrated under reduced pressure to give a residue which was isolated and purified by TLC (DCM: MeOH = 10:1, Rf = 0.5) to give the title compound (22 mg, yield: 31.8%) as a white solid. MS (ESI) M/Z: 407.17 [M+1]+.

Step 3: Preparation of 3-(methoxymethyl)-1-(4-((2-oxo-2,3-dihydro-1H-pyrrolo[3,2-b]pyridin-1-yl)methyl)benzyl)-1H-pyrazole-4-carboxylic acid: Compound 113-3 (22 mg, 0.054 mmol) was added to methanol (3 mL) at room temperature. A solution of sodium hydroxide (10 mg, 0.25 mmol) in water (1 mL) was added and the mixture was stirred at room temperature for 24 hours. The pH of the reaction solution was adjusted to acidic with aqueous 1 N hydrogen chloride, the reaction solution was extracted with DCM (10 mL × 3), the organic phases were combined, dried over anhydrous sodium sulfate, and concentrated under reduced pressure to give the title compound (25 mg, crude) as a white solid. MS (ESI) M/Z: 393 $[M+1]^+$.

Step 4: Preparation of N-((3-fluoro-4-methoxypyridin-2-yl)methyl)-3-(methoxymethyl)-1-(4-((2-oxo-2,3-dihydro-1H-pyrrolo[3,2-b]pyridin-1-yl)methyl]benzyl)-1H-pyrazole-4-carboxamide: Compound 113-4 (25 mg, 0.063 mmol), Intermediate 3 (11.8 mg, 0.075 mmol), HATU (28.72 mg, 0.075 mmol) and triethylamine (25.45 mg, 0.252 mmol) were added successively to DMF (2 mL) and the mixture was stirred at room temperature for 1 h. Water (10 mL) was added. After extraction with DCM (10 mLx3), the combined organic phases were dried over anhydrous $Na_2SO_4$, filtered and concentrated under reduced pressure to give a residue which was purified by column chromatography (methanol: dichloromethane = 0-100%) to give the title compound (4 mg, yield: 11.86%) as a white solid. MS (ESI) M/Z: 531.21 [M+1]+; $^1H$ NMR (400 MHz, DMSO-$D_6$) δ 8.39 (t, J = 5.4 Hz, 1H), 8.22 (s, 1H), 8.20 (d, J = 5.5 Hz, 1H), 7.55 (d, J = 6.8 Hz, 1H), 7.25 (d, J = 8.1 Hz, 2H), 7.20 (d, J = 8.2 Hz, 2H), 7.15 (dd, J = 6.7, 5.5 Hz, 1H), 6.76 (d, J = 7.1 Hz, 1H), 6.45 (t, J = 7.0 Hz, 1H), 5.25

(s, 2H), 5.21 (s, 2H), 4.49 (dd, J = 5.4, 2.3 Hz, 2H), 4.46 (s, 2H), 3.88 (s, 3H), 3.47 (s, 2H), 3.21 (s, 3H).

Using the appropriate starting materials, the compounds listed below were prepared using procedures similar to those described in Examples 21, 22, 23, and 24.

Table 4.

| NO. | Structural formula | Mass spectrum ESI (+) m/z [M + 1]$^+$ |
|---|---|---|
| 32 | 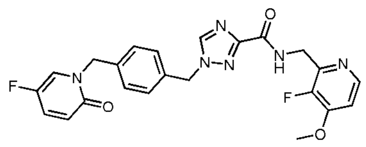 | 467.2 |
| 77 | 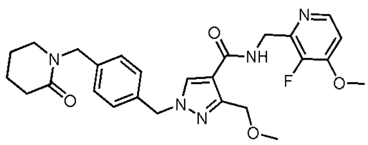 | 496.2 |
| 85 | 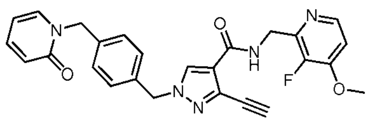 | 472.1 |
| 88 | 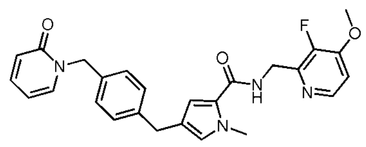 | 461.2 |
| 89 | 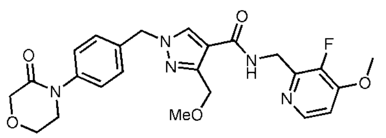 | 484.2 |
| 90 | 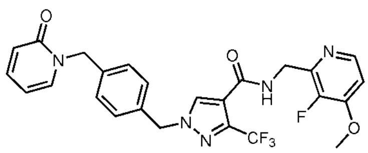 | 516. 2 |
| 91 | 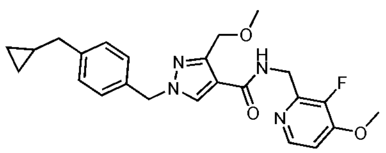 | 439.2 |
| 92 | 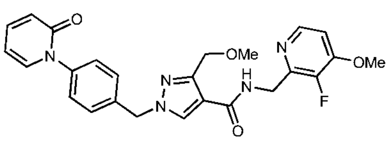 | 478.2 |
| 98 | 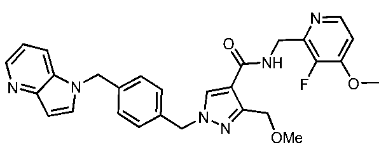 | 515.2 |
| 101 | 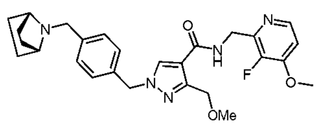 | 494.3 |

(continued)

| NO. | Structural formula | Mass spectrum ESI (+) m/z [M + 1]$^+$ |
|---|---|---|
| 102 | 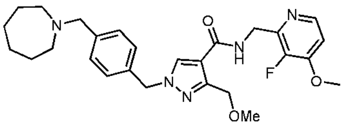 | 497.3 |
| 103 | 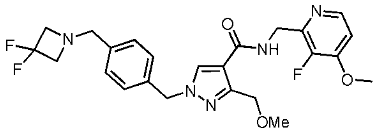 | 490.2 |
| 106 | 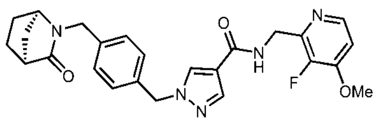 | 464.2 |
| 113 | 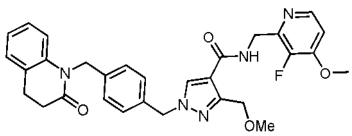 | 544.2 |
| 117 | 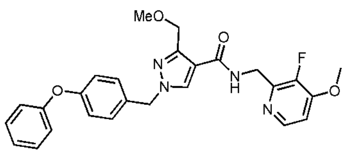 | 477.2 |
| 124 | 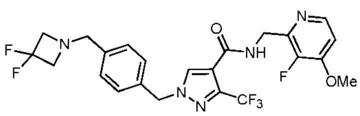 | 514.2 |
| 138 | 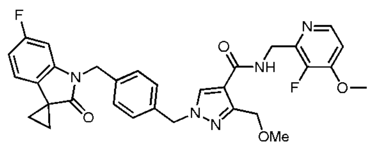 | 574.2 |
| 140 | 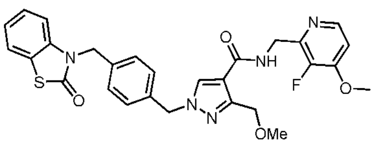 | 548.2 |
| 149 | 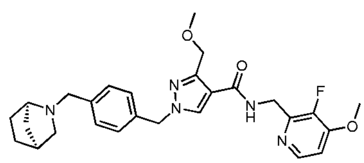 | 494.3 |
| 150 | 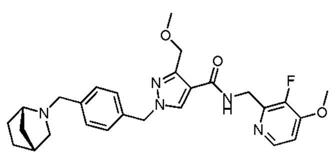 | 494.3 |

(continued)

| NO. | Structural formula | Mass spectrum ESI (+) m/z [M + 1]+ |
|---|---|---|
| 151 | 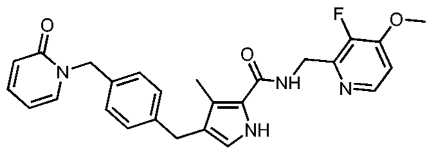 | 461.2 |
| 159 | 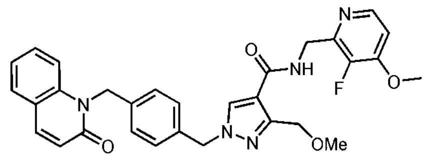 | 542.2 |
| 160 | 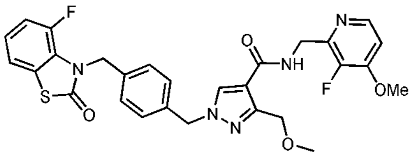 | 566.2 |
| 181 | 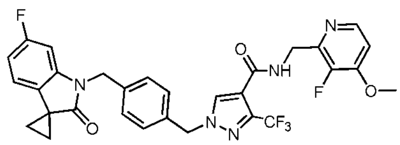 | 598.2 |
| 198 | 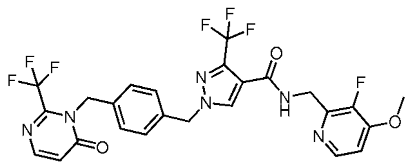 | 585.2 |

Example 25. N-(4-carbamoylbenzyl)-1-(4-(cyclopropylmethyl)benzyl)-1H-pyrazole-4-carboxamide (Compound 105) was prepared according to the following synthetic scheme:

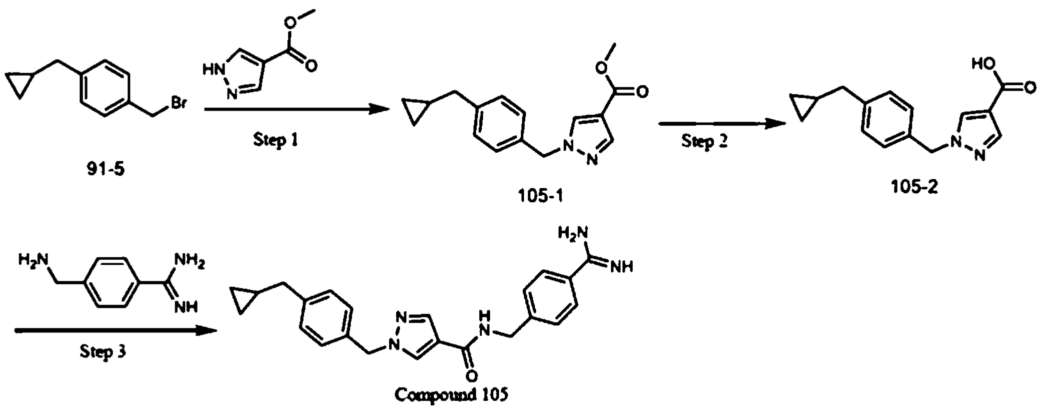

91-5
105-1
105-2
Compound 105

Step 1: Preparation of methyl 1-(4-(cyclopropylmethyl)benzyl)-1H-pyrazole-4-carboxylate: To a solution of 4-(cyclopropylmethyl)benzyl bromide (200 mg, 0.89 mmol) in DMF (8 mL) was added methyl 1H-pyrazole-4-carboxylate (134.69 mg, 1.07 mmol) and $K_2CO_3$ (246.01 mg, 1.78 mmol), and the mixture was stirred at room temperature for 1 h. After monitoring the completion of the reaction by LCMS, the mixture was diluted with water (20 mL), extracted with EA (10 mL * 3), and dried with $Na_2SO_4$. The organic layer was filtered and the filtrate was concentrated under reduced pressure. The residue was purified by preparative TLC (PE: EA = 10:1) to give the desired Compound 105-1 (202 mg, 84.11% yield) as a white solid. MS (ESI) m/z: 271.2[m+H]+.

Step 2: Preparation of 1-(4-(cyclopropylmethyl)benzyl)-1H-pyrazole-4-carboxylic acid: To a solution of Compound 105-1 (50 mg, 0.18 mmol) in MeOH (2 mL) was added NaOH (2 M, 0.3 mL, 0.6 mmol) and the mixture was stirred at room temperature overnight. After monitoring the completion of the reaction by LCMS, the reaction mixture was quenched by the addition of HCl solution (2 M, 0.35 mL, 0.7 mmol) and concentrated under reduced pressure to afford the title compound (82.51 mg, 100% yield) as a white solid. MS (ESI) m/z: 257.2[m+H]+.

Step 3: Preparation of N-(4-carbamoylbenzyl)-1-(4-(cyclopropylmethyl)benzyl)-1H-pyrazole-4-carboxamide: To a solution of Compound 105-2 (47.41 mg, 0.18 mmol) in DMF (3 mL) was added HATU (68.44 mg, 0.18 mmol) and DIEA (69.79 mg, 0.54 mmol), and the mixture was stirred at room temperature for 0.5 h. 4-carbamoylbenzylamine (29.54 mg, 0.20 mmol) was then added to the reaction. The mixture was stirred at room temperature for 2 hours. After monitoring the completion of the reaction by LCMS, the reaction mixture was diluted with $H_2O$ (20 mL) and extracted with DCM: MeOH = 8:1 (10 mL * 3) and dried over $Na_2SO_4$. The organic phase was filtered and the filtrate was concentrated under reduced pressure. The residue was purified by preparative TLC (DCM: MeOH = 8:1) to give the desired Compound 105 (54.03 mg, 75.38% yield) as a white solid. MS (ESI) m/z: 38.82[m+H]+; HPLC: 254 nm: 97.718%, 214 nm: 98.699%; $^1$H NMR (400 MHz, DMSO-d6) δ 8.65 (t, J = 5.7 Hz, 1H), 8.25 (s, 1H), 7.90 (s, 1H), 7.70 (d, J = 8.1 Hz, 2H), 7.31 (d, J = 8.0 Hz, 2H), 7.22 (dd, J = 17.4, 8.1 Hz, 7H), 5.30 (s, 2H), 4.42 (d, J = 5.9 Hz, 2H), 2.47 (d, J = 7.0 Hz, 2H), 0.98 - 0.87 (m, 1H), 0.48 - 0.40 (m, 2H), 0.17 (q, J = 5.0 Hz, 2H).

Example 26. (Z)-1-(4-(cyclopropylmethyl)benzyl)-N-(4-(N'-hydroxycarbamoyl)benzyl)-1H-pyrazole-4-carboxamide (Compound 348) was prepared according to the following synthetic scheme:

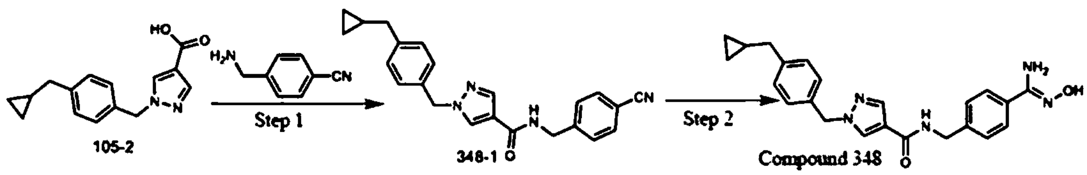


Step 1: Preparation of N-(4-cyanobenzyl)-1-(4-(cyclopropylmethyl)benzyl)-1H-pyrazole-4-carboxamide: To a solution of 1-(4-(cyclopropylmethyl)benzyl)-1H-pyrazole-4-carboxylic acid (50 mg, 0.20 mol) in DMF (2 mL) was added DIEA (77 mg, 0.60 mmol) and HATU (91 mg, 0.24 mmol), and the mixture was stirred at room temperature for 10 min. Compound 4-(aminomethyl)benzonitrile (34 mg, 0.26 mmol) was then added to the reaction. The mixture was stirred at room temperature for 30 minutes. After monitoring the completion of the reaction by LCMS, the reaction mixture was concentrated under reduced pressure. The residue was purified by preparative TLC (DCM: MeOH = 60:1) to give the title compound (30.0 mg, 41.51% yield) as a white solid. MS (ESI) m/z: 371.2 $[M+H]^+$.

Step 2: Preparation of (Z)-1-(4-(cyclopropylmethyl)benzyl)-N-(4-(N'-hydroxycarbamoyl)benzyl)-1H-pyrazole-4-carboxamide: To a solution of Compound 348-1 (20 mg, 0.054 mmol) in EtOH (6 mL) and $H_2O$ (2 mL) was added hydroxylamine hydrochloride (18.76 mg, 0.27 mmol) and $Na_2CO_3$ (28.62 mg, 0.27 mol). The mixture was refluxed at 85°C under nitrogen for 2 h. After LCMS monitored the completion of the reaction, the reaction mixture was concentrated under reduced pressure. The residue was purified by preparative TLC (DCM: MeOH = 8:1) and reverse phase column to give the title compound 348 (7.50 mg, 34.4% yield) as a white solid. MS (ESI) m/z: 404.3 $[M+H]^+$; HPLC: 254 nm: 98.695%, 214 nm: 99.018%; $^1$H NMR (400 MHz, DMSO-d6) δ 12.85 (s, 1H), 11.22 (s, 1H), 9.01 (s, 2H), 8.83 (t, J = 5.9 Hz, 1H), 8.27 (s, 1H), 7.92 (s, 1H), 7.67 (d, J = 8.0 Hz, 2H), 7.48 (d, J = 8.0 Hz, 2H), 7.22 (q, J = 8.0 Hz, 4H), 5.30 (s, 2H), 4.47 (d, J = 5.9 Hz, 2H), 2.47 (d, J = 7.0 Hz, 2H), 1.24 (s, 1H), 0.93 (d, J = 6.5 Hz, 1H), 0.53 - 0.38 (m, 2H), 0.17 (d, J = 4.7 Hz, 2H).

Example 27. N-(4-carbamoylbenzyl)-1-(4-cyclohexyl-3-(trifluoromethyl)benzyl)-3-(methoxymethyl)-1H-pyrazole-4-carboxamide (Compound 153) was prepared according to the following synthetic scheme:

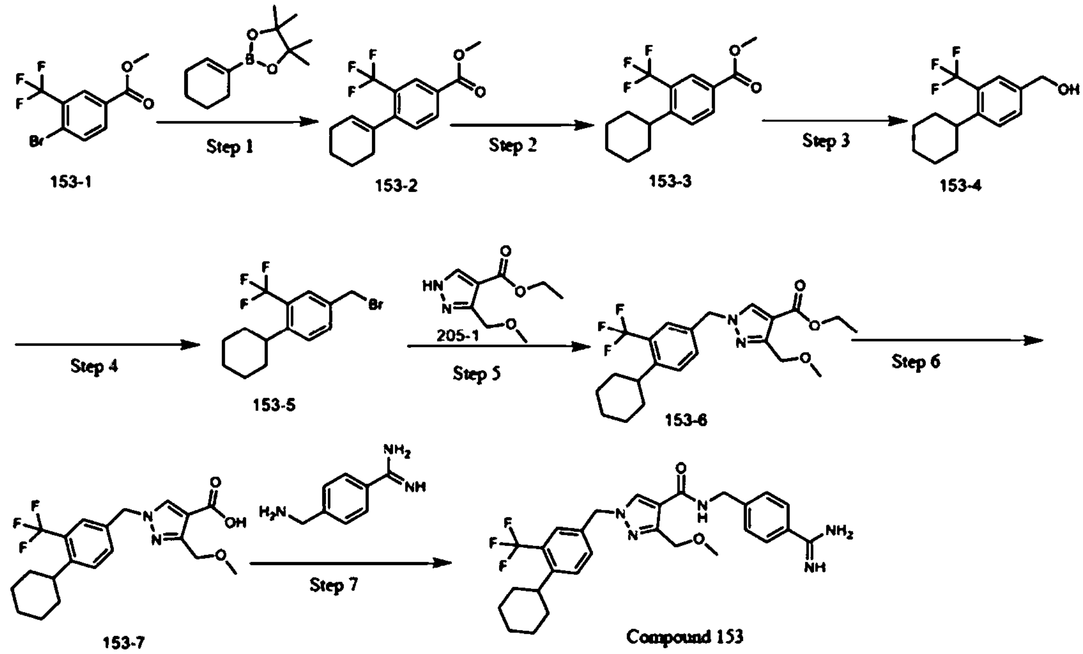


153-1 153-2 153-3 153-4 153-5 153-6 153-7 Compound 153

Step 1: Preparation of methyl 2-(trifluoromethyl)-2',3',4',5'-tetrahydro-[1,1'-biphenyl]-4-carboxylate: To a solution of Compound 153-1 (850 mg, 3.0 mmol) in 1,4-dioxane (15 mL) and water (1.5 mL) was added Compound SM2 (686 mg, 3.3 mmol) and $K_2CO_3$ (829 mg, 6.0 mmol). The mixture was purged with nitrogen for 5 minutes. Then Pd(dppf)$Cl_2$ (219 mg, 0.3 mmol) was added and the mixture was stirred at 100°C for 4 h. After monitoring the completion of the reaction by LCMS, the reaction mixture was cooled to room temperature and filtered through a pad of celite. The reaction mixture was poured into 100 ml water and extracted with EA (100 ml * 3). The combined organic layers were dried over anhydrous $Na_2SO_4$. The residue was purified by silica gel chromatography (eluting with PE: EA = 100:0 to 90:10) to give the title compound (805 mg, 94.30% yield). $^1$H NMR(400 MHz, DMSO) δ 8.16(d,J=9.8 Hz,2H), 7.51(d,J=7.9 Hz,1H), 5.58(s,1H).

Step 2: Preparation of methyl 4-cyclohexyl-3-(trifluoromethyl)benzoate: To a solution of Compound 153-2 (805 mg, 2.83 mmol) in MeOH (15 mL) was added Pd/C (200 mg) and the mixture was stirred at room temperature for 16 h. After monitoring the completion of the reaction by LCMS, the reaction mixture was filtered and the filtrate was concentrated under reduced pressure. The residue was purified by silica gel chromatography (eluting with PE: EA = 100:0 to 90:10) to give the title compound (800 mg, 98.68% yield). $^1$H NMR(400 MHz, DMSO) δ 8.24 - 8.01 (m, 2H), 7.81 (d, J = 8.2 Hz, 1H), 3.90 (d, J = 10.4 Hz, 3H), 2.87 (t, J = 11.1 Hz, 1H), 1.77 (dd, J = 44.5, 10.7 Hz, 5H), 1.56 (dd, J = 22.3, 10.6 Hz, 2H), 1.32 (dt, J = 23.8, 11.9 Hz, 3H).

Step 3: Preparation of (4-cyclohexyl-3-(trifluoromethyl)phenyl)methanol: To a solution of Compound 153-3 (200 mg, 0.7 mmol) in dry THF (10 mL) cooled to -5°C was added $LiAlH_4$ (1M in THF) (1.05 mL, 1.05 mmol) dropwise. The mixture was brought to room temperature and the solution was stirred at room temperature for 2 hours. After completion of the reaction was monitored by LCMS, the reaction was quenched with saturated $NH_4Cl$ solution. The reaction mixture was diluted with water (50 mL) and extracted with EA (50 mL * 3). The combined organics were concentrated. The residue was purified by preparative TLC (PE: EA = 8:1) to afford the title compound (152 mg, 84.24% yield). $^1$H NMR (400 MHz, DMSO) δ 7.63 - 7.43 (m, 3H), 5.30 (q, J = 5.8 Hz, 1H), 4.52 (d, J = 5.7 Hz, 2H), 2.80 (t, J = 11.2 Hz, 1H), 1.82 - 1.67 (m, 5H), 1.51 (dd, J = 22.6, 10.6 Hz, 2H), 1.34 (dd, J = 23.8, 10.3 Hz, 3H).

Step 4: Preparation of 4-(bromomethyl)-1-cyclohexyl-2-(trifluoromethyl)benzene: To a solution of Compound 153-4 (152 mg, 0.59 mmol) in DCM (10 mL) cooled to -0°C was added phosphorus tribromide (160 mg, 0.59 mmol). The mixture was brought to room temperature and the solution was stirred at room temperature for 10 minutes. After monitoring the completion of the reaction by LCMS, the reaction mixture was diluted with water (50 mL) and extracted with DCM (50 mL * 3). The combined organics were concentrated. The residue was purified by preparative TLC (DCM: MeOH = 15:1) to give the title compound (115 mg, 60.8% yield). $^1$H NMR (400 MHz, DMSO) δ 7.77 - 7.56 (m, 3H), 4.77 (s, 2H), 2.80 (t, J = 11.7 Hz, 1H), 1.83 - 1.65 (m, 5H), 1.52 (dd, J = 21.7, 9.9 Hz, 2H), 1.38 - 1.29 (m, 3H).

Step 5: Preparation of ethyl 1-(4-cyclohexyl-3-(trifluoromethyl)benzyl)-3-(methoxymethyl)-1H-pyrazole-4-carboxylate: To a solution of Compound 153-5 (115 mg, 0.36 mmol) in DMF (8 mL) was added Compound 205-1 (72 mg, 0.40 mmol) and $K_2CO_3$ (124 mg, 0.90 mmol), and the mixture was stirred at room temperature for 3 h. After monitoring the completion of the reaction by LCMS, the reaction mixture was diluted with water (50 mL) and extracted with DCM (50 mL * 3). The combined organic phases were concentrated and the residue was purified by preparative TLC (PE: EA = 2:1) to give the desired title compound (60 mg, 39.48% yield). $^1$H NMR (400 MHz, DMSO) δ 8.50 (s, 1H), 7.68 - 7.57 (m, 2H), 7.52

(d, J = 8.1 Hz, 1H), 5.40 (s, 2H), 4.52 (s, 2H), 4.21 (p, J = 6.9 Hz, 2H), 3.25 (s, 3H), 2.80 (t, J = 11.4 Hz, 1H), 1.85 - 1.62 (m, 5H), 1.49 (dd, J = 23.5, 11.7 Hz, 2H), 1.37 - 1.22 (m, 6H).

Step 6: Preparation of 1-(4-cyclohexyl-3-(trifluoromethyl)benzyl)-3-(methoxymethyl)-1H-pyrazole-4-carboxylic acid: To a solution of Compound 153-6 (34 mg, 0.08 mmol) in MeOH (2 mL) was added NaOH (2 M, 0.5 mL, 1 mmol), and the mixture was stirred at room temperature for 1 hour. The reaction mixture was quenched by the addition of HCl solution (2 M, 0.5 mL, 1 mmol) and concentrated under reduced pressure to afford the title compound (90 mg, 100% yield) as a white solid. MS (ESI) M/Z: 397.0 $[M+H]^+$.

Step 7: Preparation of N-(4-carbamoylbenzyl)-1-(4-cyclohexyl-3-(trifluoromethyl)benzyl)-3-(methoxymethyl)-1H-pyrazole-4-carboxamide: To a solution of Compound 153-7 (30 mg, 0.076 mmol) in DMF (3 mL) was added HATU (30 mg, 0.08 mmol) and DIEA (30 mg, 0.24 mmol), and the mixture was stirred at room temperature for 10 min. Compound 4-carbamoylbenzylamine (12 mg, 0.84 mmol) was then added to the reaction. The mixture was stirred at room temperature for 1 hour. After monitoring the completion of the reaction by LCMS, the reaction mixture was concentrated under reduced pressure. The residue was purified by reverse phase HPLC to afford the hydrochloride salt of the title compound (22.18 mg, 55.55% yield) as a white solid. MS (ESI) m/z: 528.3[m+H]+; $^1$H NMR (400 MHz, DMSO) δ 9.31 (s, 2H), 9.03 (s, 2H), 8.59 (t, J = 5.8 Hz, 1H), 8.35 (s, 1H), 7.78 (d, J = 8.2 Hz, 2H), 7.69 - 7.57 (m, 2H), 7.51 (d, J = 8.1 Hz, 3H), 5.39 (s, 2H), 4.64 - 4.33 (m, 4H), 3.22 (s, 3H), 2.78 (d, J = 10.6 Hz, 1H), 1.79 (s, 2H), 1.74 - 1.59 (m, 3H), 1.56 - 1.41 (m, 2H), 1.32 (t, J = 10.8 Hz, 3H).

Example 28. N-(4-carbamoylbenzyl)-1-(4-cyclopropoxy-3-(trifluoromethyl)benzyl)-1H-pyrazole-4-carboxamide (Compound 379) was prepared according to the following synthetic scheme:

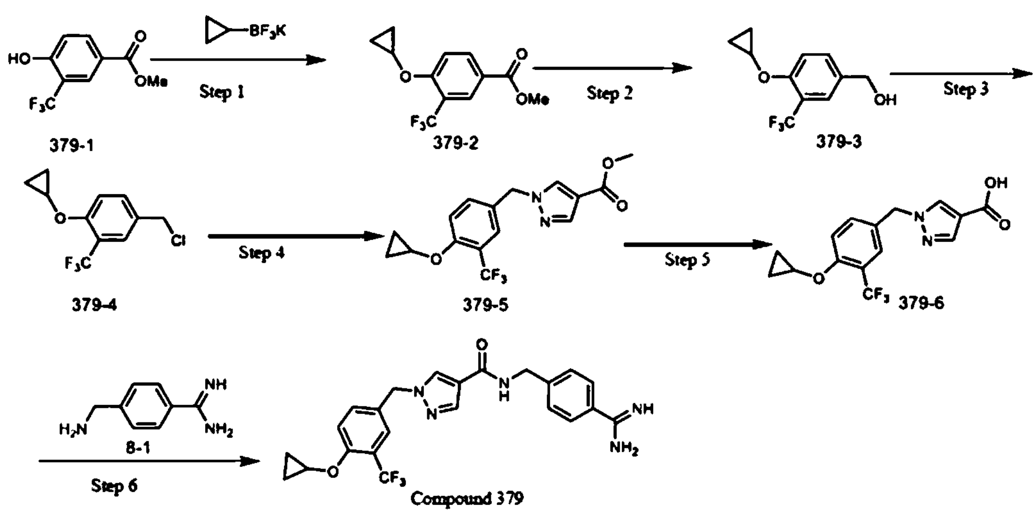


Step 1: Preparation of methyl 4-cyclopropoxy-3-(trifluoromethyl)benzoate: To a solution of Compound 379-1 (400 mg, 1.82 mmol) in toluene (16 mL) and $H_2O$ (4 mL) was added potassium cyclopropyltrifluoroborate (673 mg, 4.55 mmol), 1,10-phenanthroline (131 mg, 0.73 mmol), $Cu(OAc)_2$ (132 mg, 0.73 mmol) and $K_2CO_3$ (754 mg, 5.46 mmol). The mixture was purged with oxygen for 2 minutes. The mixture was then stirred at 70°C under oxygen overnight. After monitoring the completion of the reaction by LCMS, the reaction mixture was cooled to room temperature and filtered through a pad of celite. The reaction mixture was poured into $H_2O$ (150 mL) and extracted with EA (150 mL * 3). The combined organics were concentrated. The residue was purified by reverse phase column to give the desired title compound (94 mg, 19.88% yield) as an oil. MS (ESI) m/z: 261.1[m+H]+.

Step 2: Preparation of (4-cyclopropoxy-3-(trifluoromethyl)phenyl)methanol: To a solution of Compound 379-2 (50 mg, 0.34 mmol) in dry THF (15 mL) cooled to 0°C was added $LiBH_4$ (39 mg, 1.80 mmol) dropwise. The mixture was allowed to come to room temperature and the solution was stirred at room temperature overnight. After completion of the reaction, the reaction was monitored by LCMS. The reaction was quenched with saturated $NH_4Cl$ solution. The reaction mixture was diluted with water (100 mL) and extracted with EA (100 mL * 3). The combined organics were concentrated. The residue was purified by chromatography on a silica gel column (PE: EA = 100:0 to PE: EA = 0:100) to give the desired title compound (36 mg, 42.92% yield). MS (ESI) m/z: 215.0[m-OH]+.

Step 3: Preparation of 4-(chloromethyl)-1-cyclopropoxy-2-(trifluoromethyl)benzene: To a solution of Compound 379-3 (36 mg, 0.16 mmol) in DCM (5 mL) was slowly added $SOCl_2$ (38 mg, 0.32 mmol) at 0°C. The reaction mixture was stirred at room temperature for 0.5 h. After monitoring the completion of the reaction by LCMS, the reaction mixture was

diluted with a saturated solution of $NaHCO_3$ (10 mL) and extracted with DCM (30 mL * 3). The combined organic phases were concentrated under reduced pressure to obtain the title compound (26 mg, yield 66.91%). MS(ESI) m/z: 251.0[m+H]+.

Step 4: Preparation of methyl 1-(4-cyclopropoxy-3-(trifluoromethyl)benzyl)-1H-pyrazole-4-carboxylate: To a solution of Compound 379-4 (26 mg, 0.10 mmol) in DMF (5 mL) was added methyl pyrrole-3-carboxylate (15 mg, 0.12 mmol) and $K_2CO_3$ (27 mg, 0.20 mmol). The mixture was stirred overnight at room temperature. After monitoring the completion of the reaction by LCMS, the reaction mixture was concentrated under reduced pressure. The residue was purified by flash chromatography (PE: EA = 100:0 to 50:50) to give the title compound (18 mg, 50.99% yield). MS (ESI) m/z: 341.1[m+H]+.

Step 5: Preparation of 1-(4-cyclopropoxy-3-(trifluoromethyl)benzyl)-1H-pyrazole-4-carboxylic acid: To a solution of Compound 379-5 (18 mg, 0.053 mmol) in MeOH (2 mL) was added NaOH (2 M, 1 mL, 2 mmol) and the mixture was stirred overnight at room temperature. After completion of the reaction, the reaction was monitored by LCMS. The reaction mixture was quenched by the addition of HCl solution (2 M, 1 mL, 2 mmol) and concentrated under reduced pressure to give the title compound (17 mg). MS (ESI) m/z: 327.0[m+H]+.

Step 6: Preparation of N-(4-carbamoylbenzyl)-1-(4-cyclopropoxy-3-(trifluoromethyl)benzyl)-1H-pyrazole-4-carboxamide: To a solution of Compound 379-6 (17 mg, 0.052 mmol) in DMF (2 mL) was added DIEA (20 mg, 0.16 mmol) and HATU (22 mg, 0.057 mmol), and the mixture was stirred at room temperature for 5 min. Compound 8-1 (14 mg, 0.062 mmol) was then added to the reaction. The mixture was stirred at room temperature for 15 minutes. The completion of the reaction was monitored by LCMS, the reaction mixture was then purified by reverse phase column to give the title Compound 379 (5.45 mg, 21.18% yield) as a white solid.

MS (ESI) m/z: 458.4[m+H]+; $^1H$ NMR (400 MHz, DMSO) δ 9.31 (s, 2H), 9.04 (s, 2H), 8.83 (t, J = 5.7 Hz, 1H), 8.32 (s, 1H), 7.94 (s, 1H), 7.77 (d, J = 8.2 Hz, 2H), 7.61 (d, J = 7.6 Hz, 2H), 7.55 - 7.41 (m, 3H), 5.36 (s, 2H), 4.48 (d, J = 5.6 Hz, 2H), 4.02 (dt, J = 8.3, 4.3 Hz, 1H), 0.88 - 0.74 (m, 2H), 0.69 - 0.56 (m, 2H).

Using the appropriate starting materials, the compounds listed in the table below were prepared using procedures similar to those described in Examples 25, 26, 27, and 28.

Table 5.

| NO. | Structural formula | Mass spectrum ESI (+) m/z [M + 1]+ |
|---|---|---|
| 39 | 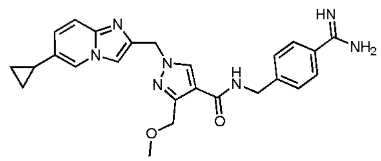 | 458.2 |
| 40 | 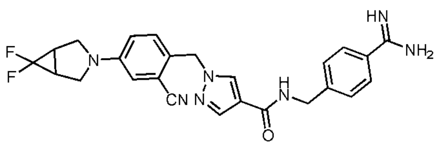 | 476.2 |
| 109 | 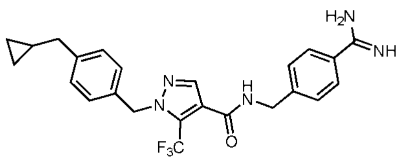 | 456.2 |
| 121 | 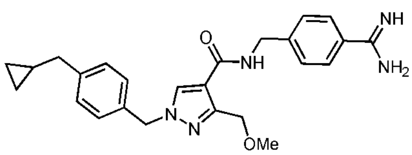 | 432.2 |
| 128 | 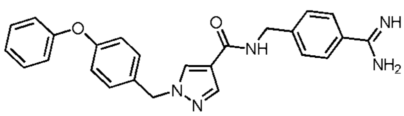 | 426.2 |

(continued)

| NO. | Structural formula | Mass spectrum ESI (+) m/z [M + 1]+ |
|---|---|---|
| 158 | 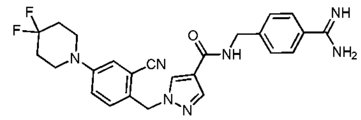 | 478.2 |
| 241 | 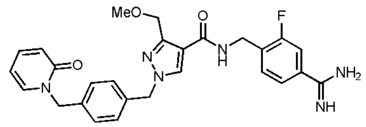 | 503.2 |
| 242 | 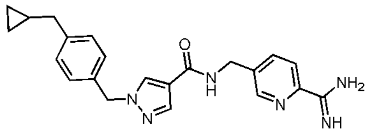 | 389.2 |
| 243 | 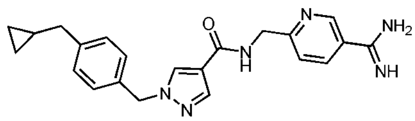 | 389.2 |
| 255 | 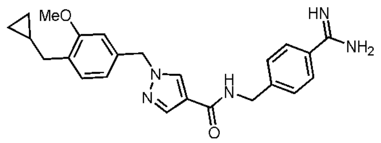 | 418.2 |
| 262 | 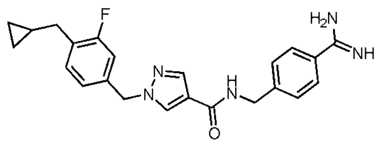 | 406.2 |
| 264 | 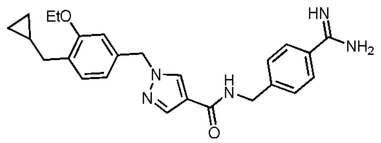 | 432.2 |
| 270 | 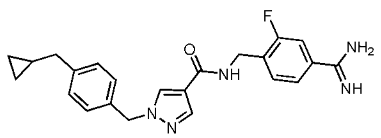 | 406.2 |
| 271 | 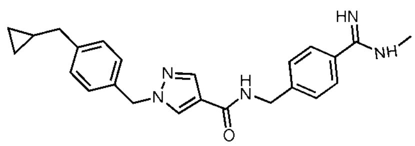 | 402.2 |
| 275 | 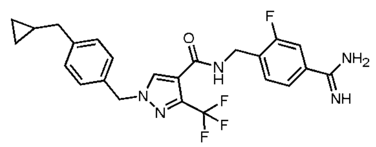 | 474.2 |
| 312 | 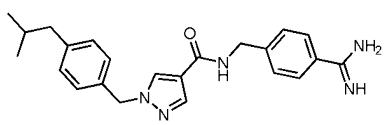 | 390.2 |

(continued)

| NO. | Structural formula | Mass spectrum ESI (+) m/z [M + 1]+ |
| --- | --- | --- |
| 314 | 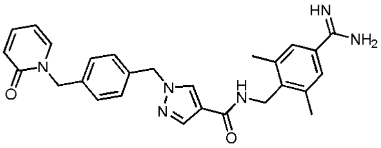 | 469.2 |
| 321 | 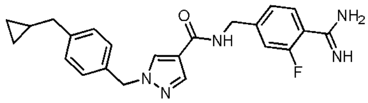 | 406.2 |
| 322 | 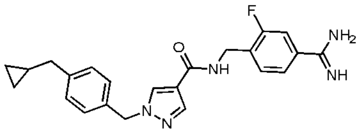 | 406.2 |
| 323 | 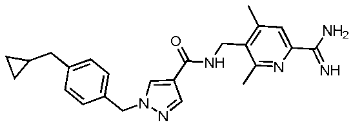 | 417.2 |
| 329 | 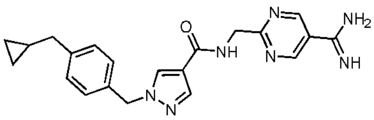 | 390.2 |
| 330 | 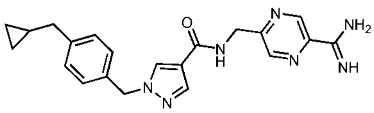 | 390.2 |
| 341 | 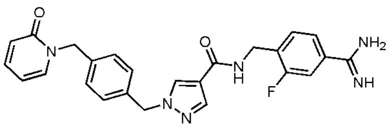 | 459.2 |
| 345 | 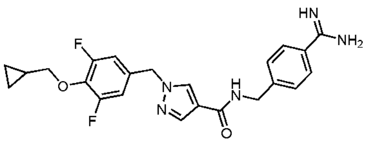 | 440.2 |
| 347 | 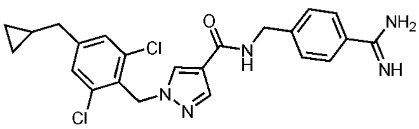 | 456.2 |
| 351 | 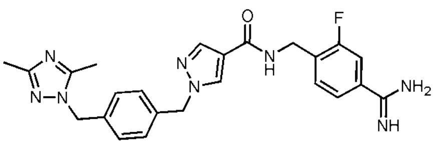 | 461.2 |
| 353 | 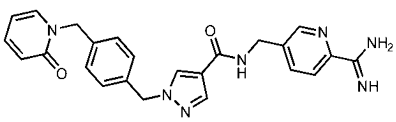 | 442.2 |

(continued)

| NO. | Structural formula | Mass spectrum ESI (+) m/z [M + 1]+ |
|---|---|---|
| 354 | 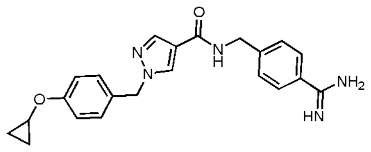 | 390.2 |
| 355 | 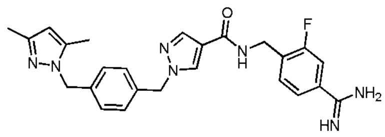 | 460.2 |
| 358 | 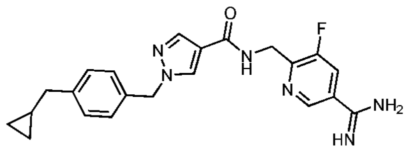 | 407.2 |
| 359 | 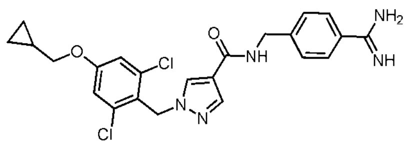 | 472.1 |
| 360 | 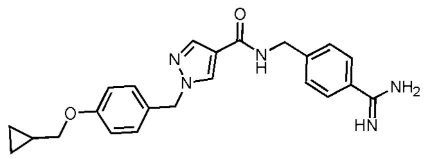 | 404.2 |
| 361 | 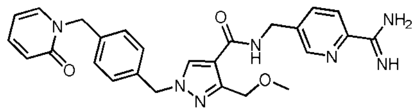 | 486.2 |
| 365 | 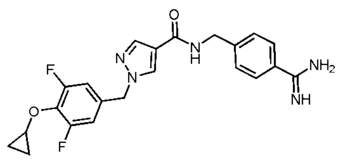 | 426.2 |
| 366 | 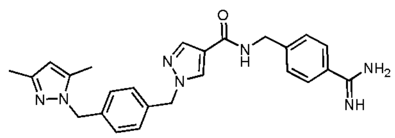 | 442.2 |
| 368 | 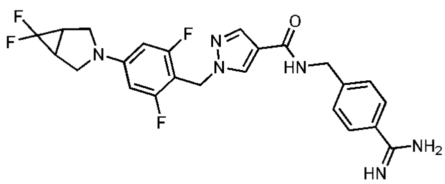 | 487.2 |
| 371 | 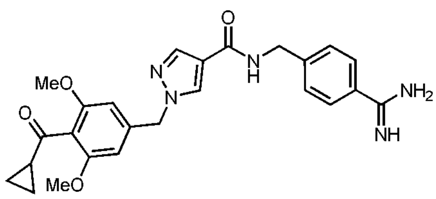 | 462.2 |

(continued)

| NO. | Structural formula | Mass spectrum ESI (+) m/z [M + 1]+ |
|---|---|---|
| 372 | 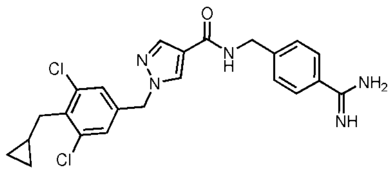 | 456.1 |
| 373 | 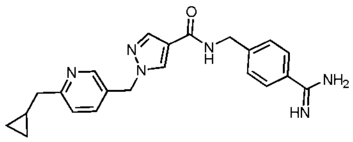 | 389.2 |
| 374 | 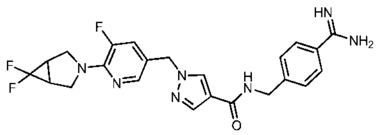 | 470.2 |
| 375 | 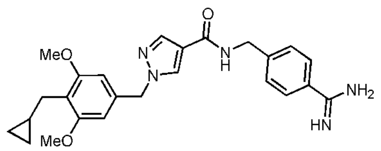 | 448.2 |
| 379 | 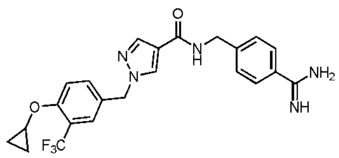 | 458.2 |
| 380 | 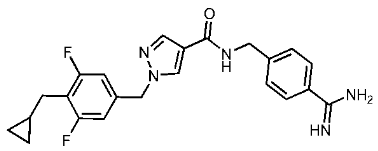 | 424.2 |
| 381 | 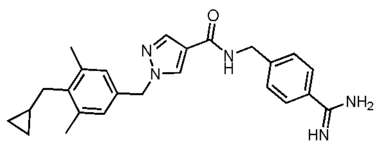 | 416.2 |
| 382 | 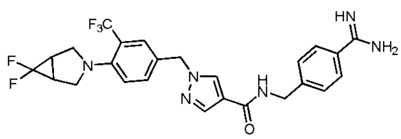 | 519.2 |
| 385 | 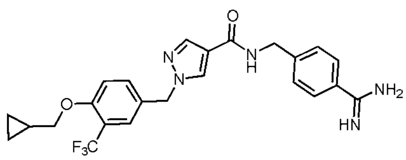 | 472.2 |
| 386 | 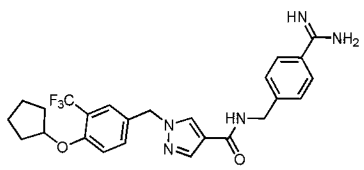 | 486.2 |

(continued)

| NO. | Structural formula | Mass spectrum ESI (+) m/z [M + 1]+ |
|---|---|---|
| 387 | 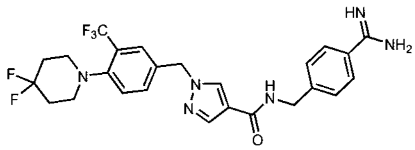 | 521.2 |
| 388 | 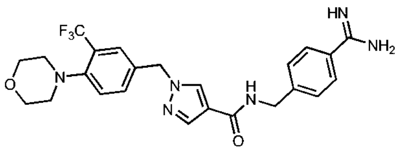 | 487.2 |
| 393 | 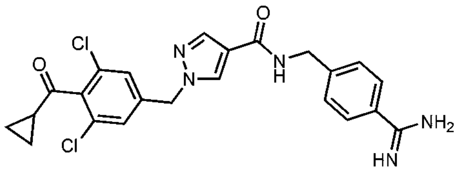 | 470.1 |
| 394 | 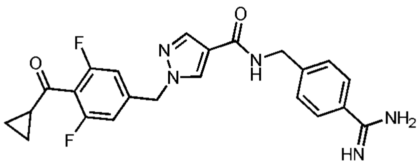 | 438.2 |

Example 29. N-(4-carbamoylbenzyl)-1-(4-phenoxybenzyl)-1H-pyrazole-4-carboxamide (Compound 128) was prepared according to the following synthetic scheme:

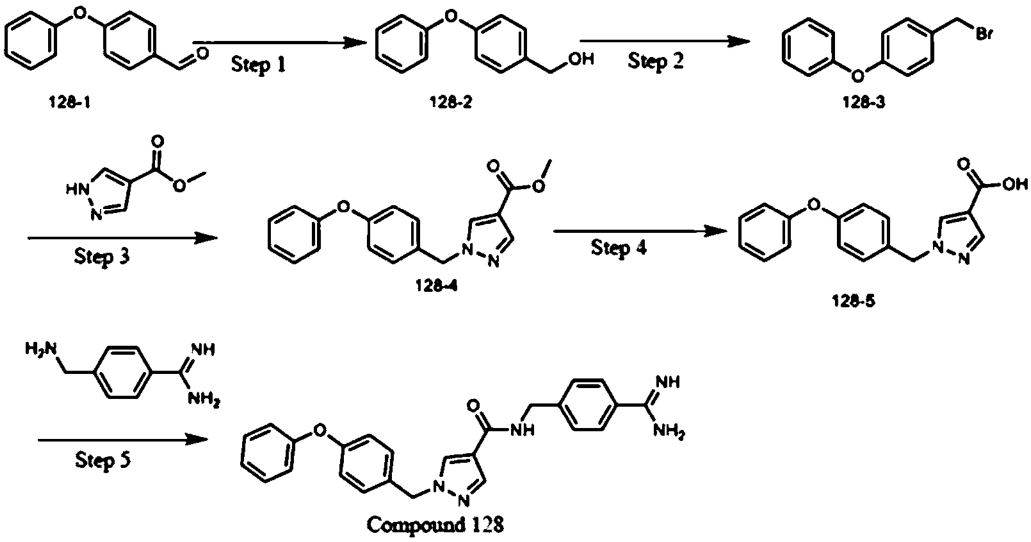


Step 1: Preparation of 4-phenoxybenzyl alcohol: Compound 128-1 (1.0 g, 5.05 mmol) was added to methanol (10 mL) under the protection of nitrogen, the mixture was cooled to 0°C, sodium borohydride (575 mg, 15.14 mmol) was added, the mixture was naturally returned to room temperature and stirred for 1 hour. Saturated aqueous ammonium chloride (50 mL) was added, the mixture was extracted with EA (50 mL × 3), and the combined organic phases were washed separately with water (100 mL) and saturated aqueous sodium chloride (100 mL), dried over anhydrous sodium sulfate, filtered, and concentrated under reduced pressure to give a residue. The residue was purified by column chromatography (EA: PE = 0-100%) to give the title compound (850 mg, yield: 85%) as a white solid. MS (ESI) M/Z: 202.1 [M+H]+.

Step 2: Preparation of 4-phenoxybenzyl bromide: Compound 128-2 (800 mg, 4.00 mmol) was added to DCM (10 mL) under nitrogen, the mixture was cooled to 0°C, and phosphorus tribromide (1.7 g, 6.00 mmol) dissolved in DCM (5 mL) was added dropwise. The mixture was naturally returned to room temperature and stirred for 1 hour. The temperature was lowered to 0°C, the pH of the reaction solution was adjusted to about 8 with saturated sodium bicarbonate aqueous solution (20 mL), and the reaction solution was extracted with DCM (30 mL × 3). The organic phases were combined, dried over anhydrous sodium sulfate, filtered, and concentrated under reduced pressure to give the title compound (850 mg, yield: 80%) as a white solid. MS (ESI) M/Z: 263.0 $[M+H]^+$.

Step 3: Preparation of methyl 1-(4-phenoxybenzyl)-1H-pyrazole-4-carboxylate: Compound 128-3 (780 mg, 2.52 mmol) was added to ethanol (10 mL) at room temperature, a solution of sodium hydroxide (504 mg, 12.60 mmol) in water (2 mL) was added, and the mixture was stirred at room temperature for 16 hours. The pH of the reaction mixture was adjusted to acidic with aqueous 1 N hydrogen chloride, the mixture was extracted with DCM (10 mL × 3), the organic phases were combined, dried over anhydrous sodium sulfate, filtered and concentrated under reduced pressure to give the title compound (700 mg, yield: 94%) as a white solid. MS (ESI) M/Z: 295.1 $[M+H]^+$.

Step 4: Preparation of 1-(4-phenoxybenzyl)-1H-pyrazole-4-carboxylic acid: At room temperature, methyl pyrazole-4-carboxylate (354 mg, 2.70 mmol) and potassium carbonate (2.1 g, 15.26 mmol) were added successively to DMF (10 mL) and the mixture was stirred at room temperature for 5 min. Compound 128-4 (800 mg, 3.05 mmol) was added and stirred at room temperature for 2 h. Water (50 mL) was added, the mixture was extracted with dichloromethane (50 mL × 3), and the combined organic phases were washed separately with water (50 mL) and saturated aqueous sodium chloride solution (50 mL), dried over anhydrous sodium sulfate, filtered, and concentrated under reduced pressure to give a residue. The residue was purified by column chromatography (EA: PE = 0-100%) to give the title compound (780 mg, yield: 94%) as a white solid. MS (ESI) M/Z: 309.1 $[M+H]^+$.

Step 5: Preparation of N-(4-carbamoylbenzyl)-1-(4-phenoxybenzyl)-1H-pyrazole-4-carboxamide: Compound 128-5 (50 mg, 0.17 mmol), HATU (77 mg, 0.20 mmol) and triethylamine (52 mg, 0.51 mmol) were sequentially added to DMF (2 mL) and stirred at room temperature for 0.5 h. Compound 4-aminomethylbenzamidine (45 mg, 0.20 mmol) was added and the mixture was stirred at room temperature for 0.5 h. Water (10 mL) was added, the mixture was extracted DCM (10 mL × 3), the organic phases were combined, dried over anhydrous $Na_2SO_4$, filtered and concentrated under reduced pressure to a residue which was purified by column chromatography (methanol: dichloromethane = 0-100%) to give the title compound (25 mg, yield: 35%) as a yellow solid. MS (ESI) M/Z: 426.2 $[M+H]^+$; $^1H$ NMR(400 MHz, DMSO-$d_6$) δ 9.09 (s, 3H), 8.78 (t, J = 8.4 Hz, 1H), 8.28 (s, 1H), 7.92 (s, 1H), 7.78 - 7.73 (m, 2H), 7.53 - 7.46 (m, 2H), 7.42 - 7.35 (m, 2H), 7.33 - 7.29 (m, 2H), 7.19 - 7.12 (m, 1H), 7.02 - 6.96 (m, 4H), 5.33 (s, 2H), 4.48 (d, J = 6.0 Hz, 2H).

Using the appropriate starting materials, the compounds listed in the table below were prepared using procedures similar to those described in Examples 22 and 29

Table 6.

| NO. | Structural formula | Mass spectrum ESI (+) m/z$[M+1]^+$ |
|---|---|---|
| 115 | 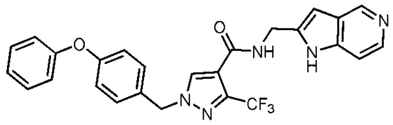 | 492.2 |
| 117 | 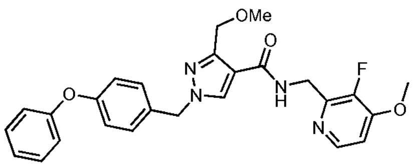 | 477.2 |
| 133 | 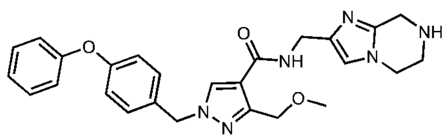 | 473.2 |
| 134 | 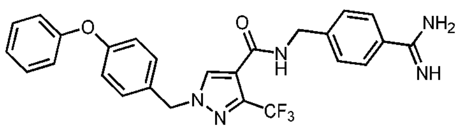 | 494.2 |

(continued)

| NO. | Structural formula | Mass spectrum ESI (+) m/z[M+1]+ |
|---|---|---|
| 141 | 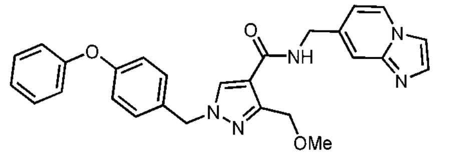 | 468.2 |

Example 30. 3-((3-fluoro-3-(methoxymethyl)azetidin-1-yl)methyl)-N-(3-fluoro-4-methoxypyridin-2-yl)methyl (Compound 205) was prepared according to the following synthetic scheme:

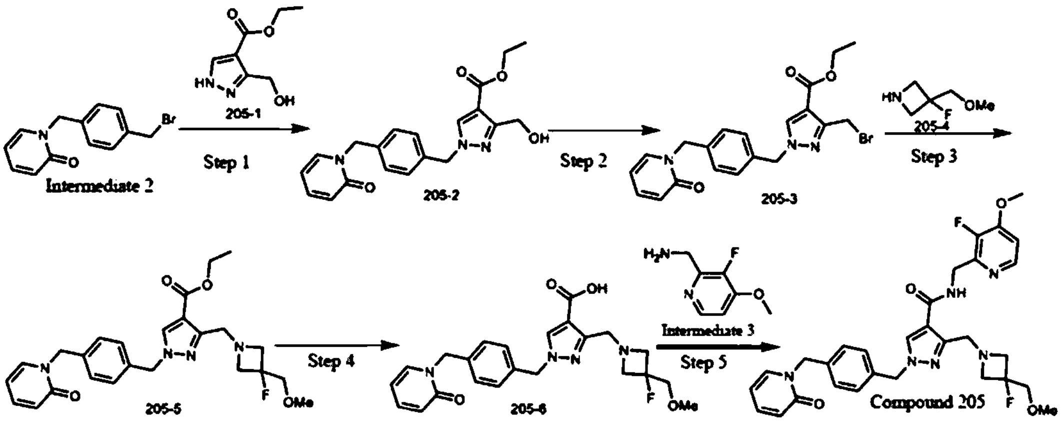


Step 1: Preparation of ethyl 3-(hydroxymethyl)-1-(4-((2-oxopyridin-1(2H)yl)methyl)benzyl)-1H-pyrazole-4-carboxylate: To a mixture of Intermediate 2 (1.00 g, 3.60 mmol, 1.0 eq) and Compound 205-1 (734 mg, 4.32 mmol, 1.2 eq) in DMF (10 mL) was added $K_2CO_3$ (994 mg, 7.20 mmol, 2.0 eq), and the mixture was stirred overnight at room temperature. The reaction mixture was diluted with $H_2O$ (30 mL), extracted with EtOAc (20 mL * 3), and dried over anhydrous $Na_2SO_4$. The organic phase was filtered and the filtrate was concentrated under reduced pressure. The residue was purified by silica gel column (PE/EA = 1/2) to give the title compound (700 mg, 53.0% yield) as a white solid. MS (ESI) m/z: 368.2 $[M+H]^+$.

Step 2: Preparation of ethyl 3-(bromomethyl)-1-(4-((2-oxopyridin-1(2H)yl)methyl)benzyl)-1H-pyrazole-4-carboxylate: a solution of Compound 205-2 (30 mg, 0.08 mmol, 1.0 eq) in anhydrous MeCN (5 mL) was added $PBr_3$ (43 mg, 0.16 mmol, 2.0 eq) at room temperature and the mixture was stirred at 80°C for 1 h. After monitoring the completion of the reaction by TLC, the reaction mixture was diluted with $H_2O$ (10 mL), extracted with EtOAc (10 mL * 3), and dried over anhydrous $Na_2SO_4$. The organic phase was filtered and the filtrate was concentrated under reduced pressure. The residue was purified by preparative TLC (PE/EA = 1/1) to give the title compound (24 mg, 68.6% yield) as a white solid. MS (ESI) m/z: 431.2 $[M+H]^+$.

Step 3: Preparation of ethyl 3-((3-fluoro-3-(methoxymethyl)azetidin-1-yl)methyl)-1-(4-((2-oxopyridin-1(2H)-yl)methyl)benzyl)-1H-pyrazole-4-carboxylate: a mixture of Compounds 205-3 (24 mg, 0.06 mmol, 1.0 eq) and 205-4 (11 mg, 0.09 mmol, 1.5 eq) in DMF (2 mL) was added DIEA (16 mg, 0.12 mmol, 2.0 eq) and the mixture was stirred at room temperature for 2 hours. After the reaction was monitored for completion by LCMS, the reaction mixture was diluted with $H_2O$ (10 mL), extracted with EtOAc (10 mL * 3), and dried over anhydrous $Na_2SO_4$. The organic phase was filtered and the filtrate was concentrated under reduced pressure. The residue was purified by preparative TLC (DCM/MeOH = 10/1) to afford the title compound (12 mg, 46.2% yield) as a white solid. MS (ESI) m/z: 469.3 $[M+H]^+$.

Step 4: Preparation of 3-((3-fluoro-3-(methoxymethyl)azetidin-1-yl)methyl)-1-(4-((2-oxopyridin-1(2H)-yl)methyl)benzyl)-1H-pyrazole-4-carboxylic acid: a solution of Compound 205-5 (12 mg, 0.03 mmol, 1.0 eq) in EtOH (0.8 mL) and $H_2O$ (0.2 mL) was added NaOH (5 mg, 0.12 mmol, 4.0 eq) at room temperature and the mixture was stirred at 60°C for 2 h. After monitoring the completion of the reaction by LCMS, the pH of the reaction mixture was adjusted to 4-5 with HCl solution (4.0 M). The crude title compound, after concentration under vacuum, was used directly in the next step. MS (ESI) m/z: 441.3 $[M+H]^+$.

Step 5: Preparation of 3-((3-fluoro-3-(methoxymethyl)azetidin-1-yl)methyl)-N-(3-fluoro-4-methoxypyridin-2-yl)methyl: a mixture of Compound 205-6 (concentrate from the previous step) and Intermediate 3 (8 mg, 0.04 mmol, 1.2 eq) in

DMF (2 mL) was added HATU (19 mg, 0.05 mmol, 1.5 eq) and TEA (10 mg, 0.10 mmol, 3.0 eq), and the mixture was stirred overnight at room temperature. After monitoring the completion of the reaction by LCMS, the reaction mixture was diluted with $H_2O$ (10 mL) and extracted with EtOAc (10 mL * 3) and dried over anhydrous $Na_2SO_4$. The organic phase was filtered and the filtrate was concentrated under reduced pressure. The residue was purified by preparative TLC (DCM/MeOH = 10/1) to give the title compound (3.5 mg, 23.6% yield in two steps) as a white solid. MS (ESI) m/z: 579.3 $[M+H]^+$; $^1H$ NMR (400 MHz, Methanol-$d_4$) δ 8.23 (d, J = 5.6 Hz, 1H), 8.06 (s, 1H), 7.65 (dd, J = 6.7, 2.0 Hz, 1H), 7.51-7.47 (m, 1H), 7.30-7.25 (m, 2H), 7.22-7.19 (m, 2H), 7.16-7.13 (m, 1H), 6.57-6.50 (m, 1H), 6.35 (td, J = 6.7, 1.4 Hz, 1H), 5.26 (s, 2H), 5.16 (s, 2H), 4.64 (d, J = 2.4 Hz, 2H), 3.95 (s, 3H), 3.78 (s, 2H), 3.49-3.43 (m, 2H), 3.39-3.31 (m, 2H), 3.31 (s, 3H), 3.22-3.08 (m, 2H).

Example 31. N-((3-fluoro-4-methoxypyridin-2-yl)methyl)-3-((3-fluorooxetan-3-yl)methoxy)methyl (Compound 235) was prepared according to the following synthetic scheme:

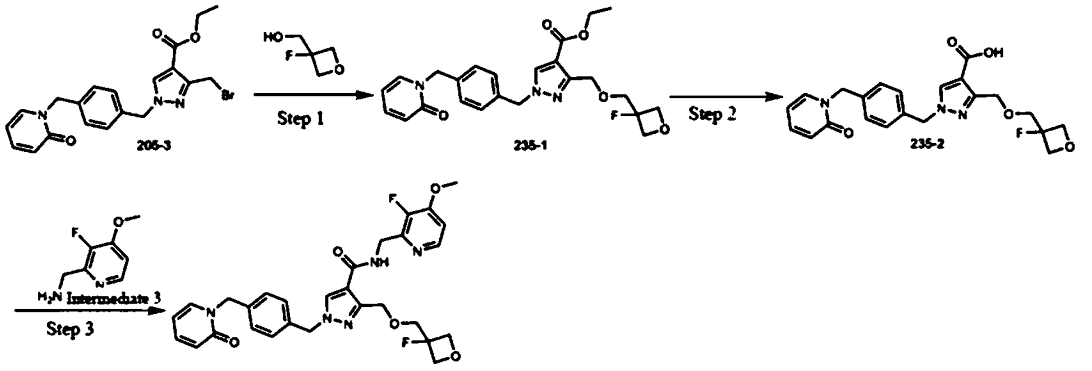


Compound 235

Step 1: Preparation of ethyl 3-((3-fluorooxetan-3-yl)methoxy)methyl)-1-(4-(2-oxopyridin-1 (2H)-yl)methylbenzyl)-1H-pyrazole-4-carboxylate: A solution of Compound (3-fluorooxetan-3-yl)methanol (25 mg, 0.24 mmol, 2.0 eq) in THF (2 mL) was added NaH (11 mg, 0.26 mmol, 2.2 eq) at 0°C and the mixture was stirred for 1 h. Compound 205-3 (50 mg, 0.12 mmol, 1.0 eq) was added dropwise to the above mixture at 0°C. The resulting mixture was stirred at room temperature for an additional hour. After monitoring the completion of the reaction by LCMS, the reaction mixture was diluted with $H_2O$ (10 mL), extracted with EtOAc (10 mL * 3), and dried over anhydrous $Na_2SO_4$. The organic phase was filtered and the filtrate was concentrated under reduced pressure. The residue was purified by preparative TLC (DCM/MeOH = 10/1) to give the title compound (25 mg, 47.3% yield) as a white solid. MS (ESI) m/z: 456.3[m+H]+.

Step 2: Preparation of 3-((3-fluorooxetan-3-yl)methoxy)methyl)-1-(4-((2-oxopyridin-1(2H)-yl)methyl)benzyl)-1H-pyrazole-4-carboxylic acid: To a solution of Compound 235-1 (25 mg, 0.06 mmol, 1.0 eq) in MeOH (0.8 mL) and $H_2O$ (0.2 mL) was added $LiOH \cdot H_2O$ (10 mg, 0.24 mmol, 4.0 eq) at room temperature and the mixture was stirred at 60°C for 2 h. After monitoring the completion of the reaction by LCMS, the pH of the reaction mixture was adjusted to 4-5 with HCl solution (4.0 M). After concentration in vacuo, the crude title compound was obtained, which was used directly in the next step. MS(ESI)m/z: 428.3[m+H]+.

Step 3: Preparation of N-((3-fluoro-4-methoxypyridin-2-yl)methyl)-3-((3-fluorooxetan-3-yl)methoxy)methyl: a mixture of Compound 235-1 (from the previous step) and Intermediate 3 (14 mg, 0.07 mmol, 1.2 eq) in DMF (3 mL) was added HATU (34 mg, 0.09 mmol, 1.5 eq) and TEA (18 mg, 0.18 mmol, 3.0 eq) and the mixture was stirred overnight at room temperature. After the reaction was monitored for completion by LCMS, the reaction mixture was diluted with $H_2O$ (10 mL) and extracted with EtOAc (10 mL * 3) and dried over anhydrous $Na_2SO_4$. The organic phase was filtered and the filtrate was concentrated under reduced pressure. The residue was purified by preparative TLC (DCM/MeOH = 10/1) to give the title compound (3.5 mg, two-step yield 10.3%) as a white solid. MS (ESI) m/z: 566.3[m+H]+. $^1H$ NMR (400 MHz, Chloroform-d) δ 8.21 (d, J = 5.5 Hz, 1H), 8.12-8.09 (d, J = 11.0 Hz, 1H), 7.90 (s, 1H), 7.36-7.26 (m, 3H), 7.23-7.19 (m, 2H), 6.82 (t, J = 6.0 Hz, 1H), 6.60 (d, J = 9.1, 1H), 6.15 (td, J = 6.7, 1.4 Hz, 1H), 5.22 (s, 2H), 5.12 (s, 2H), 4.78 (s, 2H), 4.75-4.64 (m, 4H), 4.58-4.46 (m, 2H), 3.93 (s, 3H), 3.92-3.88 (m, 2H).

Example 32. N-((3-fluoro-4-methoxypyridin-2-yl)methyl)-3-(3-hydroxy-3-methylazetidin-1-yl)-1-(4-(2-oxopyridin-1(2H)-yl)benzyl)-1H-pyrazole-4-carboxamide (Compound 296) was prepared according to the following synthetic scheme:

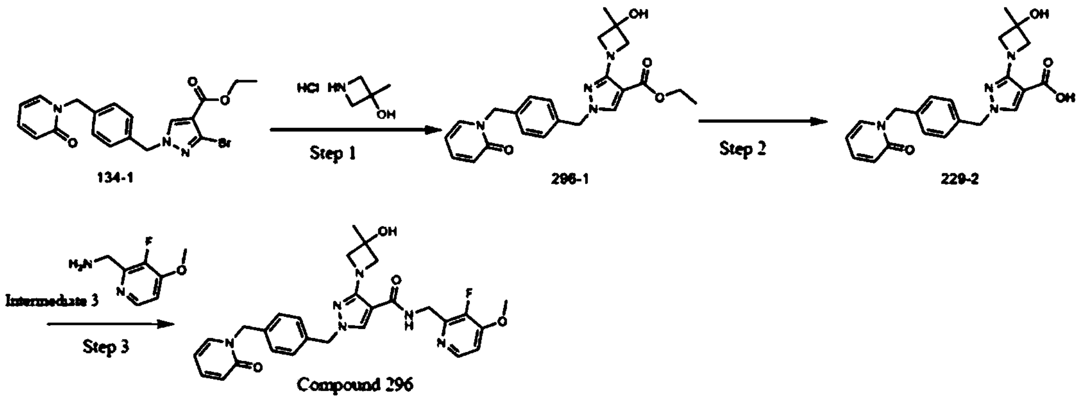


Step 1: Preparation of ethyl 3-(3-hydroxy-3-methylazetidin-1-yl)-1-(4-((2-oxopyridin-1(2H)-yl)methyl)benzyl)-1H-pyrazole-4-carboxylate: Compound 134-1 (40 mg, 0.096 mmol), 3-methylazetidin-3-ol hydrochloride (18 mg, 0.14 mmol), XantphosPdG3 (18 mg, 0.019 mmol) and cesium carbonate (94 mg, 0.29 mmol) were added to NMP (2 mL) and the mixture was reacted at 120°C overnight. The mixture was cooled to room temperature, added with water (20 mL), extracted with ethyl acetate (30 mL × 3). The organic phases were combined, washed separately with water (50 mL × 1) and saturated aqueous sodium chloride solution (50 mL × 1), dried over anhydrous sodium sulfate, and concentrated under reduced pressure to give a residue. The residue was purified on preparative plates (dichloromethane/methanol = 10:1) to give the title compound (25 mg, yield: 61.7%) as a white solid. MS (ESI) M/Z: 423.2 $[M+H]^+$.

Step 2: Preparation of 3-(3-hydroxy-3-methylazetidin-1-yl)-1-(4-((2-oxopyridin-1(2H)-yl)methyl)benzyl)-1H-pyrazole-4-carboxylic acid: Compound 296-1 (25 mg, 0.059 mmol) and lithium hydroxide monohydrate (5 mg, 0.12 mmol) were added to methanol (2 mL) and water (0.4 mL), and the mixture was reacted overnight at room temperature. The reaction solution was neutralized to pH = 5-6 with dioxane hydrochloride solution, and the reaction solution was concentrated under reduced pressure to obtain the crude product of the title compound, which can be directly used in the next step. MS (ESI) M/Z: 395.1 $[M+H]^+$.

Step 3: Preparation of N-((3-fluoro-4-methoxypyridin-2-yl)methyl)-3-(3-hydroxy-3-methylazetidin-1-yl)-1-(4-(2-oxopyridin-1(2H)-yl)benzyl)-1H-pyrazole-4-carboxamide: Crude product 296-2, Intermediate 3 (17 mg, 0.089 mmol), HOBt (24 mg, 0.18 mmol), EDCl (35 mg, 0.18 mmol) and DIEA (0.041 mL, 0.24 mmol) were sequentially added to DMF (2 mL), and the mixture was reacted at room temperature for 1 hour. After addition of water (20 mL) and extraction with dichloromethane (30 mL × 3), the organic phases were combined, washed separately with water (50 mL × 1) and saturated aqueous sodium chloride solution (50 mL × 1), dried over anhydrous sodium sulfate, and concentrated under reduced pressure to give a residue, which was isolated on a preparative plate (DCM: MeOH = 10:1) to give the title compound (10 mg, yield: 31.8%) as a white solid. MS (ESI) M/Z: 533.2 $[M+H]^+$; $^1$H NMR (400 MHz, Methanol-$d_4$) δ 8.15 (d, J = 7.8, 5.6 Hz, 1H), 7.87 (s, 1H), 7.69 - 7.62 (m, 1H), 7.49 (ddd, J = 8.9, 6.7, 2.1 Hz, 1H), 7.31 - 7.25 (m, 2H), 7.25 - 7.18 (m, 2H), 7.09 (dd, J = 6.7, 5.6 Hz, 1H), 6.58 - 6.50 (m, 1H), 6.36 (td, J = 6.7, 1.4 Hz, 1H), 5.16 (s, 2H), 5.08 (s, 2H), 4.56 (d, J = 2.2 Hz, 2H), 3.94 (s, 3H), 3.54 - 3.43 (m, 2H), 3.38 - 3.29 (m, 2H), 1.23 (s, 3H).

Using the appropriate starting materials, the compounds listed in the table below were prepared using procedures similar to those described in Examples 8, 30, 31, and 32.

Table 7.

| NO. | Structural formula | Mass spectrum ESI (+) m/z $[M + 1]^+$ |
|---|---|---|
| 162 | 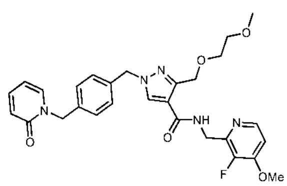 | 536.2 |
| 164 | 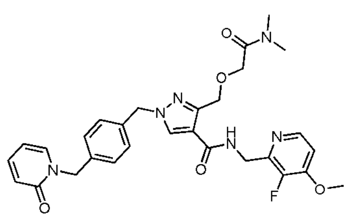 | 563.2 |

(continued)

| NO. | Structural formula | Mass spectrum ESI (+) m/z [M + 1]⁺ |
|---|---|---|
| 169 | 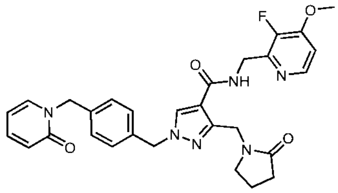 | 545.2 |
| 171 | 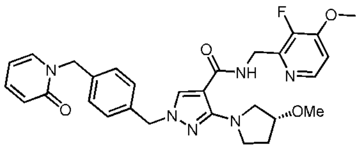 | 547.2 |
| 172 | 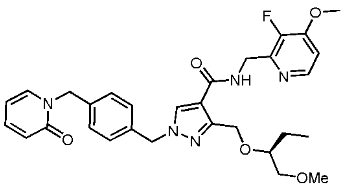 | 564.2 |
| 174 | 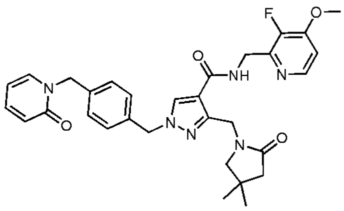 | 573.3 |
| 191 | 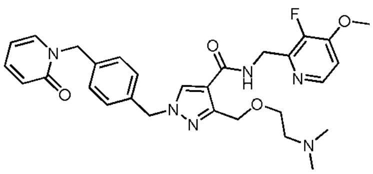 | 549.3 |
| 201 | 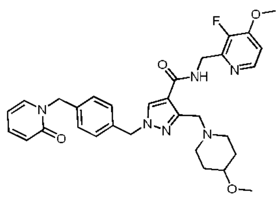 | 575.3 |
| 213 | 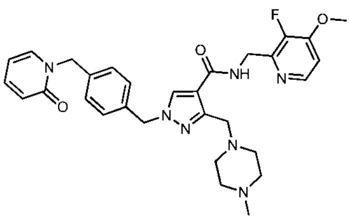 | 560.3 |
| 219 | 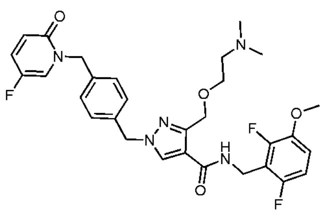 | 584.2 |

(continued)

| NO. | Structural formula | Mass spectrum ESI (+) m/z [M + 1]+ |
|---|---|---|
| 220 | 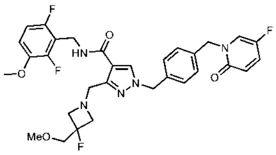 | 614.2 |
| 222 | 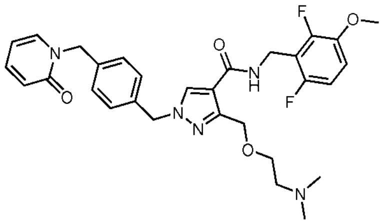 | 566.3 |
| 223 | 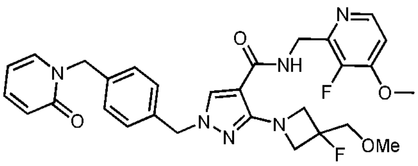 | 565.2 |
| 224 | 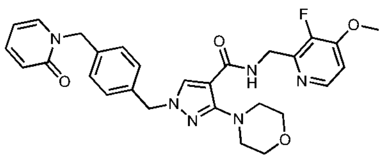 | 533.2 |
| 225 | 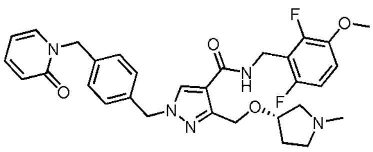 | 578.3 |
| 226 | 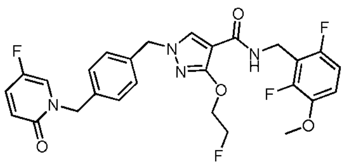 | 545.2 |
| 229 | 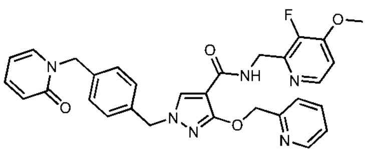 | 555.2 |
| 230 | 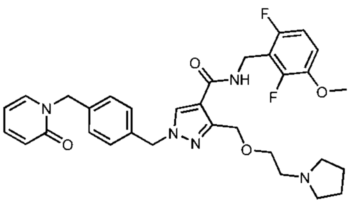 | 592.3 |

(continued)

| NO. | Structural formula | Mass spectrum ESI (+) m/z [M + 1]+ |
|---|---|---|
| 231 | 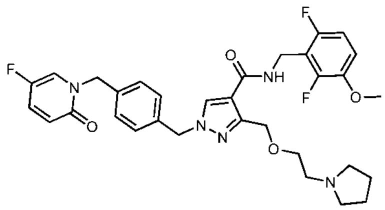 | 610.3 |
| 232 | 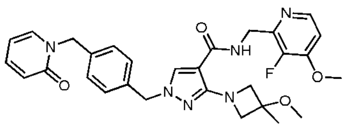 | 545.2 |
| 233 | 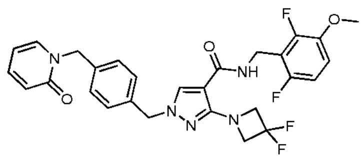 | 556.2 |
| 234 | 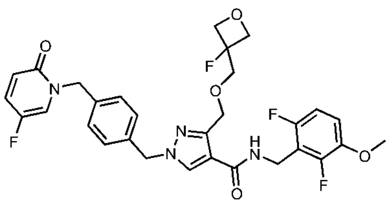 | 601.2 |
| 235 | 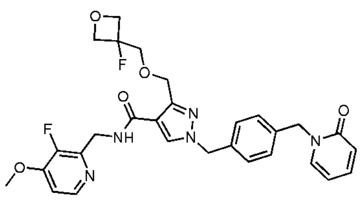 | 566.2 |
| 236 | 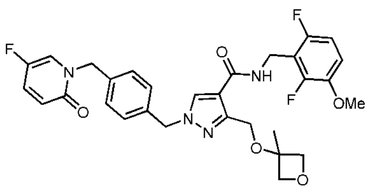 | 583.2 |
| 237 | 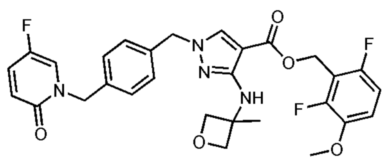 | 569.2 |
| 238 | 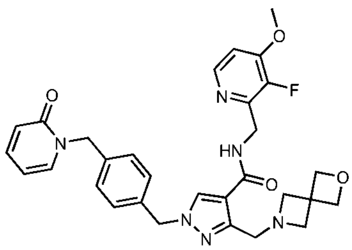 | 559.2 |

(continued)

| NO. | Structural formula | Mass spectrum ESI (+) m/z [M + 1]+ |
|---|---|---|
| 239 | 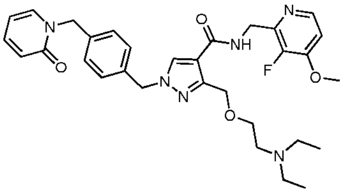 | 577.3 |
| 240 | 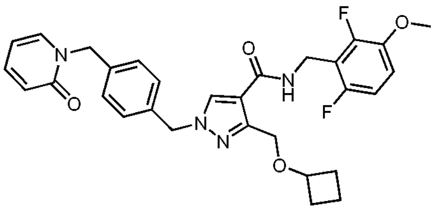 | 549.2 |
| 247 | 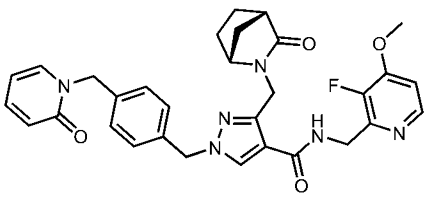 | 571.2 |
| 248 | 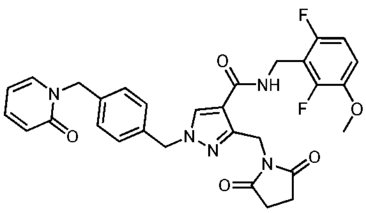 | 576.2 |
| 249 | 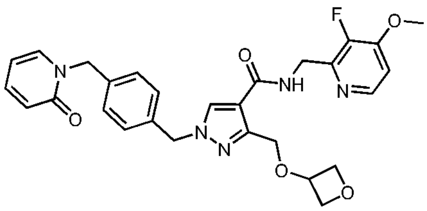 | 534.2 |
| 252 | 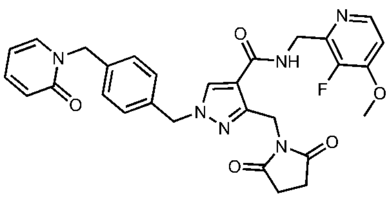 | 539.2 |
| 265 | 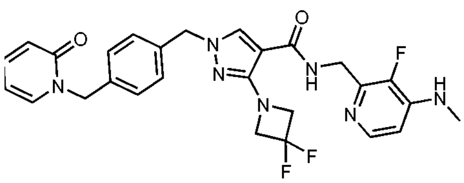 | 538.2 |
| 268 | 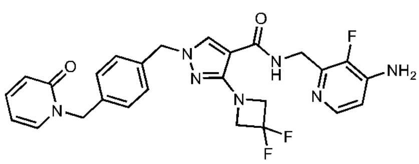 | 524.2 |

(continued)

| NO. | Structural formula | Mass spectrum ESI (+) m/z [M + 1]+ |
|---|---|---|
| 272 | 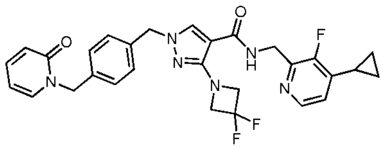 | 549.2 |
| 277 | 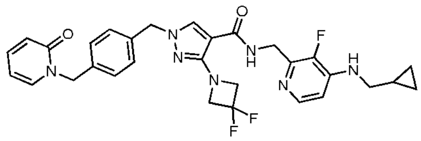 | 578.2 |
| 278 | 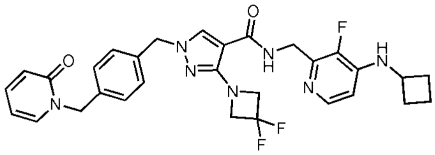 | 578.2 |
| 279 | 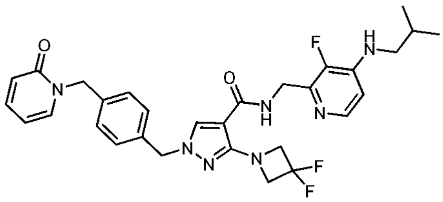 | 580.3 |
| 281 | 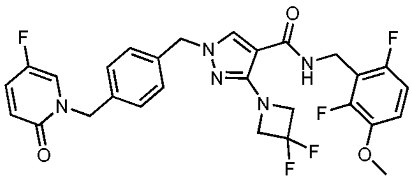 | 574.2 |
| 284 | 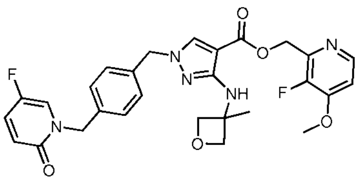 | 552.2 |
| 285 | 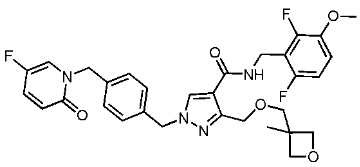 | 597.2 |
| 286 | 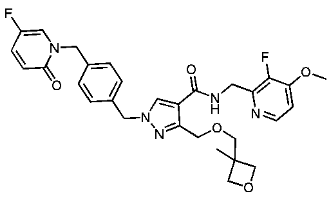 | 580.2 |
| 288 | 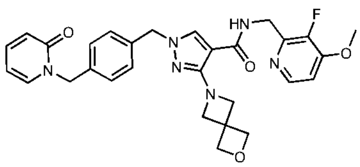 | 545.2 |

(continued)

| NO. | Structural formula | Mass spectrum ESI (+) m/z [M + 1]+ |
|---|---|---|
| 289 | 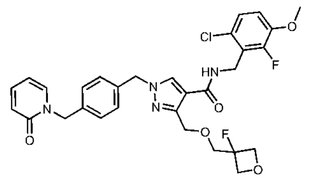 | 599.2 |
| 290 | 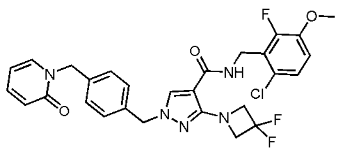 | 572.2 |
| 291 | 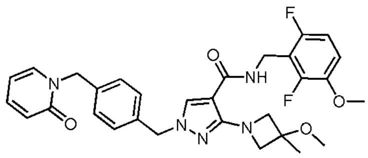 | 564.2 |
| 294 | 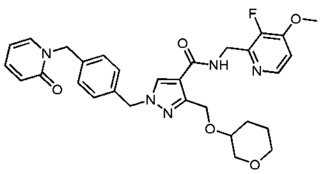 | 562.2 |
| 295 | 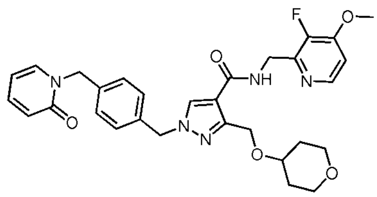 | 562.2 |
| 296 | 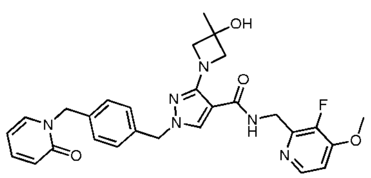 | 533.2 |
| 297 | 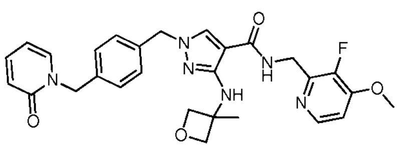 | 533.2 |
| 298 | 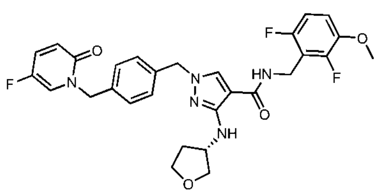 | 568.2 |
| 299 | 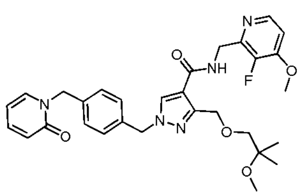 | 564.3 |

(continued)

| NO. | Structural formula | Mass spectrum ESI (+) m/z [M + 1]+ |
|---|---|---|
| 300 | 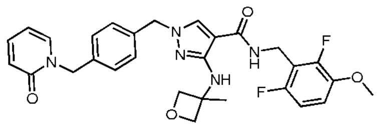 | 550.2 |
| 305 | 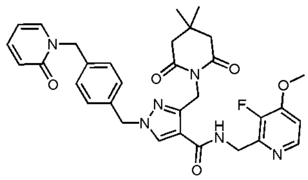 | 601.3 |
| 306 | 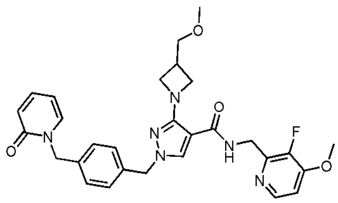 | 547.2 |
| 307 | 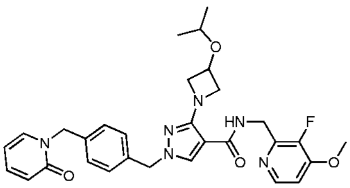 | 561.3 |
| 308 | 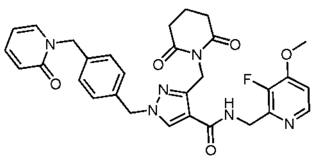 | 573.2 |
| 309 | 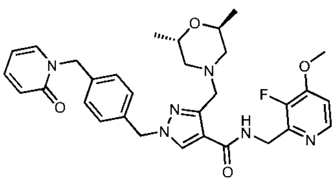 | 575.3 |
| 313 | 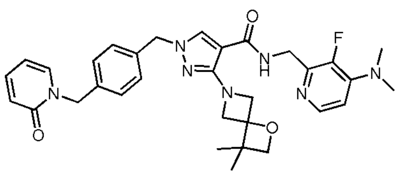 | 586.3 |
| 315 | 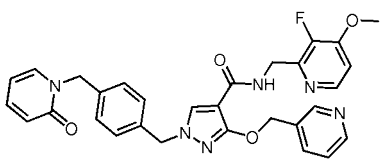 | 555.2 |

(continued)

| NO. | Structural formula | Mass spectrum ESI (+) m/z [M + 1]+ |
|---|---|---|
| 316 | 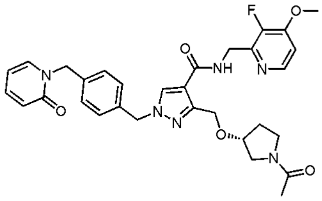 | 589.3 |
| 318 | 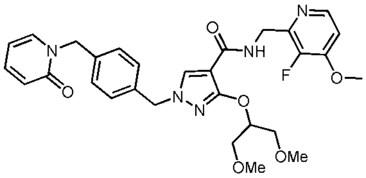 | 566.2 |
| 319 | 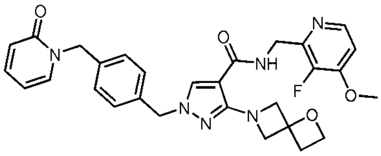 | 545.2 |
| 320 | 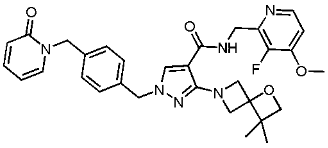 | 573.3 |
| 324 | 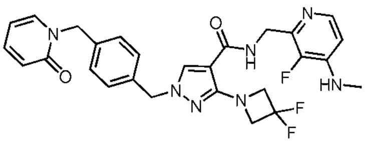 | 538.2 |
| 332 | 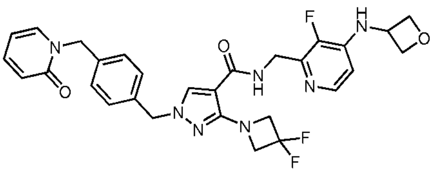 | 580.2 |

Example 33. N-((3-fluoro-4-methoxypyridin-2-yl)methyl)-2',4'-dimethyl-1-(4-((2-oxopyridin-1(2H)yl)methyl)benzyl)-1H,2'H-[3,3'-bispyrazole]-4-carboxamide (Compound 221) was prepared according to the following synthetic scheme:

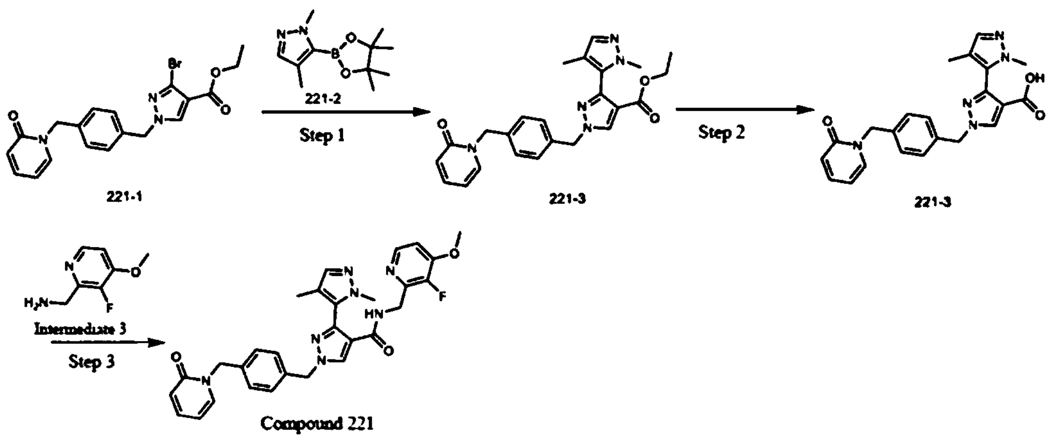


Step 1: Preparation of 2',4'-dimethyl-1-(4-((2-oxopyridin-1(2H)yl)methyl)benzyl)-1H,2'H-[3,3'-bispyrazolyl]-4-carboxylic acid: Under nitrogen, Compounds 221-1 (30 mg, 0.072 mmol), 221-2 (24 mg 0.11 mmol), $Pd(dppf)_2Cl_2$ (6 mg, 0.0072 mmol) and potassium phosphate (54 mg, 0.25 mmol) were added to 1,4-dioxane (3 mL) and water (0.6 mL), respectively, and the mixture was reacted at 110°C overnight. The mixture was cooled to room temperature, added with water (30 mL), extracted with ethyl acetate (50 mL × 3). The organic phases were combined, washed separately with water (50 mL × 1) and saturated aqueous sodium chloride solution (50 mL × 1), dried over anhydrous sodium sulfate, and concentrated under reduced pressure to give a residue. The residue was isolated on preparative plates (DCM: MeOH = 10:1) to give the title compound (12 mg, yield: 38.7%) as a white solid. MS (ESI) M/Z: 432.2 $[M+H]^+$.

Step 2: Preparation of ethyl 2',4'-dimethyl-1-(4-((2-oxopyridin-1(2H)yl)methyl)benzyl)-1H,2'H-[3,3'-bispyrazole]-4-carboxylate: Compound 221-3 (12 mg, 0.028 mmol) and lithium hydroxide monohydrate (3 mg, 0.056 mmol) were added to methanol (2 mL) and water (0.5 mL) and the mixture was reacted overnight at room temperature. The reaction solution was neutralized to pH = 5-6 with dioxane hydrochloride solution, and the reaction solution was concentrated under reduced pressure to obtain the crude product of the title compound, which was directly used in the next step. MS (ESI) M/Z: 404.2 $[M+H]^+$.

Step 3: Preparation of N-((3-fluoro-4-methoxypyridin-2-yl)methyl)-2',4'-dimethyl-1-(4-((2-oxopyridin-1(2H)yl)methyl)benzyl)-1H,2'H-[3,3'-bispyrazole]-4-carboxamide: Crude product 221-4, Intermediate 3 (7 mg, 0.034 mmol), HATU (13 mg, 0.034 mmol) and TEA (0.016 mL, 0.11 mmol) were sequentially added to DMF (2 mL), and the mixture was reacted at room temperature for 1 hour. After addition of water (20 mL) and extraction with dichloromethane (30 mL × 3), the organic phases were combined, washed separately with water (50 mL × 1) and saturated aqueous sodium chloride solution (50 mL × 1), dried over anhydrous sodium sulfate, and concentrated under reduced pressure to give a residue, which was isolated on preparative plates (DCM: MeOH = 10:1) to give the title compound (6 mg, yield: 39.6%) as a white solid. MS (ESI) M/Z: 542.2 $[M+H]^+$; $^1H$ NMR (400 MHz, Methanol-$d_4$) δ 8.26 (s, 1H), 8.14 - 8.08 (m, 1H), 7.67 (ddd, J = 6.8, 2.1, 0.7 Hz, 1H), 7.53 - 7.45 (m, 1H), 7.33 - 7.28 (m, 5H), 7.11 - 7.03 (m, 1H), 6.58 - 6.51 (m, 1H), 6.36 (td, J = 6.7, 1.4 Hz, 1H), 5.39 (s, 2H), 5.17 (s, 2H), 4.52 (d, J = 2.2 Hz, 2H), 3.93 (s, 3H), 3.62 (s, 3H), 1.86 (s, 3H).

The compounds listed in the table below were prepared using the appropriate starting materials using procedures similar to those described in Example 33.

Table 8.

| NO. | Structural formula | Mass spectrum ESI (+) m/z $[M+1]^+$ |
|---|---|---|
| 165 | 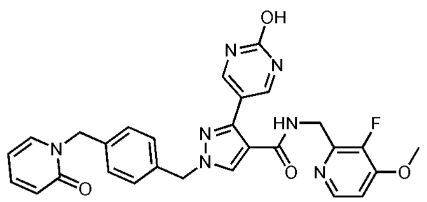 | 542.2 |
| 166 | 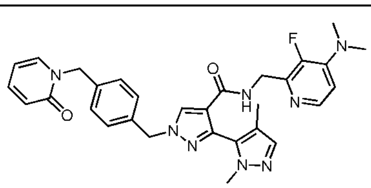 | 555.3 |

(continued)

| NO. | Structural formula | Mass spectrum ESI (+) m/z [M+1]+ |
|---|---|---|
| 301 | 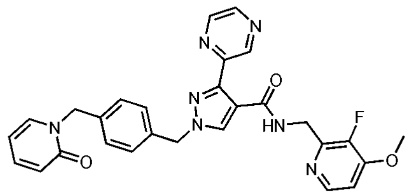 | 526.2 |
| 304 | 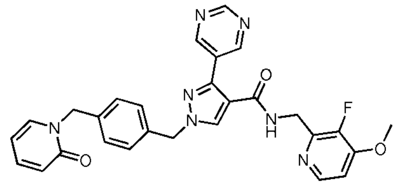 | 526.2 |

Example 34. N-(2,6-difluoro-3-methoxybenzyl)-3-(3,3-difluorocyclobutyl)-1-(4-((5-fluoro-2-oxopyridin-1(2H)yl)methyl) benzyl)-1H-pyrazole-4-carboxamide (Compound 209) was prepared according to the following synthetic scheme:

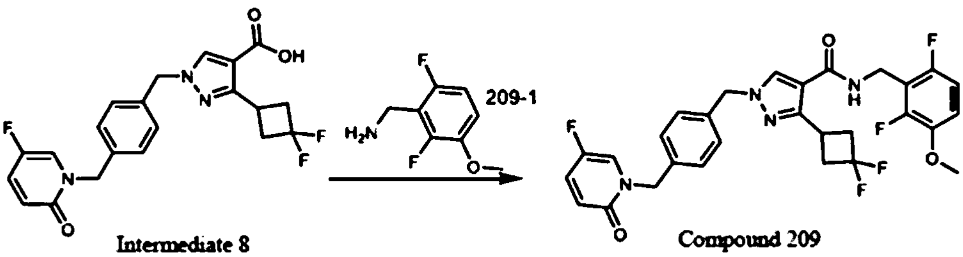


Intermediate 8 Compound 209

To a solution of Intermediate 8 (38 mg, 0.091 mmol) in DMF (2 mL) was added DIEA (35 mg, 0.27 mmol) and HATU (38 mg, 0.10 mmol) and the mixture was stirred at room temperature for 10 min. Compound 209-1 (16 mg, 0.091 mmol) was then added to the reaction. The mixture was stirred at room temperature for 30 minutes. After monitoring the completion of the reaction by LCMS, the reaction mixture was concentrated under reduced pressure. The residue was purified by preparative TLC (DCM: MeOH = 11:1) to give the title compound (11.41 mg, 21.89% yield) as a white solid. MS (ESI) m/z: 573.4 $[M+H]^+$; HPLC: 254 nm: 99.478%, 214 nm: 99.303%; $^1$H NMR (400 MHz, DMSO) δ 8.27 (t, J = 5.1 Hz, 1H), 8.18 (s, 1H), 8.10 - 7.94 (m, 1H), 7.57 (ddd, J = 10.3, 7.2, 3.4 Hz, 1H), 7.29 (d, J = 8.1 Hz, 2H), 7.21 (d, J = 8.1 Hz, 2H), 7.11 (td, J = 9.2, 5.1 Hz, 1H), 7.00 (t, J = 9.1 Hz, 1H), 6.43 (dd, J = 10.0, 5.4 Hz, 1H), 5.26 (s, 2H), 5.01 (s, 2H), 4.39 (d, J = 4.9 Hz, 2H), 3.81 (s, 3H), 3.75 (dd, J = 8.7, 6.1 Hz, 1H), 2.93 - 2.69 (m, 4H).

Example 35. N-((3-fluoro-4-(methylamino)pyridin-2-yl)methyl)-3-(oxetan-3-yl)-1-(4-((2-oxopyridin-1(2H)-yl)methyl)benzyl)-1H-pyrazole-4-carboxamide (Compound 260) was prepared according to the following synthetic scheme:

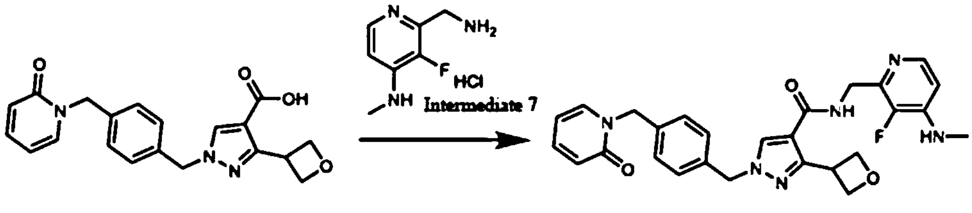


260-1 Compound 260

Compound 260-1 (220 mg, 0.60 mmol), 1-hydroxybenzotriazole (122 mg, 0.90 mmol), 1-(3-dimethylaminopropyl)-3-ethylcarbodiimide hydrochloride (172 mg, 0.9 mmol) and triethylamine (181 mg, 1.80 mmol) were sequentially

added to N,N-dimethylformamide (3 mL) and the mixture was stirred at room temperature for 10 min. Intermediate 7 (126 mg, 0.73 mmol) was added and the mixture was stirred at room temperature for 16 h, added with water (25 mL), and extracted with ethyl acetate (25 mL × 3). The organic phases were combined, washed separately with water (50 mL) and saturated sodium chloride (50 mL), dried over anhydrous sodium sulfate and filtered. The filtrate was collected and concentrated under reduced pressure to give a residue. The residue was purified by column chromatography (dichloromethane: methanol = 0-10%) to give the title Compound 260 (130 mg, yield: 43%) as a white solid. MS (ESI) M/Z: 503.21 $[M+H]^+$; $^1H$ NMR (400 MHz, DMSO-$d_6$) δ 8.30 (d, J = 6.0 Hz, 1H), 8.28 (s, 1H), 7.90 (d, J = 5.6 Hz, 1H), 7.77 (dd, J = 6.8, 2.0 Hz, 1H), 7.45 - 7.36 (m, 1H), 7.30 - 7.21 (m, 4H), 6.58 - 6.49 (m, 2H), 6.42 - 6.37 (m, 1H), 6.26 - 6.19 (m, 1H), 5.30 (s, 2H), 5.07 (s, 2H), 4.79 (dd, J = 8.4, 5.6 Hz, 2H), 4.68 (dd, J = 6.8, 5.6 Hz, 2H), 4.55 - 4.46 (m, 1H), 4.37 (dd, J = 5.6, 2.0 Hz, 2H), 2.73 (d, J = 4.8 Hz, 3H).

Using the appropriate starting materials, the compounds listed below were prepared using procedures similar to those described in Examples 34 and 35.

Table 9.

| NO. | Structural formula | Mass spectrum ESI (+) m/z $[M + 1]^+$ |
|---|---|---|
| 173 | 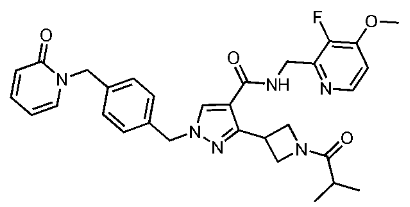 | 573.3 |
| 179 | 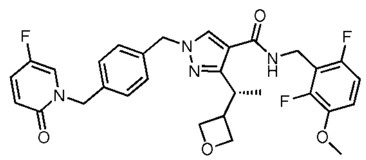 | 567.2 |
| 180 | 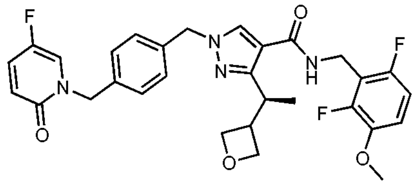 | 567.2 |
| 208 | 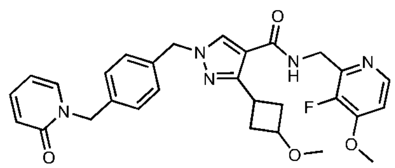 | 532.2 |
| 209 | 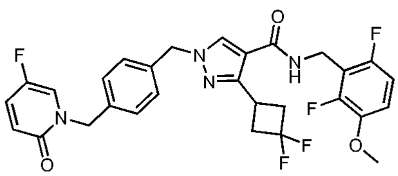 | 573.2 |
| 210 | 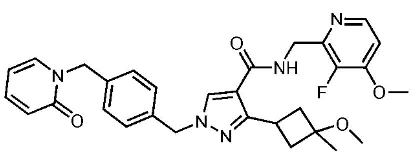 | 546.2 |
| 211 | 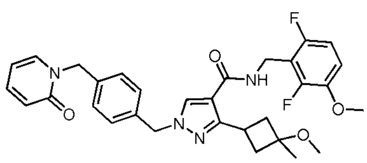 | 563.2 |

(continued)

| NO. | Structural formula | Mass spectrum ESI (+) m/z [M + 1]+ |
|---|---|---|
| 216 | 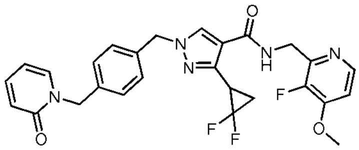 | 524.2 |
| 218 | 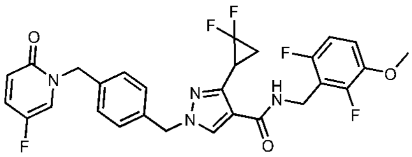 | 559.2 |
| 227 | 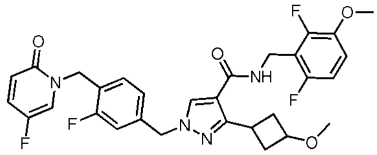 | 585.2 |
| 228 | 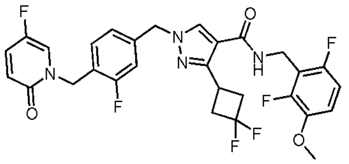 | 591.2 |
| 258 | 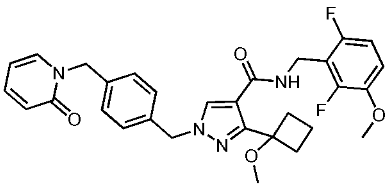 | 549.2 |
| 260 | 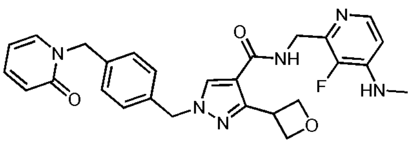 | 503.2 |
| 261 | 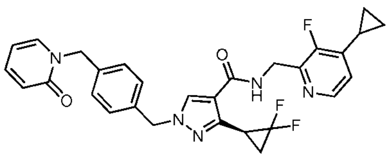 | 534.2 |
| 263 | 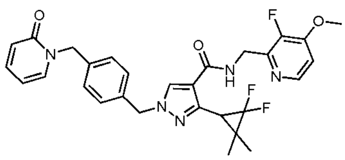 | 552.2 |
| 267 | 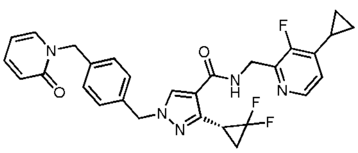 | 534.2 |

(continued)

| NO. | Structural formula | Mass spectrum ESI (+) m/z [M + 1]+ |
|---|---|---|
| 269 | 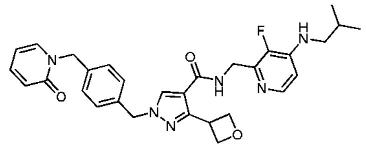 | 545.3 |
| 273 | 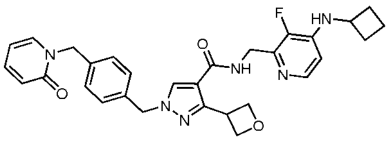 | 543.2 |
| 274 | 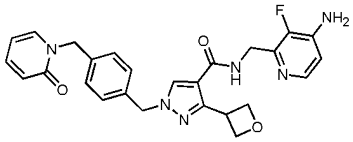 | 489.2 |
| 276 | 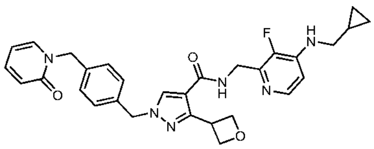 | 543.2 |
| 282 | 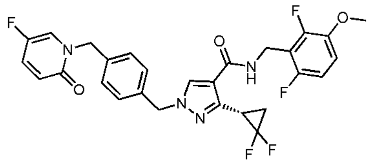 | 559.2 |
| 287 | 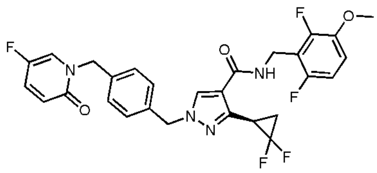 | 559.2 |
| 310 | 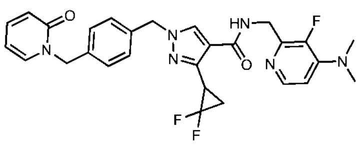 | 537.2 |
| 317 | 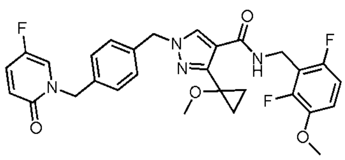 | 553.2 |
| 325 | 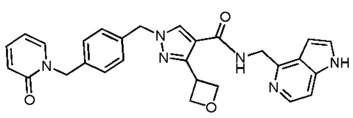 | 495.2 |
| 326 | 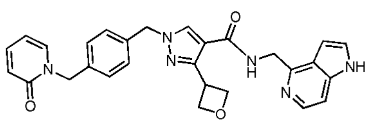 | 565.2 |

(continued)

| NO. | Structural formula | Mass spectrum ESI (+) m/z [M + 1]+ |
|---|---|---|
| 331 | 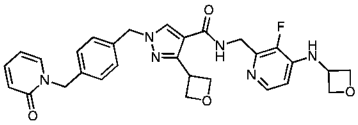 | 545.2 |
| 333 | 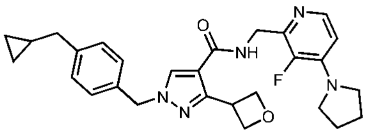 | 490.3 |
| 334 | 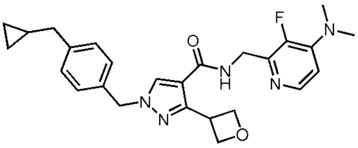 | 464.3 |
| 335 | 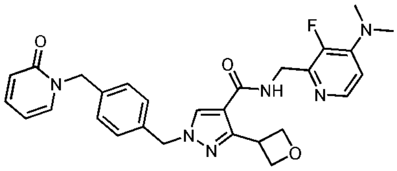 | 517.2 |
| 336 | 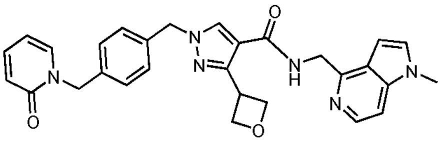 | 509.2 |
| 337 | 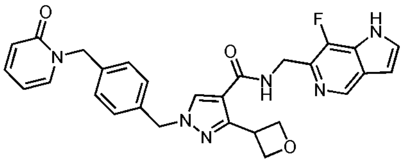 | 513.2 |
| 338 | 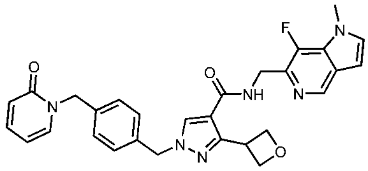 | 527.2 |
| 339 | 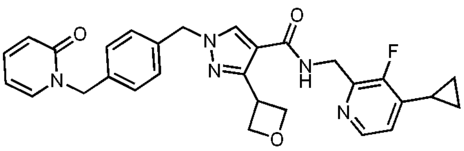 | 514.2 |
| 340 | 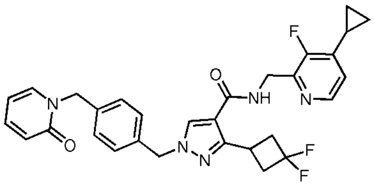 | 548.2 |

(continued)

| NO. | Structural formula | Mass spectrum ESI (+) m/z [M + 1]+ |
|---|---|---|
| 342 | 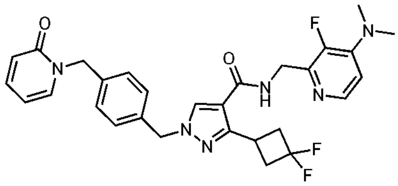 | 551.2 |
| 343 | 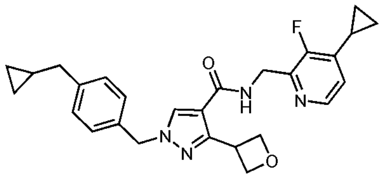 | 461.2 |
| 344 | 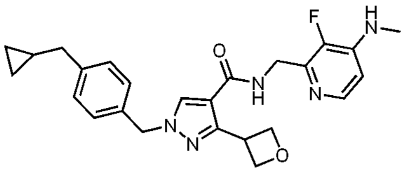 | 450.2 |
| 346 | 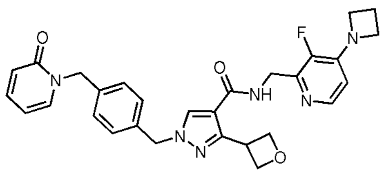 | 529.3 |
| 352 | 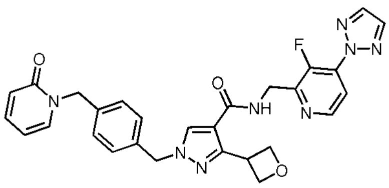 | 541.2 |

Example 36. N-((6,7-dihydro-5H-pyrrolo[3,4-d]pyrimidin-2-yl)methyl)-3-(methoxymethyl)-1-(4-(2-oxopyridin-1(2H)-yl) methyl]benzyl)-1H-pyrazole-4-carboxamide (Compound 144) was prepared according to the following synthetic scheme:

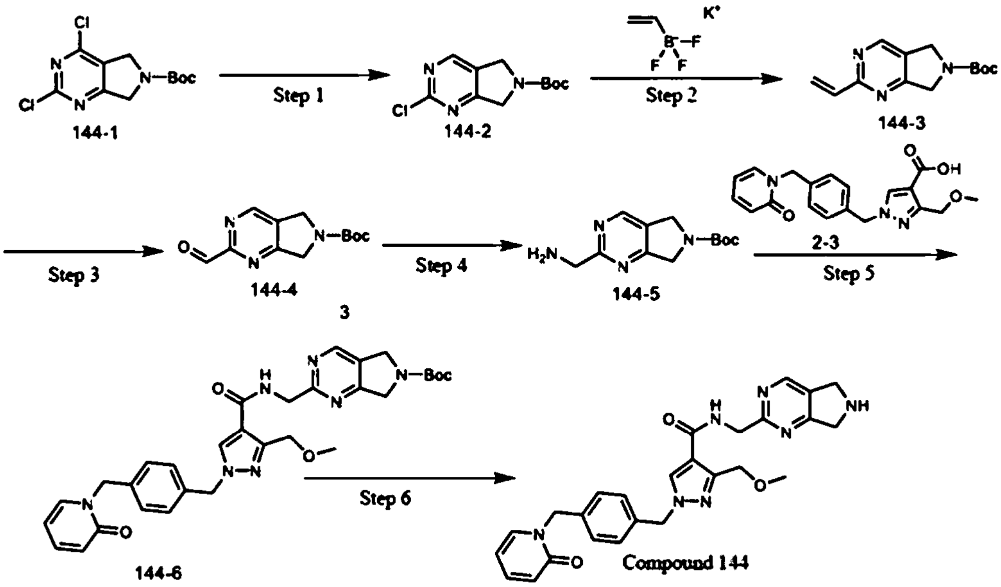


Step 1: Preparation of tert-butyl 2-chloro-5,7-dihydro-6H-pyrrolo[3,4-d]pyrimidine-6-carboxylate: To solution of tert-butyl 2,4-dichloro-5,7-dihydro-6-pyrrolo[3,4-d]pyrimidine-6-carboxylate (1.0 g, 3.45 mmol) in a MeOH (30 mL) was added AcOH (2.07 g, 34.5 mmol) and Zn (900 mg, 13.8 mmol), and the mixture was stirred at 70°C for 3 h. After monitoring the completion of the reaction by LCMS, the reaction mixture was cooled to room temperature and filtered through a pad of celite. The filtrate was concentrated under reduced pressure and the crude compound was purified by silica gel chromatography (eluting with PE: EA = 100:0 to 60:40) to give the desired title compound (600 mg, 68.03% yield). MS (ESI) M/Z: 256.0[M+H]+.

Step 2: Preparation of tert-butyl 2-vinyl-5,7-dihydro-6-pyrrolo[3,4-d]pyrimidine-6-carboxylate: To a solution of Compound 144-2 (730 mg, 2.85 mmol) in 1,4-dioxane (20 mL) and water (4 mL) was added Compound SM2 (572 mg, 4.28 mmol) and $Na_2CO_3$ (906 mg, 8.55 mmol). $Pd(dppf)Cl_2$ (208 mg, 0.29 mmol) was added and the mixture was stirred at 100°C for 3 h. After monitoring the completion of the reaction by LCMS, the reaction mixture was cooled to room temperature and filtered through a pad of celite. The reaction mixture was poured into 200 mL water and extracted with EA (200 mL * 3). The combined organic layers were dried over anhydrous $Na_2SO_4$. The organic phase was filtered and the filtrate was concentrated under reduced pressure. The residue was purified by silica gel chromatography (eluting with PE: EA = 100:0 to 50:50) to afford the desired title compound (600 mg, 84.99% yield) as a white solid. $^1H$ NMR (400 MHz, DMSO) δ 8.74 (d, J = 7.9 Hz, 1H), 6.82 (dd, J = 17.3, 10.5 Hz, 1H), 6.52 (d, J = 17.4 Hz, 1H), 5.74 (d, J = 10.7 Hz, 1H), 4.63 (d, J = 12.8 Hz, 2H), 4.54 (t, J = 16.2 Hz, 2H), 1.46 (s, 9H).

Step 3: Preparation of tert-butyl 2-formyl-5,7-dihydro-6-pyrrolo[3,4-d]pyrimidine-6-carboxylate: To a solution of Compound 144-3 (500 mg, 2.02 mmol) in 1,4-dioxane (20 mL) and water (20 mL) was added $NaIO_4$ (1.3 g, 6.06 mmol) and $K_2OsO_4$*$2H_2O$ (74 mg, 0.2 mmol), and the mixture was stirred at room temperature for 2 h. After monitoring the completion of the reaction by LCMS, the reaction mixture was poured into 100 mL water and extracted with EA (100 mL * 3). The combined organics were concentrated. The residue was purified by preparative TLC (EA: PE = 1:1) to give the desired title compound (218 mg, 43.25% yield) as a white solid. MS (ESI) M/Z: 268.2[M+H]+.

Step 4: Preparation of tert-butyl 2-(aminomethyl)-5,7-dihydro-6-pyrrolo[3,4-d]pyrimidine-6-carboxylate: To a solution of Compound 144-4 (218 mg, 0.87 mmol) in MeOH (2 mL) was added $NH_3$-MeOH (7.0 M, 5 mL) and AcOH (0.1 mL). The mixture was stirred at room temperature for 30 minutes. $NaBH_4$ (20 mg, 0.52 mmol) was added and the mixture was stirred at room temperature for 30 min. After monitoring the completion of the reaction by LCMS, the reaction mixture was diluted with $H_2O$ (80 mL) and extracted with DCM (80 mL * 3). The combined organic layers were dried over anhydrous $Na_2SO_4$. The organic phase was filtered and the filtrate was concentrated under reduced pressure. The residue was purified by reverse phase HPLC to afford the title compound (105 mg, 47.97% yield). MS (ESI) M/Z: 251.2[M+H]+.

Step 5: Preparation of tert-butyl 2-((3-(methoxymethyl)-1-(4-(2-oxopyridin-1(2H)-yl)ymethyl)benzyl)-1H-pyrazol-4-carboxamido)methyl-5,7-dihydro-6-pyrrolo[3,4-d]pyrimidine-6-carboxylate: To a solution of Compound 2-3 (46 mg, 0.13 mmol) in DMF (3 mL) was added HATU (54 mg, 0.14 mmol) and DIEA (50 mg, 0.39 mmol), and the mixture was stirred at room temperature for 10 min. Compound 144-5 (55 mg, 0.22 mmol) was then added to the reaction. The mixture was stirred at room temperature for 1 hour. After monitoring the completion of the reaction by LCMS, the reaction mixture was diluted with water (50 mL) and extracted with DCM (50 mL * 3). The combined organics were concentrated. The residue was purified by preparative TLC (DCM: MeOH = 15:1) to afford the desired title compound (36 mg, 47.22% yield) as a white solid. MS (ESI) M/Z: 586.7[M+H]+.

Step 6: Preparation of N-((6,7-dihydro-5H-pyrrolo[3,4-d]pyrimidin-2-yl)methyl)-3-(methoxymethyl)-1-(4-(2-oxopyridin-1(2H)-yl)methyl]benzyl)-1H-pyrazole-4-carboxamide: To a solution of Compound 144-6 (30 mg, 0.051 mmol) in DCM (2 mL) was added TFA (0.5 mL). The mixture was stirred at room temperature for 5 hours. After completion of the reaction, the reaction was monitored by LCMS. TEA (0.5 mL) was added to the reaction mixture. The mixture was concentrated. The residue was purified by preparative TLC (DCM: MeOH = 10:1) to give the title compound (13.44 mg, 54.04% yield). MS (ESI) M/Z: 486.2[M+H]+. $^1$H NMR (400 MHz, DMSO) δ 8.62 (t, J = 38.9 Hz, 2H), 8.28 (s, 1H), 7.80 - 7.68 (m, 1H), 7.41 (ddd, J = 8.8, 6.7, 2.0 Hz, 1H), 7.26 (q, J = 8.3 Hz, 4H), 6.40 (d, J = 9.1 Hz, 1H), 6.22 (t, J = 6.7 Hz, 1H), 5.29 (s, 2H), 5.07 (s, 2H), 4.72 - 4.43 (m, 6H), 4.06 (d, J = 39.7 Hz, 2H), 3.28 (s, 3H).

The compound listed in the table below was prepared using the appropriate starting materials using procedures similar to those described in Example 36.

Table 10.

| NO. | Structural formula | Mass spectrum ESI (+) m/z $[M+1]^+$ |
|---|---|---|
| 156 | 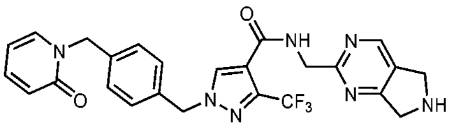 | 510.2 |

Example 37. N-((7-fluoro-1H-pyrrolo[3,2-c]pyridin-6-yl)methyl)-3-(oxetan-3-yl)-1-(4-((2-oxopyridin-1(2H)-yl)methyl) benzyl)-1H-pyrazole-4-carboxamide (Compound 337) was prepared according to the following synthetic scheme:

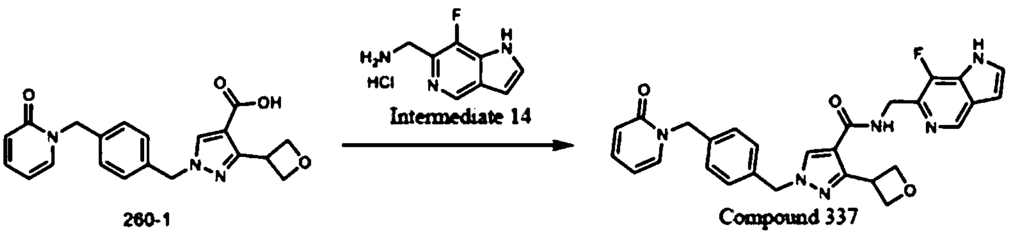


260-1

Compound 337

To a solution of Compound 260-1 (40 mg, 0.11 mmol) in DMF (3 mL) was added DIEA (43 mg, 0.33 mmol) and HATU (46 mg, 0.12 mmol), and the mixture was stirred at room temperature for 5 min. Intermediate 14 (25 mg, 0.12 mmol) was then added to the reaction. The mixture was stirred at room temperature for 30 minutes. After monitoring the completion of the reaction by LCMS, the reaction mixture was concentrated under reduced pressure. The residue was purified by preparative TLC (DCM: MeOH = 11:1) and reverse phase chromatography to give the title compound (23.56 mg, 41.99% yield) as a white solid. MS (ESI) m/z: 513.5 $[M+H]^+$; HPLC: 254 nm: 98.115%, 214 nm: 98.620%; $^1$H NMR(400 MHz, DMSO) δ 12.07 (s, 1H), 8.60 (d, J = 2.3 Hz, 1H), 8.35 (t, J = 5.4 Hz, 1H), 8.28 (s, 1H), 7.76 (dd, J = 6.8, 1.7 Hz, 1H), 7.51 (d, J = 3.1 Hz, 1H), 7.41 (ddd, J = 8.9, 6.6, 2.1 Hz, 1H), 7.26 (q, J = 8.4 Hz, 4H), 6.65 (t, J = 3.3 Hz, 1H), 6.40 (d, J = 9.1 Hz, 1H), 6.22 (td, J = 6.7, 1.3 Hz, 1H), 5.29 (s, 2H), 5.07 (s, 2H), 4.80 (dd, J = 8.5, 5.6 Hz, 2H), 4.69 (dd, J = 6.9, 5.7 Hz, 2H), 4.55 (ddd, J = 23.4, 10.4, 4.9 Hz, 3H).

Example 38. N-((7-fluoro-1H-pyrrolo[3,2-c]pyridin-6-yl)methyl)-1-(4-((2-oxopyridin-1(2H)-yl)methyl)benzyl)-1H-pyrazole-4-carboxamide (Compound 362) and N-((7-fluoro-1-(2-hydroxyethyl)-1H-pyrrolo[3,2-c]pyridin-6-yl) methyl)-1-(4-((2-oxopyridin-1(2H)-yl)methyl]benzyl)-1H-pyrazole-4-carboxamide (Compound 369) were prepared according to the following synthetic scheme:

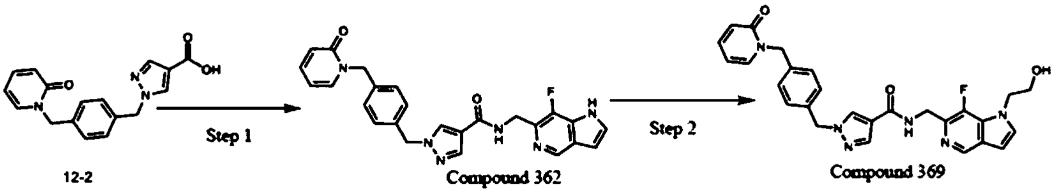


12-2

Compound 362

Compound 369

Step 1: To a solution of Compound 12-2 (50 mg, 0.14 mmol) in DMF (3 mL) was added DIEA (54 mg, 0.42 mmol) and HATU (59 mg, 0.15 mmol) and the mixture was stirred at room temperature for 5 min. Intermediate 14 (33 mg, 0.15 mmol) was then added to the reaction. The mixture was stirred at room temperature for 30 minutes. After monitoring the completion of the reaction by LCMS, the reaction mixture was concentrated under reduced pressure. The residue was purified by preparative TLC (DCM: MeOH = 15:1) and reverse phase chromatography to give the title Compound 362 (27.57 mg, 38.26% yield) as a white solid. MS (ESI) m/z: 457.2 $[M+H]^+$.

Step 2: Compound NaH (1.31 mg, 0.03 mmol, 60% in oil) was added to DMF (2 mL) at room temperature, the reaction was replaced with $N_2$ three times, Compound 362 (10 mg, 0.02 mmol) was slowly added to the reaction solution dropwise at 0°C and the mixture was reacted for 0.5 h. 2 (7.5 mg, 0.04 mmol) was slowly added to the reaction dropwise and the mixture was stirred at 70°C for 72 hours. Pre-TLC (DCM: MeOH = 9:1, Rf = 0.4) gave compound 369 (4.2 mg, 38.3% yield) as a white solid.

MS (ESI) M/Z: 313.2$[M+1]^+$; $^1$H NMR (400 MHz, DMSO-$D_6$) δ 8.53 (d, $J$ = 2.1 Hz, 1H), 8.46 (t, $J$ = 5.6 Hz, 1H), 8.20 (d, J= 0.7 Hz, 1H), 7.84 (d, J= 0.7 Hz, 1H), 7.71 (ddd, $J$ = 6.8, 2.1, 0.7 Hz, 1H), 7.43 (d, $J$ = 3.2 Hz, 1H), 7.37 (ddd, $J$ = 8.9, 6.6, 2.1 Hz, 1H), 7.22 (d, $J$ = 8.2 Hz, 2H), 7.17 (d, $J$ = 8.3 Hz, 2H), 6.58 (t, $J$ = 2.9 Hz, 1H), 6.36 (ddd, $J$ = 9.2, 1.4, 0.7 Hz, 1H), 6.18 (td, $J$ = 6.7, 1.4 Hz, 1H), 5.26 (s, 2H), 5.02 (s, 2H), 4.91 (t, $J$ = 5.3 Hz, 1H), 4.57 (dd, $J$ = 5.6, 2.5 Hz, 2H), 4.29 (t, $J$ = 5.6 Hz, 2H), 3.69 (q, $J$ = 5.4 Hz, 2H).

Example 39. N-((7-fluoro-1-methyl-2,3-dihydro-1H-pyrrolo[3,2-c]pyridin-6-yl)methyl)-1-(4-(2-oxopyridin-1(2H)-yl) methyl]benzyl)-1H-pyrazole-4-carboxamide (Compound 383) was prepared according to the following synthetic scheme:

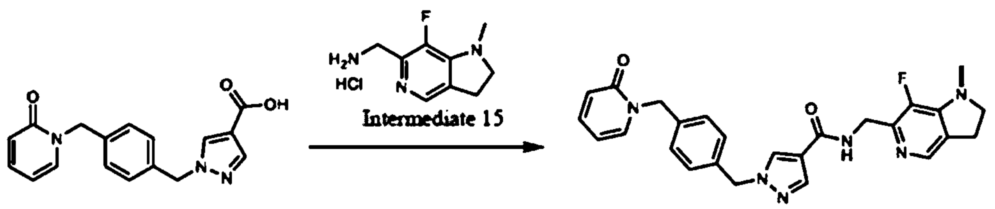


12-2

Compound 383

To a solution of Compound 12-2 (35 mg, 0.11 mmol) in DMF (2.5 mL) was added DIEA (42 mg, 0.33 mmol) and HATU (46 mg, 0.12 mmol) and the mixture was stirred at room temperature for 5 min. Intermediate 15 (47 mg, 0.22 mmol) was then added to the reaction. The mixture was stirred at room temperature for 15 minutes. After monitoring the completion of the reaction by LCMS, the reaction mixture was concentrated under reduced pressure. The residue was purified by preparative TLC (DCM: MeOH = 15:1) and reverse phase chromatography to give the title compound (35.72 mg, 66.81% yield) as a white solid. MS(ESI)m/z:473.5[m+H]+; $^1$H NMR (400 MHz, DMSO) δ 8.38 (t, J = 5.6 Hz, 1H), 8.24 (s, 1H), 7.88 (s, 1H), 7.78 - 7.70 (m, 2H), 7.41 (ddd, J = 8.9, 6.6, 2.1 Hz, 1H), 7.24 (q, J = 8.3 Hz, 4H), 6.40 (d, J = 9.1 Hz, 1H), 6.22 (td, J = 6.7, 1.3 Hz, 1H), 5.30 (s, 2H), 5.07 (s, 2H), 4.40 (dd, J = 5.5, 2.0 Hz, 2H), 3.46 (t, J = 8.7 Hz, 2H), 3.01 - 2.92 (m, 5H).

Using the appropriate starting materials, the compounds listed below were prepared using procedures analogous to those described in Examples 37, 38, and 39.

Table 11.

| NO. | Structural formula | Mass spectrum ESI (+) m/z $[M+1]^+$ |
|---|---|---|
| 146 | 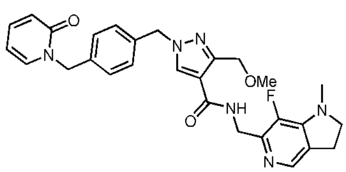 | 517.2 |
| 336 | 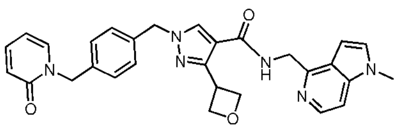 | 509.2 |

(continued)

| NO. | Structural formula | Mass spectrum ESI (+) m/z [M+1]+ |
|---|---|---|
| 356 | 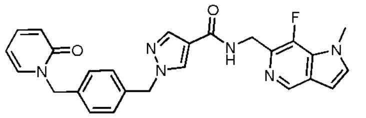 | 471.2 |
| 357 | 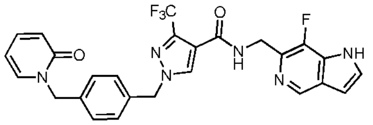 | 525.2 |
| 363 | 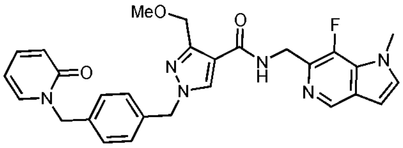 | 515.2 |
| 364 | 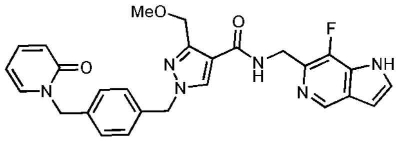 | 501.2 |
| 367 | 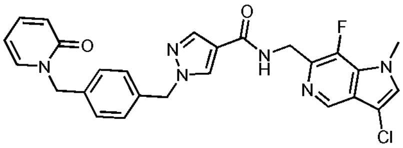 | 505.2 |
| 370 | 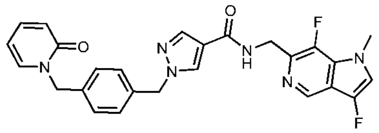 | 489.1 |
| 376 | 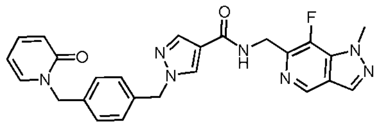 | 472.2 |
| 377 | 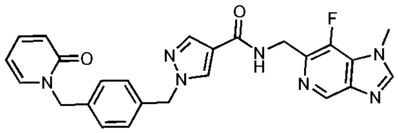 | 472.2 |
| 378 | 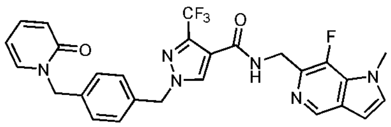 | 539.2 |
| 384 | 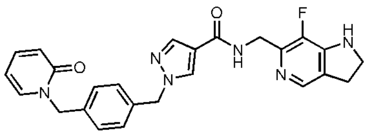 | 459.2 |
| 389 | 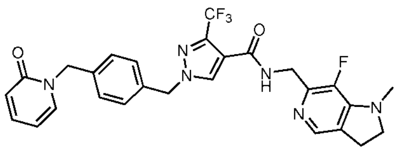 | 541.2 |

(continued)

| NO. | Structural formula | Mass spectrum ESI (+) m/z [M+1]+ |
|---|---|---|
| 390 | 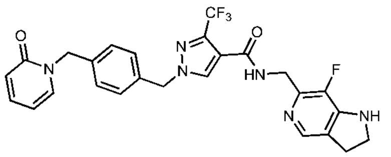 | 527.2 |
| 391 | 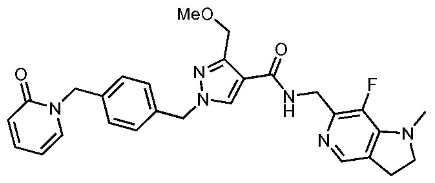 | 517.2 |
| 392 | 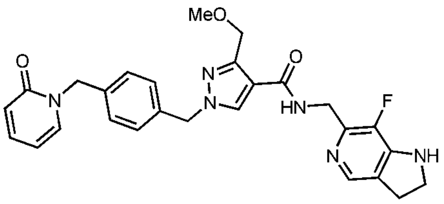 | 503.2 |
| 395 | 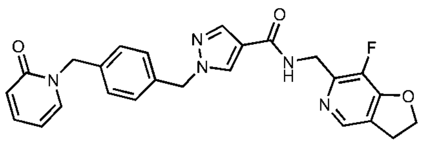 | 460.2 |
| 396 | 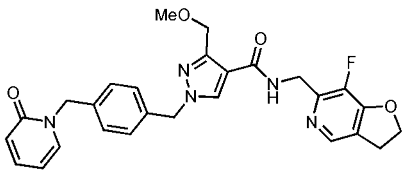 | 504.2 |

Test Example 1. Test of the inhibitory activities of the compounds of the present invention against plasma kallikrein (PKa), KLK1 and FXIa in vitro.

The ability of the compounds of the present invention to inhibit plasma kallikrein (PKK), KLK1 and FXIa was determined in the presence of 1% (v/v) DMSO by using the following biochemical assay in assay buffer (100 mM Tris, 150 mM NaCl, adjusting to pH 7.8 with HCl, and containing 0.1% (w/v) BSA and 0.05% (v/v) Tween20): compounds were tested for inhibition of PKK enzymatic activity by the FI method. PKK (R & D, Cat # 2497-SE) and Pro-Phe-Arg-AMC (Nanjing Peptide, Cat # NJP23946) solutions were prepared in reaction buffer (50 mM Tris-HCl, pH 7.5, 250 mM NaCl). The final concentrations of enzyme protein and Pro-Phe-Arg-AMC in the reaction mixture were 10 nM and 60 μM, respectively. The starting concentration of the positive reference substance Avoralstat was 100 nM, 3-fold dilution, 10 + 0 dose. 0.05 μL of compound in 100% DMSO was delivered to a 384-well plate by an acoustic liquid delivery technique (Echo665; nanoliter range). Compound duplex test. 5 μL of 2 × enzyme solution was transferred to a 384 reaction plate and centrifuged at 1000 rpm for 1 minute, incubated at 25°C for 15 minutes; 5 μL of 2 × Pro-Phe-Arg-AMC solution was transferred to a 384 reaction plate, centrifuged at 1000 rpm for 1 minute, incubated at 25°C for 3 hours. Finally, the fluorescence was measured and the FI signal was read (ex380/em460) with BMG using an Envision reader with an excitation wavelength set at 355 nm and an emission wavelength set at 460 nm. $IC_{50}$ values and nonlinear regression curve fitting were obtained using GraphPad Prism software.

Fitting of these data yielded the best-fit $IC_{50}$ values, and the results of these determinations are shown in Table 12 below, with the numbers corresponding to the numbers of the compounds. Control compounds were PKa inhibitors KVD900 and Berotralstat.

Table 12.

| NO. | PKa $IC_{50}$/nM | KLK1 $IC_{50}$ | FXIa $IC_{50}$ |
|---|---|---|---|
| KVD900 | 6.65 | >10 µM | >10 µM |
| Berotralstat | 2.21 | >10 µM | >10 µM |
| 1 | 0.178 | >10 µM | >10 µM |
| 2 | 0.517 | >10 µM | >10 µM |
| 3 | 9.27 | <500 nM | <500 nM |
| 4 | 0.155 | >10 µM | >10 µM |
| 5 | 0.60 | <500 nM | <500 nM |
| 6 | 0.032 | <500 nM | <500 nM |
| 7 | <0.051 | >10 µM | >10 µM |
| 8 | 1.22 | <500 nM | <500 nM |
| 9 | 0.406 | <500 nM | <500 nM |
| 10 | 0.132 | >10 µM | >10 µM |
| 11 | 0.030 | >500nM | >500nM |
| 12 | 1.62 | >10 µM | >10 µM |
| 13 | 0.516 | >10 µM | >10 µM |
| 14 | 0.213 | >500nM | >500nM |
| 15 | 0.898 | >10 µM | >10 µM |
| 16 | 1.24 | >10 µM | >10 µM |
| 17 | 36.2 | - | - |
| 18 | 0.408 | >10 µM | >10 µM |
| 19 | 0.6332 | >10 µM | >10 µM |
| 20 | 0.1553 | >10 µM | >10 µM |
| 21 | 2.977 | - | - |
| 22 | 0.63 | >10 µM | >10 µM |
| 23 | <0.05 | >10 µM | >10 µM |
| 24 | 0.175 | >10 µM | >10 µM |
| 25 | 0.085 | >10 µM | >10 µM |
| 26 | 699 | - | - |
| 27 | 1.48 | >10 µM | >10 µM |
| 28 | 2.33 | >500nM | >10 µM |
| 29 | >1000 | - | - |
| 30 | >1000 | - | - |
| 31 | >1000 | - | - |
| 32 | >100 | - | - |
| 33 | >100 | - | - |
| 34 | >100 | - | - |
| 35 | 1.65 | >10 µM | >10 µM |
| 36 | 523 | - | - |
| 37 | 0.16 | >10 µM | >10 µM |

(continued)

| NO. | PKa $IC_{50}$/nM | KLK1 $IC_{50}$ | FXIa $IC_{50}$ |
|---|---|---|---|
| 38 | <0.05 | >10 μM | >10 μM |
| 39 | <0.05 | >10 μM | >10 μM |
| 40 | <0.05 | - | - |
| 41 | 63.7 | - | - |
| 42 | 12.9 | - | - |
| 43 | 0.92 | >10 μM | >10 μM |
| 44 | 0.39 | >10 μM | >10 μM |
| 45 | >100 | - | - |
| 46 | >100 | - | - |
| 47 | 0.18 | >10 μM | >10 μM |
| 48 | 0.38 | >10 μM | >10 μM |
| 49 | 2.78 | >10 μM | >10 μM |
| 50 | 0.89 | >10 μM | >10 μM |
| 51 | >100 | - | - |
| 52 | >100 | - | - |
| 53 | >100 | - | - |
| 54 | 0.46 | >10 μM | >10 μM |
| 55 | 0.16 | >10 μM | >10 μM |
| 56 | 0.16 | >10 μM | >10 μM |
| 57 | 10.27 | >10 μM | >10 μM |
| 58 | 17.42 | - | - |
| 59 | >100 | - | - |
| 60 | 0.65 | >10 μM | >10 μM |
| 61 | 0.12 | >10 μM | >10 μM |
| 62 | 0.12 | >10 μM | >10 μM |
| 63 | 16.71 | - | - |
| 64 | 2.99 | >10 μM | >10 μM |
| 65 | >100 | - | - |
| 66 | >100 | - | - |
| 67 | 0.29 | >10 μM | >10 μM |
| 68 | 0.32 | >10 μM | >10 μM |
| 69 | 0.076 | >10 μM | >10 μM |
| 70 | 1.87 | >10 μM | >10 μM |
| 71 | 0.19 | >10 μM | >10 μM |
| 72 | >100 | - | - |
| 73 | 3.07 | >10 μM | >10 μM |
| 74 | 5.7 | >10 μM | >10 μM |
| 75 | 0.22 | >10 μM | >10 μM |
| 76 | 0.96 | >10 μM | >10 μM |

(continued)

| NO. | PKa $IC_{50}$/nM | KLK1 $IC_{50}$ | FXIa $IC_{50}$ |
|---|---|---|---|
| 77 | 62.9 | - | - |
| 78 | 3.72 | - | - |
| 79 | >100 | - | - |
| 80 | 0.096 | >10 μM | >10 μM |
| 81 | 76.99 | - | - |
| 82 | 26.55 | - | - |
| 83 | >100 | - | - |
| 84 | 64.71 | - | - |
| 85 | 58.67 | - | - |
| 86 | 21.2 | - | - |
| 87 | 12.9 | - | - |
| 88 | 20.1 | - | - |
| 89 | >100 | - | - |
| 90 | 3.09 | - | - |
| 91 | >100 | - | - |
| 92 | >100 | - | - |
| 93 | 0.88 | >10 μM | >10 μM |
| 94 | 0.53 | >10 μM | >10 μM |
| 95 | 9.05 | - | - |
| 96 | >100 | - | - |
| 97 | 503 | - | - |
| 98 | 245 | - | - |
| 99 | 128 | - | - |
| 100 | >100 | - | - |
| 101 | >100 | - | - |
| 102 | >100 | - | - |
| 103 | >100 | - | - |
| 104 | >100 | - | - |
| 105 | 0.26 | >10 μM | >10 μM |
| 106 | >100 | - | - |
| 107 | >100 | - | - |
| 108 | 36.79 | - | - |
| 109 | 0.35 | >10 μM | >10 μM |
| 110 | >100 | - | - |
| 111 | >100 | - | - |
| 112 | >100 | - | - |
| 113 | >100 | - | - |
| 114 | >100 | - | - |
| 115 | >100 | - | - |

(continued)

| NO. | PKa $IC_{50}$/nM | KLK1 $IC_{50}$ | FXIa $IC_{50}$ |
|---|---|---|---|
| 116 | 0.07 | >10 μM | >10 μM |
| 117 | >1000 | - | - |
| 118 | 0.92 | >10 μM | >10 μM |
| 119 | >1000 | - | - |
| 120 | >1000 | - | - |
| 121 | 0.48 | >10 μM | >10 μM |
| 122 | 124 | - | - |
| 123 | 29.45 | - | - |
| 124 | >1000 | - | - |
| 125 | 329 | - | - |
| 126 | 229 | - | - |
| 127 | 2.37 | >10 μM | >10 μM |
| 128 | 0.56 | >10 μM | >10 μM |
| 129 | 406 | - | - |
| 130 | 1.09 | >10 μM | >10 μM |
| 131 | 27.9 | - | - |
| 132 | 7.29 | - | - |
| 133 | >1000 | - | - |
| 134 | 4.47 | - | - |
| 135 | 131 | - | - |
| 136 | 36.2 | - | - |
| 137 | 0.9 | >10 μM | >10 μM |
| 138 | >1000 | - | - |
| 139 | 12.6 | - | - |
| 140 | 78 | - | - |
| 141 | >1000 | - | - |
| 142 | 0.35 | >10 μM | >10 μM |
| 143 | 0.65 | >10 μM | >10 μM |
| 144 | 25.9 | - | - |
| 145 | >1000 | - | - |
| 146 | 1.25 | >10 μM | >10 μM |
| 147 | 1.57 | >10 μM | >10 μM |
| 148 | 347 | - | - |
| 149 | 481 | - | - |
| 150 | 979 | - | - |
| 151 | 544 | - | - |
| 152 | 594 | - | - |
| 153 | 0.18 | >10 μM | >10 μM |
| 154 | 68.8 | - | - |

(continued)

| NO. | PKa $IC_{50}$/nM | KLK1 $IC_{50}$ | FXIa $IC_{50}$ |
|---|---|---|---|
| 155 | 1.48 | - | - |
| 156 | 669 | - | - |
| 157 | 1.89 | >10 μM | >10 μM |
| 158 | >1000 | - | - |
| 159 | 93.8 | - | - |
| 160 | 413 | - | - |
| 161 | 12.7 | - | - |
| 162 | 8.68 | - | - |
| 163 | 33.4 | - | - |
| 164 | 26.3 | - | - |
| 165 | 34.5 | - | - |
| 166 | 4.59 | - | - |
| 117 | >1000 | - | - |
| 118 | 0.92 | >10 μM | >10 μM |
| 119 | >1000 | - | - |
| 120 | >1000 | - | - |
| 121 | 0.48 | >10 μM | >10 μM |
| 122 | 124 | - | - |
| 123 | 29.45 | - | - |
| 124 | >1000 | - | - |
| 125 | 329 | - | - |
| 126 | 229 | - | - |
| 127 | 2.37 | >10 μM | >10 μM |
| 128 | 0.56 | >10 μM | >10 μM |
| 129 | 406 | - | - |
| 130 | 1.09 | >10 μM | >10 μM |
| 131 | 27.9 | - | - |
| 132 | 7.29 | - | - |
| 133 | >1000 | - | - |
| 134 | 4.47 | - | - |
| 135 | 131 | - | - |
| 136 | 36.2 | - | - |
| 137 | 0.9 | >10 μM | >10 μM |
| 138 | >1000 | - | - |
| 139 | 12.6 | - | - |
| 140 | 78 | - | - |
| 141 | >1000 | - | - |
| 142 | 0.35 | >10 μM | >10 μM |
| 143 | 0.65 | >10 μM | >10 μM |

(continued)

| NO. | PKa $IC_{50}$/nM | KLK1 $IC_{50}$ | FXIa $IC_{50}$ |
|---|---|---|---|
| 144 | 25.9 | - | - |
| 145 | >1000 | - | - |
| 146 | >1000 | - | - |
| 147 | 1.57 | >10 μM | >10 μM |
| 148 | 347 | - | - |
| 149 | 481 | - | - |
| 150 | 979 | - | - |
| 151 | 544 | - | - |
| 152 | 594 | - | - |
| 153 | 0.18 | >10 μM | >10 μM |
| 154 | 68.8 | - | - |
| 155 | 1.48 | >10 μM | >10 μM |
| 156 | 669 | - | - |
| 157 | 1.89 | >10 μM | >10 μM |
| 158 | 0.05 | >10 μM | >10 μM |
| 159 | 93.8 | - | - |
| 160 | 413 | - | - |
| 161 | 12.7 | - | - |
| 162 | 8.68 | - | - |
| 163 | 33.4 | - | - |
| 164 | 26.3 | - | - |
| 165 | 34.5 | - | - |
| 166 | 4.59 | - | - |
| 167 | 144.7 | - | - |
| 168 | 274 | - | - |
| 169 | 11.9 | - | - |
| 170 | 18.2 | - | - |
| 171 | 86 | - | - |
| 172 | 113 | - | - |
| 173 | 11.8 | - | - |
| 174 | 11.5 | - | - |
| 175 | 46 | - | - |
| 176 | 18 | - | - |
| 177 | 17.5 | - | - |
| 178 | 7.74 | - | - |
| 179 | 15 | - | - |
| 180 | 7.88 | - | - |
| 181 | 613 | - | - |
| 182 | >1000 | - | - |

(continued)

| NO. | PKa $IC_{50}$/nM | KLK1 $IC_{50}$ | FXIa $IC_{50}$ |
|---|---|---|---|
| 183 | 633 | - | - |
| 184 | >1000 | - | - |
| 185 | 84.5 | - | - |
| 186 | 709 | - | - |
| 187 | 10.8 | - | - |
| 188 | 2.74 | - | - |
| 189 | 180 | - | - |
| 190 | >1000 | - | - |
| 191 | 16.3 | - | - |
| 192 | >1000 | - | - |
| 193 | 0.32 | >10 μM | >10 μM |
| 194 | 487 | - | - |
| 195 | 192 | - | - |
| 196 | 15.3 | - | - |
| 197 | 164 | - | - |
| 198 | >1000 | - | - |
| 199 | 19.8 | - | - |
| 200 | 5.66 | - | - |
| 201 | 10.8 | - | - |
| 202 | >1000 | - | - |
| 203 | 0.53 | >10 μM | >10 μM |
| 204 | 1.23 | >10 μM | >10 μM |
| 205 | 8.98 | - | - |
| 206 | 318 | - | - |
| 207 | 165 | - | - |
| 208 | 7.99 | - | - |
| 209 | 10.6 | - | - |
| 210 | 5.99 | - | - |
| 211 | 8.34 | - | - |
| 212 | 5.85 | - | - |
| 213 | 35 | >10 μM | >10 μM |
| 214 | 0.16 | >10 μM | >10 μM |
| 215 | 797 | - | - |
| 216 | 2.64 | - | - |
| 217 | 15.7 | - | - |
| 218 | 2.16 | >10 μM | >10 μM |
| 219 | 4.98 | - | - |
| 220 | 12 | - | - |
| 221 | 2.61 | >10 μM | >10 μM |

(continued)

| NO. | PKa $IC_{50}$/nM | KLK1 $IC_{50}$ | FXIa $IC_{50}$ |
|---|---|---|---|
| 222 | 1.94 | >10 μM | >10 μM |
| 223 | 11.9 | - | - |
| 224 | 28.2 | - | - |
| 225 | 2.86 | >10 μM | >10 μM |
| 226 | 10.9 | - | - |
| 227 | 9.15 | - | - |
| 228 | 16.4 | - | - |
| 229 | 22.9 | - | - |
| 230 | 9.8 | - | - |
| 231 | 3.66 | - | - |
| 232 | 7.15 | - | - |
| 233 | 9.73 | - | - |
| 234 | 4.97 | - | - |
| 235 | 3.33 | - | - |
| 236 | 16.6 | - | - |
| 237 | 2.78 | - | - |
| 238 | 9.15 | - | - |
| 239 | 6.95 | - | - |
| 240 | 12.6 | - | - |
| 241 | 0.08 | >10 μM | >10 μM |
| 242 | 2.27 | >10 μM | >10 μM |
| 243 | 14.3 | - | - |
| 244 | 5.8 | - | - |
| 245 | 66.8 | - | - |
| 246 | 4.39 | - | - |
| 247 | 2.87 | - | - |
| 248 | 2.85 | - | - |
| 249 | 7.89 | - | - |
| 250 | 1.9 | >10 μM | >10 μM |
| 251 | 3.92 | - | - |
| 252 | 4.63 | - | - |
| 253 | 0.18 | >10 μM | >10 μM |
| 254 | 0.72 | >10 μM | >10 μM |
| 255 | 2.35 | >10 μM | >10 μM |
| 256 | 0.44 | >10 μM | >10 μM |
| 257 | 0.67 | >10 μM | >10 μM |
| 258 | 63 | - | - |
| 259 | 32.8 | - | - |
| 260 | 7.78 | - | - |

(continued)

| NO. | PKa $IC_{50}$/nM | KLK1 $IC_{50}$ | FXIa $IC_{50}$ |
|---|---|---|---|
| 261 | 0.59 | >10 μM | >10 μM |
| 262 | 2.67 | >10 μM | >10 μM |
| 263 | 0.59 | >10 μM | >10 μM |
| 264 | 0.82 | >10 μM | >10 μM |
| 265 | 6.91 | - | - |
| 266 | 88 | - | - |
| 267 | 23 | - | - |
| 268 | 68.6 | - | - |
| 269 | >1000 | - | - |
| 270 | 0.18 | >10 μM | >10 μM |
| 271 | 855 | - | - |
| 272 | 42 | - | - |
| 273 | >1000 | - | - |
| 274 | 0.6 | >10 μM | >10 μM |
| 275 | 56 | - | - |
| 276 | >1000 | - | - |
| 277 | >1000 | - | - |
| 278 | >1000 | - | - |
| 279 | >1000 | - | - |
| 280 | 2.64 | - | - |
| 281 | 24.9 | - | - |
| 282 | 2.36 | >10 μM | >10 μM |
| 283 | 3.55 | - | - |
| 284 | 3.41 | - | - |
| 285 | 10.5 | - | - |
| 286 | 10.8 | - | - |
| 287 | 4.54 | - | - |
| 288 | 11.5 | - | - |
| 289 | 2.31 | >10 μM | >10 μM |
| 290 | 4.62 | - | - |
| 291 | 5.9 | - | - |
| 292 | 1.59 | - | - |
| 293 | 162 | - | - |
| 294 | 10.6 | - | - |
| 295 | 16.3 | - | - |
| 296 | 2.22 | >10 μM | >10 μM |
| 297 | 2.83 | - | - |
| 298 | 2.79 | - | - |
| 299 | 11.2 | - | - |

(continued)

| NO. | PKa $IC_{50}$/nM | KLK1 $IC_{50}$ | FXIa $IC_{50}$ |
|---|---|---|---|
| 300 | 1.94 | - | - |
| 301 | 24.9 | - | - |
| 302 | 18.1 | - | - |
| 303 | 274.7 | - | - |
| 304 | 7.24 | - | - |
| 305 | 12.4 | - | - |
| 306 | 12.8 | - | - |
| 307 | 4.57 | - | - |
| 308 | 3.49 | - | - |
| 309 | 273 | - | - |
| 310 | 1.6 | >10 μM | >10 μM |
| 311 | 3.36 | - | - |
| 312 | 2 | - | - |
| 313 | 3.24 | - | - |
| 314 | 0.32 | >10 μM | >10 μM |
| 315 | - | - | - |
| 316 | - | - | - |
| 317 | - | - | - |
| 318 | - | - | - |
| 319 | - | - | - |
| 320 | - | - | - |
| 321 | 2.71 | >10 μM | >10 μM |
| 322 | 0.18 | >10 μM | >10 μM |
| 323 | 65.6 | - | - |
| 324 | 6.91 | - | - |
| 325 | 38.6 | - | - |
| 326 | 182 | - | - |
| 327 | 491 | - | - |
| 328 | 14 | - | - |
| 329 | 42.4 | - | - |
| 330 | 855 | - | - |
| 331 | >1000 | - | - |
| 332 | 540 | - | - |
| 333 | >1000 | - | - |
| 334 | 421 | - | - |
| 335 | 1.92 | >10 μM | >10 μM |
| 336 | 13.9 | - | - |
| 337 | 2.23 | >10 μM | >10 μM |
| 338 | 1.38 | >10 μM | >10 μM |

(continued)

| NO. | PKa $IC_{50}$/nM | KLK1 $IC_{50}$ | FXIa $IC_{50}$ |
|---|---|---|---|
| 339 | 31.6 | - | - |
| 340 | 48 | - | - |
| 341 | 0.07 | >10 μM | >10 μM |
| 342 | 6.29 | - | - |
| 343 | >1000 | - | - |
| 344 | 237 | - | - |
| 345 | 1.98 | >10 μM | >10 μM |
| 346 | 5.64 | - | - |
| 347 | 1.04 | >10 μM | >10 μM |
| 348 | 3.75 | - | - |
| 349 | 4.47 | - | - |
| 350 | 4.51 | - | - |
| 351 | 1.13 | >10 μM | >10 μM |
| 352 | 90 | - | - |
| 353 | 0.79 | >10 μM | >10 μM |
| 354 | 2.45 | >10 μM | >10 μM |
| 355 | 0.12 | >10 μM | >10 μM |
| 356 | 2.23 | >10 μM | >10 μM |
| 357 | 1.9 | >10 μM | >10 μM |
| 358 | 6.06 | - | - |
| 359 | 3.15 | - | - |
| 360 | 3.29 | - | - |
| 361 | 0.09 | >10 μM | >10 μM |
| 362 | 2.57 | >10 μM | >10 μM |
| 363 | 2.14 | >10 μM | >10 μM |
| 364 | 2.06 | >10 μM | >10 μM |
| 365 | 1.87 | >10 μM | >10 μM |
| 366 | 0.07 | >10 μM | >10 μM |
| 367 | 43 | - | - |
| 368 | 0.19 | >10 μM | >10 μM |
| 369 | 787 | - | - |
| 370 | 10.2 | >10 μM | >10 μM |
| 371 | >100 | >10 μM | >10 μM |
| 372 | 0.85 | >10 μM | >10 μM |
| 373 | 0.56 | >10 μM | >10 μM |
| 374 | 0.06 | >10 μM | >10 μM |
| 375 | 0.85 | >10 μM | >10 μM |
| 376 | 67 | - | - |
| 377 | 1.42 | >10 μM | >10 μM |

(continued)

| NO. | PKa $IC_{50}$/nM | KLK1 $IC_{50}$ | FXIa $IC_{50}$ |
|---|---|---|---|
| 378 | 1.41 | >10 μM | >10 μM |
| 379 | 0.97 | >10 μM | >10 μM |
| 380 | 0.27 | >10 μM | >10 μM |
| 381 | 0.03 | >10 μM | >10 μM |
| 382 | 0.15 | >10 μM | >10 μM |
| 383 | 1.39 | >10 μM | >10 μM |
| 384 | 2.17 | >10 μM | >10 μM |
| 385 | 0.88 | >10 μM | >10 μM |
| 386 | 1.68 | >10 μM | >10 μM |
| 387 | 0.08 | >10 μM | >10 μM |
| 388 | 0.18 | >10 μM | >10 μM |
| 389 | 1.45 | >10 μM | >10 μM |
| 390 | 2.02 | >10 μM | >10 μM |
| 391 | 1.40 | >10 μM | >10 μM |
| 392 | 2.44 | >10 μM | >10 μM |
| 393 | 1.05 | >10 μM | >10 μM |
| 394 | 1.56 | >10 μM | >10 μM |
| 395 | 1.68 | >10 μM | >10 μM |
| 396 | 2.10 | >10 μM | >10 μM |

The above results indicated that the compounds of the present invention have a strong inhibitory effect and a high selectivity against plasma kallikrein (PKa).

Test Example 2. Evaluation of the compounds of the present invention in terms of PKa Inhibition in Kaolin-Activated Human PPP:

Platelet-poor plasma (PPP) obtained from human whole blood, anticoagulated with Na - Citrate, was incubated with various concentrations of the test compounds and 25, 75, 250, or 750 μg/mL kaolin in assay buffer for 20 minutes at 37°C, so that a concentration response of the test compounds was obtained with respect to various kaolin concentrations. The positive drug was diluted in accordance with the reference concentration and diluted 3-fold with the diluent as the first point.

50 nL of compounds were then transferred to a 384 assay plate containing 2 replicates per well. The 384 assay plate was centrifuged at 1000 rpm/min. 2.5 μL of human plasma working solution was added to each assay well. The 384 assay plate was centrifuged at 1000 RPM. 2.5 μL of kaolin working solution was added to each assay well. The 384 assay plate was centrifuged at 1000 RPM and incubated at 25°C for 20 minutes. 5 μL of Pro-Phe-Arg-AMC working solution was added to each assay well. The 384 assay plate was centrifuged at 1000 RPM and incubated at 25°C for 60 minutes. BMG reads the fluorescence signal.

The percent inhibition of the compound was calculated according to the following formula:

Compound (% inhibition) = 100 * (average of negative control well values-compound well values)/(average of negative control well values-average of positive control well values)

The $IC_{50}$ values of the compounds according to the invention for PKa inhibition in kaolin-activated human PPP were shown in the table below, with the numbers corresponding to the numbers of the compounds. Control compounds were PKa inhibitors KVD900 and Berotralstat.

Table 13.

| NO. | PKa $IC_{50}$/nM |
|---|---|
| Berotralstat | 56.25 |

(continued)

| NO. | PKa $IC_{50}$/nM |
|---|---|
| KVB900 | 69.13 |
| 1 | 7.6 |
| 2 | 6.8 |
| 4 | 13.31 |
| 11 | 6.08 |
| 14 | 7.51 |
| 18 | 7.92 |
| 19 | 23.55 |
| 23 | 7.99 |
| 37 | 6.701 |
| 38 | 10.19 |
| 39 | 73.42 |
| 40 | 2.15 |
| 76 | 12.51 |
| 109 | 8.38 |
| 127 | 16.74 |
| 128 | 13.2 |
| 214 | 14.9 |
| 241 | 19.29 |
| 242 | 40.69 |

The above results indicated that the compounds of the present invention have a strong inhibitory effect against plasma kallikrein (PKa) in kaolin-activated human PPP and are superior to the control compounds.

To those skilled in the art, the disclosure is not limited to the foregoing illustrative examples, but may be embodied in other specific forms without departing from the essential attributes thereof. It is therefore intended that all aspects are considered as illustrative and not restrictive, the examples referred to in the appended claims are not the foregoing examples, the references cited are only with respect to the appended claims rather than foregoing examples, and that all changes which come within the meaning and range of equivalency of the claims are therefore intended to be embraced therein.

All patents, patent applications, and references cited in this specification are hereby incorporated by reference in their entirety. In the event of inconsistency, this disclosure, including the definitions, will be persuasive.

## Claims

1. A compound represented by the general formula (I) or a pharmaceutically acceptable prodrug or salt thereof:

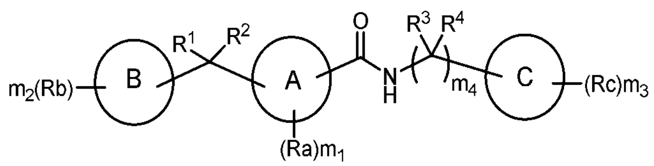

(I)

wherein,

ring A is 5-6 membered heteroaryl or phenyl;
ring B is 5-10 membered monocyclic or bicyclic aryl or heteroaryl;
$m_4$ is 0 or 1;
when $m_4$ is 1, ring C is selected from the group consisting of 5-6 membered heteroaryl, phenyl, and 9-10 membered bicyclic heteroaryl;
when $m_4$ is 0, ring C is selected from the group consisting of 5-6 membered heteroaryl fused to 5-6 membered heterocyclyl, phenyl fused to 5-6 membered heterocyclyl, 5-6 membered heteroaryl fused to $C_{3-6}$cycloalkyl, and phenyl fused to $C_{3-6}$cycloalkyl;
$R^1$, $R^2$, $R^3$, and $R^4$ are each independently selected from the group consisting of H atom, halogen, $C_{1-6}$alkyl, $C_{3-6}$cycloalkyl, -($C_{1-6}$alkylene)$_n$-$C_{1-6}$alkoxy, and $C_{1-6}$hydroxyalkyl;
each Ra is independently selected from the group consisting of -($C_{1-6}$alkylene)$_n$-$C_{1-6}$alkoxy, -($C_{1-6}$alkylene)$_n$-O-$C_{1-6}$alkylene-$C_{1-6}$alkoxy, $C_{1-6}$haloalkyl, $C_{1-6}$hydroxyalkyl, $C_{1-6}$alkyl, $C_{1-6}$haloalkoxy, -($C_{1-6}$alkylene)$_n$-$C_{3-6}$cycloalkyl, -($C_{1-6}$alkylene)$_n$-O-$C_{3-6}$cycloalkyl, halogen, -OH, -SH, -$NH_2$, -C(O)$NH_2$, -$NO_2$, -CN, $C_{2-6}$alkenyl, $C_{2-6}$alkynyl, - $C_{1-6}$alkylene-O-$C_{1-6}$alkylene-C(O)-$NH_2$, -$C_{1-6}$alkylene-O-$C_{1-6}$alkylene-C(O)-N($C_{1-6}$alkyl)$_2$, -$C_{1-6}$alkylene-O-$C_{1-6}$alkylene-C(O)-NH-$C_{1-6}$alkyl, -($C_{1-6}$alkylene)$_n$-5-6 membered heteroaryl, -O-($C_{1-6}$alkylene)$_n$-5-6 membered heteroaryl, -($C_{1-6}$alkylene)$_n$-4-7 membered monocyclic or bicyclic heterocyclyl, -($C_{1-6}$alkylene)$_n$-O-($C_{1-6}$alkylene)$_n$-4-7 membered monocyclic or bicyclic heterocyclyl, -NH-4-7 membered monocyclic or bicyclic heterocyclyl, -N($C_{1-6}$alkyl)-4-7 membered monocyclic or bicyclic heterocyclyl, -$C_{1-6}$alkylene-O-$C_{1-6}$alkylene-$NH_2$, -$C_{1-6}$alkylene-O-$C_{1-6}$alkylene-N($C_{1-6}$alkyl)$_2$ and -$C_{1-6}$alkylene-O-$C_{1-6}$alkylene-NH-$C_{1-6}$alkyl, and the $C_{1-6}$ alkylene, 5-6 membered heteroaryl, 4-7 membered monocyclic or bicyclic heterocyclyl, and $C_{3-6}$cycloalkyl are each independently optionally substituented with one or more substituents selected from the group consisting of $C_{1-6}$alkyl, oxo, -($C_{1-6}$alkylene)$_n$-$C_{1-6}$alkoxy, -C(O)-$C_{1-6}$alkyl, -C(O)-$C_{1-6}$alkoxy, halogen, and -OH;
each Rb is independently selected from the group consisting of halogen, -CN, -($C_{1-6}$alkylene)$_n$-$C_{3-6}$cycloalkyl, -O-($C_{1-6}$alkylene)$_n$-$C_{3-6}$cycloalkyl, -C(O)-$C_{3-6}$cycloalkyl, $C_{1-6}$alkyl, $C_{1-6}$haloalkyl, -($C_{1-6}$alkylene)$_n$-4-7 membered monocyclic or bicyclic heterocyclyl, -($C_{1-6}$alkylene)$_n$-5-6 membered heteroaryl, -($C_{1-6}$alkylene)$_n$-9-10 membered bicyclic heteroaryl, -$C_{2-6}$alkenyl-$C_{3-6}$cycloalkyl, phenyl, $C_{1-6}$hydroxyalkyl, $C_{1-6}$haloalkoxy, -($C_{1-6}$alkylene)$_n$-$C_{1-6}$ alkoxy, -OH, -SH, -$NH_2$, -C(O)$NH_2$, -$NO_2$, $C_{2-6}$alkenyl, $C_{2-6}$alkynyl, -($C_{1-6}$alkylene)$_n$-4-7 membered monocyclic or bicyclic heterocyclyl fused to phenyl and -O-phenyl, and the $C_{3-6}$cycloalkyl, 4-7 membered monocyclic or bicyclic heterocyclyl, 9-10 membered bicyclic heteroaryl, phenyl, and 5-6 membered heteroaryl are each independently optionally substituented with one or more substituents selected from the group consisting of halogen, oxo, $C_{1-6}$alkyl, $C_{1-6}$haloalkyl, $C_{1-6}$haloalkoxy, -($C_{1-6}$alkylene)$_n$-$C_{1-6}$ alkoxy, $C_{1-6}$hydroxyalkyl, -CN, -OH, -SH, -$NH_2$, -C(O)$NH_2$, and -$NO_2$;
each Rc is independently selected from the group consisting of $C_{1-6}$alkyl, $C_{1-6}$haloalkyl, halogen, -$NH_2$, -NH-$C_{1-6}$alkyl, -N($C_{1-6}$alkyl)$_2$, -($C_{1-6}$alkylene)$_n$-$C_{1-6}$alkoxy, formamidinyl, N-hydroxyformamidinyl, -C(=NH)-NH-$C_{1-6}$alkyl, -C(=NH)-N($C_{1-6}$alkyl)$_2$, $C_{1-6}$haloalkoxy, $C_{1-6}$hydroxyalkyl, -CN, -OH, -SH, -S-$C_{1-6}$ alkyl, -C(O)$NH_2$, -$NO_2$, -$C_{1-6}$alkylene-$NH_2$, -$C_{1-6}$alkylene-NH-$C_{1-6}$alkyl, $C_{2-6}$alkenyl, $C_{2-6}$alkynyl, $C_{3-6}$cycloalkyl, 3-6-membered heterocyclyl, -NH-($C_{1-6}$alkylene)$_n$-$C_{3-6}$cycloalkyl, -NH-($C_{1-6}$alkylene)$_n$-3-6 membered heterocyclyl and 5-6 membered heteroaryl, and the 3-6 membered heterocyclyl and 5-6 membered heteroaryl are each independently optionally substituted with one or more substituents selected from the group consisting of $C_{1-6}$alkyl, oxo, -($C_{1-6}$alkylene)$_n$-$C_{1-6}$alkoxy, -C(O)-$C_{1-6}$alkyl, -C(O)-$C_{1-6}$alkoxy, halogen and -OH;
or, two adjacent Rc together with the atoms to which they are attached form a $C_{3-6}$cycloalkyl or 5-6 membered heterocyclyl, and the $C_{3-6}$cycloalkyl or 5-6 membered heterocyclyl are each independently optionally substituted with one or more substituents selected from the group consisting of $C_{1-6}$alkyl, oxo, -($C_{1-6}$alkylene)$_n$-$C_{1-6}$alkoxy, -C(O)-$C_{1-6}$alkyl, -C(O)-$C_{1-6}$alkoxy, halogen and -OH;
the heterocyclyl and heteroaryl each containing 1, 2, 3 or 4 heteroatoms selected from the group consisting of N, O and S;
$m_1$ is 0, 1 or 2;
$m_2$ is 0, 1, 2, or 3;
$m_3$ is 0, 1, 2, or 3; and
each n is independently 0 or 1.

**2.** The compound or a pharmaceutically acceptable prodrug or salt thereof according to claim 1, which is a compound represented by the general formula (II) or a pharmaceutically acceptable prodrug or salt thereof,

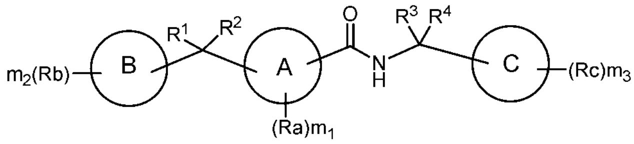

(II)

wherein,
ring A, ring B, ring C, $R^1$, $R^2$, $R^3$, $R^4$, Ra, Rb, Rc, $m_1$, $m_2$ and $m_3$ are as defined in claim 1.

**3.** The compound or a pharmaceutically acceptable prodrug or salt thereof according to claim 1 or 2, which is a compound represented by the general formula (III) or a pharmaceutically acceptable prodrug or salt thereof,

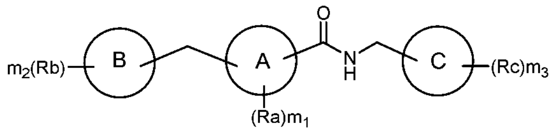

(III)

wherein,
ring A, ring B, ring C, Ra, Rb, Rc, $m_1$, $m_2$ and $m_3$ are as defined in claim 1.

**4.** The compound or a pharmaceutically acceptable prodrug or salt thereof according to claim 1 or 2, which is a compound represented by the general formula (IV) or a pharmaceutically acceptable prodrug or salt thereof,

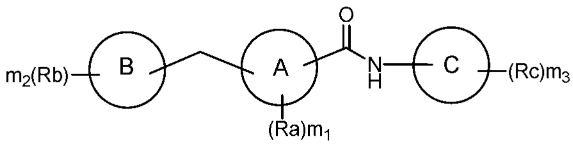

(IV)

wherein,

ring A, ring B, Ra, Rb, Rc, $m_1$, $m_2$ and $m_3$ are as defined in claim 1;
ring C is selected from the group consisting of 5-6 membered heteroaryl fused to 5-6 membered heterocyclyl, phenyl fused to 5-6 membered heterocyclyl, 5-6 membered heteroaryl fused to $C_{3-6}$cycloalkyl, and phenyl fused to $C_{3-6}$cycloalkyl; and
in particular, ring C is selected from the group consisting of cyclopentopyridinyl, cyclopentoimidazolyl and dihydrofuropyridinyl.

**5.** The compound or a pharmaceutically acceptable prodrug or salt thereof according to any one of claims 1 to 4, wherein

ring A is selected from triazolyl, pyrazolyl, thiazolyl, oxadiazolyl, oxazolyl, thienyl, thiadiazolyl, pyridyl, isoxazolyl, imidazolyl, pyrrolyl, furyl, isothiazolyl, phenyl, pyrimidinyl, pyrazinyl, pyridazinyl, triazinyl, and tetrazolyl;
in particular,

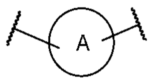

is selected from the group consisting of

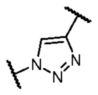, 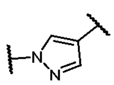, 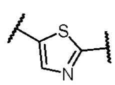,

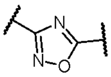, 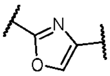, 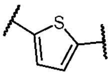, 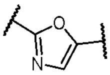, 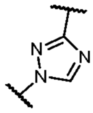, 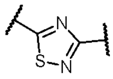, 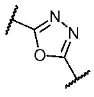, 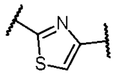, 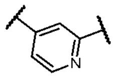,

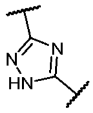, 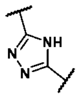, 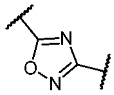, 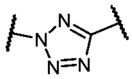, 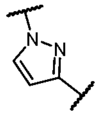, 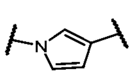, 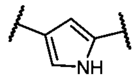 and 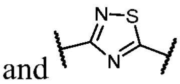.

**6.** The compound or a pharmaceutically acceptable prodrug or salt thereof according to any one of claims 1 to 5, wherein

ring B is selected from the group consisting of phenyl, quinolinyl, imidazopyridinyl, pyridinyl, imidazolyl, naphthyl, triazolyl, pyrazolyl, thiazolyl, oxadiazolyl, oxazolyl, thienyl, thiadiazolyl, isoxazolyl, pyrrolyl, furanyl, isothiazolyl, phenyl, pyrimidinyl, pyrazinyl, pyridazinyl, triazinyl, isoquinolinyl, indolyl, indazolyl, quinoxalinyl, phthalazinyl, benzimidazolyl, benzothiophenyl, quinazolinyl, and benzothiazolyl;
in particular,

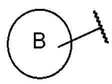

is selected from the group consisting of

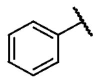, 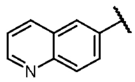, 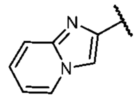,

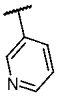 and 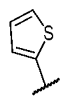.

**7.** The compound or a pharmaceutically acceptable prodrug or salt thereof according to any one of claims 1 to 6, wherein

ring C is selected from the group consisting of pyridinyl, phenyl, pyrimidinyl, thiadiazolyl, thiazolyl, triazolyl, pyrazolyl, oxadiazolyl, oxazolyl, thienyl, isoxazolyl, imidazolyl, pyrrolyl, furanyl, isothiazolyl, pyrazinyl, pyridazinyl, triazinyl, isoquinolinyl, pyrrolopyridinyl, imidazopyridinyl, triazolopyridinyl, benzisoxazolyl, benzothiazolyl and pyrazolopyridinyl;
in particular,

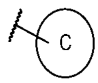

is selected from the group consisting of

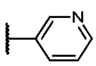, 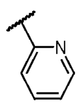, 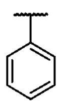, 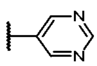,

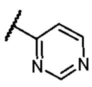, 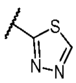, 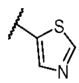, 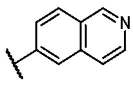, 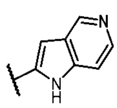, 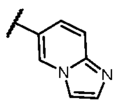, 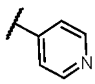, 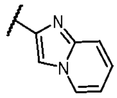, 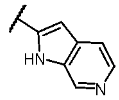,

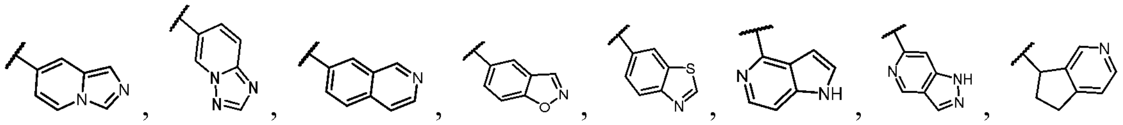,

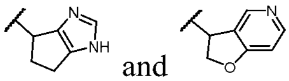 and .

**8.** The compound or a pharmaceutically acceptable prodrug or salt thereof according to any one of claims 1 to 7, wherein

each Ra is independently selected from the group consisting of -($C_{1-6}$alkylene)$_n$-$C_{1-6}$alkoxy, -($C_{1-6}$alkylene)$_n$-O-$C_{1-6}$alkylene-$C_{1-6}$alkoxy, -($C_{1-6}$alkylene)$_n$-4-7 membered monocyclic or bicyclic heterocyclyl, -($C_{1-6}$alkylene)$_n$-O-($C_{1-6}$alkylene)$_n$-4-7 membered monocyclic or bicyclic heterocyclyl, -NH-4-7 membered monocyclic or bicyclic heterocyclyl, $C_{1-6}$haloalkyl, $C_{1-6}$hydroxyalkyl, $C_{1-6}$alkyl, $C_{1-6}$haloalkoxy, -($C_{1-6}$alkylene)$_n$-$C_{3-6}$cycloalkyl, -($C_{1-6}$alkylene)$_n$-O-$C_{3-6}$cycloalkyl, halogen, -CN, -$C_{1-6}$alkylene-O-$C_{1-6}$alkylene-C(O)-N($C_{1-6}$alkyl)$_2$, -($C_{1-6}$alkylene)$_n$-5-6 membered heteroaryl, -O-($C_{1-6}$alkylene)$_n$-5-6 membered heteroaryl, and -$C_{1-6}$alkylene-O-$C_{1-6}$ alkylene-N($C_{1-6}$alkyl)$_2$, and the $C_{1-6}$alkylene, 5-6 membered heteroaryl, 4-7 membered monocyclic or bicyclic heterocyclyl, and $C_{3-6}$cycloalkyl are each independently optionally substituted with one, two, three or four substituents selected from the group consisting of $C_{1-6}$alkyl, oxo, -($C_{1-6}$ alkylene)$_n$-$C_{1-6}$ alkoxy, -C(O)-$C_{1-6}$alkyl, -C(O)-$C_{1-6}$alkoxy, halogen, and -OH, each n is independently 0 or 1, the 4-7 membered monocyclic or bicyclic heterocyclyl and the 5-6 membered heteroaryl each independently contain 1, 2, or 3 heteroatoms selected from the group consisting of N, O, and S, preferably selected from the group consisting of pyrimidinyl, pyrazolyl, pyridinyl, pyrrolidinyl, azetidinyl, piperidinyl, piperazinyl, oxetanyl, 2-oxa-6-azaspiro[3.3]heptyl, 2-azabicyclo [2.2.1]heptyl, isoxazolyl, tetrahydropyranyl, pyrazinyl and 1-oxa-6-azaspiro[3.3]heptyl;
in particular, each Ra is independently selected from the group consisting of

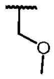,

tetrahydrofuryl, oxetanyl, difluoromethyl, trifluoromethyl, methyl, Cl atom, -CN,

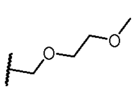, 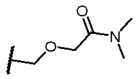, 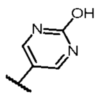, 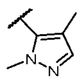, 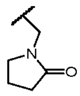, 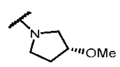, 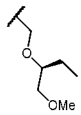, 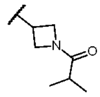, 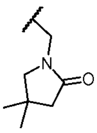, 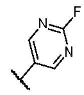,

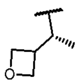, 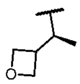, 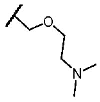, 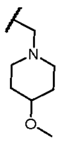, 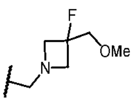, 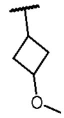, 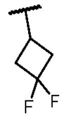, 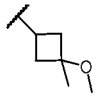, 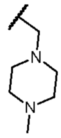, 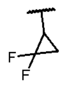, 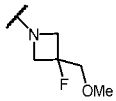,

morpholinyl,

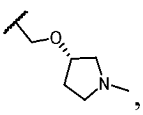, 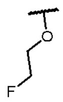, 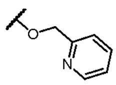, 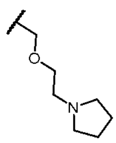, 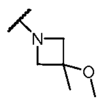, 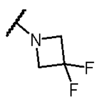, 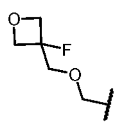, 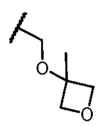,

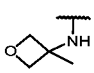, 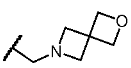, 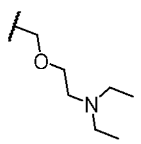, 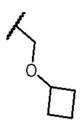, 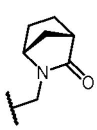, 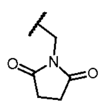, 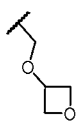, 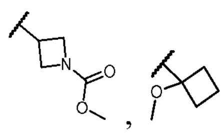,

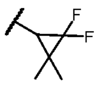, 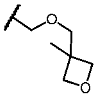, 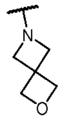, 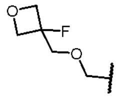, 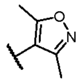, 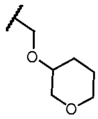, 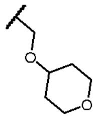, 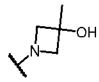, 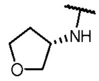, 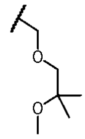,

pyrazinyl, pyrimidinyl,

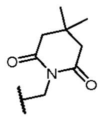, 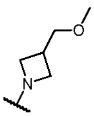, 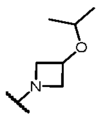, 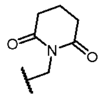, 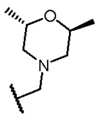, 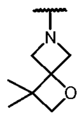, 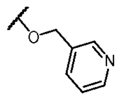,

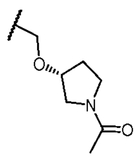, 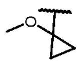, 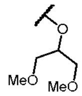 and 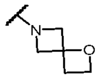;

and/or

each Rb is independently selected from the group consisting of halogen, -CN, -($C_{1-6}$alkylene)$_n$-$C_{3-6}$cycloalkyl, -O-$C_{1-6}$alkylene-$C_{3-6}$cycloalkyl, -C(O)-$C_{3-6}$cycloalkyl, $C_{1-6}$alkyl, $C_{1-6}$haloalkyl, $C_{1-6}$alkoxy, -($C_{1-6}$alkylene)$_n$-4-7 membered monocyclic or bicyclic heterocyclyl, -$C_{1-6}$alkylene-5-6 membered heteroaryl, -$C_{1-6}$alkylene-9-10 membered bicyclic heteroaryl, -$C_{2-6}$alkenyl-$C_{3-6}$cycloalkyl, -$C_{1-6}$alkylene-4-7 membered monocyclic or bicyclic heterocyclyl fused to phenyl and -O-phenyl, each n being independently 0 or 1, the 4-7 membered monocyclic or bicyclic heterocyclyl, 9-10 membered bicyclic heteroaryl, phenyl, and 5-6 membered heteroaryl are each independently optionally substituted with one, two or three substituents selected from the group consisting of halogen, oxo, $C_{1-6}$alkyl, and $C_{1-6}$haloalkyl, and the 4-7 membered monocyclic or bicyclic heterocyclyl, the 9-10 membered bicyclic heteroaryl group, and the 5-6 membered heteroaryl each independently contain 1, 2, or 3 heteroatoms selected from the group consisting of N, O, and S, preferably selected from the group consisting of dihydropyridinyl, 3-azabicyclo[3.1.0]hexyl, pyrazolyl, morpholinyl, piperidinyl, pyrrolopyridinyl, 7-azabicyclo [2.2.1]heptyl, azepinyl, azetidinyl, 2-azabicyclo[2.2.1]heptyl, pyrrolidinyl, 5-azaspiro[2.4]heptyl, thiazolidinyl, indazolyl, dihydropyrimidinyl and triazolyl;

in particular, each Rb is independently selected from the group consisting of

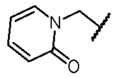,

Cl atom, -CN,

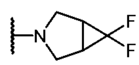,

cyclopropyl, F atom,

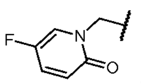,

methyl, ethyl,

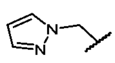, 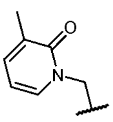, 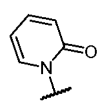, 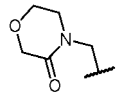,

Cl atom,

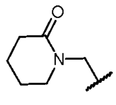, 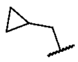, 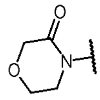, 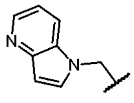,

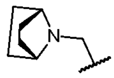, 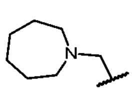, 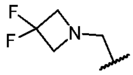, 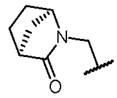, 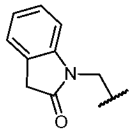, 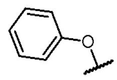, 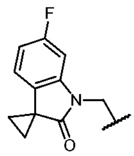, 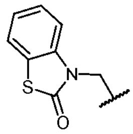

, 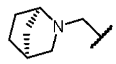, 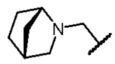,

trifluoromethyl, cyclohexyl,

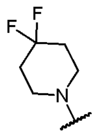, 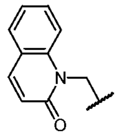, 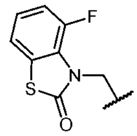,

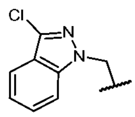, 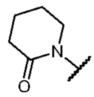, 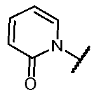, 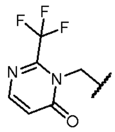,

methoxy, ethoxy,

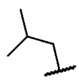, 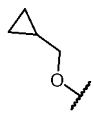, 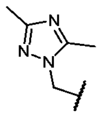,

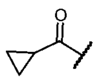, 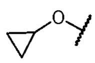, 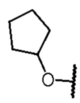 and 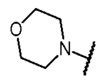;

and/or

each Rc is independently selected from the group consisting of $C_{1-6}$alkyl, $C_{1-6}$haloalkyl, halogen, $-NH_2$, $-C_{1-6}$alkylene-$NH_2$, $-C_{1-6}$alkylene-NH-$C_{1-6}$alkyl, $-(C_{1-6}$alkylene$)_n$-$C_{1-6}$alkoxy, formamidinyl, N-hydroxyformamidinyl, -C(=NH)-NH-$C_{1-6}$ alkyl, -S-$C_{1-6}$alkyl, -NH-$C_{1-6}$alkyl, -N($C_{1-6}$alkyl$)_2$, $C_{1-6}$hydroxyalkyl, -CN, $C_{3-6}$cycloalkyl, 3-6 membered heterocyclyl, -NH-($C_{1-6}$alkylene$)_n$-$C_{3-6}$cycloalkyl, -NH-($C_{1-6}$alkylene$)_n$-3-6 membered heterocyclyl, and 5-6 membered heteroaryl, each n is independently 0 or 1, the 3-6 membered heterocyclyl and 5-6 membered heteroaryl are each independently optionally substituted with one, two or three substituents selected from the group consisting of $C_{1-6}$alkyl, oxo, $C_{1-6}$alkoxy, halogen, and -OH, the 3-6 membered heterocyclyl and 5-6 membered heteroaryl each independently containing 1, 2 or 3 heteroatoms selected from the group consisting of N, O and S, preferably selected from pyrrolidinyl, azetidinyl, oxetanyl and triazolyl;

or two adjacent Rc together with the atoms to which they are attached form piperazinyl, piperidinyl, pyrrolidinyl, or dihydrofuran, each independently optionally substituted with one, two, or three substituents selected from the group consisting of $C_{1-6}$alkyl, oxo, $C_{1-6}$alkoxy, halogen, and -OH;

in particular, each Rc is independently selected from the group consisting of methyl, $-NH_2$, $-CH_2NH_2$, $-CH_2NHCH_3$, F atom, methoxy, difluoromethyl, formamidinyl, N-hydroxyformamidinyl, Cl atom, ethyl, methylthio,

$-N(CH_3)_2$, $-NH-CH_3$, -CN,

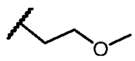

, pyrrolidinyl, cyclopropyl,

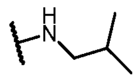,

$-C(=NH)-NH-CH_3$, -NH-cyclobutyl, $-NH-CH_2$-cyclopropyl,

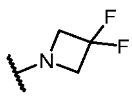,

$-NH-CH_2$-oxetanyl, azetidinyl,

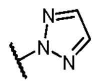

and hydroxyethyl.

**9.** The compound or a pharmaceutically acceptable prodrug or salt thereof according to any one of claims 1 to 8, wherein

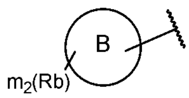

is selected from the group consisting of

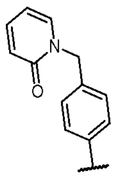, 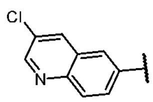,

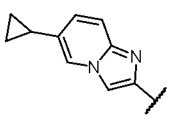, 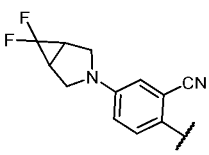, 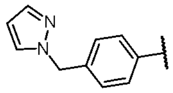, 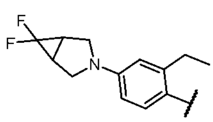, 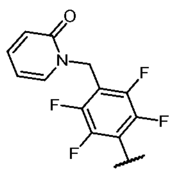,

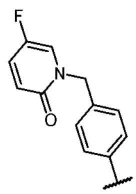, 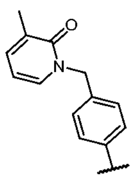, 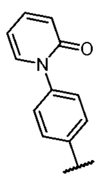, 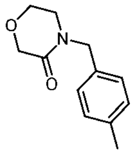, 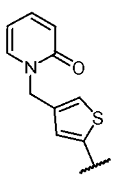, 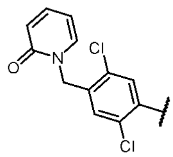, 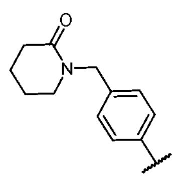,

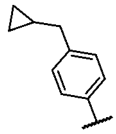, 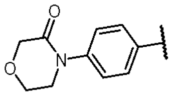, 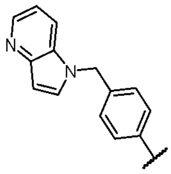, 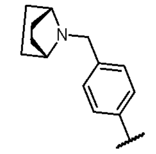, 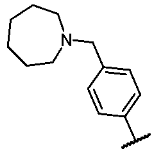, 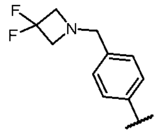,

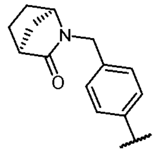, 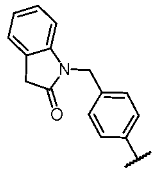, 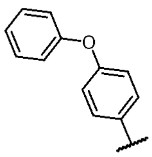, 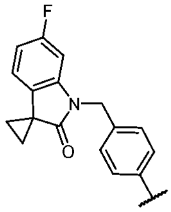, 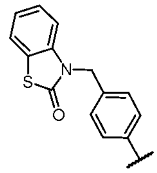, 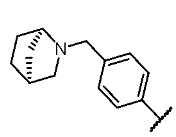,

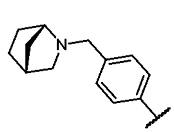, 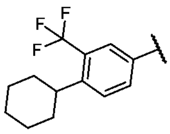, 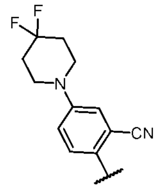, 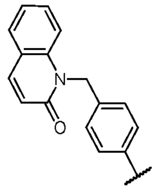, 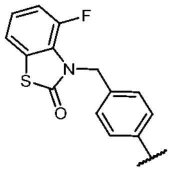, 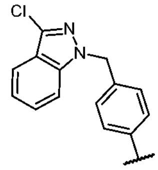,

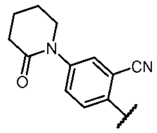, 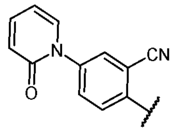, 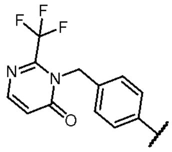, 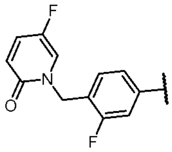, 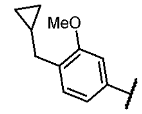, 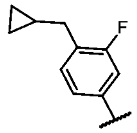,

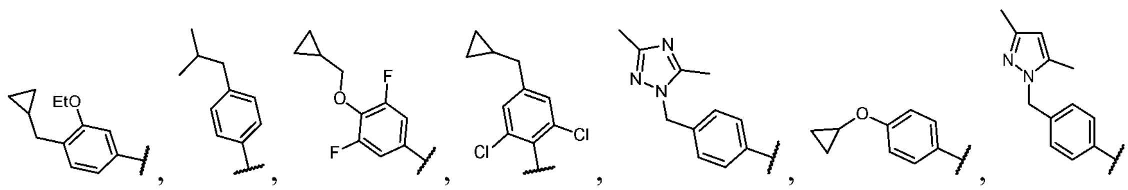
, , , , , , ,

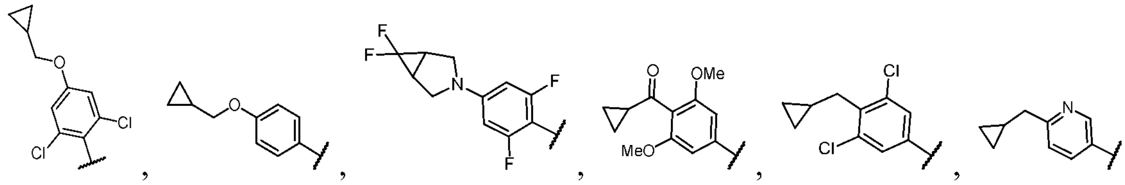
, , , , , ,

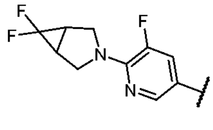, 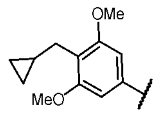, 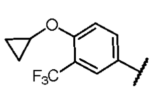, 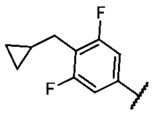, 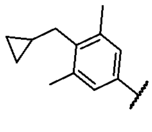, 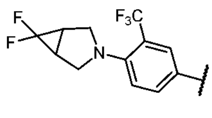,

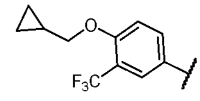, 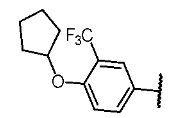, 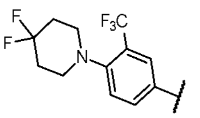, 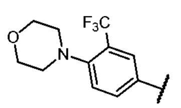, 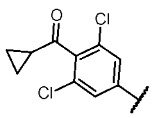

and

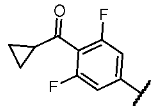.

**10.** The compound or a pharmaceutically acceptable prodrug or salt thereof according to any one of claims 1 to 9, wherein

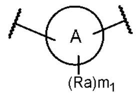

is selected from the group consisting of

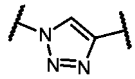, 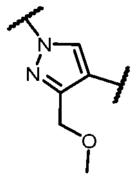,

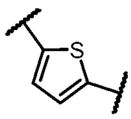, 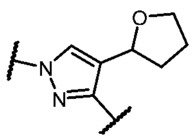, 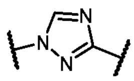, 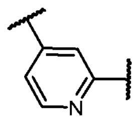, 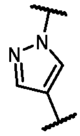, 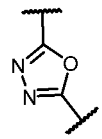, 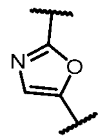, 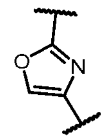,

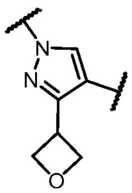, 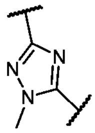, 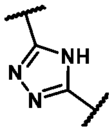, 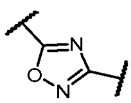, 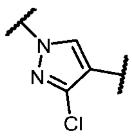, 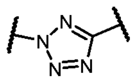, 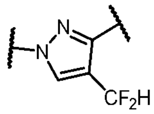,

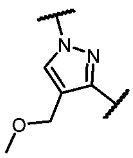, 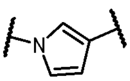, 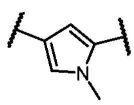, 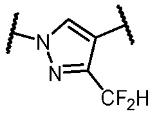, 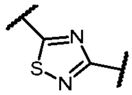, 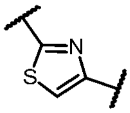, 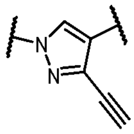,

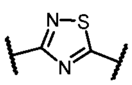, 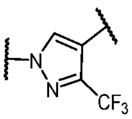, 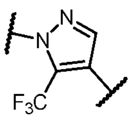, 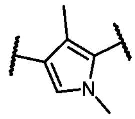, 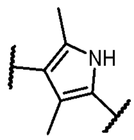, 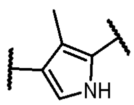,

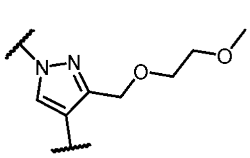, 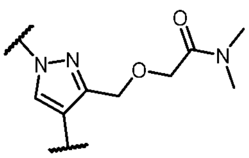, 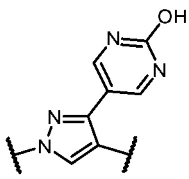, 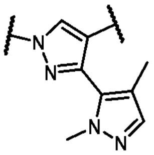, 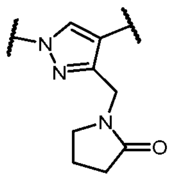,

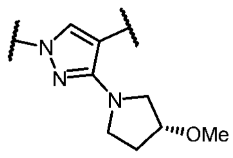 , 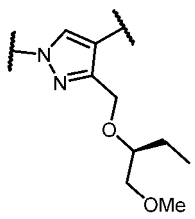 , 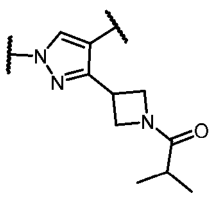 , 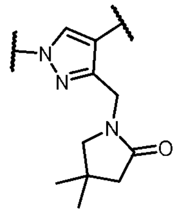 , 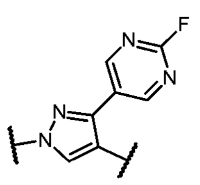 ,

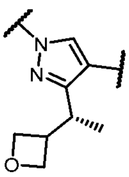 , 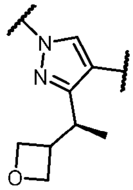 , 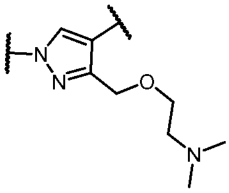 , 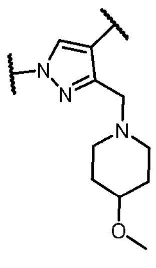 , 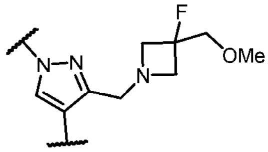 , 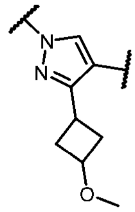

, 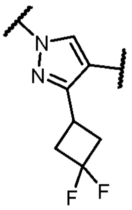 , 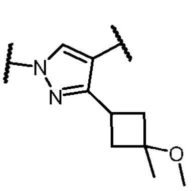 , 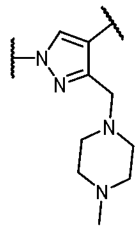 , 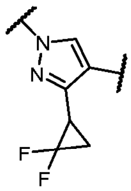 , 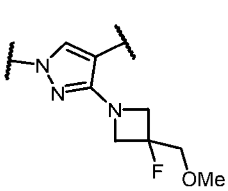 , 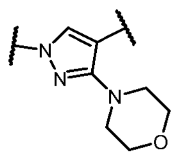 ,

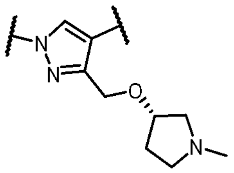 , 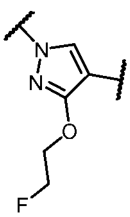 , 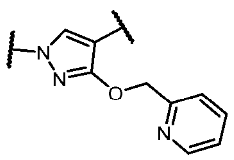 , 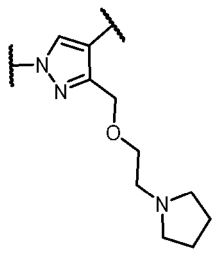 , 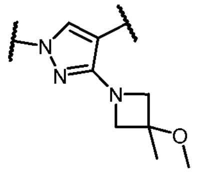 ,

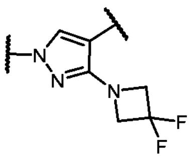 , 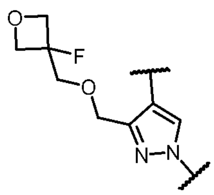 , 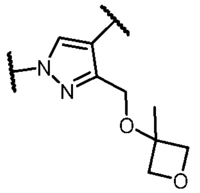 , 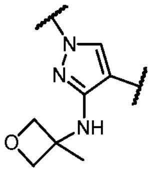 , 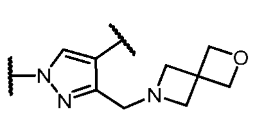 ,

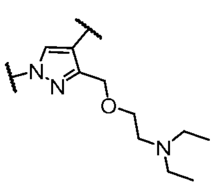 , 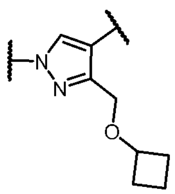 , 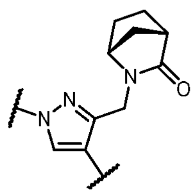 , 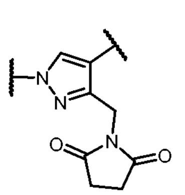 , 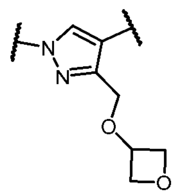 ,

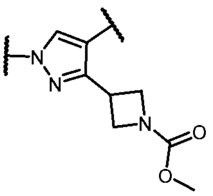, 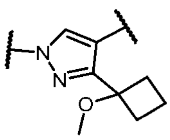, 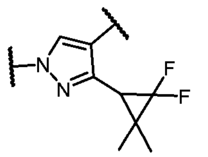, 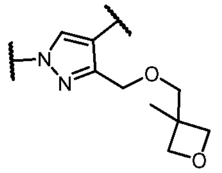, 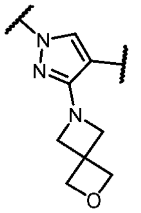,

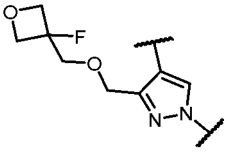, 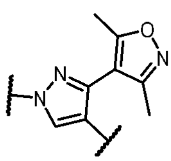, 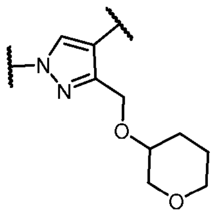, 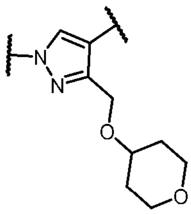, 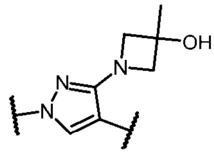,

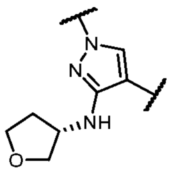, 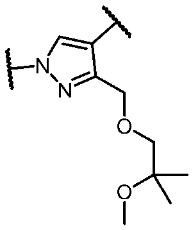, 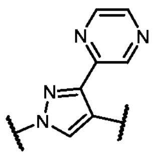, 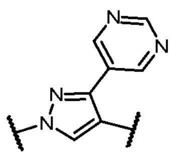, 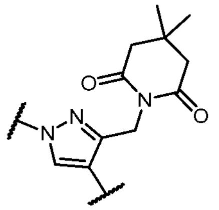,

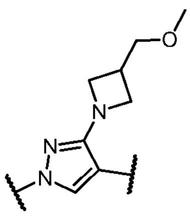, 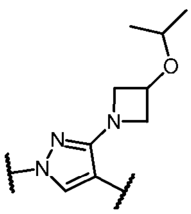, 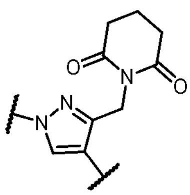, 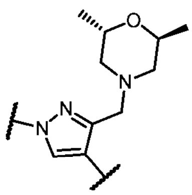, 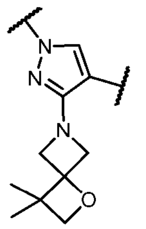,

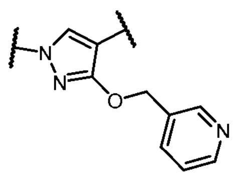, 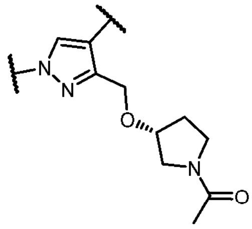, 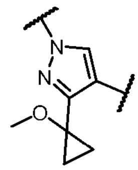, 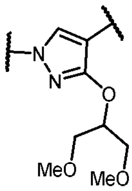 and 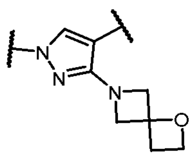.

**11.** The compound or a pharmaceutically acceptable prodrug or salt thereof according to claim 1 to 10, wherein

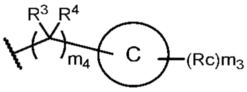, 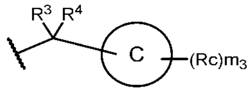 or 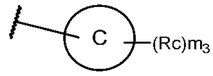

is selected from the group consisitng of

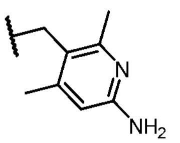, 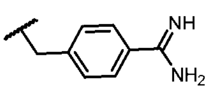, 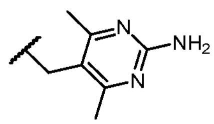,

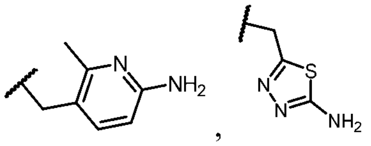 , 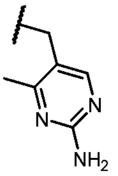 , 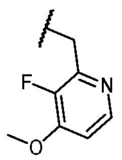 , 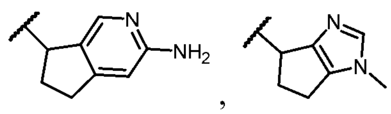 ,

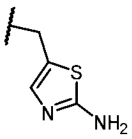 , 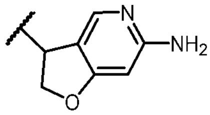 , 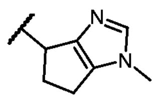 , 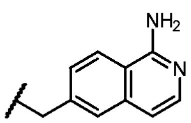 , 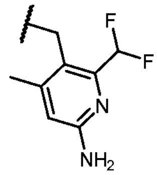 ,

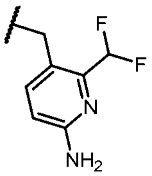 , 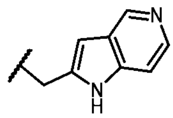 , 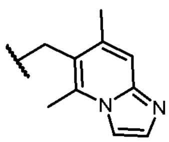 , 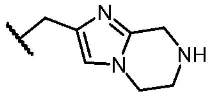 , 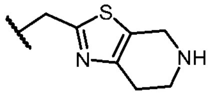 ,

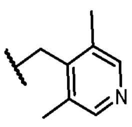 , 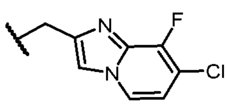 , 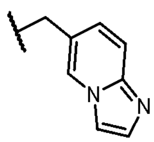 , 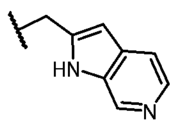 , 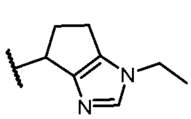 ,

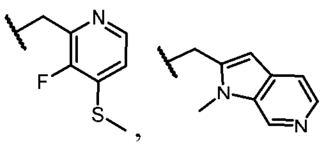 , 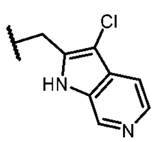 , 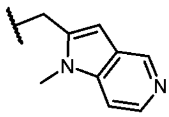 , 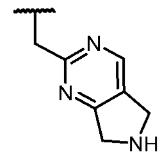 , 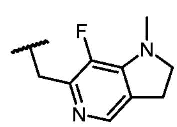 ,

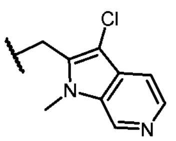 , 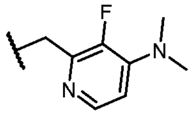 , 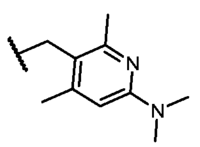 , 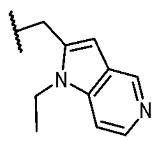 , 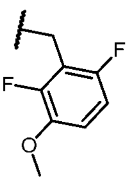 ,

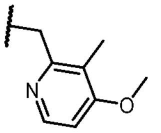 , 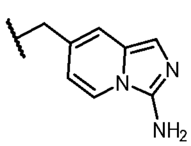 , 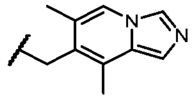 , 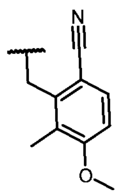 , 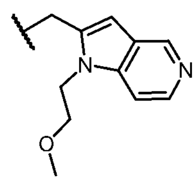 ,

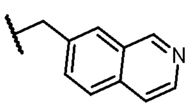 , 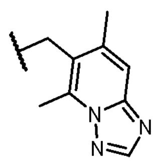 , 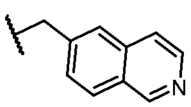 , 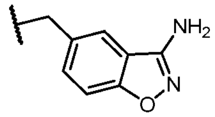 , 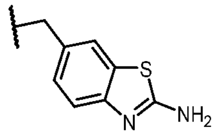 ,

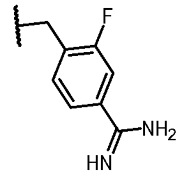, 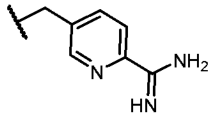, 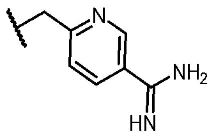, 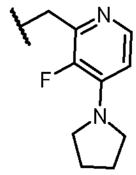, 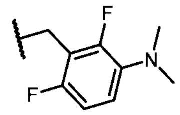,

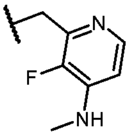, 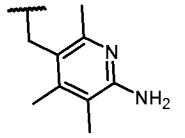, 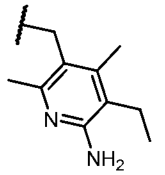, 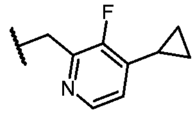, 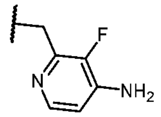,

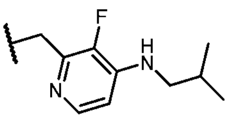, 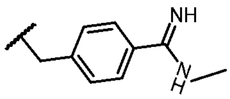, 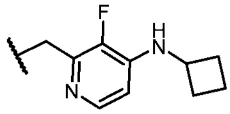, 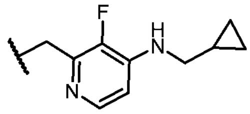,

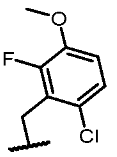, 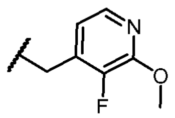, 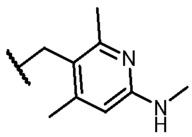, 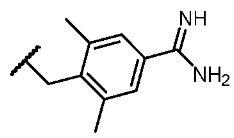, 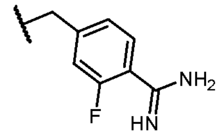,

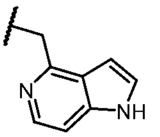, 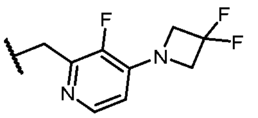, 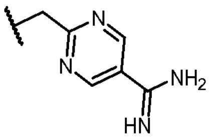, 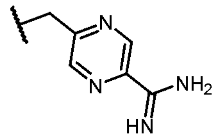,

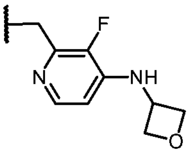, 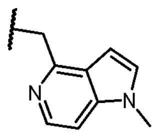, 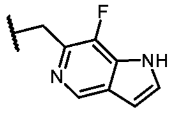, 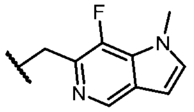, 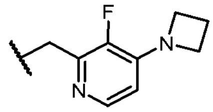,

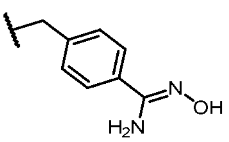, 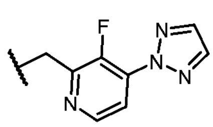, 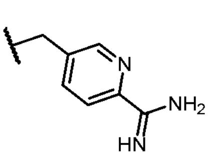, 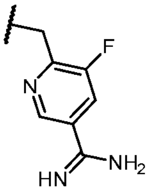, 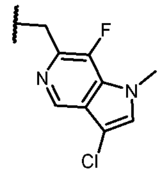,

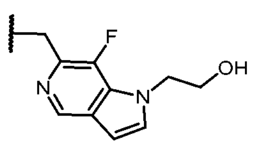, 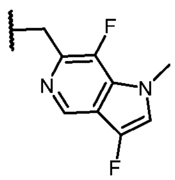, 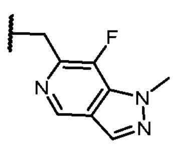, 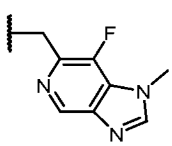, 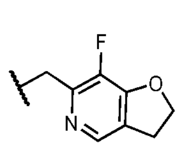,

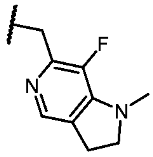 and 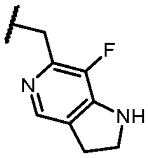 .

12. The compound or a pharmaceutically acceptable prodrug or salt thereof according to any one of claims 1 to 11, wherein
when each Rc is selected from the group consisting of formamidinyl, N-hydroxyformamidinyl, or $-NH_2$, the pharmaceutically acceptable prodrug of the compound is selected from the group consisting of:

$C_{1-6}$carbonate of formamidinyl and $-NH_2$;
$C_{1-6}$carbonate, $C_{1-6}$carboxylate, phenylcarboxylate or pyridylcarboxylate of N-hydroxyformamidinyl, and phenylcarboxylate and pyridylcarboxylate may be substituted with one or more halogens, $C_{1-6}$alkyl or $C_{1-6}$alkoxy; and/or
the pharmaceutically acceptable salt is selected from the group consisting of tosylate, mesylate, hydrochloride, acetate, and citrate.

13. The compound or a pharmaceutically acceptable prodrug or salt thereof according to any one of claims 1 to 12, selected from the group consisting of:

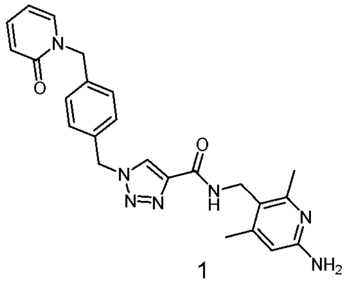
1 ,

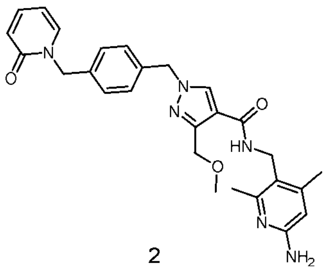
2 ,

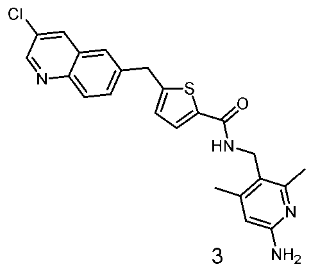
3 ,

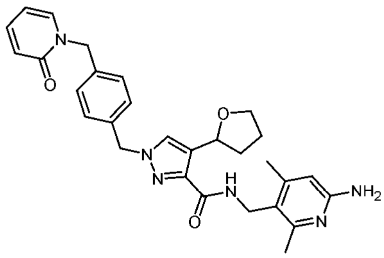
4 ,

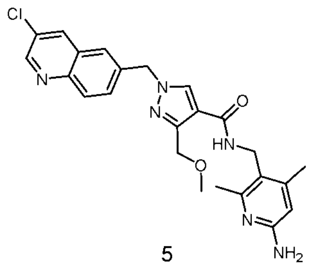
5 ,

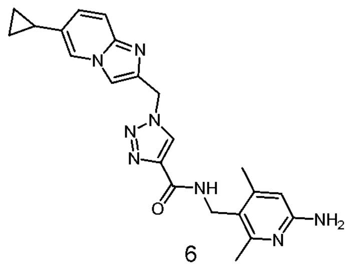
6 ,

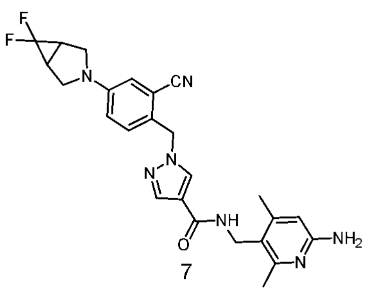
7 ,

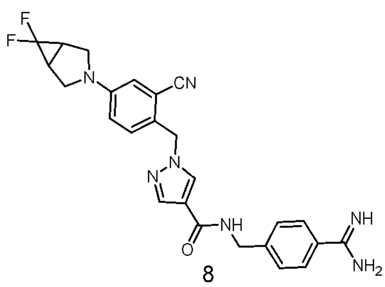
8 ,

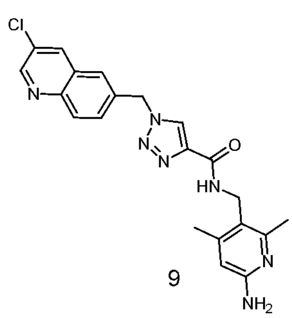
9 ,

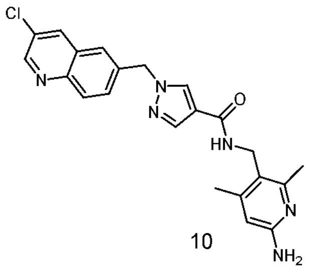
10 ,
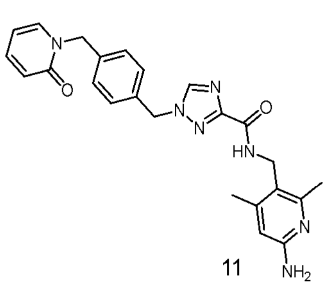
11 ,
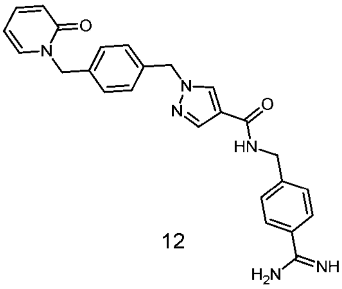
12 ,

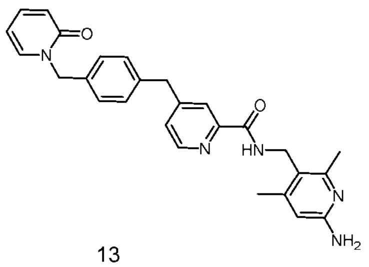
13 ,
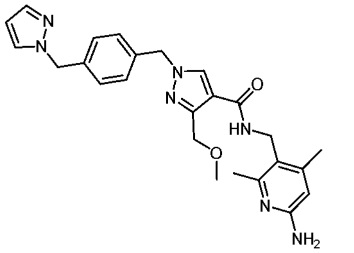
14 ,
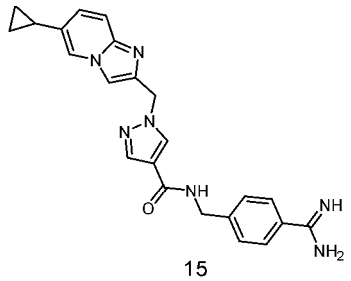
15 ,

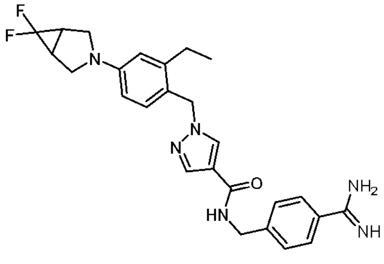
16 ,
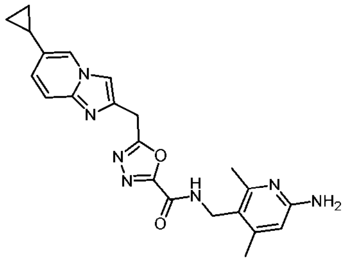
17 ,
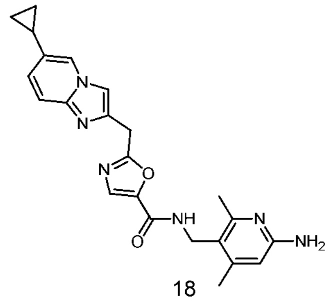
18 ,

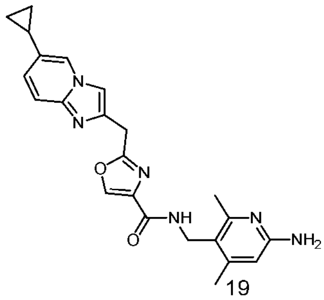
19 ,
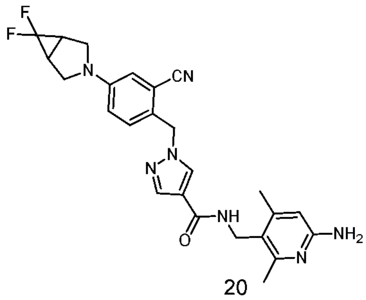
20 ,
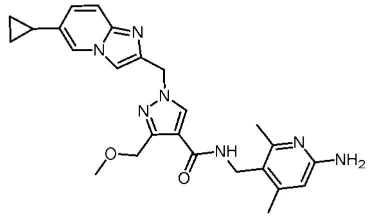
21 ,

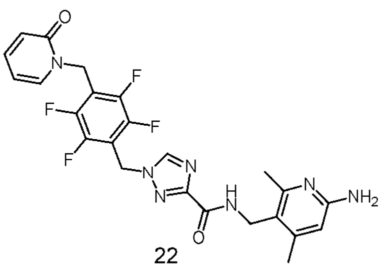

22

20

,

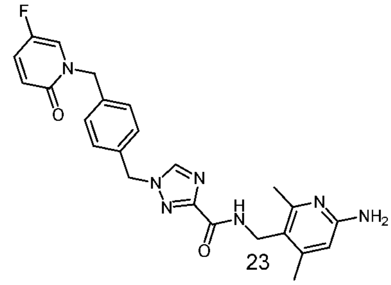

23

,

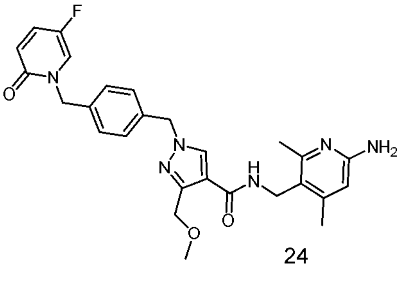

24

,

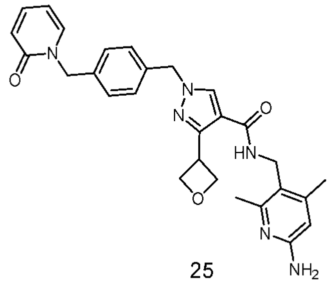

25

,

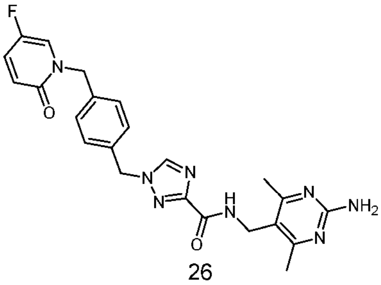

26

,

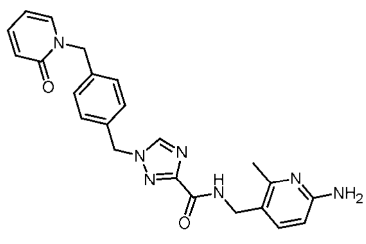

27

,

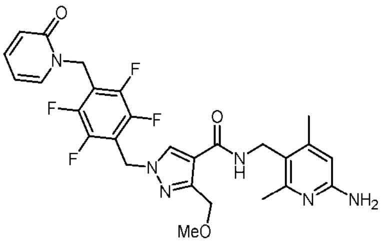

28

,

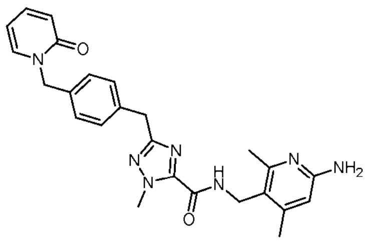

29

,

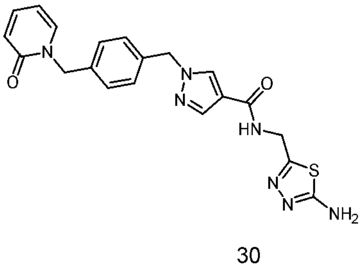

30

,

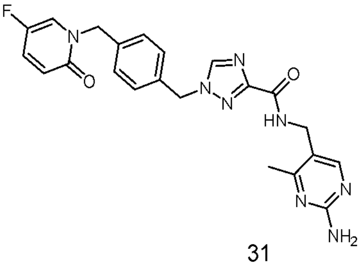

31

,

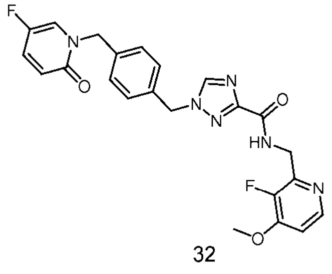

32

,

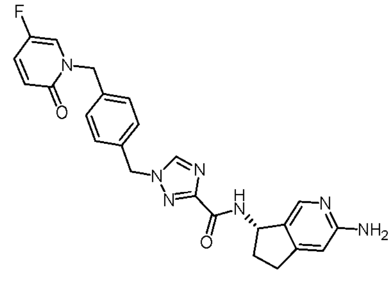

33 ,

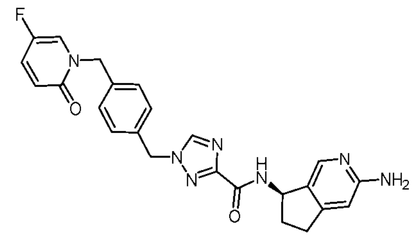

34 ,

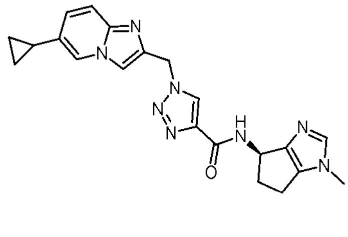

35 ,

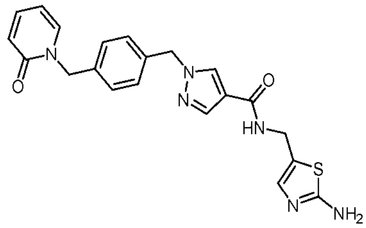

36 ,

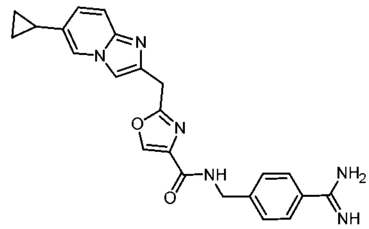

37 ,

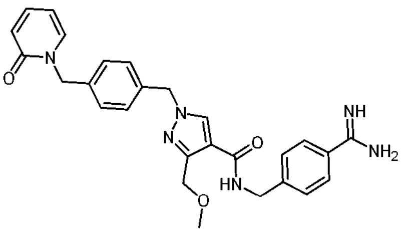

38 ,

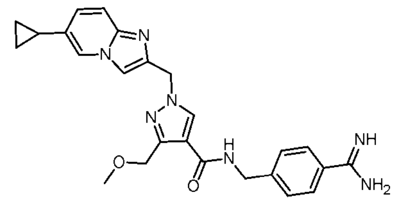

39 ,

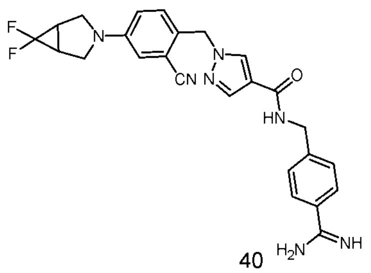

40 ,

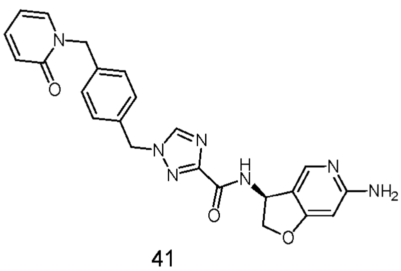

41 ,

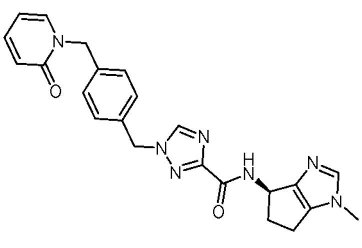

42 ,

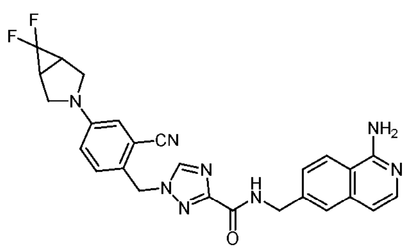

43 ,

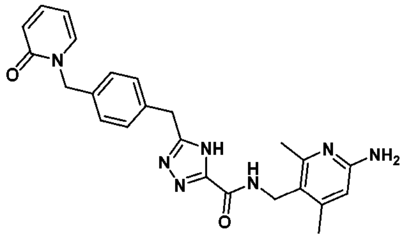
44 ,

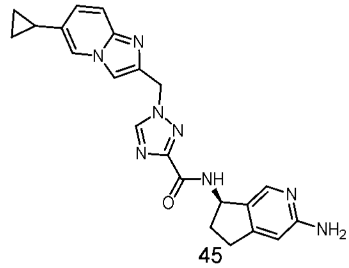
45 ,

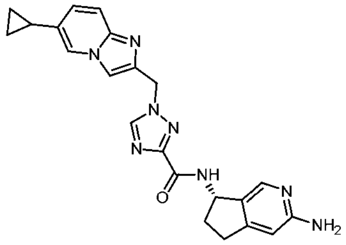
46

,

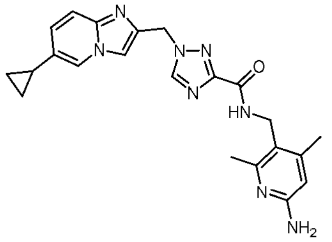
47 ,

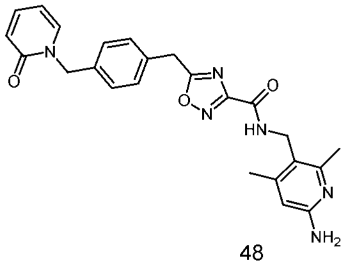
48 ,

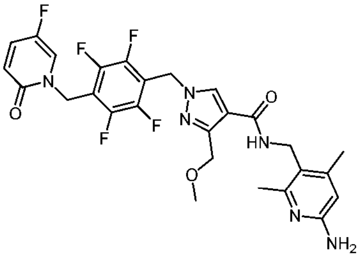
49 ,

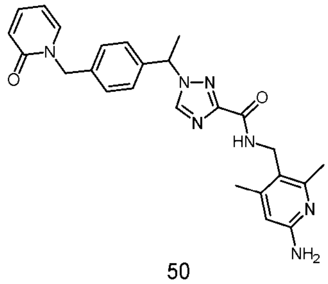
50 ,

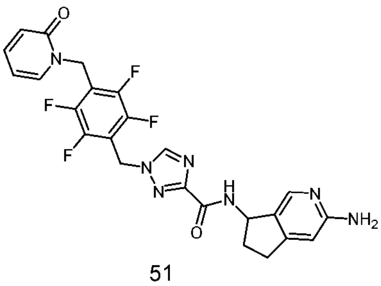
51 ,

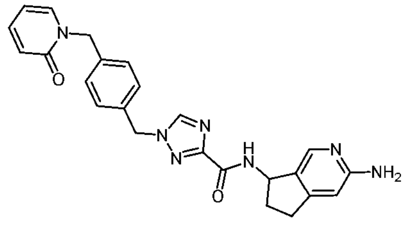
52 ,

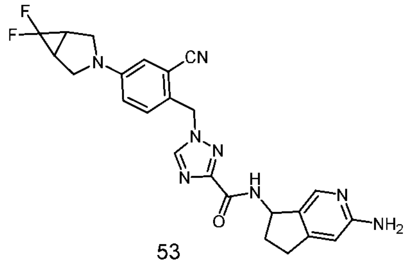
53 ,

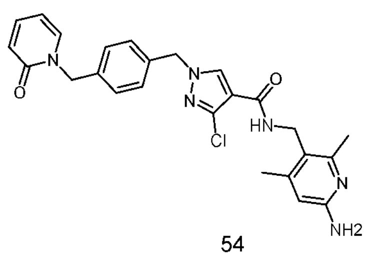
54 ,

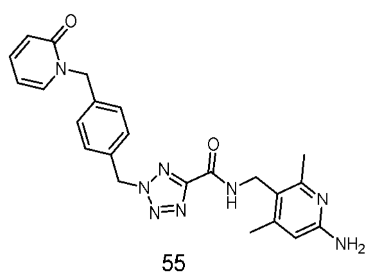
55 ,

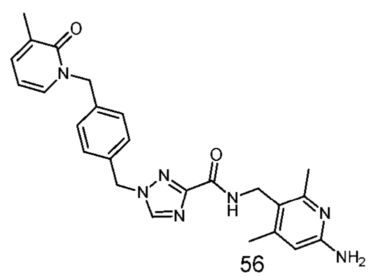
56 ,

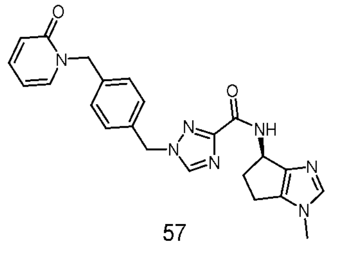
57 ,

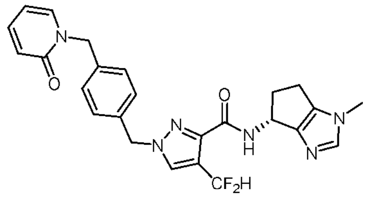
58 ,

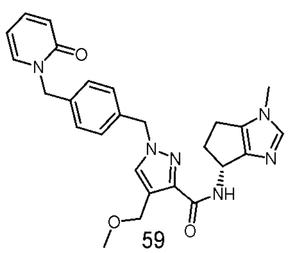
59 ,

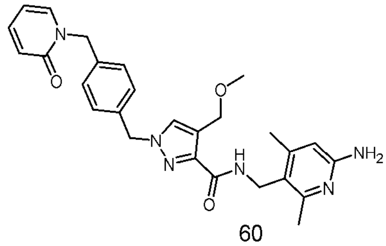
60 ,

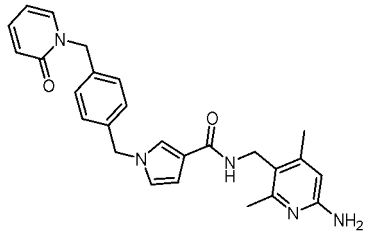
61 ,

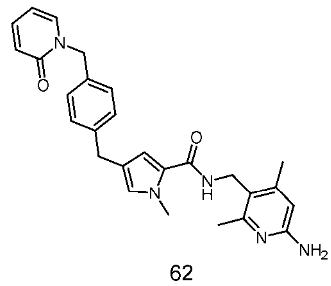
62 ,

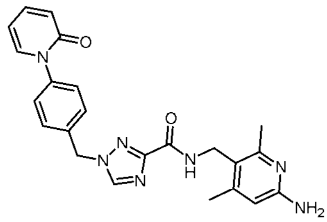
63 ,

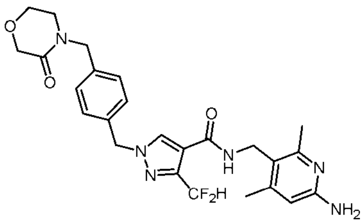
64 ,

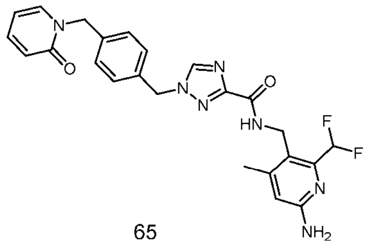
65 ,

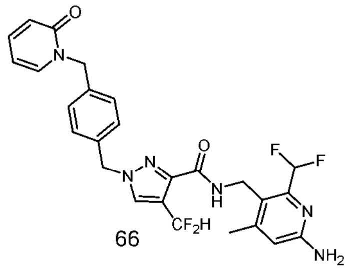
66 ,

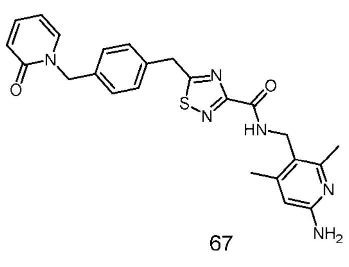
67 ,

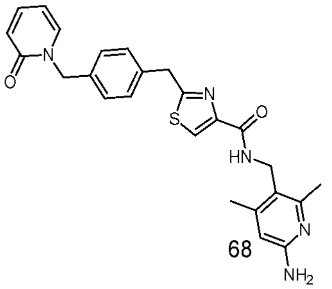
68 ,

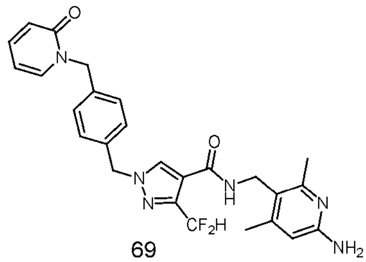
69 ,

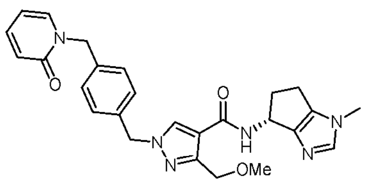
70 ,

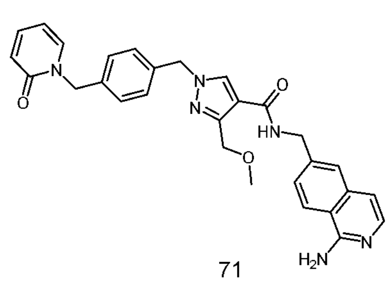

71 ,

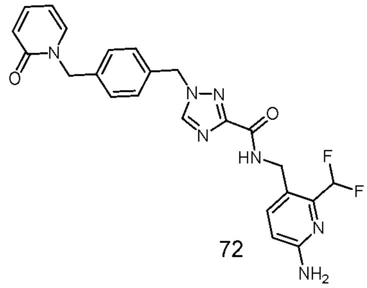

72 ,

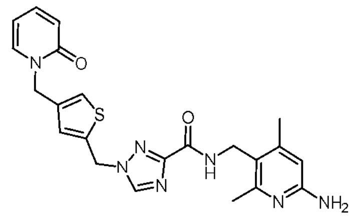

73 ,

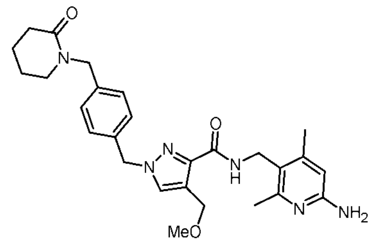

74 ,

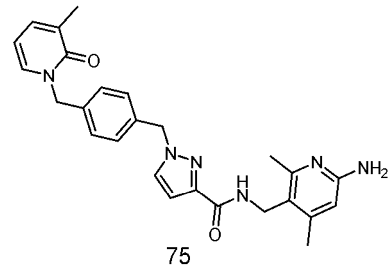

75 ,

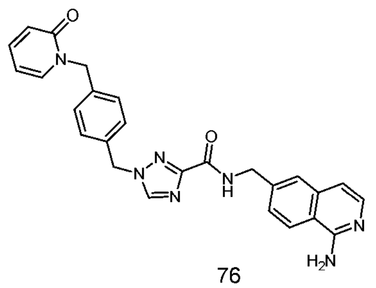

76 ,

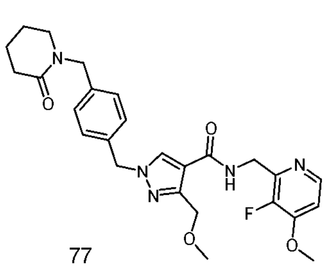

77 ,

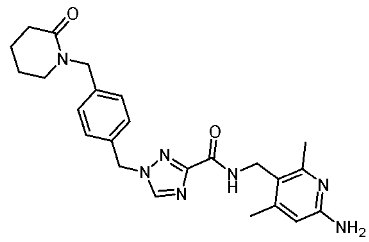

78 ,

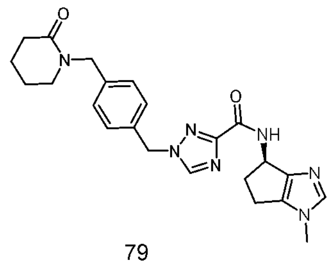

79 ,

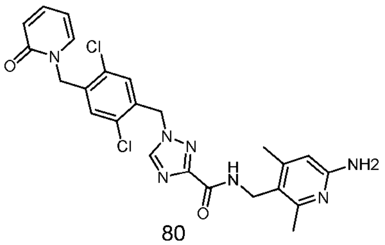

80 ,

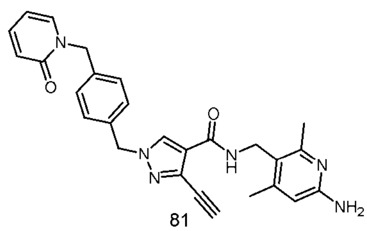

81 ,

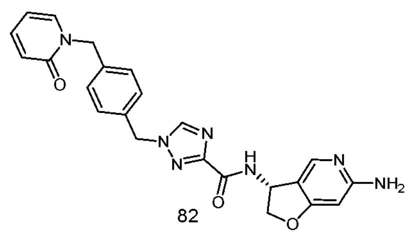

82 ,

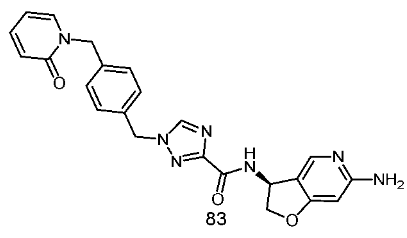

83 ,

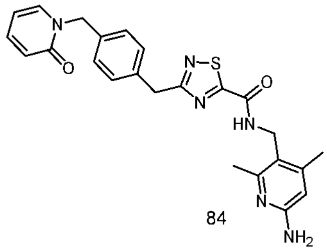
84 ,

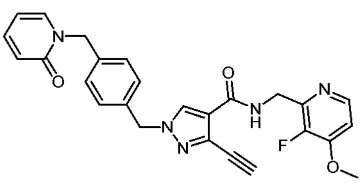
85 ,

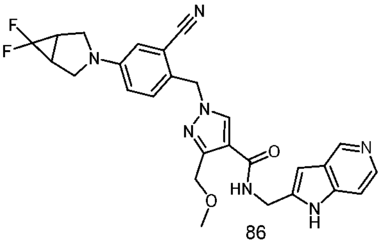
86 ,

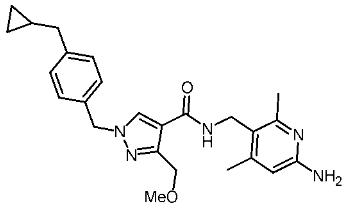
87 ,

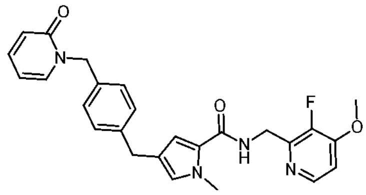
88 ,

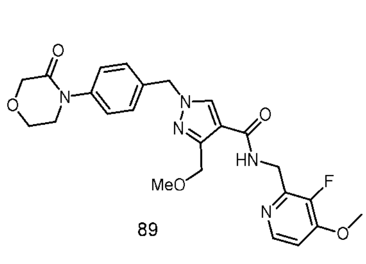
89 ,

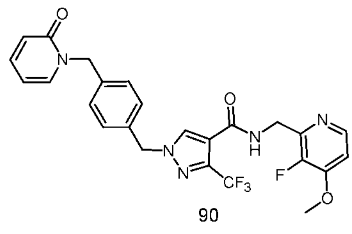
90 ,

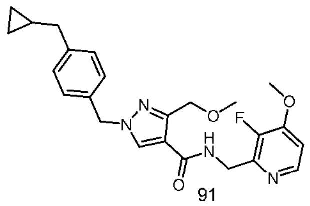
91 ,

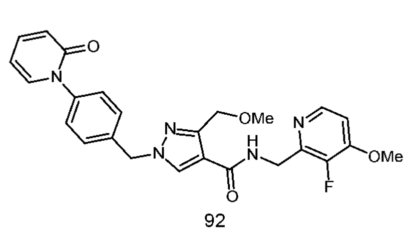
92

,

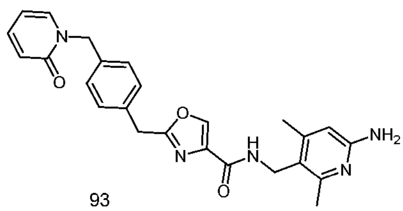
93 ,

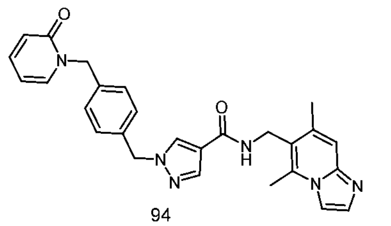
94 ,

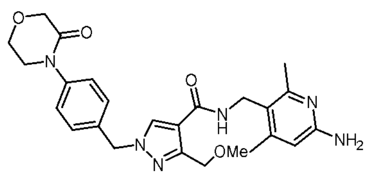
95 ,

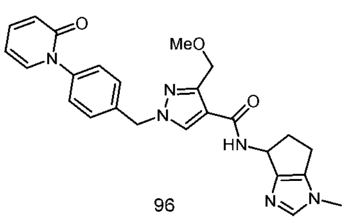
96 ,

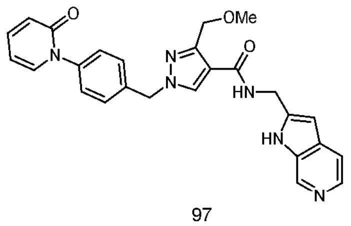
97 ,

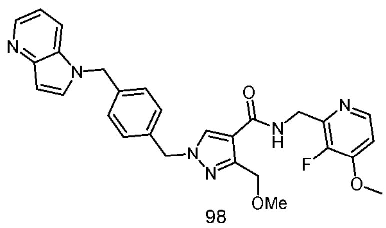
98 ,

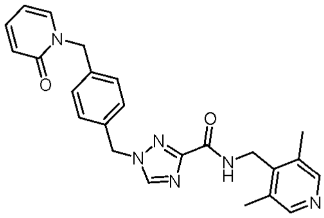
99 ,

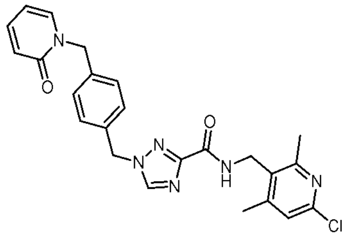
100 ,

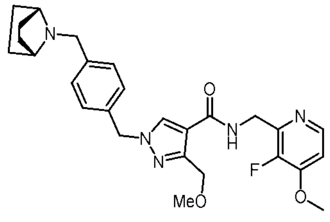
101,

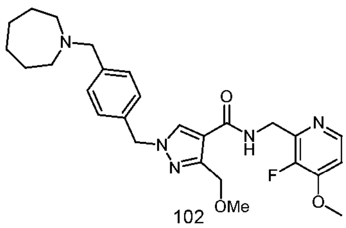
102,

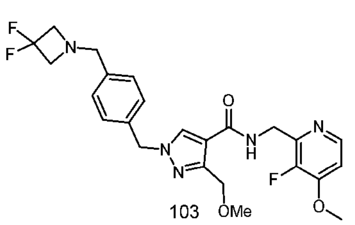
103,

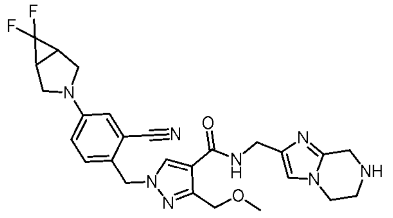
104,

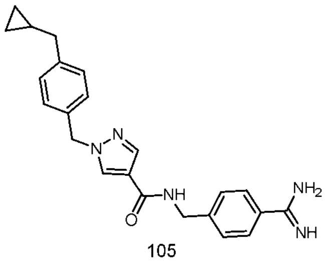
105,

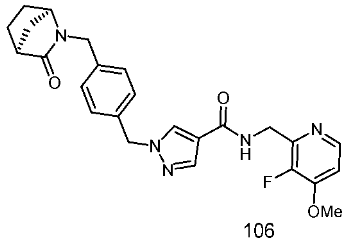
106,

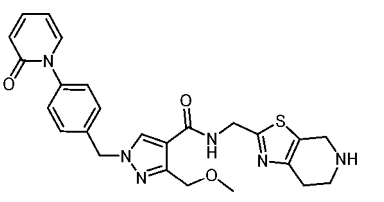
107,

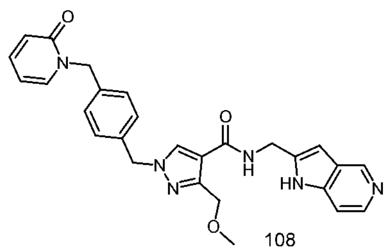
108,

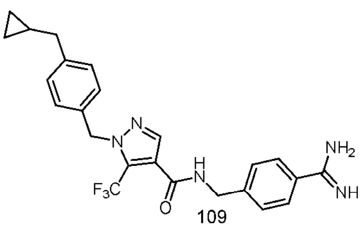
109,

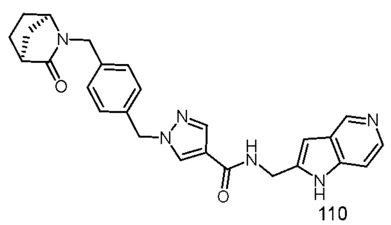
110,

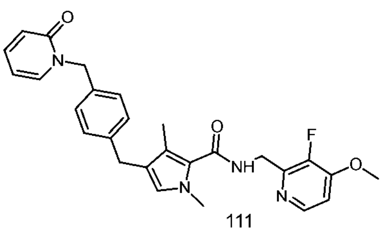
111,

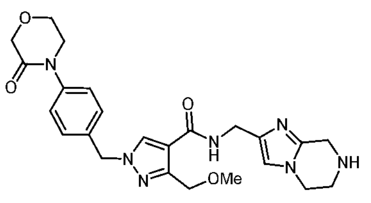
112,

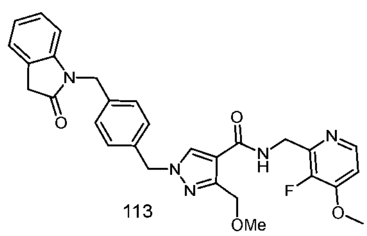
113,

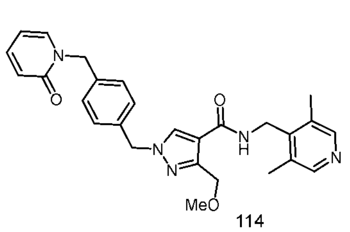
114,

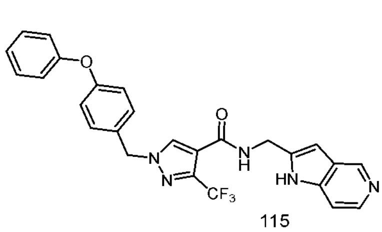
115,

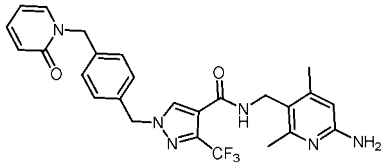

116 ,

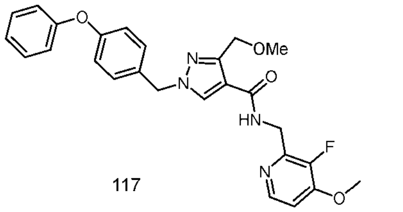

117 ,

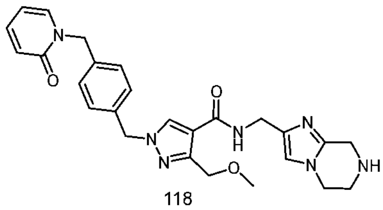

118 ,

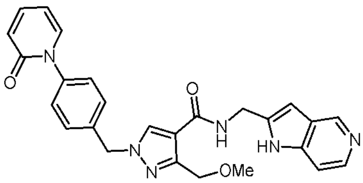

119 ,

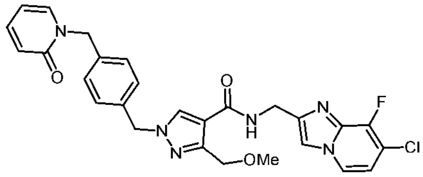

120 ,

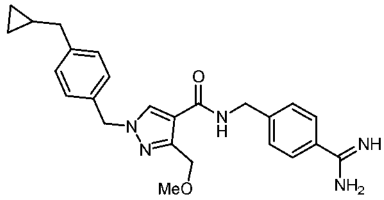

121 ,

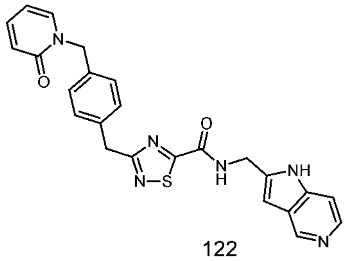

122 ,

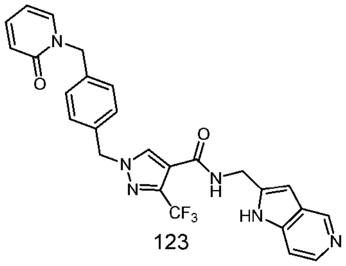

123 ,

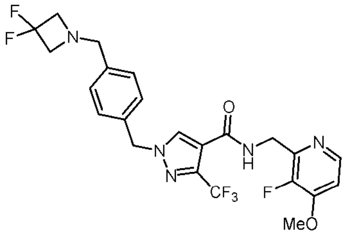

124 ,

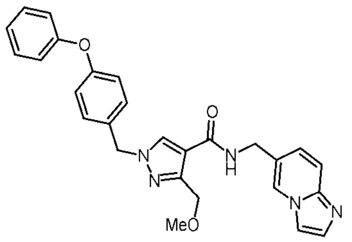

125 ,

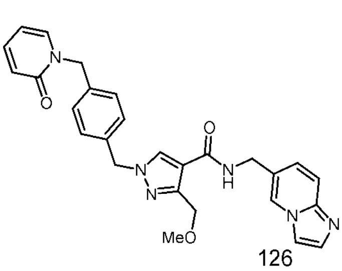

126 ,

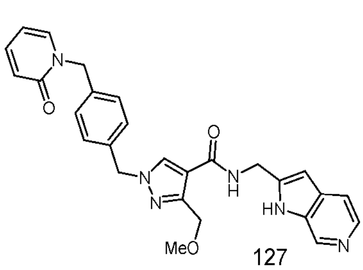

127 ,

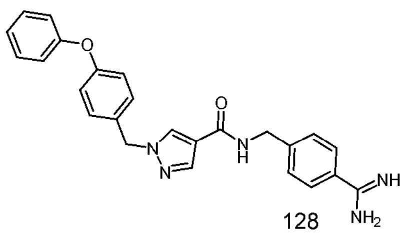

128 ,

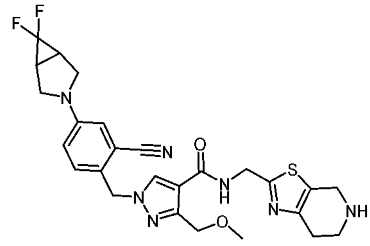

129 ,

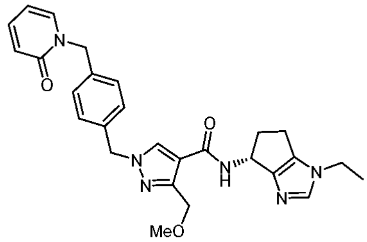
130 ,
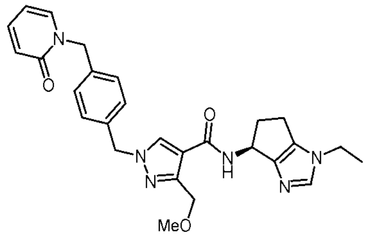
131 ,
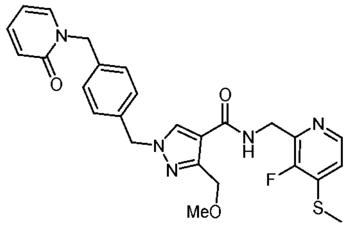
132 ,

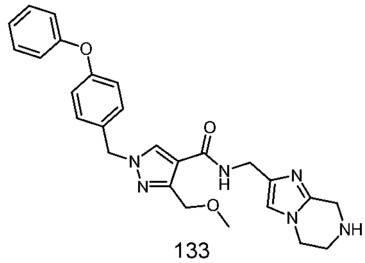
133 ,
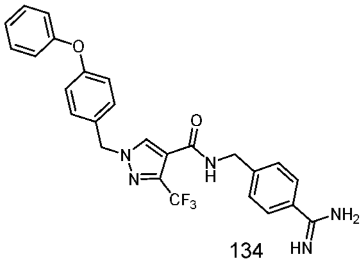
134 ,
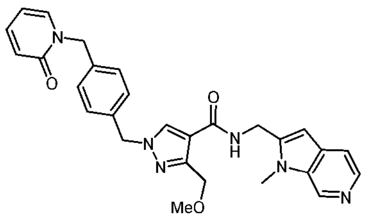
135 ,

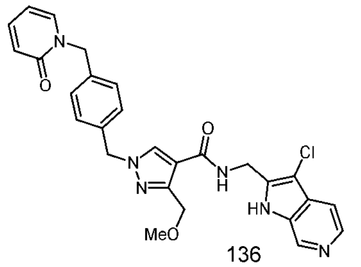
136 ,
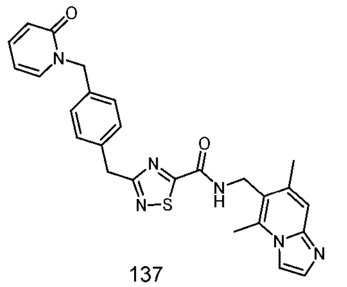
137 ,
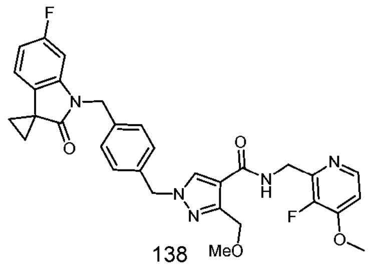
138 ,

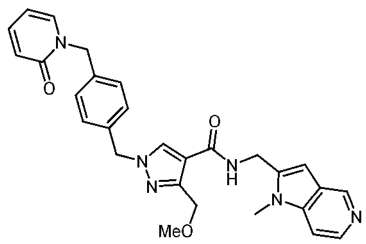
139 ,
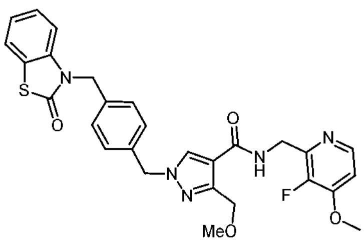
140 ,
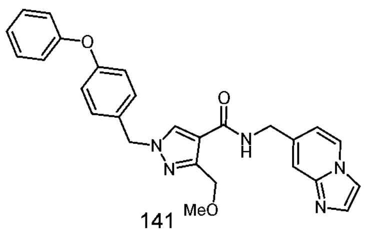
141 ,

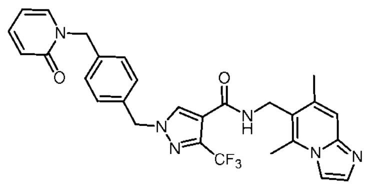
142 ,
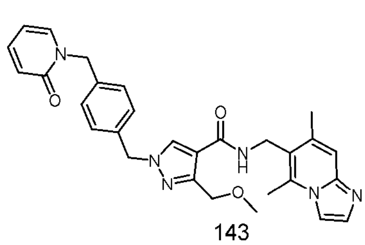
143 ,
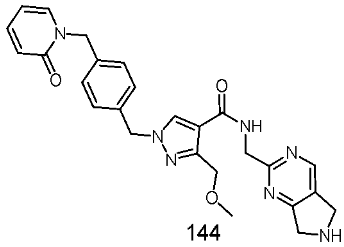
144 ,

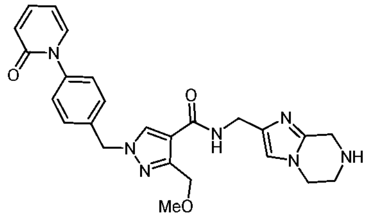

145 ,

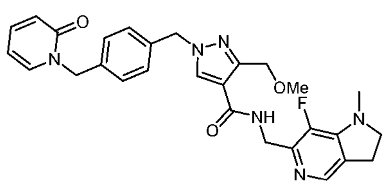

146 ,

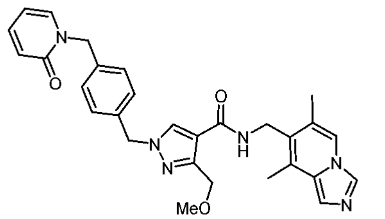

147 ,

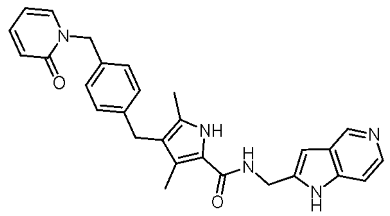

148 ,

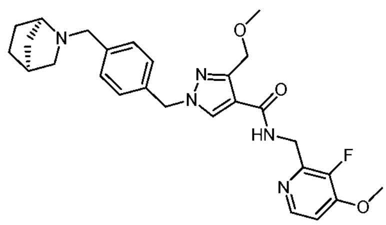

149 ,

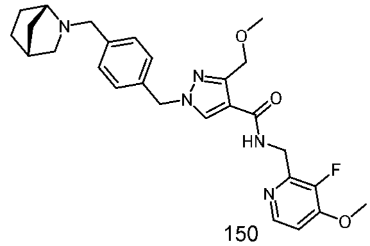

150 ,

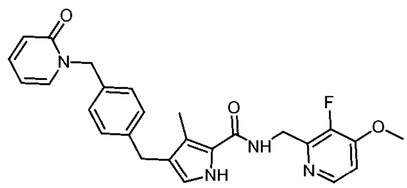

151 ,

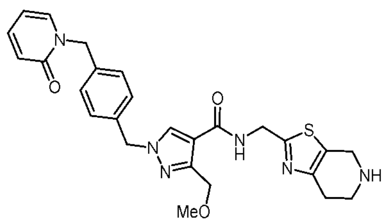

152 ,

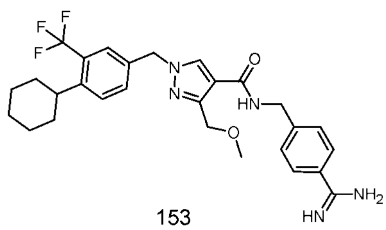

153 ,

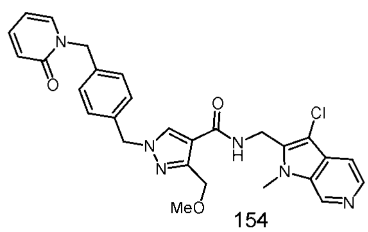

154 ,

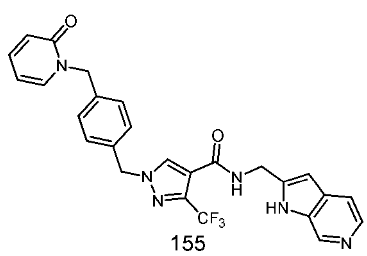

155 ,

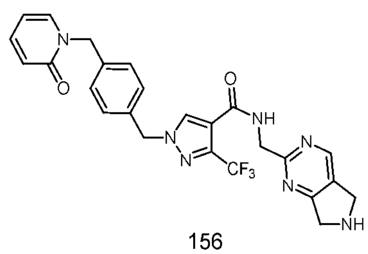

156 ,

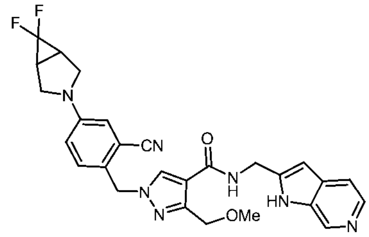

157 ,

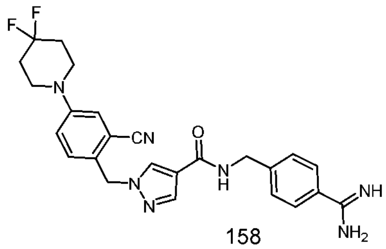

158

,

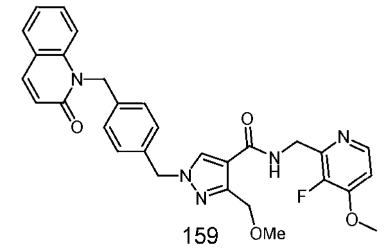

159

,

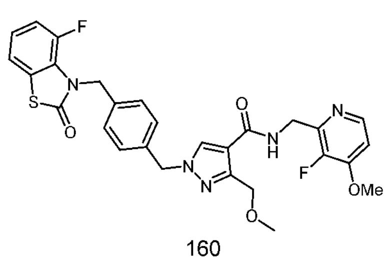

160

,

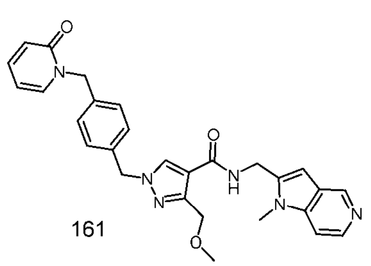

161

,

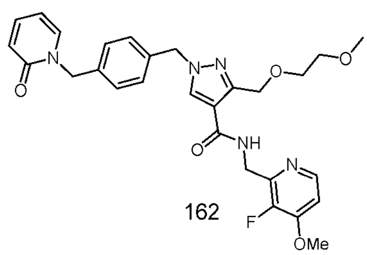

162

,

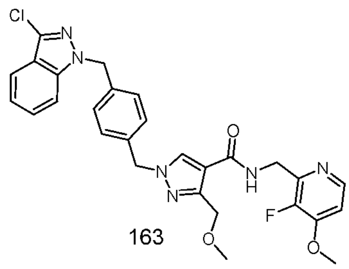

163

,

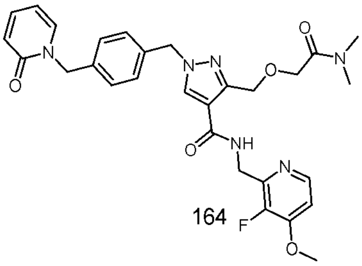

164

,

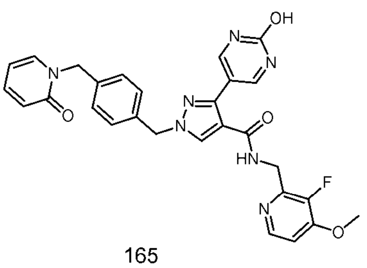

165

,

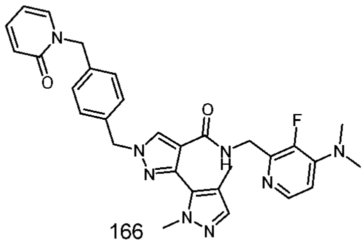

166

,

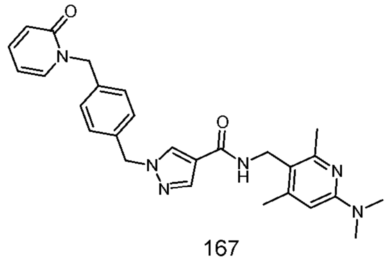

167

,

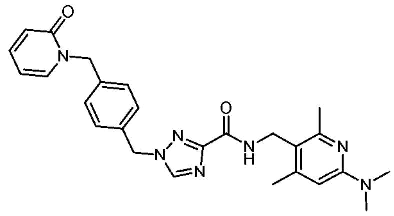

168

,

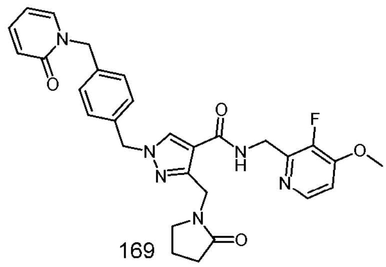
169,

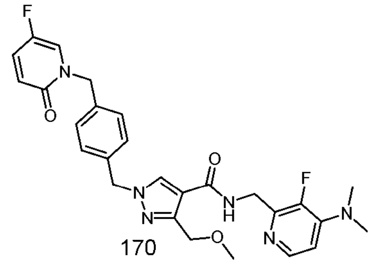
170,

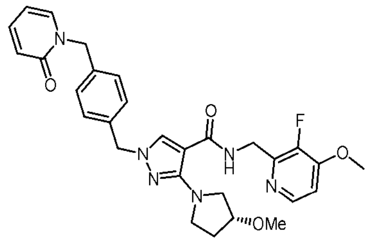
171,

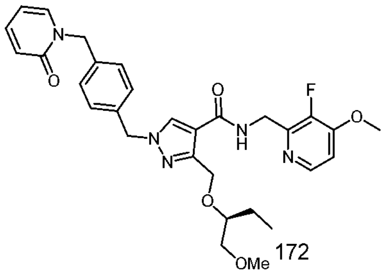
172,

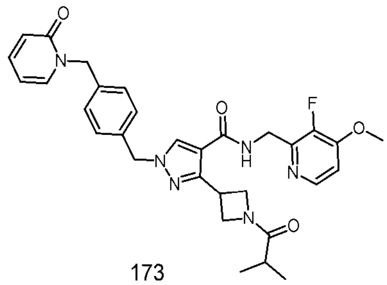
173,

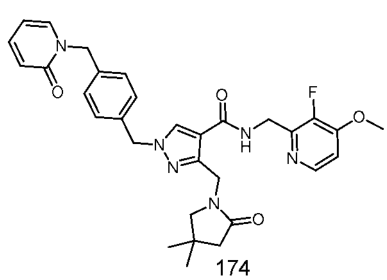
174,

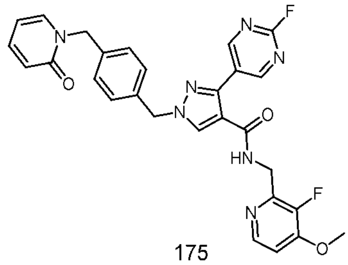
175,

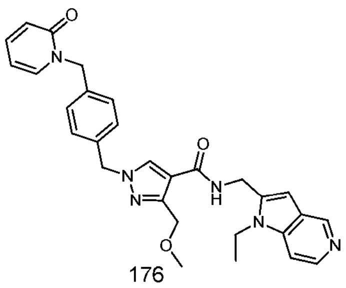
176,

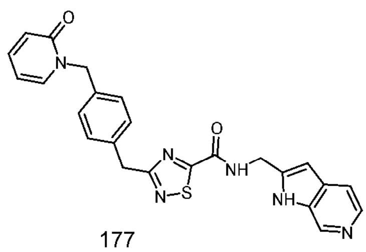
177,

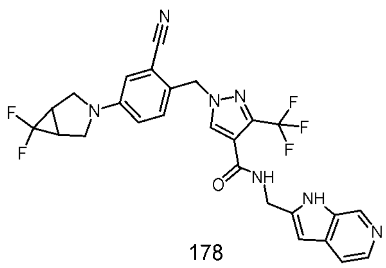
178,

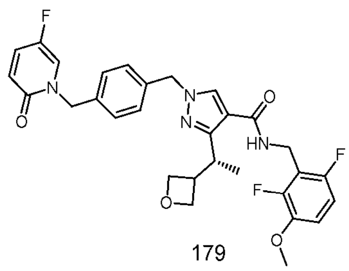
179,

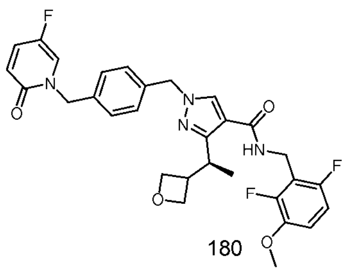
180,

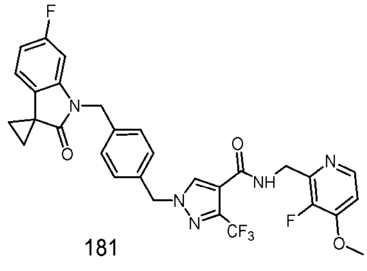
181 ,

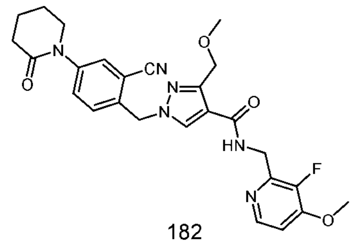
182 ,

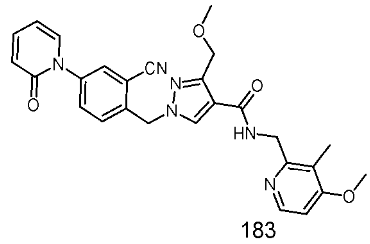
183 ,

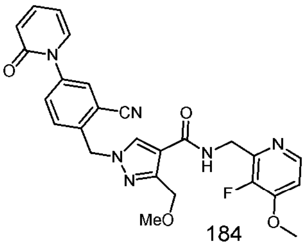
184 ,

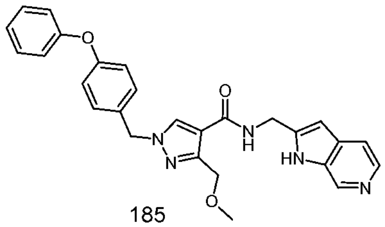
185 ,

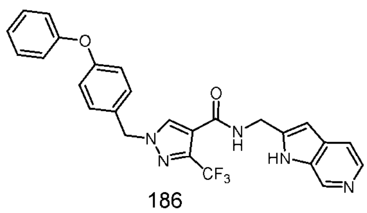
186 ,

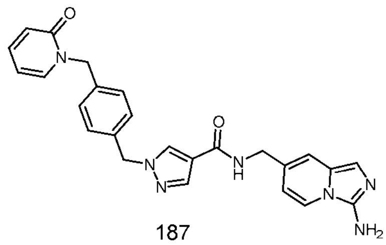
187 ,

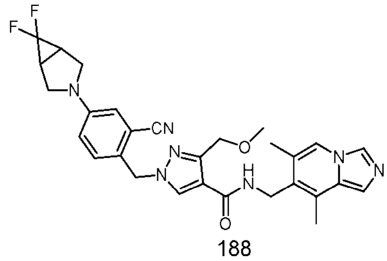
188 ,

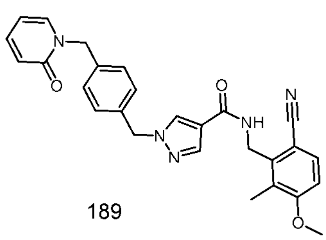
189 ,

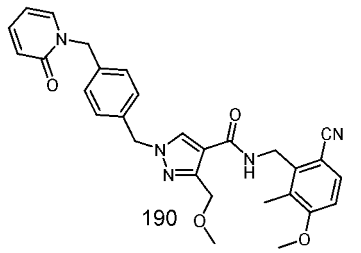
190 ,

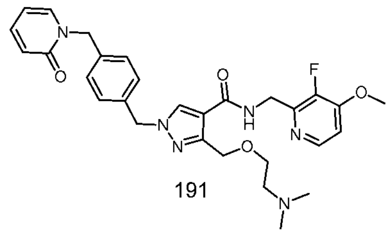
191 ,

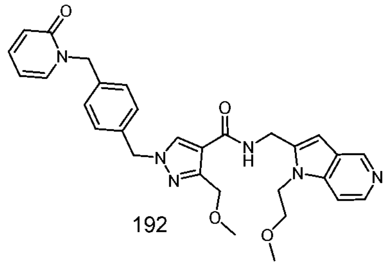
192 ,

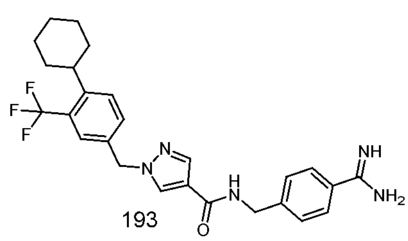
193 ,

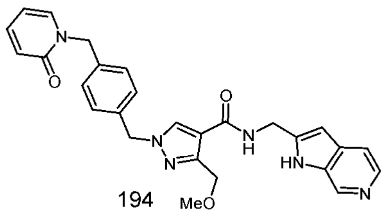
194 ,

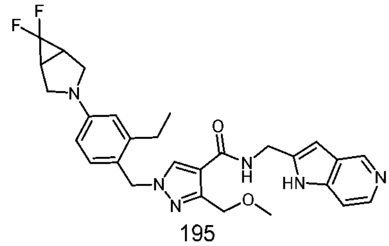
195 ,

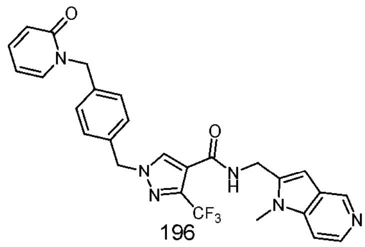
196 ,

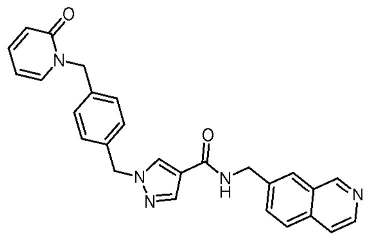
197 ,

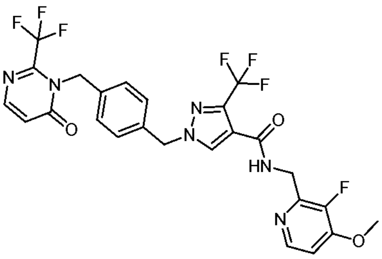
198 ,

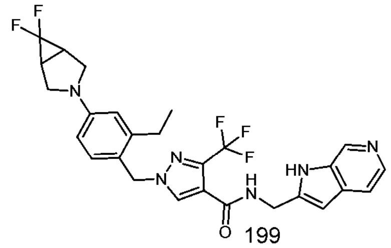
199 ,

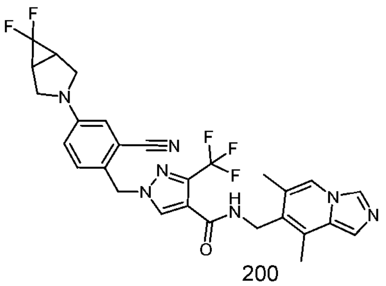
200 ,

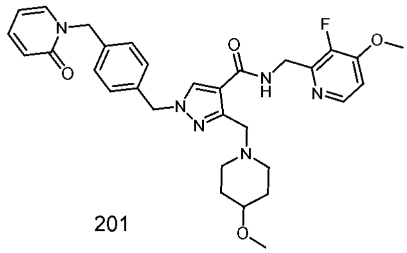
201 ,

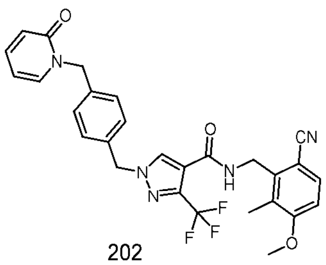
202 ,

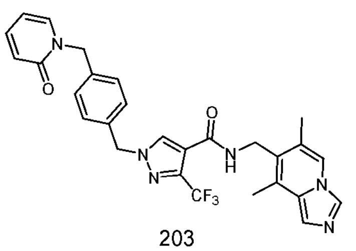
203 ,

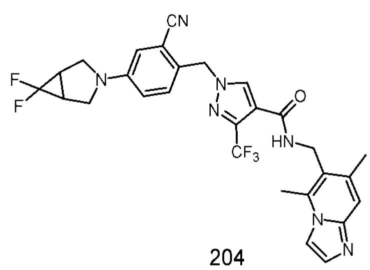
204 ,

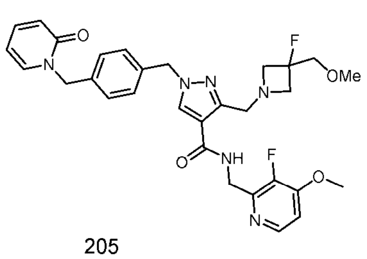

205 ,

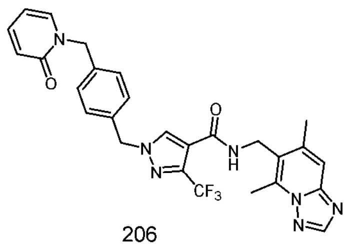

206 ,

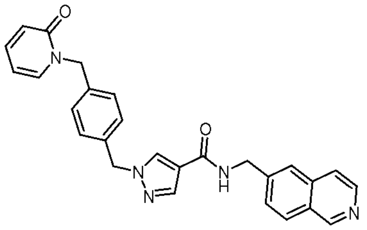

207 ,

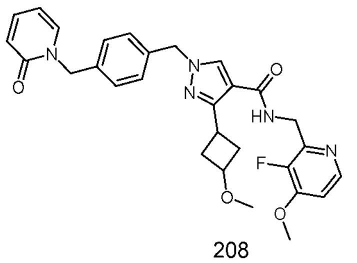

208 ,

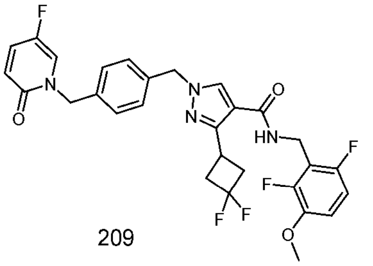

209 ,

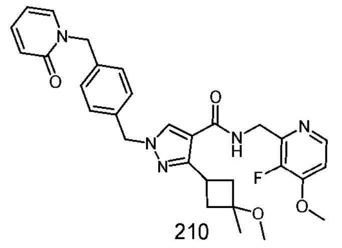

210 ,

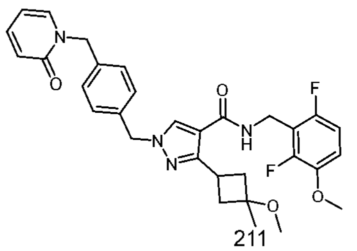

211 ,

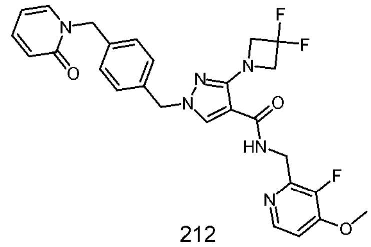

212 ,

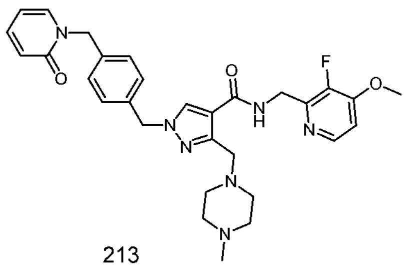

213 ,

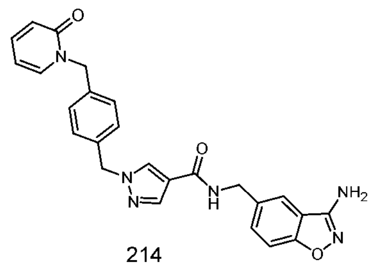

214 ,

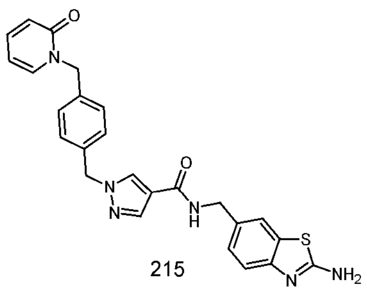

215 ,

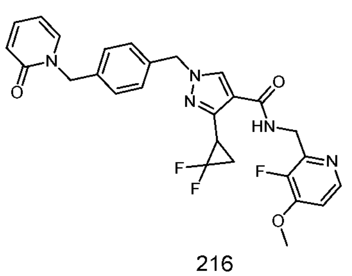

216 ,

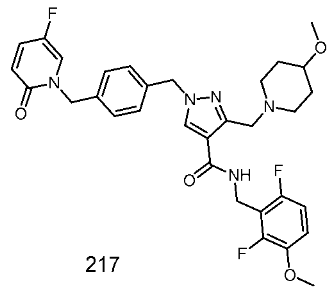

217 ,

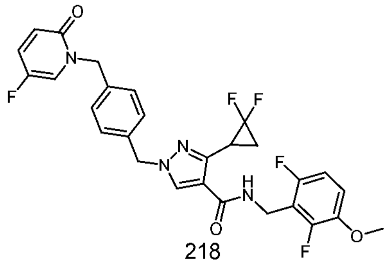
218
,

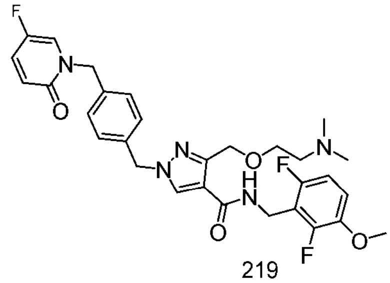
219
,

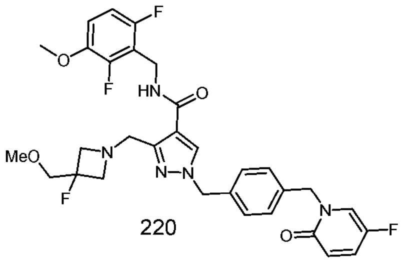
220
,

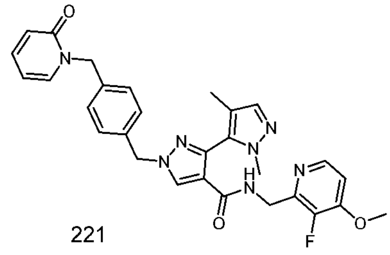
221
,

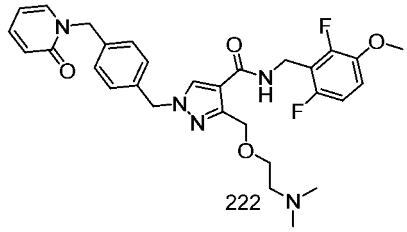
222
,

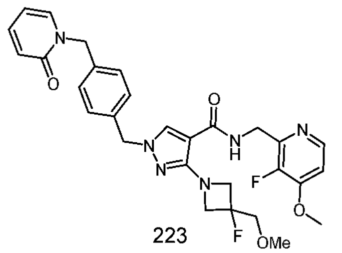
223
,

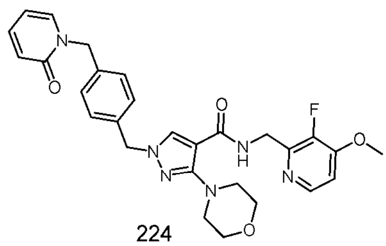
224
,

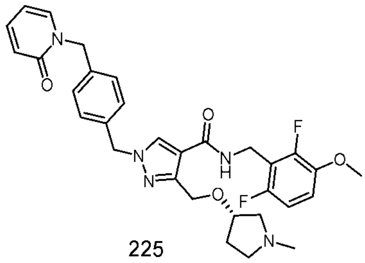
225
,

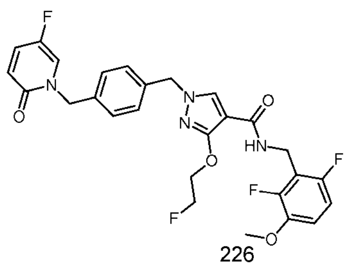
226
,

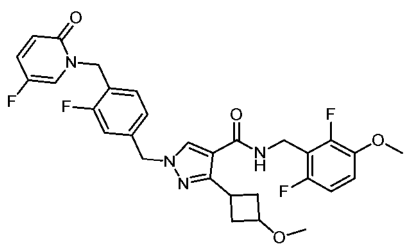
227
,

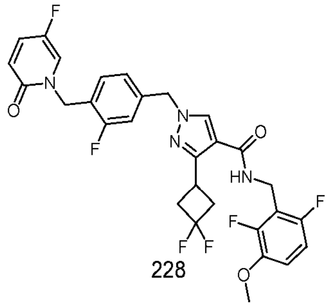
228
,

225

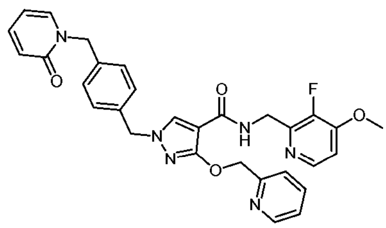

229,

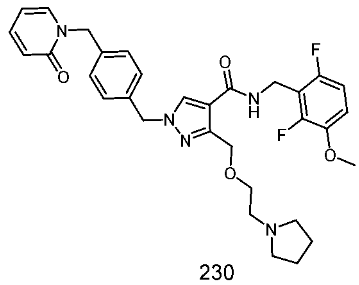

230,

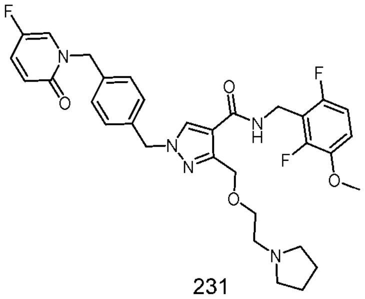

231,

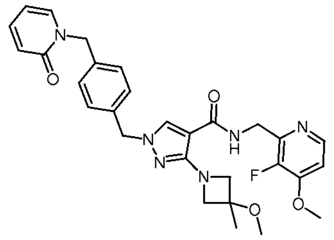

232,

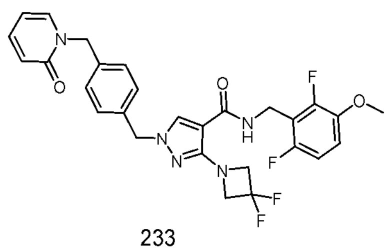

233,

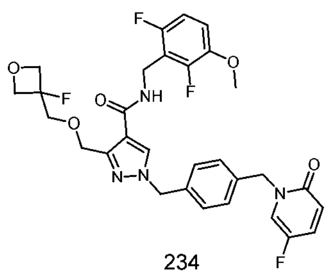

234,

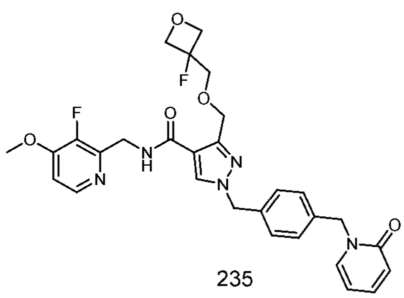

235,

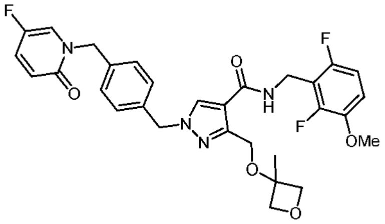

236,

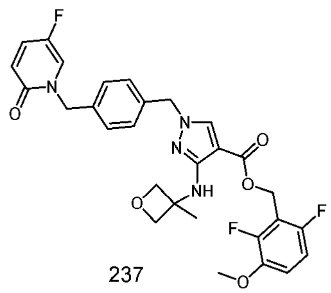

237,

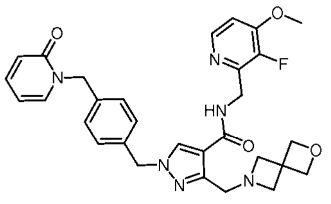

238,

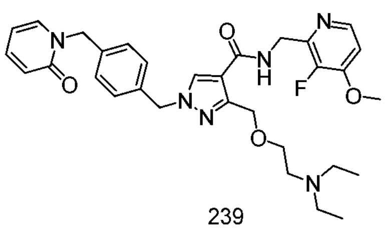

239,

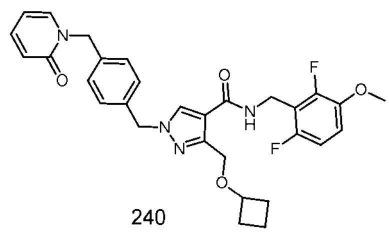

240,

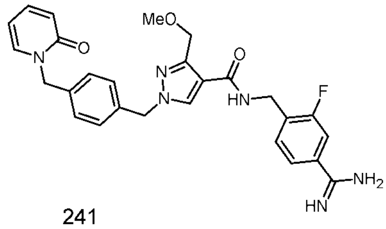

241 ,

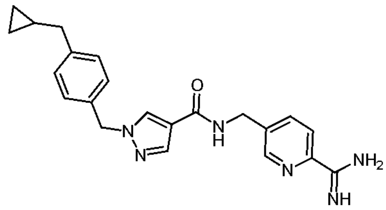

242 ,

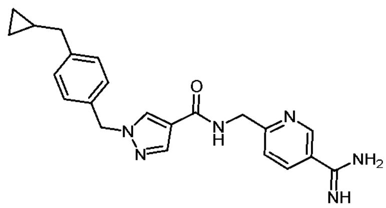

243 ,

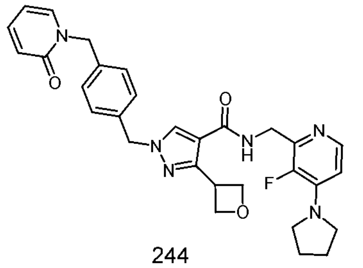

244 ,

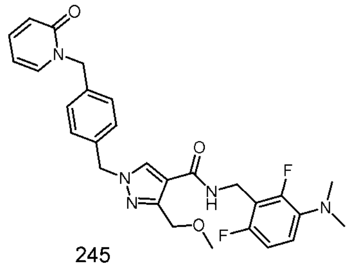

245 ,

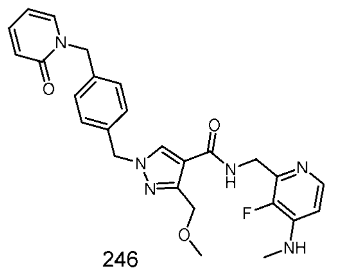

246 ,

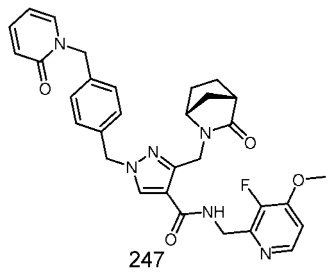

247 ,

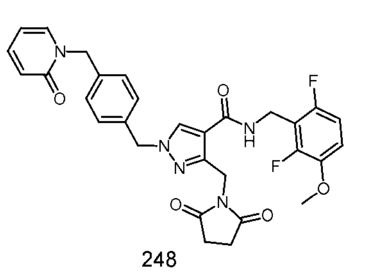

248 ,

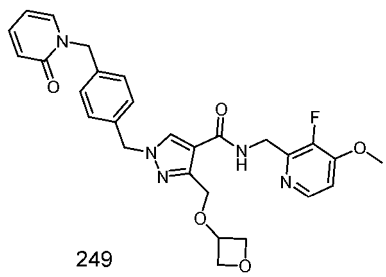

249 ,

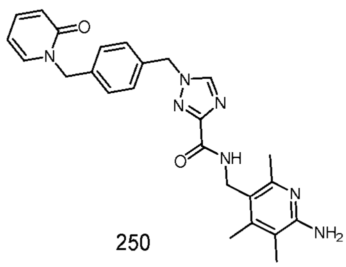

250 ,

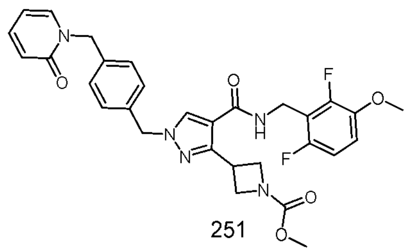

251 ,

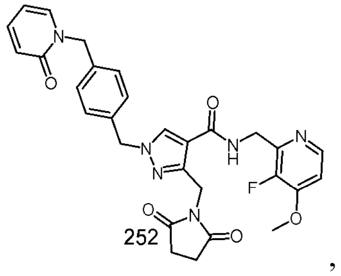

252 ,

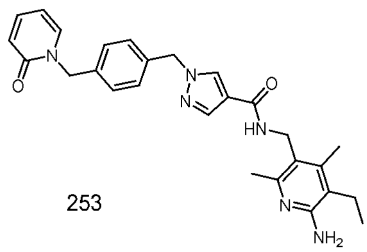

253 ,

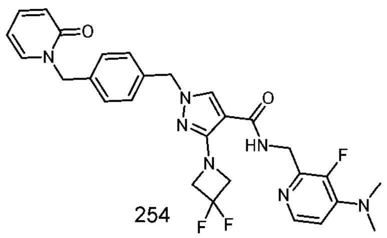

254,

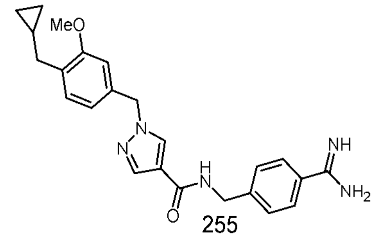

255,

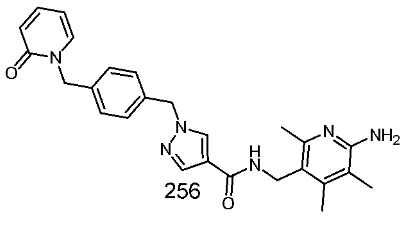

256,

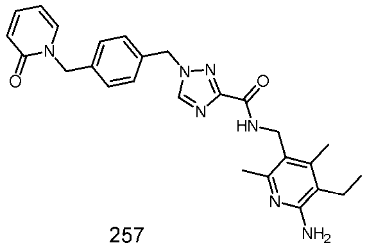

257,

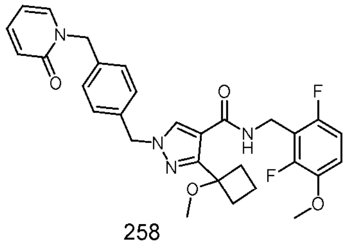

258,

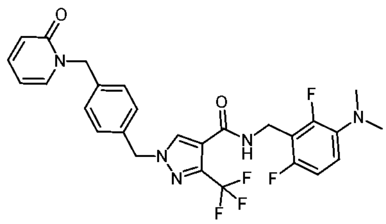

259,

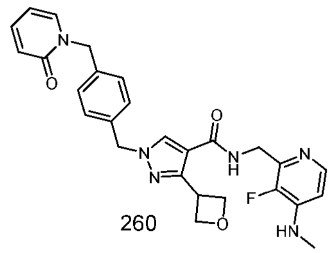

260,

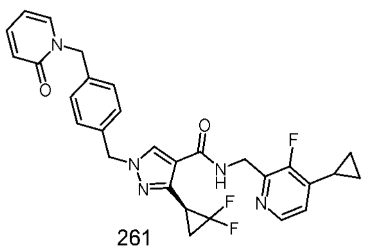

261,

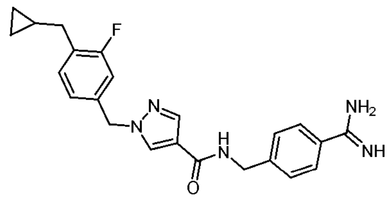

262,

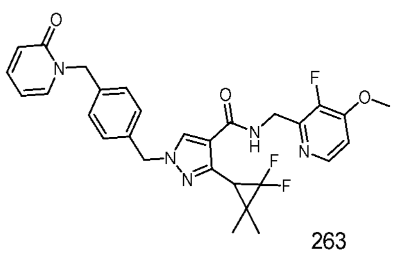

263,

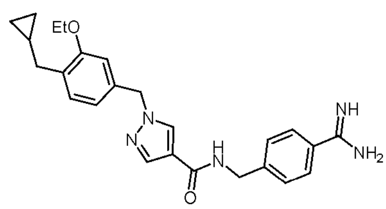

264,

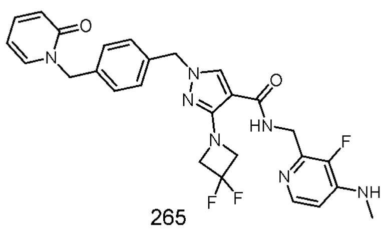

265,

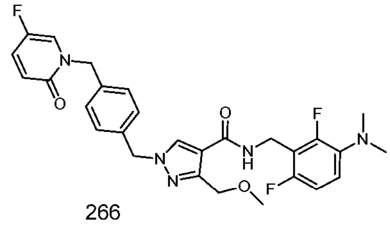

266,

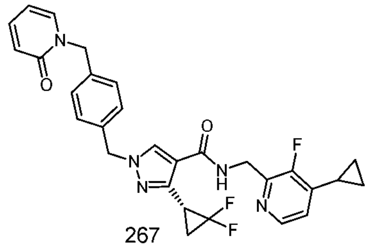
267 ,

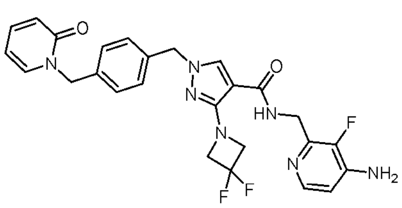
268 ,

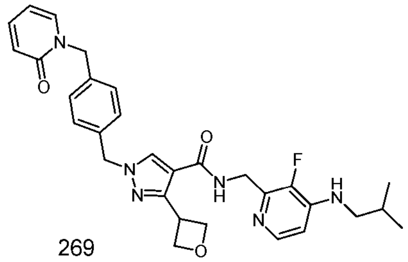
269 ,

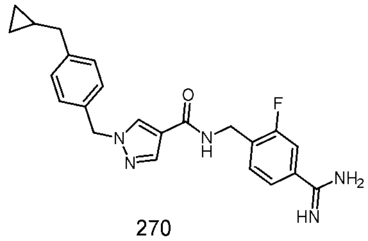
270 ,

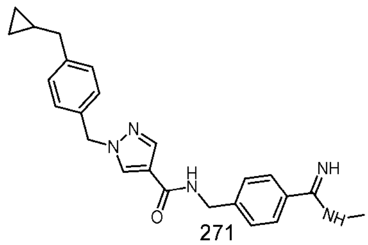
271 ,

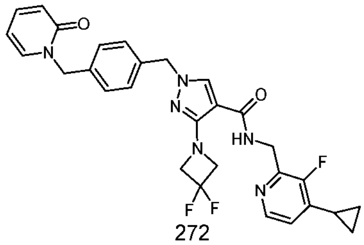
272 ,

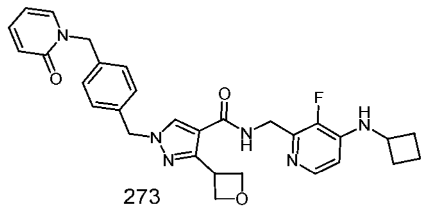
273 ,

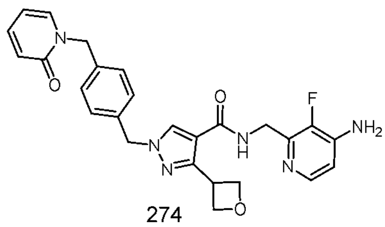
274 ,

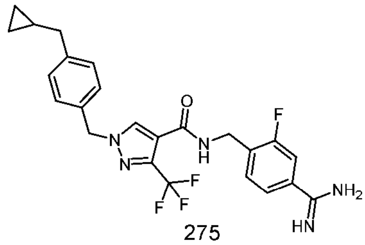
275 ,

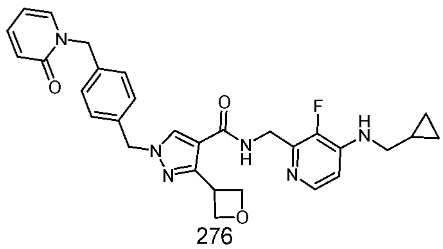
276 ,

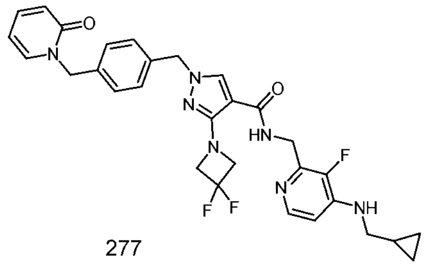
277 ,

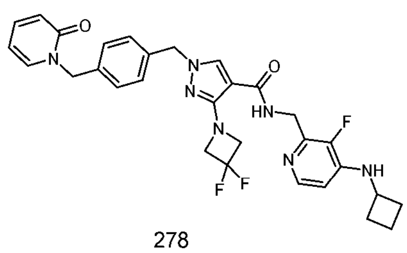
278 ,

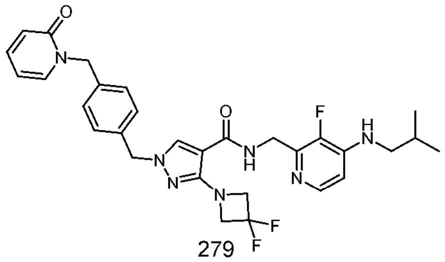

279 ,

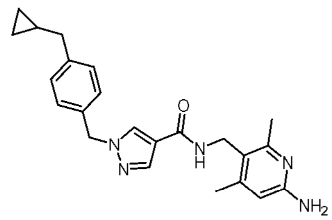

280 ,

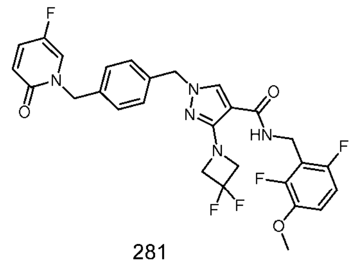

281 ,

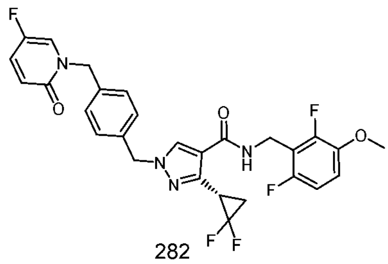

282 ,

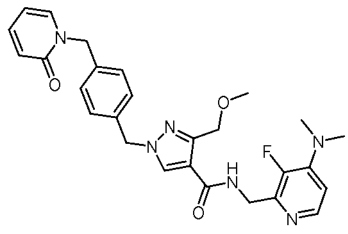

283 ,

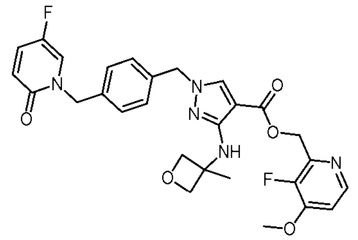

284 ,

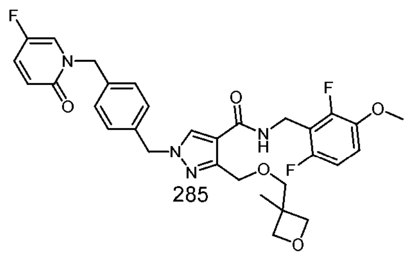

285 ,

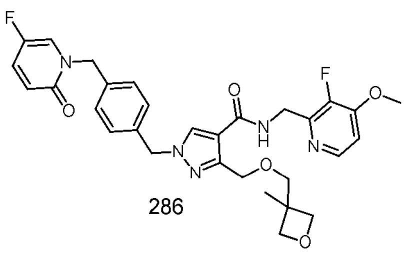

286 ,

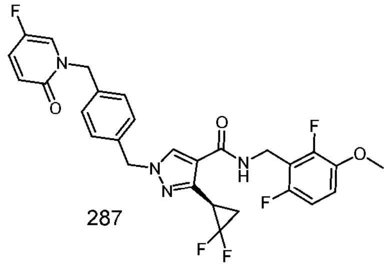

287 ,

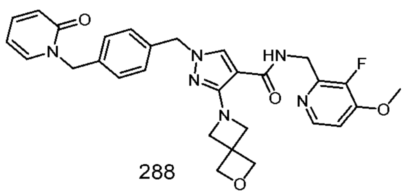

288 ,

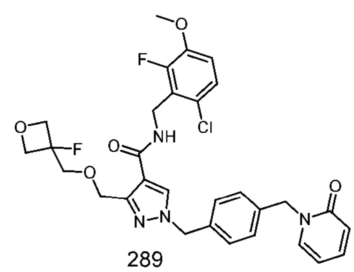

289 ,

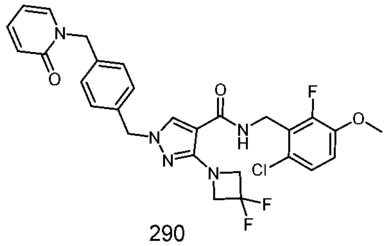

290 ,

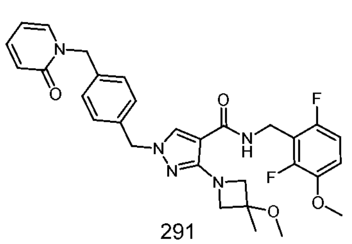

291 ,

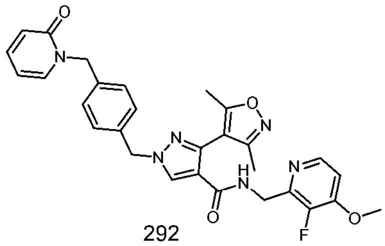

292 ,

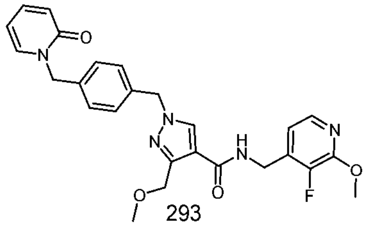

293 ,

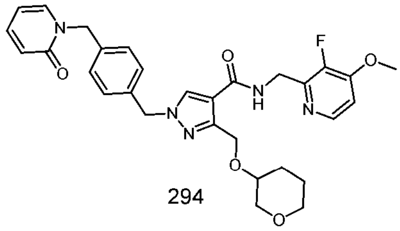

294 ,

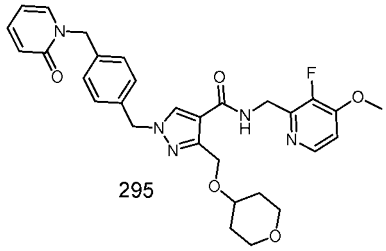

295 ,

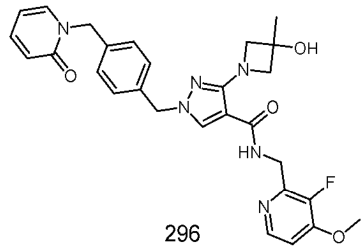

296 ,

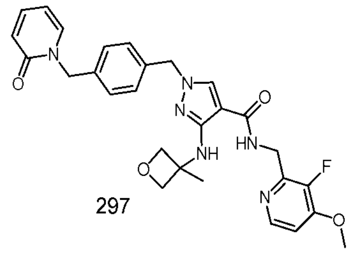

297 ,

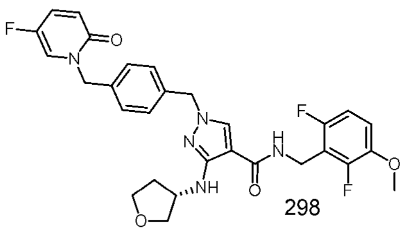

298 ,

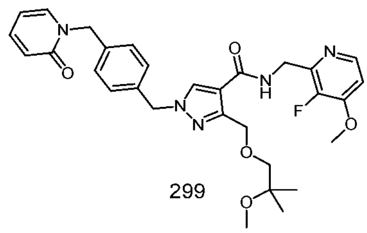

299 ,

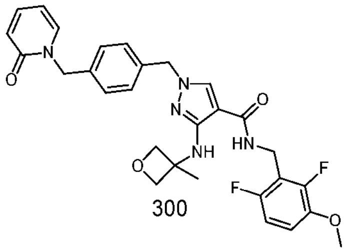

300 ,

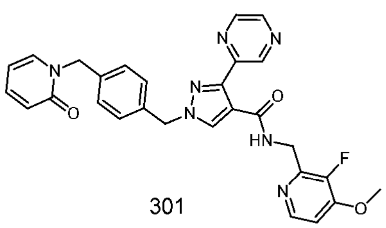

301 ,

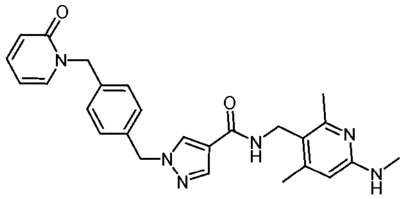
302
,

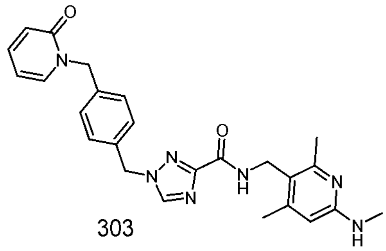
303
,

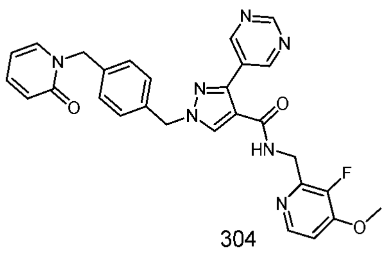
304
,

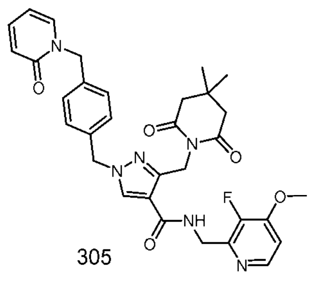
305
,

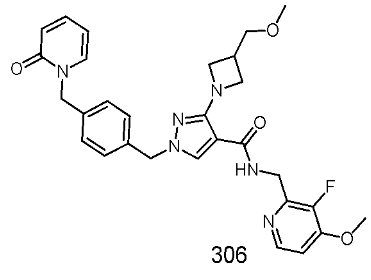
306
,

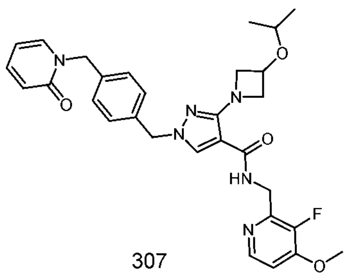
307
,

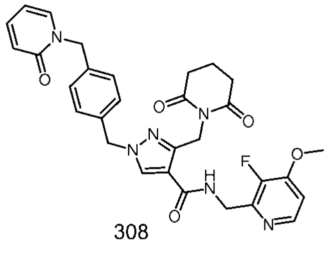
308
,

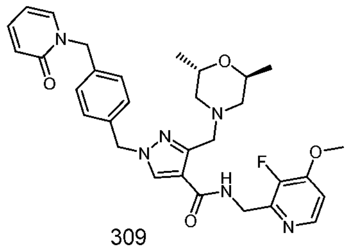
309
,

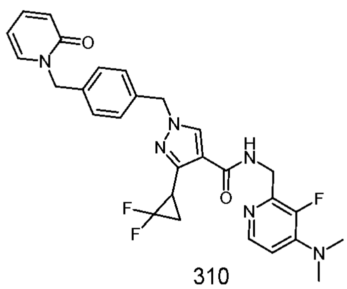
310
,

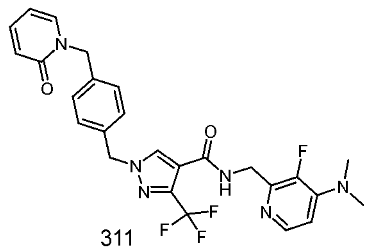
311
,

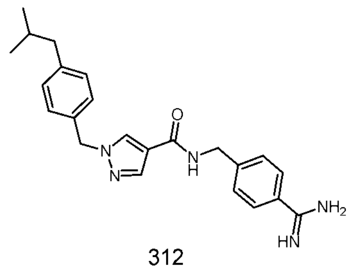
312
,

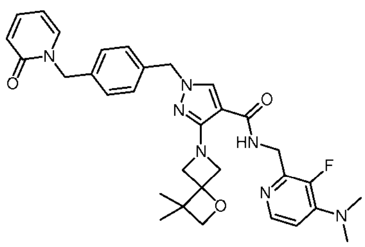
313
,

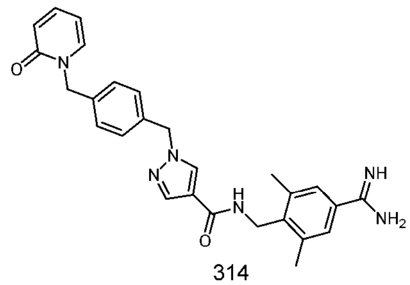
314
,

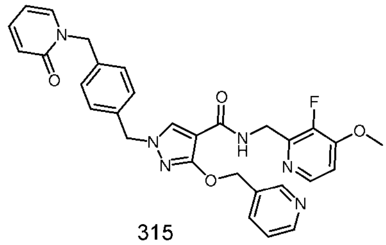
315 ,

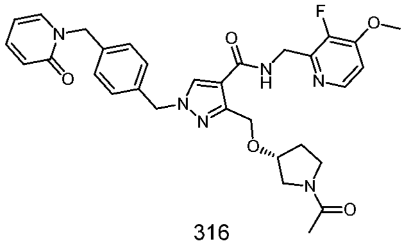
316 ,

313

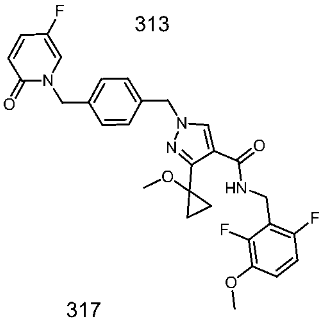
317 ,

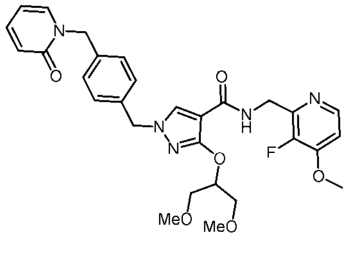
318 ,

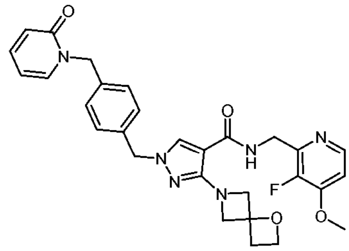
319 ,

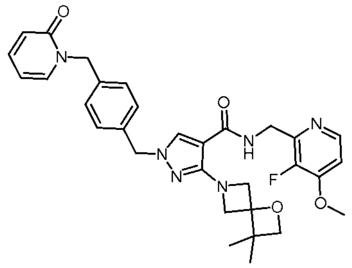
320 ,

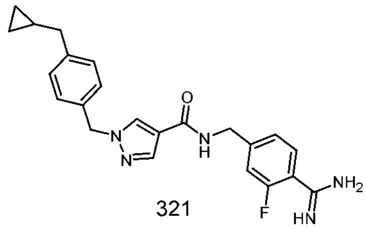
321 ,

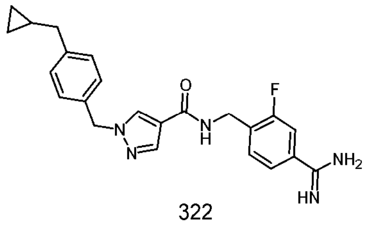
322 ,

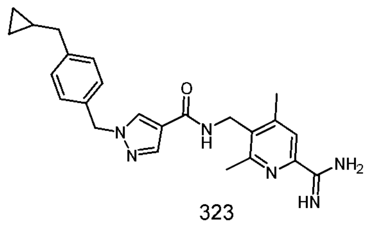
323 ,

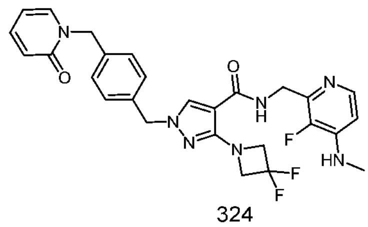
324 ,

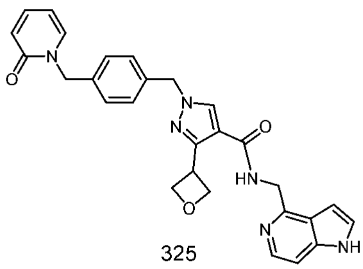
325 ,

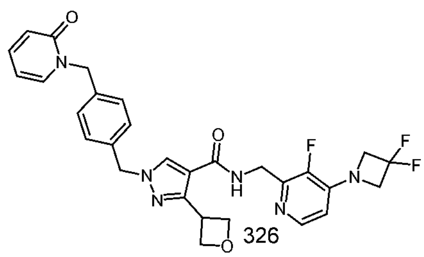
326 ,

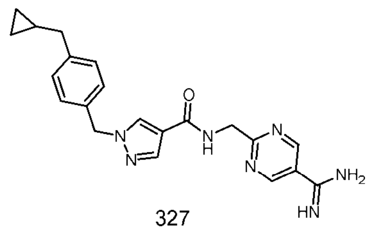
327 ,

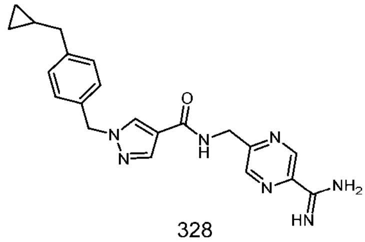
328 ,

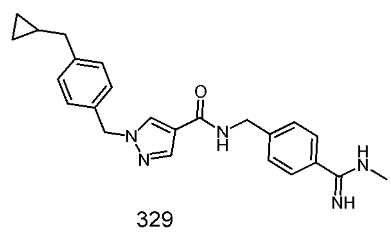
329 ,

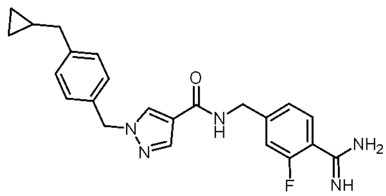
330 ,

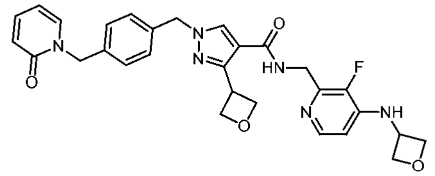
331 ,

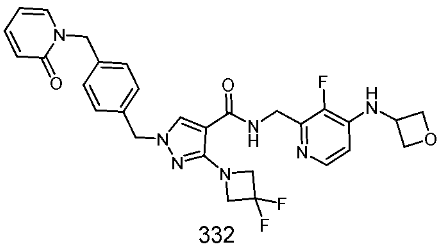
332 ,

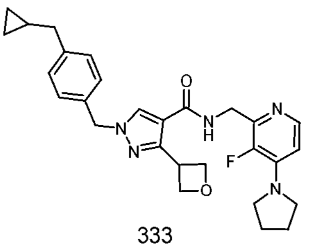
333 ,

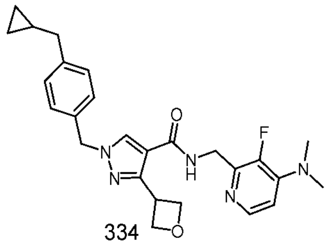
334 ,

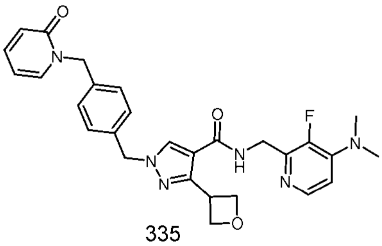
335 ,

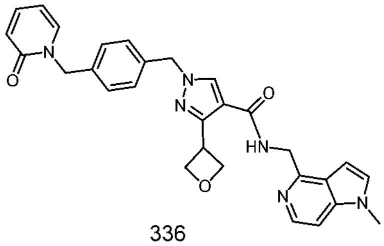
336 ,

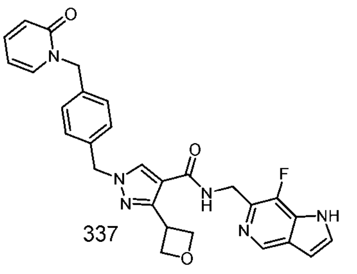
337 ,

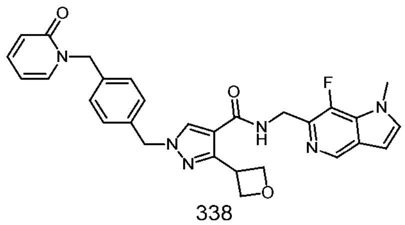
338 ,

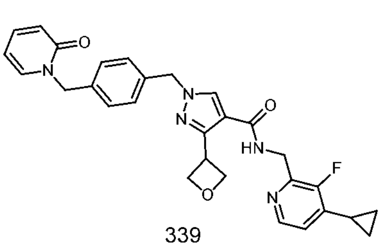
339 ,

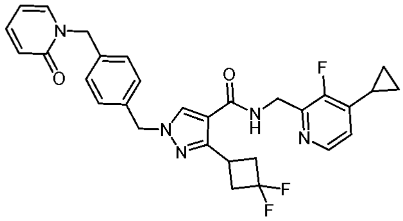
340 ,

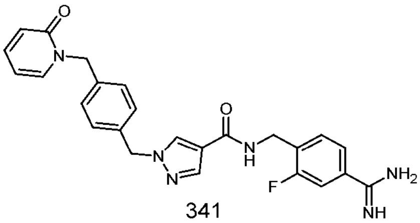
341 ,

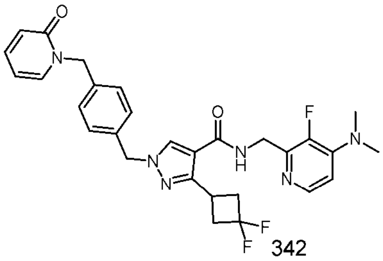
342 ,

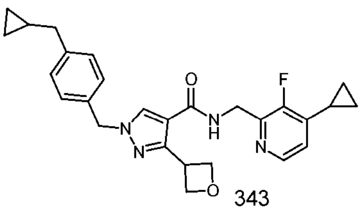
343 ,

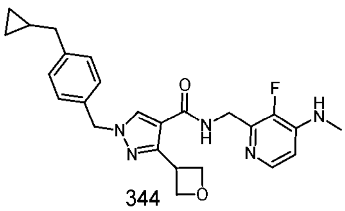
344 ,

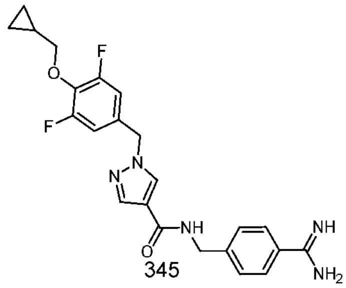
345 ,

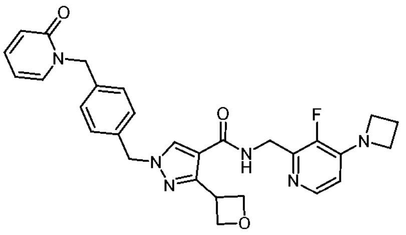
346 ,

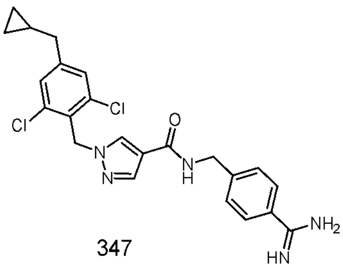
347 ,

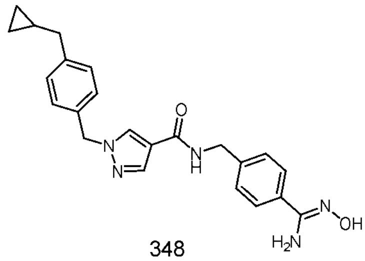
348 ,

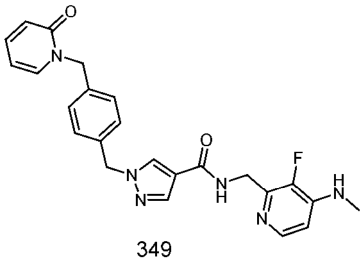
349 ,

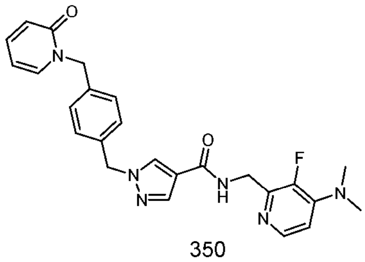
350 ,

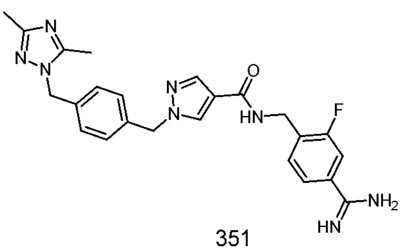
351 ,

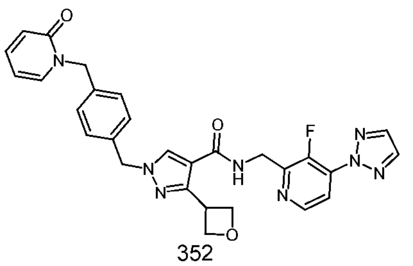
352 ,

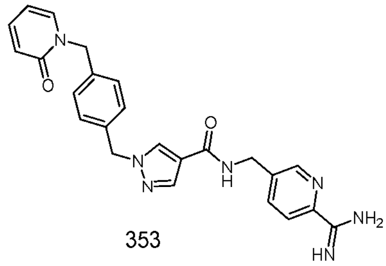
353 ,

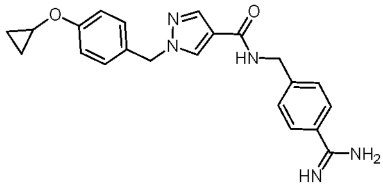
354 ,

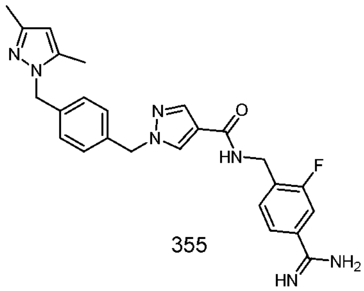
355 ,

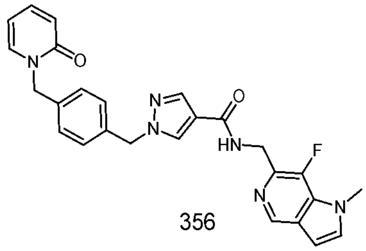
356 ,

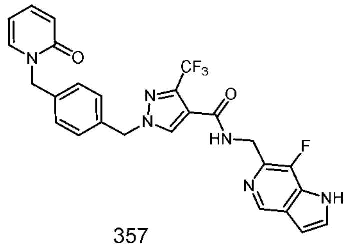
357 ,

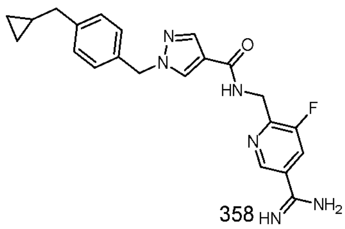
358 ,

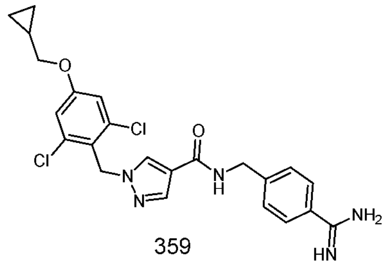
359 ,

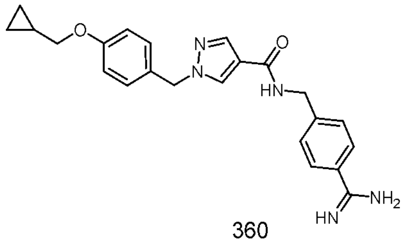
360 ,

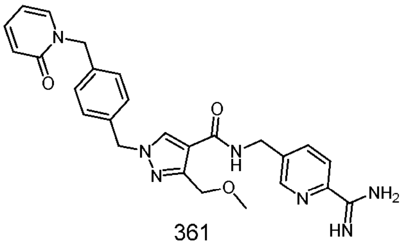
361 ,

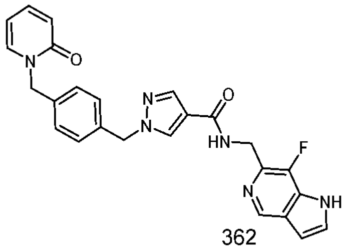
362 ,

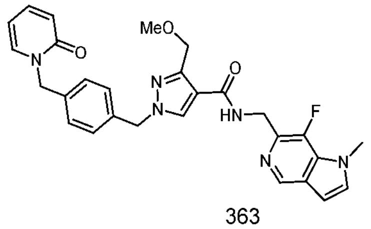
363 ,

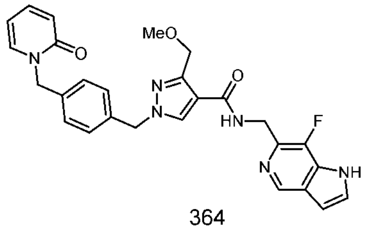
364 ,

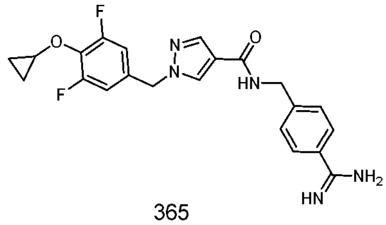
365 ,

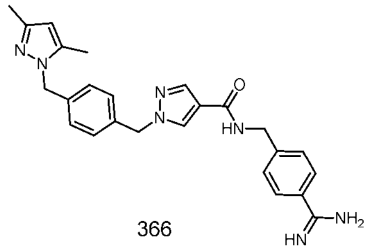
366 ,

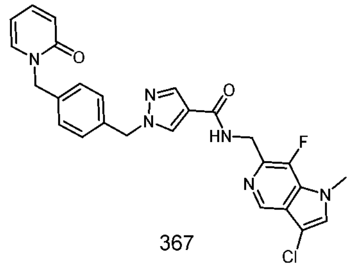
367 ,

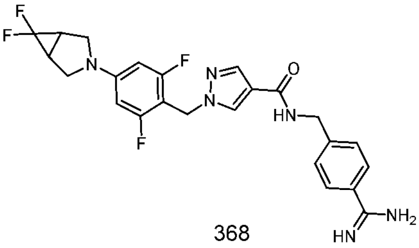
368 ,

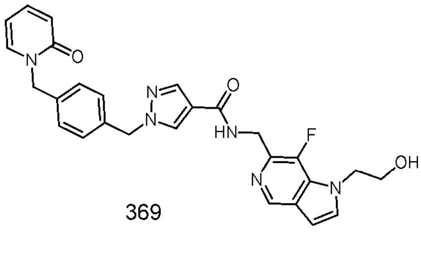
369 ,

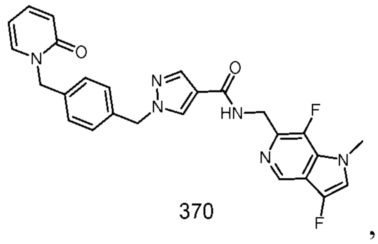
370 ,

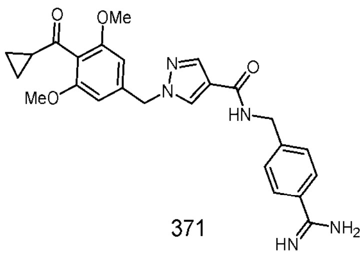
371 ,

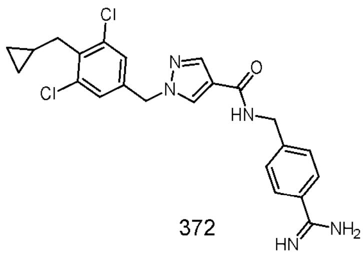
372 ,

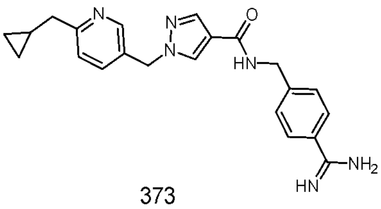
373 ,

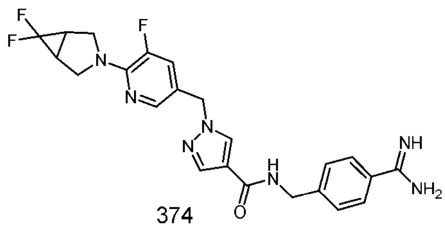
374 ,

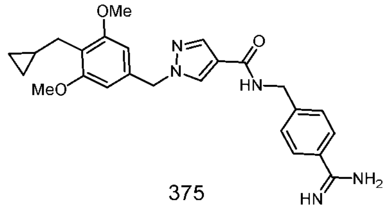
375 ,

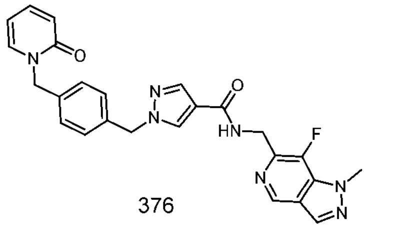
376 ,

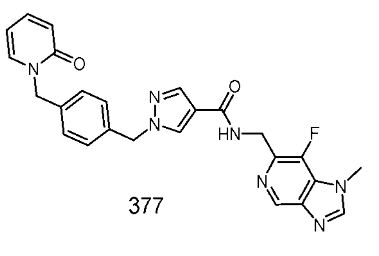
377 ,

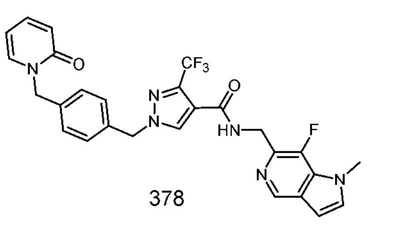
378 ,

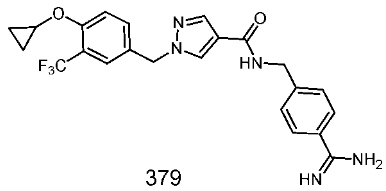
379
,

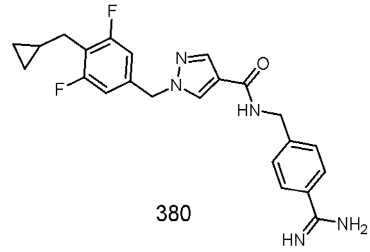
380
,

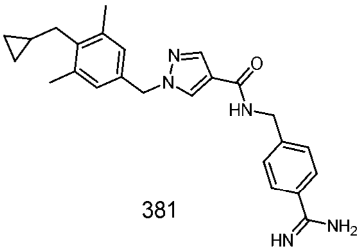
381
,

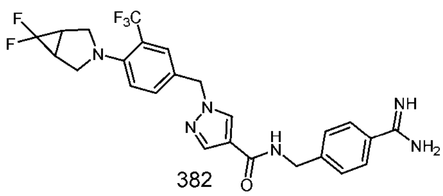
382
,

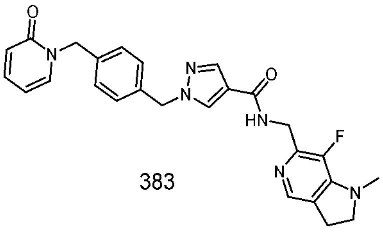
383
,

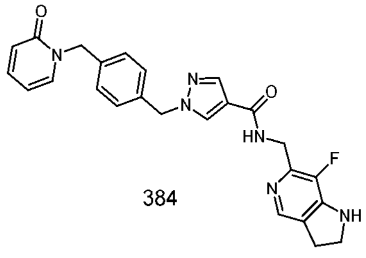
384
,

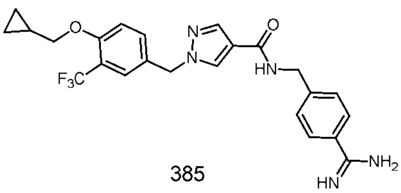
385
,

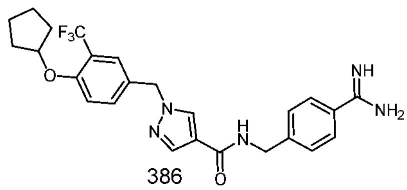
386
,

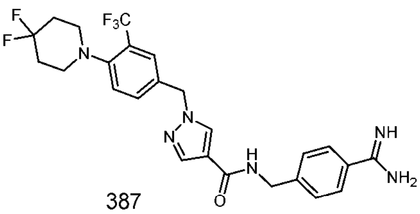
387
,

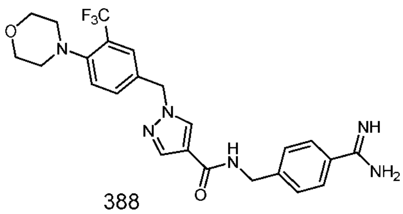
388
,

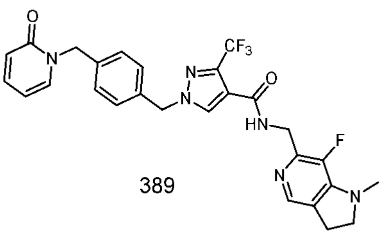
389
,

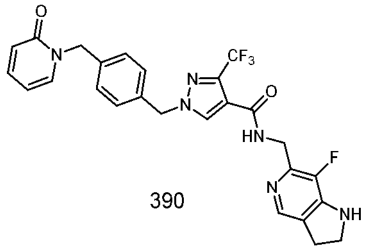
390
,

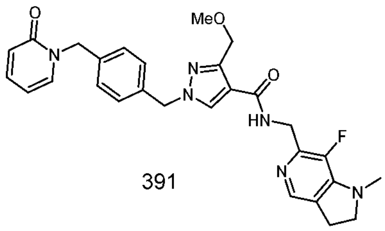

391

,

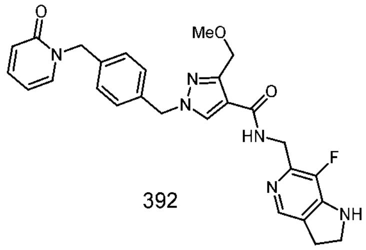

392

,

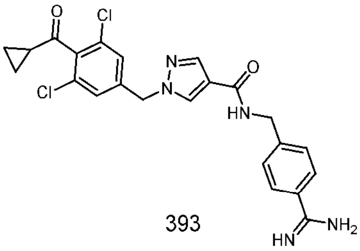

393

,

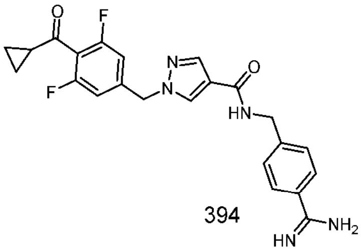

394

,

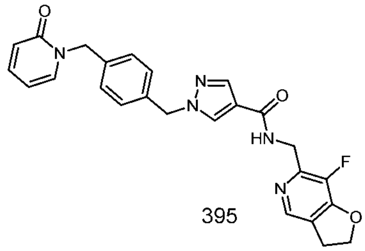

395

and

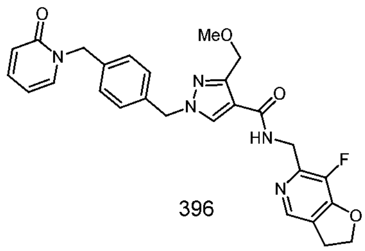

396

.

**14.** A pharmaceutical composition comprising a compound or a pharmaceutically acceptable prodrug or salt thereof according to any one of claims 1 to 13, and one or more pharmaceutically acceptable carriers, diluents or excipients.

**15.** Use of a compound or a pharmaceutically acceptable prodrug or salt thereof according to any one of claims 1 to 13 or a pharmaceutical composition according to claim 14 for the manufacture of a medicament for the treatment of hereditary angioedema (HAE) and cerebral edema after stroke and neuroprotection.

**16.** Use of a compound or a pharmaceutically acceptable prodrug or salt thereof according to any one of claims 1 to 13, or a pharmaceutical composition according to claim 14 for the manufacture of a medicament for the treatment of cerebral edema caused by stroke and nerve damage.

**17.** Use of a compound or a pharmaceutically acceptable prodrug or salt thereof according to any one of claims 1 to 13 or a pharmaceutical composition according to claim 14 for the manufacture of a medicament for the treatment of an ocular microangiopathy-associated disorder;
in particular, the ocular microangiopathy-associated disorder is selected from the group consisting of diabetic retinopathy, diabetic macular edema, retinal vein occlusion, central retinal vein occlusion, macular degeneration, retinopathy of prematurity, neovascular glaucoma, retinitis pigmentosa, proliferative and non-proliferative retinopathy, retinal angiomatous hyperplasia, macular telangiectasia, ischemic retinopathy, iris neovascularization, intraocular neovascularization, corneal neovascularization, retinal neovascularization, polypoidal choroidal vasculopathy, choroidal neovascularization, retinal degeneration, diabetic complications of retinal vascular permeability associated with diabetic retinopathy and diabetic macular edema; particularly selected from the group consisting of diabetic retinopathy, diabetic macular edema, retinal vein occlusion, central retinal vein occlusion and wet age-related macular degeneration.

**INTERNATIONAL SEARCH REPORT**

International application No.
**PCT/CN2024/100315**

**A. CLASSIFICATION OF SUBJECT MATTER**

C07D405/14(2006.01)i; C07D403/12(2006.01)i; C07D403/14(2006.01)i; A61K31/4439(2006.01)i; A61P7/02(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNTXT; VEN; CNKI; WOTXT; EPTXT; USTXT; STN: 远森制药, 王永钢, 余飞, 高燕珊, 付登荣, 孙喜成, 吴功雄, 酰胺, 水肿, 糖尿病, 血浆激肽释放酶, 杂芳, 吡唑, 吡啶, 吡啶酮, 咪唑, 抑制剂, 遗传性血管性水肿, 视网膜病变, 结构式, plasma kallikrein, inhibitor, hereditary angioedema, retinopathy, diabetic, carboxamide, pyrazole, imidazole, pyridine, pyridinone, heteroaromatic

**C. DOCUMENTS CONSIDERED TO BE RELEVANT**

| Category* | Citation of document, with indication, where appropriate, of the relevant passages | Relevant to claim No. |
|---|---|---|
| X | CN 103080104 A (NOVARTIS AG) 01 May 2013 (2013-05-01)<br>description, paragraphs [0539] and [1396], and claims 1, 9, 14 and 15 | 1-17 |
| X | CN 110022875 A (LIFESCI PHARMACEUTICALS, INC.) 16 July 2019 (2019-07-16)<br>description, paragraphs [0153], [0165], [0171], [0173] and [0183], and claims 45 and 46 | 1-17 |
| X | CN 114728962 A (TAKEDA PHARMACEUTICAL CO., LTD.) 08 July 2022 (2022-07-08)<br>description, paragraphs [0169] and [0205], and claims 39 and 40 | 1-17 |
| A | CN 106061480 A (LIFESCI PHARMACEUTICALS, INC.) 26 October 2016 (2016-10-26)<br>entire document | 1-17 |
| A | CN 115052873 A (BOEHRINGER INGELHEIM INTERNATIONAL GMBH) 13 September 2022 (2022-09-13)<br>entire document | 1-17 |
| A | US 2014378474 A1 (NOVARTIS AG) 25 December 2014 (2014-12-25)<br>entire document | 1-17 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 September 2024** | **04 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/100315**

**C. DOCUMENTS CONSIDERED TO BE RELEVANT**

| Category* | Citation of document, with indication, where appropriate, of the relevant passages | Relevant to claim No. |
|---|---|---|
| A | WO 2019142053 A2 (LIFESCI PHARMACEUTICALS, INC.) 25 July 2019 (2019-07-25) entire document | 1-17 |
| A | WO 2021175290 A1 (MEDSHINE DISCOVERY INC.) 10 September 2021 (2021-09-10) entire document | 1-17 |

Form PCT/ISA/210 (second sheet) (July 2022)

**INTERNATIONAL SEARCH REPORT**
**Information on patent family members**

International application No.
**PCT/CN2024/100315**

| Patent document cited in search report | Publication date (day/month/year) | Patent family member(s) | Publication date (day/month/year) |
|---|---|---|---|
| CN 103080104 A | 01 May 2013 | TW 201206900 A | 16 February 2012 |
| | | CA 2806015 A1 | 09 February 2012 |
| | | KR 20130096253 A | 29 August 2013 |
| | | WO 2012017020 A1 | 09 February 2012 |
| | | JP 2013532713 A | 19 August 2013 |
| | | JP 5809267 B2 | 10 November 2015 |
| | | US 2012035168 A1 | 09 February 2012 |
| | | US 9290485 B2 | 22 March 2016 |
| | | AU 2011287574 A1 | 28 February 2013 |
| | | AU 2011287574 B2 | 22 January 2015 |
| | | EP 2601189 A1 | 12 June 2013 |
| | | EP 2601189 B1 | 15 April 2015 |
| | | ES 2542764 T3 | 11 August 2015 |
| | | EA 021359 B1 | 29 May 2015 |
| | | IN 201300637 P1 | 17 October 2014 |
| | | CN 103080104 B | 08 April 2015 |
| | | MX 2013001363 A | 28 February 2013 |
| CN 110022875 A | 16 July 2019 | EP 3481391 A1 | 15 May 2019 |
| | | EP 3481391 A4 | 11 March 2020 |
| | | US 2020239435 A1 | 30 July 2020 |
| | | US 10781200 B2 | 22 September 2020 |
| | | WO 2018011628 A1 | 18 January 2018 |
| | | US 2018297984 A1 | 18 October 2018 |
| | | US 10301284 B2 | 28 May 2019 |
| | | HK 40006420 A0 | 22 May 2020 |
| | | HK 40007606 A0 | 05 June 2020 |
| CN 114728962 A | 08 July 2022 | US 2021078999 A1 | 18 March 2021 |
| | | US 11787796 B2 | 17 October 2023 |
| | | WO 2021055621 A1 | 25 March 2021 |
| | | EP 4031547 A1 | 27 July 2022 |
| | | EP 4031547 B1 | 17 July 2024 |
| | | JP 2022548696 W | 21 November 2022 |
| | | HK 40074859 A0 | 06 January 2023 |
| CN 106061480 A | 26 October 2016 | CA 2935683 A1 | 09 July 2015 |
| | | AU 2014373735 A1 | 11 August 2016 |
| | | AU 2014373735 B2 | 12 March 2020 |
| | | MX 2016008688 A | 12 January 2017 |
| | | EA 032713 B1 | 31 July 2019 |
| | | US 2018170906 A1 | 21 June 2018 |
| | | US 10266515 B2 | 23 April 2019 |
| | | WO 2015103317 A1 | 09 July 2015 |
| | | JP 6759100 B2 | 23 September 2020 |
| | | EP 3089746 A1 | 09 November 2016 |
| | | EP 3089746 A4 | 30 August 2017 |
| | | US 2020031799 A1 | 30 January 2020 |
| | | IN 201617024513 A | 31 August 2016 |
| | | JP 2017501205 W | 12 January 2017 |
| | | BR 112016015449 A2 | 08 August 2017 |
| | | US 2017260163 A1 | 14 September 2017 |
| | | HK 1230502 A0 | 08 December 2017 |

Form PCT/ISA/210 (patent family annex) (July 2022)

**INTERNATIONAL SEARCH REPORT**
**Information on patent family members**

International application No.
**PCT/CN2024/100315**

| Patent document cited in search report | Publication date (day/month/year) | Patent family member(s) | Publication date (day/month/year) |
|---|---|---|---|
| | | US 2018170906 A1 | 21 June 2018 |
| | | US 10259803 B2 | 16 April 2019 |
| | | CN 106061480 B | 28 February 2020 |
| | | HK 1230502 A1 | 16 October 2020 |
| | | KR 20160106627 A | 12 September 2016 |
| CN 115052873 A | 13 September 2022 | WO 2021160718 A1 | 19 August 2021 |
| | | TW 202144330 A | 01 December 2021 |
| | | KR 20220141333 A | 19 October 2022 |
| | | JP 2023067881 A | 16 May 2023 |
| | | AR 121341 A1 | 11 May 2022 |
| | | IL 295125 A | 01 September 2022 |
| | | US 2021292301 A1 | 23 September 2021 |
| | | US 11760745 B2 | 19 September 2023 |
| | | BR 112022013979 A2 | 11 October 2022 |
| | | CA 3168690 A1 | 19 August 2021 |
| | | EP 4103562 A1 | 21 December 2022 |
| | | JP 7230277 B2 | 28 February 2023 |
| | | AU 2021218952 A1 | 28 July 2022 |
| | | US 2024076286 A1 | 07 March 2024 |
| | | US 2024002363 A1 | 04 January 2024 |
| | | TW 202233592 A | 01 September 2022 |
| | | MX 2022009941 A1 | 12 September 2022 |
| | | JP 2022543494 W | 12 October 2022 |
| | | VN 90591 A | 25 October 2022 |
| | | CN 116120287 A | 16 May 2023 |
| | | IN 202217050903 A | 09 June 2023 |
| | | HK 40084822 A0 | 21 July 2023 |
| US 2014378474 A1 | 25 December 2014 | WO 2013111108 A1 | 01 August 2013 |
| | | EP 2807157 A1 | 03 December 2014 |
| WO 2019142053 A2 | 25 July 2019 | WO 2019142053 A3 | 14 November 2019 |
| WO 2021175290 A1 | 10 September 2021 | JP 7245397 B2 | 23 March 2023 |
| | | EP 4116299 A1 | 11 January 2023 |
| | | EP 4116299 A4 | 27 March 2024 |
| | | US 2023167098 A1 | 01 June 2023 |
| | | US 11814371 B2 | 14 November 2023 |
| | | CN 115210230 A | 18 October 2022 |
| | | JP 2023508243 W | 01 March 2023 |
| | | HK 40082202 A0 | 02 June 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Seminars in Immunopathology*, 2023, vol. 45, 389-410
- *Stroke*, 2014, vol. 45 (1)
- *Diabetes*, 2015, vol. 64, 3588-3599
- *Diabetes*, 2011, vol. 60, 590-1598
- *Investig. Ophthalmol. Vis. Sci.*, 2016, vol. 57, 2390-2399
- *Invest Ophthalmol Vis Sci.*, 2016, vol. 57 (6), 2390-2399
- *CHEMICAL ABSTRACTS*, 64-19-7
- *CHEMICAL ABSTRACTS*, 865-47-4
- *CHEMICAL ABSTRACTS*, 534-17-8
- *CHEMICAL ABSTRACTS*, 202289-38-1
- *CHEMICAL ABSTRACTS*, 878-23-9
- *CHEMICAL ABSTRACTS*, 128625-52-5
- *CHEMICAL ABSTRACTS*, 3945-69-5
- *CHEMICAL ABSTRACTS*, 208-488-9
- *CHEMICAL ABSTRACTS*, 7681-65-4
- *CHEMICAL ABSTRACTS*, 7758-89-6
- *CHEMICAL ABSTRACTS*, 7758-98-7
- *CHEMICAL ABSTRACTS*, 7646-69-7
- *CHEMICAL ABSTRACTS*, 124-41-4
- *CHEMICAL ABSTRACTS*, 26628-22-8
- *CHEMICAL ABSTRACTS*, 1066-33-7
- *CHEMICAL ABSTRACTS*, 497-19-8
- *CHEMICAL ABSTRACTS*, 584-08-7
- *CHEMICAL ABSTRACTS*, 16940-66-2
- *CHEMICAL ABSTRACTS*, 148893-10-1
- *CHEMICAL ABSTRACTS*, 1651823-59-4
- *CHEMICAL ABSTRACTS*, 1445086-28-1
- *CHEMICAL ABSTRACTS*, 72287-26-4
- *CHEMICAL ABSTRACTS*, 51364-51-3
- *CHEMICAL ABSTRACTS*, 13566-03-5
- *CHEMICAL ABSTRACTS*, 14221-01-3
- *CHEMICAL ABSTRACTS*, 53199-31-8
- *CHEMICAL ABSTRACTS*, 787618-22-8
- *CHEMICAL ABSTRACTS*, 1445085-77-7
- *CHEMICAL ABSTRACTS*, 161265-03-8
- *CHEMICAL ABSTRACTS*, 141-78-6
- *CHEMICAL ABSTRACTS*, 7087-68-5
- *CHEMICAL ABSTRACTS*, 68-12-2
- *CHEMICAL ABSTRACTS*, 13292-87-0
- *CHEMICAL ABSTRACTS*, 75-09-2
- *CHEMICAL ABSTRACTS*, 140681-55-6
- *CHEMICAL ABSTRACTS*, 94790 -35-9
- *CHEMICAL ABSTRACTS*, 76-05-1
- *CHEMICAL ABSTRACTS*, 109-99-9
- *CHEMICAL ABSTRACTS*, 16949-15-8
- *CHEMICAL ABSTRACTS*, 38721-52-7
- *CHEMICAL ABSTRACTS*, 557-21-1
- *CHEMICAL ABSTRACTS*, 872-50-4
- *CHEMICAL ABSTRACTS*, 67-56-1
- *CHEMICAL ABSTRACTS*, 75-05-8
- *CHEMICAL ABSTRACTS*, 25895-60-7
- *CHEMICAL ABSTRACTS*, 128-08-5
- *CHEMICAL ABSTRACTS*, 128-09-6
- *CHEMICAL ABSTRACTS*, 109-72-8
- *CHEMICAL ABSTRACTS*, 76513-69-4
- *CHEMICAL ABSTRACTS*, 121-44-8
- *CHEMICAL ABSTRACTS*, 3087-36-3
- *CHEMICAL ABSTRACTS*, 104-15-4
- *CHEMICAL ABSTRACTS*, 113978-91-9
- *CHEMICAL ABSTRACTS*, 4472-41-7
- *CHEMICAL ABSTRACTS*, 865-49-6
- *CHEMICAL ABSTRACTS*, 2206-27-1